# EUROPEAN PATENT APPLICATION

(11) **EP 3 594 349 A1**
(43) Date of publication of application: **15.01.2020**
(21) Application number: 18764709.4
(22) Date of filing: 07.03.2018
(51) Int. Cl.: C12N 15/29, C12N 15/82, A01H 5/00

(54) **METHOD FOR EPIGENETICALLY MANIPULATING PLANT PHENOTYPIC PLASTICITY**

(30) Priority: 08.03.2017 WO PCT/CN2017/075976
(71) Applicant: Nanjing Agricultural University, Nanjing, Jiangsu 210095 (CN)
(72) Inventor: QIANG, Sheng, Nanjing Jiangsu 210095 (CN); XIE, Hongjie, Nanjing Jiangsu 210095 (CN)
(74) Representative: Engelhard, Markus
(86) International application number: PCT/CN2018/078295
(87) International publication number: WO 2018/161921

(57) **Abstract**

The present invention discloses a method for epigenetically manipulating phenotypic plasticity traits of plants, including: quantitatively replacing methylation sites of a target gene by means of a degeneration codon, and carrying out a transgenic operation by designing a synthetic gene; or constructing a plant expression vector by using a CRISPR/Cas9 technology to quantitatively replace the methylation sites with in-situ in-vivo gene degeneration codons and quantitatively regulate a degree of methylation and an expression level of an exogenous or in-situ in-vivo target gene to attain quantitative regulation of the plasticity traits of a target plant. By means of quantitative regulation of the methylation degree of an ICE1 gene, plants having different cold-resistance capabilities are cultivated, thus providing a new choice for the cultivation of cold-resistant crops by means of epigenetic techniques, and providing a technical method for reducing the impact of cold harm on crop yield, quality and the like.

## Description

### Technical Field

The present invention belongs to the field of molecule biology and plant genetic engineerings, and relates to a method for epigenetically manipulating phenotypic plasticity traits of plants

### Background Art

DNA methylation is an important epigeneticmode of genome DNA, which is to transfer the methyl in S-adenosylmethionine donor to a specific base in the presence of DNA methyltransferase as a catalyst. DNA methylation is common in bacteria, plants and animals and part of many biological processes.DNA methylation shows species, tissue, organ and age specificities in plants and plays a part in regulation of genetic function including transcription, duplication, DNA repair, transgenosis and cell differentiation from the growth to the evolution of plants. The study carried out by Jullien and others in 2012 on the dynamic change of DNA methylation in the sexual reproduction process of Arabidopsis thaliana shows that maintenance methylation hardly occur in the generation of female gamete, but the de novo methylation and maintenance methylationobviously occur in the embryo after fertilization and are maintained at some level till adult plant. The dynamic change period of DNA methylation is important to the reproduction of Arabidopsis thaliana. High salinity treatment can lead to the change of DNA methylation in Oryza sativagenome (Han Yanan and others, 2010). Study by Tan and others in 2010 shows that salt stress can lead to raise in methylation in the intron area of the ABA negative regulatory factor ZmPP2C of Zea mays and thereby greatly reduce expression quantity; however, salt stresscan induce the demethylation in ZmGST generated in reactive oxygen metabolism and increase the expression level of ZmGST. These studies prove thatadversity stress can change the epigenetic modificationstate of DNA in plants and regulate genetic expression and plant growth to react to the adversity stress.

DNA methylation is a main epigenetic modification mode and has become a hot point of study. Lots have been done on study of DNA methylation, which mainly are mainly focused on how methylation happens, biological functions of methylation, testing method of methylation, but the study on the activation of genetic expression by change of methylation is just started and not so deep. Most of studies above are done in vivo and whether methylation can be regulated in vitro has not been verified. Methylation silencing happening in transgenic plants is mainly related to DNA methylation, and based on this discovery, DNA methylation has been used in the cultivation of transgenic crops.

Low temperature is one of common adversity factors. Many plants will get higher cold resistance by low-temperature and cold damage treatment, and this is cold domestication (Thomashow, 1999).But plants originally from tropic and subtropic areas have high requirements on temperature and cannot bear shock chilling (0-15 °C).Chilling damage has big influence on the output and quality of Musa nana (Xu *et al*., 2002), Gossypiumhirsutum (Zhang & Zhang,2006), Zea mays (Ma *et al*., 2006) andOryza sativa (Quan *et al*., 2006). During severe winters and springs from 1991 to 1992, 1992 to 1993, recently 1999 to 2000, large area of banana plants in Southeast China has been damaged by strong cold wave, which leads to huge loss to banana farmers (Xu *et al*., 2002). During the whole growth period of cotton, the best temperature is 20 to 30°C and it will cause damage to cotton in growth period when going down to less than 15°C, which is known as cold damage(Yin, 2002). The continuous low temperature (11-13 °C) and raining from July 31^{st} to August 3^{rd} in 2001 reduced 40 to 50% of cotton production in 26.7^{∗}104hm² area in Changji, Shihezi and Kuitun, loweredfiber quality by 1 to 2 levels and caused 20^{∗}10⁸ CNY economic loss (Zhang & Zhang, 2006).Due to low temperature in large area in Northeast China, accumulated temperature in most years is not enough (≥10°C), so corn grows slowly and mature late after Akishimo and thereby suffer from chilling damage, which is very severein (1957, 1969, 1972 and 1976) and reduces >15% of corn production (Ma *et al*., 2006). Most rice areas in China have been threatened by chilling damage which happens frequently. Rice cold damage happened in 1954,1956,1957,1969,1971,1972,1974,1976,1979,1980,1986,1988,1993,1995, 1998,2002,2003 and 2006 in Yanbian, Jilin Province (Quan *et al*., 2006).

Protein generated in the domestication process is low-temperature induced protein because the gene expressed based on low-temperature domestication is (cold-regulated gene, COR). When plants suffer from cold stress, the transcription level of COR gene will increase and the expression of these genes can be induced by ABA, therefore experts expect that the low-temperature signal causes large amount of composition of ABA in plant bodies which will induce expression of other downstream genes. This pathwayis calledABA-dependentsignalingpathway (Skriver *et al*., 1990). Later study found that COR can be expressed in plants with mutation of ABA synthetic gene, which proves the existence of independent ABA pathway or ABA independent signalingpathway. In 1994, Yamaguchi and Skinozaki found for the first time a DRE element (dehydration responsive element) of 9bp (TACCGACAT) in the promoter area of an rd29A gene in Arabidopsis thaliana, and in the same year, another DAN regulator element CRT (C-repeat) of 5bp (CCGAC)were identified in the promoter area of a *COR15a gene.* Both of the elements have a core sequence CCGAC, that is LTRE (low-temperature responsive element). CRT/DRE or their core sequence CCGAC is common in the promoter of low-temperature induced genes and can promote the expression of genes under low-temperature, dry and high-salt conditions. The first CRT/DRE binding factor is CBF1 transcription factor and later CBF2 and CBF3 were separated by using probes. People also have cloned several kinds of CBF genes from other species besides model plant Arabidopsis thaliana. As the promoter area of CBF does not have CRT/DRE sequence and CCGAC sequence motif, the gene family does now have self-regulation and thereby some matter in upstream induces their expression. According to the expression characteristics and function prediction of CBF, Gilmour and others promoted ICE-CBF pathway model in 1998. ICE1 (inducer of *CBF* expression 1) is the only transcription factor identified to directly act on CBF promoter and is a main switch to control the CBF gene. DNA binding experiment shows ICE1can bind with the MYC recognition sequence CANNTG (ICE kit predicted by Gilmour) of the promoter of CBF gene (Chinnusamy *et al*., 2003) and ICE kit is an important cis-acting element that inactivates the transcription of CBF gene (Zarka *et al*., 2003). ICE1 is passivated by phosphorylation under normal temperature and activated at low temperature by dephosphorylation and specifically bond onto the MYC sitein the promoter of CBF, thereby inducing the expression of CBF.
JagloOhoson founded in 1998 that after being over-expressed in transgenic Arabidopsis thaliana, CBF1 enables the COR gene that contains CRT/DRE regulation element to express without low-temperature induction, which indicates that the CBF1 is an important regulation factor for low-temperature domestication of Arabidopsis thaliana and can increase the cold resistance of Arabidopsis thaliana by increasing the expression level of COR gene. CBFs expression increases the cold resistance and the transgenicArabidopsis thaliana grows slowly and blossoms late (Gilmour *et al*., 2004). Study done by Fernando and others in 2003 proves cbf2 mutants of Arabidopsis thaliana, when stressed by cold, will have increased expression of CBF1 and CBF3 and higher dry and salt resistance, which proves CBF2 is a negative regulator of CBF1 and CBF3. Compared to normal Arabidopsis thaliana plants, ICE1 transgenic plants haveobviously high cold resistance (Chinnusamy *et al*., 2003; Chinnusamy *et al*., 2006). ZhengYinying and others also transplanted cloned ICE1 gene of Arabidopsis thaliana into Nicotianatabacum, and the result shows that the transgenic Nicotianatabacumplants has higher survival rate than reference plants under condition of instant chilling damage, which indicates that the ICE1 gene can increase the cold resistance of high and low-temperature sensitive plants. The transcriptomicsstudy of mutants shows that expression defects of ICE1 leads to 50% failure of low-temperature activation of low-temperature induced gene. The studies on ICE1 gene about increasing cold resistance in receptor plants cannot control the increase level of cold resistance of receptor plants.

### Summary of the Invention

The present invention provides an epigenetic manipulation technology for quantitative regulation of gene methylation. A synthetic gene is designed by quantitatively replacing methylation sites with degenerate codons or genetic manipulation for quantitative replacementof in-situ genes with the degenerate codons is carried out by using a CRISPR/Cas9 technology, the degree of methylation of a target gene is in vivo regulated quantitatively in a transgenic receptor or a target plant to realize the regulation of gene transcription and expression level, and achieve an effect of epigenetically manipulating phenotypic plasticity traits of the plants.

A method for epigenetically manipulating phenotypic plasticity traits of plants includes: quantitatively replacing methylation sites of a target gene by means of a degenerate codon, and carrying out transgenic manipulation by designing a synthetic gene; or constructing a plant expression vector by using a CRISPR/Cas9 technology to quantitatively replace methylation sites with degenate codons of an in-situ in-vivo target gene so as to regulate the expression level of the gene and to attain quantitative regulation on plasticity traits of the plants.

The method preferably includes: by quantitatively replacing methylation cytimidine sites of the target gene with the degenerate codons, quantitatively controlling the number of sites capable of being re-methylated of the gene in an organism so as to quantitatively regulate the degree of methylation and expression level of the target gene, and to attain quantitative regulation on the plasticity traits of the plants.

The method further preferably includes: designing a target gene quantitatively replaced by a synthetic codon, carrying out the transgenic genetic manipulation, and quantitatively regulating the degree of methylation and expression level of an exogenous gene in a receptor so as to attain the quantitative regulation on the plasticity traits of the plants.

The method further preferably includes: constructing a plant expression vector by using the CRISPR/Cas9 technology to quantitatively replace the methylation cytimidine site of the target gene with the in-situ in-vivo gene degenerate codon, and quantitatively regulating the degree of methylation and expression level of the in-situ in-vivo target gene so as to attain the quantitative regulation on the plasticity traits of a modified plant.

In the method of the present invention, the plasticity trait is preferably cold resistance.

In the method of the present invention, the target gene is preferably *ICE1* gene.

The *ICE1* gene is selected from Ageratinaadenophora*AaICE1* gene, Manihotesculenta*MeICE1* gene, Actinidiachinensis*AcICE1* gene, Musa nana *MaICE1* gene, Musa basjoo*MabICE1* gene, Theobroma cacao *TcICE1* gene, Citrus trifoliata*CtICE1* gene, Vitisvinifera*VvICE1* gene, Malusdomestica*MdICE1* gene, Chorisporabungeana*CbICE1* gene, Solanumcapsicoides*ScICE1* gene, Solanumlycopersicum*SlICE1* gene, *PrunuspersicaPpICE1* gene, Zea mays *ZmICE1* gene, Gossypiumhirsutum*GhICE1* gene, Arachishypogae*AhICE1* gene, Brassica juncea*BjICE1* gene, Brassica rapa*BrICE1* gene, Chrysanthemum dichrum*CdICE1* gene, Thellungiellahalophila*ThICE1* gene, Daucuscarota*DcICE1* gene, Glycine max *GmICE1* gene, Raphanussativus*RsICE1* gene, Triticumaestivum*TaICE1* gene, Hordeumvulgare*HvICE1* gene, Heveabrasiliensis*HbICE1* gene, Juglansregia*JrICE1* gene, Lactuca sativa *LsICE1* gene, Oryza sativa *OsICE1* gene, Phalaenopsisaphrodite*PaICE1* gene, Prunusmume*PmICE1* gene, Camellia sinensis*CsICE1* gene, Arabidopsis thaliana *AtICE1* gene, Brassica *rapaBrrICE1* gene, Isatistinctoria*ItICE1* gene, Eucalyptus camaldulensis*EcICE1* gene, Jatrophacurcas*JcICE1* gene, Piceaabies*PiaICE1* gene, Populussuaveolens*PsICE1* gene, Populustomentosa*PtICE1* gene, Potamogetonperfoliatus*PopICE1* gene, Potamogetonwrightii*PwICE1* gene, wherein a nucleotide sequence of Ageratinaadenophora AaICE1 gene is SEQ ID NO: 1, a nucleotide sequence of Manihotesculenta MeICE1 gene is SEQ ID NO: 9, a nucleotide sequence of Actinidiachinensis AcICE1 gene is SEQ ID NO: 10, a nucleotide sequence of Musa nana MaICE1 gene is SEQ ID NO: 11, a nucleotide sequence of Musa basjoo MabICE1 gene is SEQ ID NO: 12, a nucleotide sequence of Theobroma cacao TcICE1 gene is SEQ ID NO: 13, a nucleotide sequence of Citrus trifoliata CtICE1 gene is SEQ ID NO: 14, a nucleotide sequence of Vitisvinifera VvICE1 gene is SEQ ID NO: 15, a nucleotide sequence of Malusdomestica MdICE1 gene is SEQ ID NO: 16, a nucleotide sequence of Chorisporabungeana CbICE1 gene is SEQ ID NO: 17, a nucleotide sequence of Solanumcapsicoides ScICE1 gene is SEQ ID NO: 18, a nucleotide sequence of Solanumlycopersicum SlICE1 gene is SEQ ID NO: 19, a nucleotide sequence of Prunuspersica PpICE1 gene is SEQ ID NO: 20, a nucleotide sequence of Zea mays ZmICE1 gene is SEQ ID NO: 21, a nucleotide sequence of Gossypiumhirsutum GhICE1 gene is SEQ ID NO: 22, a nucleotide sequence of Arachishypogaea AhICE1 gene is SEQ ID NO: 23, a nucleotide sequence of Brassica juncea BjICE1 gene is SEQ ID NO: 24, a nucleotide sequence of Brassica rapa BrICE1 gene is SEQ ID NO: 25, a nucleotide sequence of Chrysanthemum dichrum CdICE1 gene is SEQ ID NO: 26, a nucleotide sequence of Thellungiellahalophila ThICE1 gene is SEQ ID NO: 27, a nucleotide sequence of Daucuscarota DcICE1 gene is SEQ ID NO: 28, a nucleotide sequence of Glycine max GmICE1 gene is SEQ ID NO: 29, a nucleotide sequence of Raphanussativus RsICE1 gene is SEQ ID NO: 30, a nucleotide sequence of Triticumaestivum TaICE1 gene is SEQ ID NO: 31, a nucleotide sequence of Hordeumvulgare HvICE1 gene is SEQ ID NO: 32, a nucleotide sequence of Heveabrasiliensis HbICE1 gene is SEQ ID NO: 33, a nucleotide sequence of Juglansregia JrICE1 gene is SEQ ID NO: 34, a nucleotide sequence of Lactuca sativa LsICE1 gene is SEQ ID NO: 35, a nucleotide sequence of Oryza sativa OsICE1 gene is SEQ ID NO: 36, a nucleotide sequence of Phalaenopsisaphrodite PaICE1 gene is SEQ ID NO: 37, a nucleotide sequence of Prunusmume PmICE1 gene is SEQ ID NO: 38, a nucleotide sequence of Camellia sinensis CsICE1 gene is SEQ ID NO: 39, a nucleotide sequence of Arabidopsis thaliana AtICE1 gene is SEQ ID NO: 40, a nucleotide sequence of Brassica rapa BrrICE1 gene is SEQ ID NO: 41, a nucleotide sequence of Isatistinctoria ItICE1 gene is SEQ ID NO: 42, a nucleotide sequence of Eucalyptus camaldulensis EcICE1 gene is SEQ ID NO: 43, a nucleotide sequence of Jatrophacurcas JcICE1 gene is SEQ ID NO: 44, a nucleotide sequence of Piceaabies PiaICE1 gene is SEQ ID NO: 45, a nucleotide sequence of Populussuaveolens PsICE1 gene is SEQ ID NO: 46, a nucleotide sequence of Populustomentosa ICE1 gene is SEQ ID NO: 47, a nucleotide sequence of Potamogetonperfoliatus PopICE1 gene is SEQ ID NO: 48, and a nucleotide sequence of Potamogetonwrightii PwICE1 gene is SEQ ID NO: 49.

The nucleotide sequences of the ICE1 genes after the methylation cytimidine is all replaced by the degeneration codon are: Ageratinaadenophora: SEQ ID NO: 3, Manihotesculenta: SEQ ID NO: 50, Actinidiachinensis: SEQ ID NO: 51, Musa rubinea: SEQ ID NO: 52, Musa basjoo: SEQ ID NO: 53, Theobromacacao: SEQ ID NO: 54, Citrus trifoliata: SEQ ID NO: 55, Vitisvinifera: SEQ ID NO: 56, Malusdomestica: SEQ ID NO: 57, Chorisporabungeana: SEQ ID NO: 58, Solanumcapsicoides: SEQ ID NO: 59, Solanumlycopersicum: SEQ ID NO: 60, Prunuspersica: SEQ ID NO: 61, Zeamays: SEQ ID NO: 62, Gossypiumhirsutum: SEQ ID NO: 63, Arachishypogaea: SEQ ID NO: 64, Brassica juncea: SEQ ID NO: 65, Brassica rapa: SEQ ID NO: 66, Chrysanthemum dichrum: SEQ ID NO: 67, Thellungiellahalophila: SEQ ID NO: 68, Daucuscarota: SEQ ID NO: 69, Glycine max: SEQ ID NO: 70, Raphanussativus: SEQ ID NO: 71, Triticumaestivum: SEQ ID NO: 72, Hordeumvulgare: SEQ ID NO: 73, Heveabrasiliensis: SEQ ID NO: 74, Juglansregia: SEQ ID NO: 75, Lactucasativa: SEQ ID NO: 76, Oryzasativa: SEQ ID NO: 77, Phalaenopsisaphrodite: SEQ ID NO: 78, Prunusmume: SEQ ID NO: 79, Camellia sinensis: SEQ ID NO: 80, Arabidopsis thaliana: SEQ ID NO: 81, Brassica rapa: SEQ ID NO: 82, Isatistinctoria: SEQ ID NO: 83, Eucalyptus camaldulensis: SEQ ID NO: 84, Jatrophacurcas: SEQ ID NO: 85, Piceaabies: SEQ ID NO: 86, Populussuaveolens: SEQ ID NO: 87, Populustomentosa: SEQ ID NO: 88, Potamogetonperfoliatus: SEQ ID NO: 89,Potamogetonwrightii: SEQ ID NO: 90; and nucleotide sequences of the ICE1 genes after methylation cytimidine is partially replaced by the degeneration codon are: Ageratinaadenophora: SEQ ID NO: 4, Manihotesculenta: SEQ ID NO: 91, Actinidiachinensis: SEQ ID NO: 92, Musa rubinea: SEQ ID NO: 93, Musa basjoo: SEQ ID NO: 94, Theobromacacao: SEQ ID NO: 95, Citrus trifoliata: SEQ ID NO: 96, Vitisvinifera: SEQ ID NO: 97, Malusdomestica: SEQ ID NO: 98, Chorisporabungeana: SEQ ID NO: 99, Solanumcapsicoides: SEQ ID NO: 100 Solanumlycopersicum: SEQ ID NO: 101, Prunuspersica: SEQ ID NO: 102, Zeamays: SEQ ID NO: 103, Gossypiumhirsutum: SEQ ID NO: 104, Arachishypogaea: SEQ ID NO: 105, Brassica juncea: SEQ ID NO: 106, Brassica rapa: SEQ ID NO: 107, Chrysanthemum dichrum: SEQ ID NO: 108, Thellungiellahalophila: SEQ ID NO: 109, Daucuscarota: SEQ ID NO: 110, Glycine max: SEQ ID NO: 111, Raphanussativus: SEQ ID NO: 112, Triticumaestivum: SEQ ID NO: 113, Hordeumvulgare: SEQ ID NO: 114, Heveabrasiliensis: SEQ ID NO: 115, Juglansregia: SEQ ID NO: 116, Lactucasativa: SEQ ID NO: 117, Oryzasativa: SEQ ID NO: 118, Phalaenopsisaphrodite: SEQ ID NO: 119, Prunusmume: SEQ ID NO: 120, Camellia sinensis: SEQ ID NO: 121, Arabidopsis thaliana: SEQ ID NO: 122, Brassica rapa: SEQ ID NO: 123, Isatistinctoria: SEQ ID NO: 124, Eucalyptus camaldulensis: SEQ ID NO: 125, Jatrophacurcas: SEQ ID NO: 126, Piceaabies: SEQ ID NO: 127, Populussuaveolens: SEQ ID NO: 128, Populustomentosa: SEQ ID NO: 129, Potamogetonperfoliatus: SEQ ID NO: 130, Potamogetonwrightii: SEQ ID NO: 131.

As a preferred method of the present invention, the synthetic AaICE1 gene or the AaICE1 gene after the methylation cytimidine is all or partially insitu replaced with the degenerate codon by means of CRISRP/Cas9 is introduced into plants of Oryza, Manihotesculenta, Musa nana, Solanumlycopersicum, Theobroma cacao, Heveabrasiliensis, Citrus limon, Citrus sinensis, Citrus reticulata, Saccharumofficinarum, Carica papaya, Eriobotrya japonica , Litchi chinensis, Dimocarpuslongan, Mangiferaindica, Solanumtuberosum, Capsicum annuum, Sorghum bicolor, Vitisvinifera, Malusdomestica, Chorisporabungeana, Solanumcapsicoides, Actinidiachinensis, Prunuspersica, Zea mays, Gossypiumhirsutumspp, Arachishypogaea, Brassica juncea, Brassica rapa, Chrysanthemum dichrum, Daucuscarota, Glycine max, Raphanussativus, Triticumaestivum, Hordeumvulgare, Juglansregia, Lactuca sativa, Cymbidium hybridum, Phalaenopsisaphrodite, Chimonanthus praecox, Camellia sinensis and Eucalyptus camaldulensi to obtain transgenic plants with enhanced cold resistance; and the AaICE1 gene after the methylation cytimidine is all replaced has a sequence of SEQ ID NO: 3, and the AaICE1 gene after the methylation cytimidine is partially replaced has a sequence of SEQ ID NO: 4.

A cold-resistant gene is selected from an ICE1 gene after the methylation cytimidine is all or partially replaced.

The wild-type *ICE1* gene is selected from Ageratinaadenophora*AaICE1* gene, Manihotesculenta*MeICE1* gene, Actinidiachinensis*AcICE1* gene, Musa rubinea*MaICE1* gene, Musa basjoo*MabICE1* gene, Theobroma cacao*TcICE1* gene, Citrus trifoliata*CtICE1* gene, Vitisvinifera*VvICE1* gene, Malusdomestica*MdICE1* gene, Chorisporabungeana*CbICE1* gene, Solanumcapsicoides*ScICE1* gene, Solanumlycopersicum*SlICE1* gene, Prunuspersica*PpICE1* gene, Zea mays *ZmICE1* gene, Gossypiumhirsutum*GhICE1* GENE, Arachishypogaea*AhICE1* gene, Brassica juncea*BjICE1* gene, Brassica rapa*BrICE1* gene, Chrysanthemum dichrum*CdICE1* gene, Thellungiellahalophila*ThICE1* gene, Daucuscarota*DcICE1* gene, Glycine max *GmICE1* gene, Raphanussativus*RsICE1* gene, Triticumaestivum*TaICE1* gene, Hordeumvulgare*HvICE1* gene, Heveabrasiliensis*HbICE1* gene, Juglansregia*JrICE1* gene, Lactuca sativa *LsICE1* gene, Oryza sativa *OsICE1* gene, Phalaenopsisaphrodite*PaICE1* gene, Prunusmume*PmICE1* gene, Camellia sinensis*CsICE1* gene, Arabidopsis thaliana *AtICE1* gene, Brassica rapa*BrrICE1* gene, Isatistinctoria*ItICE1* gene, Eucalyptus camaldulensis*EcICE1* gene, Jatrophacurcas*JcICE1* gene, Piceaabies*PiaICE1* gene, Populussuaveolens*PsICE1* gene, Populustomentosa*PtICE1* gene, Potamogetonperfoliatus*PopICE1* gene, Potamogetonwrightii*PwICE1* gene, wherein a nucleotide sequence of Ageratinaadenophora AaICE1 gene is SEQ ID NO: 1, a nucleotide sequence of Manihotesculenta MeICE1 gene is SEQ ID NO: 9, a nucleotide sequence of Actinidiachinensis AcICE1 gene is SEQ ID NO: 10, a nucleotide sequence of Musa nana MaICE1 gene is SEQ ID NO: 11, a nucleotide sequence of Musa basjoo MabICE1 gene is SEQ ID NO: 12, a nucleotide sequence of Theobroma cacao TcICE1 gene is SEQ ID NO: 13, a nucleotide sequence of Citrus trifoliata CtICE1 gene is SEQ ID NO: 14, a nucleotide sequence of Vitisvinifera VvICE1 gene is SEQ ID NO: 15, a nucleotide sequence of Malusdomestica MdICE1 gene is SEQ ID NO: 16, a nucleotide sequence of Chorisporabungeana CbICE1 gene is SEQ ID NO: 17, a nucleotide sequence of Solanumcapsicoides ScICE1 gene is SEQ ID NO: 18, a nucleotide sequence of Solanumlycopersicum S1ICE1 gene is SEQ ID NO: 19, a nucleotide sequence of Prunuspersica PpICE1 gene is SEQ ID NO: 20, a nucleotide sequence of Zea mays ZmICE1 gene is SEQ ID NO: 21, a nucleotide sequence of Gossypiumhirsutum GhICE1 gene is SEQ ID NO: 22, a nucleotide sequence of Arachishypogaea AhICE1 gene is SEQ ID NO: 23, a nucleotide sequence of Brassica juncea BjICE1 gene is SEQ ID NO: 24, a nucleotide sequence of Brassica rapa BrICE1 gene is SEQ ID NO: 25, a nucleotide sequence of Chrysanthemum dichrum CdICE1 gene is SEQ ID NO: 26, a nucleotide sequence of Thellungiellahalophila ThICE1 gene is SEQ ID NO: 27, a nucleotide sequence of Daucuscarota DcICE1 gene is SEQ ID NO: 28, a nucleotide sequence of Glycine max GmICE1 gene is SEQ ID NO: 29, a nucleotide sequence of Raphanussativus RsICE1 gene is SEQ ID NO: 30, a nucleotide sequence of Triticumaestivum TaICE1 gene is SEQ ID NO: 31, a nucleotide sequence of Hordeumvulgare HvICE1 gene is SEQ ID NO: 32, a nucleotide sequence of Heveabrasiliensis HbICE1 gene is SEQ ID NO: 33, a nucleotide sequence of Juglansregia JrICE1 gene is SEQ ID NO: 34, a nucleotide sequence of Lactuca sativa LsICE1 gene is SEQ ID NO: 35, a nucleotide sequence of Oryza sativa OsICE1 gene is SEQ ID NO: 36, a nucleotide sequence of Phalaenopsisaphrodite PaICE1 gene is SEQ ID NO: 37, a nucleotide sequence of Prunusmume PmICE1 gene is SEQ ID NO: 38, a nucleotide sequence of Camellia sinensis CsICE1 gene is SEQ ID NO: 39, a nucleotide sequence of Arabidopsis thaliana AtICE1 gene is SEQ ID NO: 40, a nucleotide sequence of Brassica rapa BrrICE1 gene is SEQ ID NO: 41, a nucleotide sequence of Isatistinctoria ItICE1 gene is SEQ ID NO: 42, a nucleotide sequence of Eucalyptus camaldulensis EcICE1 gene is SEQ ID NO: 43, a nucleotide sequence of Jatrophacurcas JcICE1 gene is SEQ ID NO: 44, a nucleotide sequence of Piceaabies PiaICE1 gene is SEQ ID NO: 45, a nucleotide sequence of Populussuaveolens PsICE1 gene is SEQ ID NO: 46, a nucleotide sequence of Populustomentosa PtICE1 gene is SEQ ID NO: 47, a nucleotide sequence of Potamogetonperfoliatus PopICE1 gene is SEQ ID NO: 48, and a nucleotide sequence of Potamogetonwrightii PwICE1 gene is SEQ ID NO: 49.

The nucleotide sequences of the *ICE1* genes after the methylation cytimidine is all replaced by the degenerate codon are: Ageratinaadenophora: SEQ ID NO: 3, Manihotesculenta: SEQ ID NO: 50, Actinidiachinensis: SEQ ID NO: 51, Musa rubinea: SEQ ID NO: 52, Musa basjoo: SEQ ID NO: 53, Theobromacacao: SEQ ID NO: 54, Citrus trifoliata: SEQ ID NO: 55, Vitisvinifera: SEQ ID NO: 56, Malusdomestica: SEQ ID NO: 57, Chorisporabungeana: SEQ ID NO: 58, Solanumcapsicoides: SEQ ID NO: 59, Solanumlycopersicum: SEQ ID NO: 60, Prunuspersica: SEQ ID NO: 61, Zeamays: SEQ ID NO: 62, Gossypiumhirsutum: SEQ ID NO: 63, Arachishypogaea: SEQ ID NO: 64, Brassica juncea: SEQ ID NO: 65, Brassica rapa: SEQ ID NO: 66, Chrysanthemum dichrum: SEQ ID NO: 67, Thellungiellahalophila: SEQ ID NO: 68, Daucuscarota: SEQ ID NO: 69, Glycine max: SEQ ID NO: 70, Raphanussativus: SEQ ID NO: 71, Triticumaestivum: SEQ ID NO: 72, Hordeumvulgare: SEQ ID NO: 73, Heveabrasiliensis: SEQ ID NO: 74, Juglansregia: SEQ ID NO: 75, Lactucasativa: SEQ ID NO: 76, Oryzasativa: SEQ ID NO: 77, Phalaenopsisaphrodite: SEQ ID NO: 78, Prunusmume: SEQ ID NO: 79, Camellia sinensis: SEQ ID NO: 80, Arabidopsis thaliana: SEQ ID NO: 81, Brassica rapa: SEQ ID NO: 82, Isatistinctoria: SEQ ID NO: 83, Eucalyptus camaldulensis: SEQ ID NO: 84, Jatrophacurcas: SEQ ID NO: 85, Piceaabies: SEQ ID NO: 86, Populussuaveolens: SEQ ID NO: 87, Populustomentosa: SEQ ID NO: 88, Potamogetonperfoliatus: SEQ ID NO: 89, Potamogetonwrightii: SEQ ID NO: 90.

The nucleotide sequences of the ICE1 genes after the methylation cytimidine is partially replaced by the degeneration codon are: Ageratinaadenophora: SEQ ID NO: 4, Manihotesculenta: SEQ ID NO: 91, Actinidiachinensis: SEQ ID NO: 92, Musa rubinea: SEQ ID NO: 93, Musa basjoo: SEQ ID NO: 94, Theobromacacao: SEQ ID NO: 95, Citrus trifoliata: SEQ ID NO: 96, Vitisvinifera: SEQ ID NO: 97, Malusdomestica: SEQ ID NO: 98, Chorisporabungeana: SEQ ID NO: 99, Solanumcapsicoides: SEQ ID NO: 100 Solanumlycopersicum: SEQ ID NO: 101, Prunuspersica: SEQ ID NO: 102, Zeamays: SEQ ID NO: 103, Gossypiumhirsutum: SEQ ID NO: 104, Arachishypogaea: SEQ ID NO: 105, Brassica juncea: SEQ ID NO: 106, Brassica rapa: SEQ ID NO: 107, Chrysanthemum dichrum: SEQ ID NO: 108, Thellungiellahalophila: SEQ ID NO: 109, Daucuscarota: SEQ ID NO: 110, Glycine max: SEQ ID NO: 111, Raphanussativus: SEQ ID NO: 112, Triticumaestivum: SEQ ID NO: 113, Hordeumvulgare: SEQ ID NO: 114, Heveabrasiliensis: SEQ ID NO: 115, Juglansregia: SEQ ID NO: 116, Lactucasativa: SEQ ID NO: 117, Oryzasativa: SEQ ID NO: 118, Phalaenopsisaphrodite: SEQ ID NO: 119, Prunusmume: SEQ ID NO: 120, Camellia sinensis: SEQ ID NO: 121, Arabidopsis thaliana: SEQ ID NO: 122, Brassica rapa: SEQ ID NO: 123, Isatistinctoria: SEQ ID NO: 124, Eucalyptus camaldulensis: SEQ ID NO: 125, Jatrophacurcas: SEQ ID NO: 126, Piceaabies: SEQ ID NO: 127, Populussuaveolens: SEQ ID NO: 128, Populustomentosa: SEQ ID NO: 129, Potamogetonperfoliatus: SEQ ID NO: 130, Potamogetonwrightii: SEQ ID NO: 131.

The present invention also provides a CRISPR/Cas9 expression vector containing an ICE1 gene. The expression vector is a plant transformation plasmid.

When the CRISPR/Cas9 transformation plasmid containing the expression component is further constructed by using the ICE1 gene, a promoter of Cas9 may be CaMV 35S, U6, T7 and YAO promoters. The expression component can be transferred and integrated into genome of the plant through agrobacterium (such as agrobacterium strain GV3101) for stable inheritance and expression.

By using the genetic manipulation technology for quantitatively regulating the gene methylation provided by the present invention, the gene methylation site can be inherited between transgenic or gene-edited materials, thereby achieving an objective for controlling the traits of the plants.

The plants with different cold resistance are prepared by using the ICE1 genes of different degrees of methylation and can be obtained through the plant transformation plasmids containing the ICE1 expression component. Different plants are different in transformation methods and steps, and different varieties of the same plant may also be different. However, the plant transformation technology and method are well-known and mature. Immature embryos, mature embryos, undifferentiated calluses or protoplasts of the plants are introduced by agrobacterium and screened according to a marker gene, so that transformed plants can be obtained, and plantable seedlings can be obtained through the culture of a rooting culture medium. Pollen of the plant can also be introduced by the agrobacteriam through a pollen mediation method, thereby obtaining transgenic seeds. The specific preparation steps are shown as follows:
(1) constructing CRISPR/Cas9 transformation plasmids containing the ICE1 gene by using a DNA recombination technology; and
(2) introducing the plant transformation plasmids constructed in step (1) into plant tissues by an agrobacterium infection method or a pollen mediation method, and screening a transgenic plant containing the *ICE1* gene by using the marker gene.

The present invention is applicable to all plants including dicotyledon and monocotyledon plants. The plant can be selected from Musa nana, Eriobotrya japonica, Manihotesculenta, Chaenomelessinensis, Dimocarpuslongan, Oryza, Zea mays, Gossypiumhirsutumspp, Triticumaestivum, Sorghum bicolor, Glycine max, Brassica rapa, Solanumtuberosum, Dioscoreaesculenta, Hordeumvulgare, Saccharumofficinarum, Nicotianatabacum, Prunuspersica, Pyrusspp, Heveabrasiliensis, Brassica rapa, Isatistinctoria, Phalaenopsisaphrodite, Camellia sinensis, Armeniacamume, Eucalyptus camaldulensi and vegetables (Pakchoi cabbage, Celery cabbage, Cucumissativus, Petroselinumcrispum, Capsicum annuum, Solanumcapsicoides), etc.

The present invention is proposed based on the following inventive points: the research of the present invention shows that the cold resistance of Arabidopsis thaliana can be improved to different degrees by transforming the *AaICE1* gene of different degrees of methylation of different cold-resistant populations, and the cold resistance of the population is in remarkable negative correlation to the degree of methylation of the *AaICE1* gene, thus indicating that the cold resistance of the transgenic receptor plant can be controlled by controlling the degree of methylation of the gene. In the present invention, it is discovered that the expression of *ICE* is regulated by the degree of methylation of cytimidine in a gene sequence thereof. The higher the degree of methylation is, the lower the expression level is, and otherwise the higher the expression is.
Compared with the prior art, the present invention has the following advantages: first, the gene may be methylated in a recipient organism during the genetic manipulation, which may result in silencing of the genetically manipulated target gene and difficulty in achieving the effect on regulating the gene expression by means of the methylation. According to the present invention, the quantitative replacement of cytimidine bases with degenate codons is designed for a function of adjusting the gene expression of the gene of different methylation levels or the genetic manipulation for quantitative replacement of the in-vivo gene with the degenate codon is carried outby using the CRISPR/Cas9 gene editing technology, and a majority of methylation sites can recover the methylation in therecipient plant, so that the methylation sites and the degree of methylation of a source target gene can be ensured, the degree of methylation corresponding to a donor gene can be obtained by means of screening, and the in-vitro genetic manipulation of the epigenetics can be realized. Second, based on the natural methylation of biological gene in nature and epigentically diversified methylation levels and sites, cloning is carried out, and the genetic manipulation is carried out based on the artificial simulation synthesis of the quantitative replacement of cytimidine base with the degenerate codon so as to culture the transgenic plant. The quantification of the re-methylation of the target gene of the transgenic plant can be ensured, so that the genetically improved plant has higher adaptability. Third, the degenerate codon quantitative replacement is carried out by the CRISPR/Cas9 gene editing technology to quantitatively regulate the methylation site and gene expression of the target gene of the target plant, so that the plasticity trait of the target plant is in situ in vivo regulated quantitatively. Therefore, a technical method is provided for improving the plasticity traits of the organism by means of the gene editing in-vivo in-situ epigenetic manipulation. Since the gene is sourced from the same species, ecological environment and food safety risks after a better crop is cultivated is lower than those of heterologous genes of different creature types. Fourth, the present invention is based on the natural cold-resistant germplasm resource of the plants. Although the ICE1 gene works through the CBF gene, and the cold resistance of the CBF transgenic plant is improved to certain extent, the plant growth is suppressed, and the actual application value is relatively low. However, the ICE1 gene is used as a constitutive expression gene of the plant, after the overexpression of the ICE1 gene, the cold resistance of the plant can be apparently improved, and the phenotype of the plant is not affected. However, before not knowing that the gene expression of the ICE1 gene is controlled by the degree of methylation, it is blind and limited to cultivate the ICE1 transgenic plant with highcold resistance. According to the present invention, the cold resistance can be quantitatively improved by using the ICE1 gene of different degree of methylations. More particularly, an effect of regulating the plant cold resistance by means of cold response transcription pathway of CBF can be maximized so as to cultivate the plant with higher cold resistance. Moreover, the transgenic plant can be kept diversified in cold resistance.

### Detailed Description of the Invention

### Embodiment 1: Obtaining Oryza sativa by transforming AaICE1 gene of Ageratina adenophora of different degrees of methylation

The cDNA sequence of the AaICE1 gene of Ageratina adenophora is cloned, and the sequence is SEQ ID NO: 1. According to degenerate features of a codon, sites that can be theoretically methylated on the AaICE1 gene are replaced. According to requirements on target traits, the sites of different degrees of methylation on the AaICE1 gene are quantitatively replaced. The AaICE1 gene on which all methylation sites are replaced has a sequence of SEQ ID NO: 3 and is named AaICE1(DC)1; and the AaICE1 gene on which part of methylation sites are replaced has a sequence of SEQ ID NO: 4 and is named AaICE1(DC)2. A vector pBI121 and the AaICE1, AaICE1(DC)1 and AaICE1(DC)2 genes are cleaved by restriction endonucleases BsaI and Smal. A target gene is bonded to pBI121, and a Bar gene is used to replace a Kan resistant gene in pBI121 and used as a resistance screening gene for constructing a pBI121-AaICE1 expression vector of different degrees of methylation. The expression vector is introduced into Oryza sativa by an agrobacterium-mediated method.

(Husked) mature seeds of Oryza sativa cv Nipponbare are selected and sterilized by using a mercury bichloride method (immersed in 0.1% mercury bichloride for 15 min, and cleaned with sterilized water), and air-dried on a super clean bench. Thereafter, embryos are placed in a callus inducing culture medium (4.4 g/L MS + 2.5 mg/L 2,4-D + 600 mg/L casein + 30g/L sucrose + 5g/L Phytagel, regulating pH to 5.8 with KOH, and sterilization is carried out under high pressure), and subjected to the callus culture at 28□ for 2 weeks. The agrobacterium GV3101 carrying the plasmid pBI121-AaICE1 is inoculated into 5ml of YEB liquid culture medium containing 5 mg/L Bialaphos, subjected to shaking culture at 28□ until a later period of a logarithmic phase, and subjected to amplification culture in a volume ratio of 1: 100. Agrobacterial thalli are collected when OD600 of the bacterial solution is 0.10 and re-suspended in an infection culture medium. The calluses of the Oryza sativa are infected by a conventional method, placed on a subculture medium containing 500 mg/L Carb and 5 mg/L Bialaphos after the co-culture infection, cultured in dark at 28□for 12-16 d, then transferred onto a subculture medium containing 5 mg/L Bialaphos, continuously subjected toscreening culture, and finally differentiated on a re-differentiation culture medium to obtain positive seedlings with Bialaphos resistance. In the transgenic experiment, 11, 15 and 13 resistant plants with transformed AaICE1, AaICE1(DC)1 and AaICE1(DC)2 genes are obtained respectively. Under a cold treatment condition at 5□, the cold resistance of transgenic offspring plants is differentiated, and target traits can be obtained by screening. Compared with the wild-type plant of Oryza sativa cv Nipponbare, the cold resistance of the obtained resistant plants is respectively improved by 1.4□, 3.0□ and 2.0□.

### Embodiment 2: Obtaining Manihot esculenta by transforming AaICE1 gene of Ageratina adenophora of different degrees of methylation

According to the degenerate feature of a codon, sites that are theoretically methylated in AaICE1 gene of Ageratina adenophora are replaced. According to requirements on target traits, the methylation sites of different degrees on the AaICE1 gene are quantitatively replaced. The AaICE1(DC)1 gene on which all methylation sites are replaced has a sequence of SEQ ID NO: 3 and the AaICE1(DC)2 gene on which part of methylation sites are replaced has a sequence of SEQ ID NO: 4. A vector pBI121 and the AaICE1, AaICE1(DC)1 and AaICE1(DC)2 genes are cleaved by restriction endonucleases BsaI and Smal. A target gene is bonded to pBI121, and a Bar gene is used to replace a Kan resistant gene in pBI121 and used as a resistance screening gene for constructing a pBI121-AaICE1 expression vector of different degrees of methylation. The expression vector is introduced into Manihot esculenta through agrobacterium GV3101.

500ul of agrobacterium GV3101 expression vector stored in an ultralow-temperature refrigerator is added into 50 ml of YEB (mixed with 5 mg/L Bialaphos) liquid culture medium, and cultured at 240 rpm and 28□ for 18-24 h. Then the mixture is centrifuged at 4000 rpm and 4□ for 10 min, and diluted with liquid MS until an OD value of the mixture is 1.0 for standby use. Green cotyledons of Manihot esculenta somatic embryos that are mature for 10-15 d are chopped. About 500 fragments of the cotyledon embryos are placed in 30 ml of agrobacterium suspension culture solution, and the solution is shaken for several times in the meanwhile. The bacterial solution is removed by suction after 45 min. The cotyledons fragments are placed in a super clean bench for 20 min or surplus liquid is blotted up by using sterile filter paper, the cotyledons fragments are transferred onto a stem organ generation culture medium, and co-cultured in light at 26□ for 3 d (16 h for each day)°C. After the transformation materials are co-cultured for 3 d respectively, calluses are taken out, rinsed with sterile water for 3 times, then cleaned for two times with the culture medium containing 500 mg/L Carb and MS (pH 5.3), and placed in the super clean bench for 20 min or the residual liquid is blotted up by using the sterile filter paper. The calluses are transferred onto the stem organ generation culture medium containing 5 mg/L Bialaphos, and cultured in dark at 26□ for 3 weeks. Resistant adventitious buds are picked and placed on a stem elongation culture medium. The resistant adventitious buds are cultured in light at 26□, 16 h for each day. The emerging stems are cut and transferred onto an MS culture medium and cultured after 2-3 weeks. When the stems grow into a plant, the plant is cut apart for subculture. The stems of about 1 cm coming up from lateral buds are cut and transferred onto a hormone-free culture medium containing 5 mg/L Bialaphos for rooting experiment so as to eliminate the false-positive plant and the stems of a wild-type plant are used as references. After 1-2 weeks, the transgenic positive plant can be normally rooted, while the false-positive plant and the reference plant will die. A total of 18, 15 and 13 plants with transformed AaICE1, AaICE1(DC)1 and AaICE1(DC)2 genes are obtained. The transgenic offsprings are screened at a low temperature to obtain plants with target traits. Compared with the wild-type plant of Manihot esculenta, the cold resistance of the obtained transgenic plants is respectively improved by 1.8□, 3.7□ and 2.5□.

### Embodiment 3: Obtaining Musa nana by transforming AaICE gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Musa nana and are introduced into Musa nana through the agrobacterium GV3101.

An embryonic suspension cell system (ECS) of Musa nana is used as a transformation receptor. The ECS of Musa nana subjected to the subculture for 10 d is collected and centrifuged to remove the supernatant, every 1 mL of cell compact volume ECS is mixed with 40 ml of agrobacterium GV3101 containing pBI121-AaICE1 plasmids of the AaICE1 gene of different degrees of methylation. The mixture is kept still in dark at 27□ for 1-2 h, and is co-cultured. Co-culture conditions: the mixture is subjected to shaking culture in dark at a rotation speed of 50 rpm at 27□ for 24 h. Then an obtained culture is kept still to remove the supernatant and is mixed with 40 ml of liquid culture medium. The mixture is continuously co-cultured for 7 d by increasing the rotation speed to 110 rpm. The co-cultured mixture is kept still to remove the supernatant, and is added with 40 ml of Musa nana ECS liquid screening culture medium. The mixture is subjected to shaking culture at a rotation speed of 100 rpm at 27□, sub-cultured every 10-14 d and continuously sub-cultured for more than three generations. A subculture method includes: 0.1-0.5 ml of cell compact volume ECS of a previous generation is collected and added into a fresh liquid screening culture medium for continuiously shaking culture. Meanwhile, the remaining ESC after the subculture of each generation and the ESC after the original co-culture is completed are screened by a semi-solid screening culture medium as a reference. Prior to the use of the semisolid screening, the ESC is washed with an M2 culture medium containing 5 mg/L Bialaphos for 2-3 times, surplus culture medium is blotted up with sterile paper, and then the ESC is transferred onto the semisolid screening culture medium to induce embryos. A somatic embryo inducing culture medium is changed every month until the maturity of the embryos. The ECS after being subjected liquid screening and being cultured for more than 3 generations is collected and kept still to remove supernatant, and is uniformly laid on a somatic embryo inducing culture medium M3, and cultured in dark for 2-3 months. The somatic embryo inducing medium is changed every month until the maturity of the embryos. The mature resistant embryos are transferred onto a somatic embryo germination culture medium, and cultured in light/dark (for 12h/12h) until the somatic embryos are germinated to form seedlings. Then the seedlings are transferred onto a rooting culture medium, and cultured in light/dark (12h/12h), thus obtaining a complete transformed plant. A total of 22, 21 and 15 plants with transformed AaICE1, AaICE1(DC)1 and AaICE1(DC)2 genes are obtained in the experiment. Corresponding transgenic plants are obtained after the cold resistance screening. The cold resistance of the obtained resistant plants is respectively improved by 1.7□, 3.9□ and 2.9□

### Embodiment 4: Obtaining Solanum lycopersicum by transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Solanum lycopersicum and the genetic transformation for the Solanum lycopersicum is carried out by means of agrobacterium infection.

When seedlings of Solanum lycopersicum grow to a height of about 5-6 cm on a 1/2 MS basic culture medium, cotyledons of aseptic seedlings are cut, and used as a receptor material of the transformation. A small amount of agrobacterium tumefaciens pBLGC bacterial solution containing a target expression vector is collected from a glycerin tube, subjected to streaking culture on a YEP solid culture medium (mixed with 5 mg/L Bialaphos and 20 mg/L Rif) and cultured in dark at 28□ until individual colonies in a diameter of about 1 mm come up (about 36-48 h). The individual colonies are collected and transferred to a similar solid culture medium plate, and cultured until a vigorous growth period (about 36 h). The agrobacterium individual colonies are picked from a YEP plate, inoculated into a YEP liquid culture medium containing 5 mg/L Bialaphos and 20 mg/L Rif, uniformly mixed, and subjected to the shaking culture overnight at 180-200 rpm and 28□ (about 16-18 h). In the next day, the mixture which is 1% of the inoculation amount is transferred to 20 ml of YEP liquid culture medium, and subjected to the shaking culture at 180 rpm and 28□ for 3-4 h until the agrobacterium is cultured to a logarithmic phase (OD600 of the bacterial solution is about 0.6). Then the mixture is centrifuged at 4000 rpm and 4□ for 15 min to collect thalli and remove supernatant. The thalli are diluted to 3-5 times the original bacterial solution by using YEP liquid culture medium, added with 200 um acetosyringone, and shaken at 180-200 rpm and 28□ for 1-2 h. Fully-developed green cotyledon leaves are collected, scratched with a sterile scalpel, infected for about 10-20 min in a prepared agrobacterium suspension, taken out and dried with sterile filter paper, transferred into a solid differentiation culture medium (Ms + 6-BA 2m g/L + IAA 0.2 mg/L), and co-cultured in dark at 23-25□ for 2-3 d (according to the growth condition of the thalli). After being co-cultured for three days, the cotyledon leaves are transferred onto a screening culture medium (Ms + 6-BA 1 mg/L + IAA 0.2 mg/L + 5 mg/L Bialaphos + 500 mg/L carbenicillin). The culture medium is changed every three days until no agrobacterium presents. It can be observed that calluses or regenerated cluster buds grow near some incisions in 14 d, and then the regenerated buds are differentiated to form single plants as far as possible, and continuously screened under the pressure of 5 mg/L Bialaphos. In about 4 weeks, the single plants are transferred into a differentiation culture medium containing 5 mg/L Bialaphos and 500 mg/L carbenicillin, and screened for about 1 month. The obtained resistant buds (clusters) are further screened, and the carbenicillin is always kept at 500 mg/L during the screening. In about two months, the regenerated buds grow to 2-3 cm, and at the very time, the survival seedlings can be transferred into a rooting culture medium (1/2MS,IBA 1 mg/L, sucrose 30g/L), and subjected to differentiation culture for 1-2 weeks. During the rooting, the carbenicillin is removed. Finally plants with well developed root systems are transplanted in potted soil. In the transgenic experiment, 13, 18 and 11 resistant plants with transformed AaICE1, AaICE1(DC)1 and AaICE1(DC)2 genes are obtained respectively. The transgenic plants with target traits are obtained by low-temperature screening. Compared with the wild-type plant, the cold resistance of the obtained resistant plants is improved respectively by 1.9□, 4.1□ and 2.4□.

### Embodiment 5: Obtaining Theobroma cacao by transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Theobroma cacao and introduced into plants of Theobroma cacao through the agrobacterium LBA4404.

Mature fruits of Theobroma cacao are collected from the field, peeled, immersed in 75% alcohol for several seconds, then immersed in NaC10 solution with a volume percentage of 5% for 30 min, and inoculated into a germination culture medium after seed coats are removed. After 10 d, cotyledons thereof are collected as explants. Thalli of agrobacteria LBA4404/PBI121 cultured until OD₆₀₀ is about 1.37 are collected, and resuspended in a same volume of PCG liquid culture medium (DKW + 2,4-D 3.0 mg/L + KT 1.0 mg/L + TDZ 0.01 mg/L + Glucose 20 g/L+ glutamine 250 mg/L), a cotyledon material of Theobroma cacao cultured for 10 d in the germination culture medium is infected, and the mixture is co-cultured in dark at 28□ for 3 d. After being co-cultured, the mixture is transferred onto a PCG solid medium (1% agar powder) containing 500 mg/L Carb and 5 mg/L Bialaphos, subjected to bacteriostatic and resistant screening culture, subcultured on the same culture medium every 10 d, cultured in the PCG culture medium for 20 d, then transferred into an SCG culture medium (KW + 2, 4-D 3 mg/L+ KT 1 mg/L + Glucose 20 g/L+10% CW + 1% agar powder) containing 500 mg/L Carb and 5 mg/L Bialaphos, cultured for 20 d, then transferred into an ED culture medium (DKW + Sucrose 30 g/L+ Glucose 1 g/L + 1% agar powder) containing Car 500 mg/L and 5 mg/L Bialaphos, and cultured until an embryoid comes up. Meanwhile, frequent observation is required, if there is contamination or re-appearance of agribacterium, the uncontaminated material is moved out immediately and continuously cultured. The mature embryos are transferred into an embryo development culture medium. The culture medium is classified into the culture medium at two phases, the culture medium at a first phase is PEC (1/2DKW + KNO₃ 0.3 g/L+AA 1000 X stock solution 1 ml/L+ Glucose 20 g/L + Sucrose 10 g/L + 1% agar powder). During the culture at the first phase, the culture condition: the daily illumination period is 16h/8h in light/dark, and the culture medium is changed every 30 d until a bud with 1-2 pieces of euphylla comes up. The mature embryos are then transferred into an RD culture medium at the second phase (1/2DKW + KN0₃ 0.3 g/L + Glucose 10 g/L+ Sucrose 5 g/L + IBA of different concentrations 1.0 mg/L + IAA of different concentrations 1.0 mg/L). The culture conditions at the second phase are consistent with that at the first phase. The embryos are transferred to the culture medium every 30 days until the seedlings are formed. In the experiment, a total of 12, 10 and 9 plants with transformed AaICE1, AaICE(DC)1 and AaICE1(DC)2 genes are obtained. After the cold resistance screening, the cold resistance of the plant with the target traits is respectively improved by 1.5□, 3.9□ and 2.2□.

### Embodiment 6: Obtaining Hevea brasiliensis by transforming AaICE1 gene of Ageratina adenophora f different degrees of methylation

The expression vectors of the AaICE1, *AaICE1*(DC)1 and *AaICE1*(DC)2 genes of different degrees of methylation constructed in embodiment 1 are subjected to genetic transformation of Hevea brasiliensis and are introduced into plants of Hevea brasiliensis through the agrobacterium EHA105.

Anthers at a late uninucleate stage of Hevea brasiliensis are inoculated onto an embryonic callus inducing culture medium and are used for agrobacterium infection after being cultured for 30 d. After being infected in EHA105 suspension for 5 min, the calluses are transferred onto a co-culture medium containing 5uM acetosyringone and 10 mg/L silver nitrate, and cultured in dark at 22□ for 2 d. Each treatment process is repeated for 3 times. According to the bacteria generation condition, the co-cultured calluses are washed with sterile water, then transferred into 50 mg/L Timentin, immersed for 1 min, dried with sterile filter paper, then transferred onto a callus inducing culture medium containing 10 mg/L silver nitrate and 500 mg/L Timentin, and re-cultured in dark at 25□ for 7 d. The calluses recovering growth are transferred into a callus inducing culture medium containing 10mg/L silver nitrate, 5 mg/L Bialaphos and 50 mg/L Timentin for inducing the somatic embryogenesis. Finally, proliferated somatic embryos are transferred into a plant regeneration culture medium containing 5 mg/L Bialaphos for inducing the plant regeneration. In the genetic transformation experiment, 10, 6 and 9 resistant plants of Hevea brasiliensis with transformed AaICE1, AaICE1(DC)1 and AaICE1(DC)2 genes are obtained respectively. After the low-temperature screening, compared with the wild-type plant of Hevea brasiliensis, the cold resistance of the plant of hevea brasiliensis with target traits is respectively improved by 1.6□, 2.9□ and 2.1□.

### Embodiment 7: Obtaining Citrus limonby transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Citrus limon and are introduced into Citrus limon through the agrobacterium EHA105.

An engineering bacterial solution stored at an ultra-low temperature is inoculated onto an LB plate containing 5 mg/l Bialaphos and 50 mg/L Rifampicin, subjected to streaking culture, and activated at 28□ for about 36 h. Individual colonies are picked, subjected to the shaking culture overnight at 150 rpm in an LB liquid culture medium. The bacterial solution is collected, sub-packaged in sterilized centrifugal tubes, centrifuged at 4000 rpm for 10 min to remove supernatant, then subjected to suspension culture for 2 h in an MT liquid culture medium, and diluted for infection until OD600 of the mixed solution is about 0.5. Epicotyl of 30-day-old aseptic seedlings of Citrus limon is collected as an explant, transversely cut into pieces in a size of about 1 cm, infected for 15 min in an agrobacterium bacterial solution, and gently shaken in the meanwhile. The bacterial solution on the epicotyl is blotted up with filter paper, and then the epicotyl is horizontally placed in a BR culture medium with 100 uM of acetosyringone (MT + 1.0 mg/L BAP + 0.1 mg/L NAA + 3% Sucrose), and co-cultured in dark at 27□ for 3 d. The co-cultured epicotyl is washed with the sterile water for 4 times and washed with 400 mg/L cephalosporin for one time. After being dried with sterile filter paper, the epicotyl is transferred into the BR culture medium containing 5 mg/L Bialaphos and 400 mg/L cephalosporin, and cultured in light. When an obtained resistant bud is elongated to about 1 cm, the resistant bud is cut off from a base part, and transferred into a culture medium containing 5 mg/L Bialaphos to continuously grow and to induce the rooting. After the root is sufficiently developed, seedling hardening is conducted for 1-2 d by opening a flask. The plant is taken out from the culture medium and cleaned with tap water to remove agar on roots. Then the plant is transplanted into a beaker with sterilized humus soil and vermiculite mixture, covered with a breathable film, and transferred into a flowerpot with nutritional soil by gradually removing the breathable film. In the genetic transformation experiment, 8, 8 and 6 resistant plants of Citrus limon with transformed AaICE1, AaICE1(DC)1 and AaICE1(DC)2 genes are obtained respectively. After the low-temperature screening, compared with the wild-type plant of Citrus limon, the cold resistance of the plant of Citrus limon with target traits is improved respectively by 1.6□, 3.2□ and 2.3□.

### Embodiment 8: Obtaining Citrus sinensis by transforming AaICE1 gene of Ageratina adenophora ofdifferent degrees of methylation

The expression vectors of the AaICE1, *AaICE1*(DC)1 and *AaICE1*(DC)2 genes of different degrees of methylation constructed in embodiment 1 are subjected to genetic transformation of Citrus sinensis and are introduced into Citrus sinensis through the agrobacterium MSU440.

A transformation receptor material is mainly Citrus sinensis "Zao Jin". Seeds are collected from mature fruits of Citrus sinensis, cleaned with tap water, then immersed in 1 M of NaOH solution for 10 min to remove pectin, rinsed with tap water for 3-5 times, immersed and sterilized for 15-20 min in 3% sodium hypochlorite solution on a super clean bench, and rinsed with sterilized distilled water for 3 times and 5 min at each time. After inner and outer seed coats are stripped on the super clean bench, the sterilized seeds are sowed in an MT seeding culture medium (MT + 25 g/L sucrose + 8 g/L agar), and cultured in dark at 26□ for 4 weeks. Thereafter, an etiolated seedling is taken out, and put in light until epicotyl begins to turn green (about 5-7 d are required), and then epicotyl stems of the aseptic seedling are cut for agrobacterium infection. A bacterial strain of agrobacterium rhizogenes MSU440 stored at -80□ is streaked on an LB solid culture medium containing 50 mg/L spectinomycin, subjected to inverting culture for 36-48 h in dark at 28□ to obtain individual colonies. The individual colonies are picked, streaked on an LB solid culture medium containing 50 mg/L spectinomycin, and cultured for 36-48 h under the same conditions. All thalli are scraped into an antibiotic-free MT liquid culture medium by using a sterile scalpel, and subjected to shaking cultureat 200 rpm and 28□ for 1.5-2 h, and then the bacterial solution is regulated for standby use until the OD600 thereof is 0.6-0.8. Epicotyl segments are immersed and infected in the agrobacterium rhizogenes bacterial solution for 20 min, and shaken for several times in the meanwhile. After the infection is finished, surplus bacterial solution on an explant is blotted up with sterile filter paper, then the explant is transferred onto an MT co-culture medium with sterile filter paper (MT + 40 g/L sucrose + 8 g/L agar + 20 mg/L acetosyringone), co-cultured in dark at 21□ for 3 d, subsequently rinsed with sterile water for 3 times, dried with the filter paper, then transferred onto an MT basic culture medium containing 400 mg/L cephalosporin (MT + 40 g/L sucrose + 8 g/L agar + 400 mg/L cephalosporin), and cultured in dark at 26□ for inducing hairy roots. The hairy roots are cut into root segments in a length of about 1.5 cm. The root segments are cultured in light/dark (16/8 h) at 26□ on an MT germination culture medium (MT + 40 g/L sucrose + 8 g/L agar + 1 mg/L BA) for inducing the germination. When a bud grows to a length of about 0.5 cm, the bud is cut off and transferred onto an MT elongation culture medium (MT + 30 g/L sucrose + 8 g/L agar + 0.1 mg/L BA + 0.1 mg/L IAA + 0.25 mg/L GA3). When elongated to a length of about 1 cm, the bud is transferred onto an MT rooting culture medium (1/2 MT + 25 g/L sucrose + 8 g/L agar + 0.5 mg/L NAA + 0.1 mg/L IBA + 0.5 g/L activated carbon) for inducing the rooting. In the genetic transformation experiment, 14, 10 and 11 resistant plants of Citrus sinensis with transformed AaICE1, AaICE1(DC)1 and AaICE1(DC)2 genes are obtained respectively. After low-temperature screening, compared with the wild-type plant of Citrus sinensis , the cold resistance of the plant of Citrus sinensis with target traits is respectively improved by 2.0□, 3.8□ and 2.5□.

### Embodiment 9: Obtaining Citrus reticulata by transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Citrus reticulata and are introduced into Citrus reticulata through the agrobacterium EHA105.

Mature fruits of Citrus reticulata are pulverized, the seeds are taken out, and immersed in 1 mol/L NaOH for 10 min, pectins on seed surfaces are removed, and then the seeds are cleaned with tap water. The seeds are immersed for 2-3 min in 70% alcohol on a super clean bench, immersed and sterilized for 20 min by using 2% sodium hypochlorite, and shaken for several times in the meanwhile, and after the sodium hypochlorite solution is removed, the seeds are washed with sterile water for 3 times and 5 min at each time. Inner and outer seed coats of the sterilized seeds are removed on the super clean bench, and then the seeds are placed in a test tube containing an MT culture medium, and cultured in dark at 26□ for 20-30 d. Etiolated epicotyl or stems are cultured in light (at 26□, light cycle of 14/10 h) for 10-15 d and collected for transformation experiment. Agrobacterium stored in an ultralow-temperature refrigerator is streaked on a YEB solid culture medium containing 5 mg/L Bialaphos and 40 mg/L Rifampicin, and cultured in dark at 28□ for 2 d. Monoclones are picked, inoculated in a YEB liquid culture medium containing 40 mg/L Rif and 5 mg/L Bialaphos, subjected to shaking culture for 24 h (at 200 rpm and 28□), and subjected to streaking culture again on the YEB solid culture medium. In 48 h, the agrobacterium with good nutrition is scraped by using a stainless steel long-handle medicine spoon, inoculated into 40 ml of MT suspension culture medium (MT + 0.5 g/L malt extract + 1.5 g/L glutamine, PH 5.7), and subjected to shaking culture at 180 rpm and 28□ for 2 h. The cultured agrobacterium bacterial solution is diluted for standby use by using an MT suspension culture medium until OD600 of the bacterial solution is 0.8. A properly-treated explant is placed into the agrobacterium solution (containing 100 umol/L AS), and infected for 20 min (gently shaken for several times in the meanwhile). Thereafter, the bacterium solution is poured out, and an explant material is placed on sterile filter paper to blot up the bacterial solution attached thereon. The material is subsequently transferred onto a co-culture medium (MT+100 uM AS + 0.4 mg/L 2,4-D,PH 5.7). The co-culture medium is covered with a layer of filter paper. After the explant material is cultured in dark at 22□ for 3 d, the explant material is transferred into a screening culture medium and cultured. After the explant material is screened for 4 months, a regenerated adventitious bud (in a length about 1 cm) is cut off, and subjected to the greenhouse grafting. In the genetic transformation experiment, 10, 13 and 9 resistant plants of Citrus reticulata with transformed AaICE1, AaICE1(DC)1 and AaICE1(DC)2 genes are obtained respectively. The transgenic plants with the target traits are obtained by low-temperature screening. Compared with the wild-type plant of Citrus reticulata, the cold resistance of the plant of Citrus reticulate with the target trait is respectively improved by 1.8□, 3.7□ and 2.3□.

### Embodiment 10: Obtaining Saccharum officinarum by transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Saccharum officinarum and are introduced into Saccharum officinarum through the agrobacterium infection.

A field material is used as an explant. A plant of Saccharum officinarum growing strong is selected, an end portion thereof is collected, external old leaves on the end portion are gradually removed layer by layer. Tissues of young leaves that are about 10 cm away from a top growth point are collected as explants. The explants are immersed in 70% ethanol for 30 s, then treated with 0.1% mercury bichloride aqueous solution for 10 min, and then washed with sterile water for 3-4 times, water drops are blotted up, and the explants are transversely cut into slices in a thickness of 0.2-0.5 mm, and inoculated onto a solid culture medium. A solid culture medium (M1) of (MS+2, 4-D1mg/L + sucrose 30 g/L, pH 5.8) is used as an embryonic callus inducing culture medium for dark culture at 25□. Individual colonies are picked from an agrobacterium storage plate, inoculated into 5ml of YEP liquid culture medium (containing 5 mg/L Bialaphos, 25 ug/ml Rif), and subjected to shaking culture at 200 rpm and 28□ until a logarithmic phase (about 24 h). The individual colonies are subjected to amplification culture in 40ml of YEP medium containing same antibiotics in a 150 ml triangular flask until the OD600 of the bacterial solution is about 0.6, and transferred into a centrifugal tube, and centrifuged at 5000 rpm and 4□ for 5 min, supernatant is discarded, and residual liquid is blotted up. Bacteria are re-suspended in an equal volume of MR liquid culture medium containing 150 umol/L AS (1/5 MS microelements + MS other ingredients + 2,4-D 1 mg/L + AS 150 umol/L + 10 mmol/L fructose + 10 mmol/L glucose + 30 g/L sucrose, pH 5.3), and cultured at 2000 rpm and 28□ for 2 h. Bacteria are induced for Vir gene expression and used as a bacterial solution stock solution for infecting a transformed material. Calluses that are cultured in dark on an M1 culture medium and grow vigorously and consistently are transferred onto a fresh M1 culture medium, cultured for 4 d, and then used for the transformation test. When in transformation, the calluses are placed on sterile filter paper, subjected to blow-dry treatment for 1-2 h on a super clean bench until the surface of a tissue block is dry. The tissue block can be used for bacterial infection once a little bit shrinkage occurs. The material after being subjected to the immersion infection by the bacterial solution for 10 min is transferred onto the filter paper, and subjected to blow drying on the super clean bench until the surface of the material is dry and free from a water membrane. Then the material tissues are cut into pieces of 0.3-0.5 cm, then transferred onto an M1 solid culture medium containing 100 umol/L AS, and co-cultured in dark at about 23□ for 3 d. The co-cultured pieces are washed with sterile water for 3-4 times, then washed with an MS liquid culture medium for one time, and subjected to blow drying on filter paper on the super clean bench until dry. The cleaned and dried transformation material after being co-cultured is inoculated onto an M2 solid culture medium containing 500 mg/L carbenicillin and 5 mg/L Bialaphos, and subjected to screening culture. The transformation material is subcultured on the same culture medium every 20-30 d in future. Frequent observation is required, when contamination or reappearance of agrobacterium is discovered, the uncontaminated material is moved out immediately for continuous culture. Furthermore, the tissue blocks having browning contamination are frequently removed or the good or uncontaminated tissue blocks are transferred onto the similar new culture medium. After the tissue blocks are swelled, large tissues are broken into small pieces frequently. Budlets are transferred onto the culture medium immediately once emerging so as to ensure the sufficient selection pressure. When growing to a height of 4-5 cm, resistant seedlings are transferred onto an M3 culture medium containing 5 mg/L Bialaphos and 300 mg/L Cef for inducing the rooting. In the genetic transformation experiment, 15, 12 and 17 resistant plants of Saccharum officinarum with transformed AaICE1, AaICE1(DC)1 and AaICE1(DC)2 genes are obtained respectively. The transgenic plants with the target traits are obtained by low-temperature screening. Compared with the wild-type Saccharum officinarum, the cold resistance of the plant of Saccharum officinarum with target traits is respectively improved by 2.4□, 4.6□ and 3.3□.

### Embodiment 11: Obtaining Carica papaya with transformed AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Carica papaya and are introduced into Carica papaya through the agrobacterium infection.

Fruits of Carica papaya that are 70%-80% mature are selected from a healthy and virus-free plants, cleaned with tap water, then scrubbed with 70% alcohol for one time, and cut apart by using a sterilized knife on a super clean bench,and seeds are taken out. The seeds are immersed in 70% alcohol for 50 s, disinfected with 15% (v/v) sodium hypochlorite solution for 20 min, washed with sterile water for 4-5 times, and dried with filter paper for standby use. The seeds of Carica papaya are cut apart with a sterile scalpel and tweezers, and complete immature embryos are inoculated onto an inducing culture medium (improved MS + 10 mg/L 2,4-D + 2 mg/L KT + 0.5 mg/L BA + 400 mg/L glutamine + 30 g/L sucrose, pH is 6.2). The immature embryos are subjected to callus induction in dark at 25□, and subcultured every 4 weeks. Mature somatic embryosare inoculated onto a somatic embryo germination culture medium (MS + 0.1 mg/L NAA + 0.5 mg/L BA + 7 g/L agar powder + 30 g/L sugar), and cultured at 25□ under a periodic illumination condition (10 h/d light, and 14 h/d dark). A frozen strain is subjected streaking culture on a plate containing 5 mg/L Bialaphos and 100 mg/L streptomycin. Individual colonies are picked and shaken in 30 ml of LB liquid culture medium at 225 rpm and 28□ for 20 h, and centrifuged for 10 min at 5000 rpm, and thalli are collected. The thalli are re-suspended by using 30 ml of improved MS liquid culture medium with 100 umol/L ethyl phthalein eugenone and continuously shaken for 3 h. Thereafter, the bacterial solution is diluted by using improved MS liquid culture medium to an appropriate concentration (OD600 is 0.6) for standby use. The immature embryos of Carica papaya that are pre-cultured for one month are immersed in the bacterial solution for 20 min and shaken for several times in the meanwhile. The bacterial solution on the surface of the callus is blotted up with sterile filter paper, and the calluses are dried moderately by blowing in the super clean bench, then are inoculated onto an inducing culture medium, and co-cultured for 3 d at 25□. The calluses and immature embryos after being co-cultured for 3 d are rinsed with sterile water for 6 times to remove the thalli attached on the surface as far as possible, inoculated into a selection culture medium containing 5 mg/L Bialaphos and 300 mg/L carbenicillin after surplus water is blotted up, and subcultured every 20 d. The browning and dead explants are removed, the concentration of the carbenicillin is gradually reduced. The calluses and embryos are cultured in dark at 25□ for inducing somatic embryogenesis. In the genetic transformation experiment, 19, 23 and 21 resistant plants of Carica papaya with transformed AaICE1, AaICE1(DC)1 and AaICE1(DC)2 genes are obtained respectively. The transgenic plants with the target traits are obtained by low-temperature screening. Compared with the wild-type plant of Carica papaya, the cold resistance of the plant of Carica papaya with the target traits is respectively improved by 1.9□, 3.8□ and 2.6□.

### Embodiment 12: Obtaining Eriobotrya japonica transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Eriobotrya japonica and are introduced into Eriobotrya japonica through the agrobacterium infection.

Agrobacterium tumefaciens are plated 2 d before the infection, and cultured in dark for 48 h at 28□ until a logarithmic phase of the agrobacterium for transformation. Agrobacterium tumefaciens colonies are scraped, suspended in a liquid culture medium, violently shaken for 1 min, and then kept still for 1-2 h so as to enable the agrobacterium to form turbid liquid. The concentration of agrobacterium is adjusted until the OD600 of the bacterial solution is 1.0. Eriobotrya japonica EC that is pre-cultured for 10-15 d is added, gently shaken, then kept still for 30 min, and placed on sterile filter paper to blot up water on the calluses, and transferred into a solid culture medium for co-culturing. After being co-cultured for 10 d, the calluses are picked and placed in a triangular conical flask, washed with sterile water for 3 times, rinsed with carbenicillin sterile water (300 mg/L, the same below) at the last time, and kept still for 1 h. Agrobacteria adhering onto the calluses are diffused into water. Thereafter, the calluses are rinsed with carbenicillin sterile water for one time, placed on sterile filter paper to blot up the water thereon, transferred to a screening culture medium, and cultured (the screening culture medium is MS culture medium with 1 mg/L 2,4-D, 0.25 mg/L KT, 10 mg/L hygromycin, 5 mg/L Bialaphos and 300 mg/L carbenicillin). When a regenerated plant grows to a height of 2-3 cm and has 4-7 leaves, seedling hardening is conducted in natural light by opening a flask cover, and the seedling is gently taken out, transplanted into a substrate containing peat soil and vermiculite in a ratio of 1:1 after being cleaned, thoroughly watered, and covered with a beaker having a notch to control the evaporation of water. After several days, the beakers are gradually removed to avoid high humidity from causing rotting of a transplanted seedling. In the genetic transformation experiment, 12, 13 and 11 resistant plants of Eriobotrya japonica with transformed AaICE1, AaICE1(DC)1 and AaICE1(DC)2 genes are obtained respectively. The transgenic plants with the target traits are obtained by low-temperature screening. Compared with the wild-type Eriobotrya japonica, the cold resistance of the plant of Eriobotrya japonica with target traits is respectively improved by 1.5□, 3.6□ and 2.2□.

### Embodiment 13: Obtaining Litchi chinensis transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Litchi chinensis and are introduced into Litchi chinensis through the agrobacterium infection.

The variety of Litchi chinensis is"Yuan Hong". The agrobacterium is stored on a solid LB culture medium and subcultured every 1-2 months. Prior to the transformation, individual colonies are picked, and subjected to shaking culture at 28□ in a liquid LB culture medium containing corresponding antibiotics until a logarithmic phase. The bacterial solution is centrifuged at 4000 rpm for 10 min, re-suspended by using a liquid callus growing culture medium, and diluted by 10 times for standby use. Embryonic calluses that are subcultured for 15 d are dried on sterile filter paper for 1 h, then placed in the prepared bacterial solution, kept still for 10 min, transferred to a co-culture medium (MS basic medium mixed with 2,4-D 2 mg/L, STS 5 mg/L, sucrose 50 g/L, agar 7 g/L and pH 5.8), and co-cultured for 2 d after the bacterial solution on the callus surface is blotted up with sterile filter paper. After being co-cultured, the calluses are rinsed with sterile water for 3 times, re-suspended in a liquid Z1 culture medium, laid on sterile filter paper, transferred into a Z1 culture medium containing microbiotics and antibiotics at certain concentration and subjected to the selection culture. After 7 weeks, resistant clones are transferred into similar selection culture medium for further screening. The purified resistant calluses are transferred into a somatic embryo differentiation culture medium (MS basic medium mixed with KT 1 mg/L, NAA 0.1 mg/L, glutamine 500 mg/L, sucrose 80 g/L, agar 15 g/L, G418 100 mg/L, 5mg/L Bialaphos, Carb 300 mg/L, pH 6.2), and the differentiation of somatic embryos is induced. Embryoid is cultured for 2 months in a mature culture medium (MS basic medium mixed with coconut milk 50 ml/L, casein hydrolysate 500 mg/L, sucrose 50 g/L, agar 9 g/L, pH 5.8), transferred into a germination culture medium (MS basic medium mixed with KT 1mg/L, GA5 mg/L, coconut milk 50 ml/L, glutamine 200 mg/L, sucrose 30 g/L, agar 7 g/L, G418 50 mg/L, 5 mg/L Bialaphos, pH 5.8), and germinated to form seedlings. In the genetic transformation experiment, 12, 13 and 11 resistant plants of Litchi chinensi with transformed AaICE1, AaICE1(DC)1 and AaICE1(DC)2 genes are obtained respectively. The transgenic plants with the target traits are obtained by low-temperature screening. Compared with the wild-type Litchi chinensi, the cold resistance of the plant of Litchi chinensi with target traits is respectively improved by 1.7□, 3.9□ and 2.7□.

### Embodiment 14: Obtaining Dimocarpus longan with transformed AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Dimocarpus longan and are introduced into Dimocarpus longan through the agrobacterium infection.

The Dimocarpus longan EC that is normally subcultured is inoculated onto a subculture medium LMs4, and pre-cultured for 10-15 d, and light-yellow small granular EC of Dimocarpus longan is selected as a transgenic receptor material. Agrobacterium tumefaciens are plated 1 d before the infection, and cultured in dark at 28□ for 24 h until a logarithmic phase of the agrobacterium for transformation. Agrobacterium tumefaciens colonies are scraped, suspended in a liquid culture medium, violently shaken for 1 min, and are kept still for 1 h so as to enable the agrobacterium to form turbid liquid. The concentration of agrobacterium is adjusted until the OD600 value of the bacterial solution is 1-1.5. EC of Dimocarpus longan that is pre-cultured for 10-15 d is added, and gently shaken, then kept still for 30 min, and is placed on sterile filter paper to blot up water on the calluses, transferred into a solid culture medium, and co-cultured. After being co-cultured for 5 d, when agrobacterium grows until bacterial plaque has visible calluses but is not full of calluses, the calluses are picked, placed in a sterile triangular flask, washed with sterile water for 5 times, rinsed with carbenicillin sterile water (300 mg/L) at the last time and kept still for 1 h to allow the agrobacterium adhering on the calluses to be sufficiently diffused into water. Thereafter, the calluses are rinsed with carbenicillin sterile water (300 mg/L) for one time, placed on sterile filter paper to blot up the water on the calluses, transferred to a screening culture medium, and cultured. In the genetic transformation experiment, 8, 10 and 7 resistant plants of Dimocarpus longan with transformed AaICE1, AaICE1(DC)1 and AaICE1(DC)2 genes are obtained respectively. The transgenic plants with the target traits are obtained by low-temperature screening. Compared with the wild-type plant of Dimocarpus longan, the cold resistance of the plant of Dimocarpus longan with the target traits is respectively improved by 1.4□, 3.5□ and 2.3□.

### Embodiment 15: Obtaining Mangifera indica transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Mangifera indica and are introduced into Mangifera indica through the agrobacterium infection.

Agrobacterium individual colonies are picked from a YEP plate by using a sterile toothpick, inoculated into a YEP liquid culture medium having 25 mg/L rifampicin, placed onto a constant-temperature table concentrator at 28□, and subjected to shaking culture at 220 rpm for 24 h, and thereafter, the bacterial solution is added into a novel resistant YEP culture medium, and cultured to a logarithmic phase under the same conditions. The bacterial solution is centrifuged at 5000 rpm for 5 min to precipitate thalli and remove supernatant, and an MS liquid culture medium is added for suspending the thalli for standby use. Fruitlets having a length of 3-5 cm on trees of Mangifera indica are collected, and the surface is scrubbed by using alcohol-immersed cotton balls to remove the surface bacteria. Then the fruitlets are rinsed with running water for several minutes, placed on a super clean bench, immersed in sodium hypochlorite solution and Tween 20 (3 droplets/L), and the mixture is stirred continuously in the meanwhile. Thereafter, the fruitlets are rinsed with sterile water for one time and 5 min at each time. The fruitlets subjected to the surface disinfection and sterilization are placed on sterile filter paper. Young embryos are uniformly cut apart by using a sterilized scalpel along a longitudinal axis of the fruitlets, taken out with pincette, and inoculated onto a culture medium (MS + 2,4-D 1 mg/L + 6 - BA 1.5 mg/L), and a longitudinal section is contacted with the culture medium. A standby bacterial solution is collected for infecting the young embryos for 10 min. After being co-cultured for 2 d, the young embryos are inoculated onto a callus inducing culture medium, and cultured for 10-20 d, and the uncontaminated calluses are transferred into a bud inducing culture medium. During the induction of all treated calluses, a culture medium for initiating the culture is mixed with several ascorbic acids, and changed every 2 d after the inoculation and changed for 3 times in total, so that the influence of a polyphenolic substance secreted by the young embryos on the callus induction can be reduced. The obtained regenerated plant is inoculated into a rooting culture medium, and subjected to the rooting culture. In the genetic transformation experiment, 12, 11 and 14 resistant plants of Mangifera indica with transformed AaICE1, AaICE1(DC)1 and AaICE1(DC)2 genes are obtained respectively. The transgenic plants with the target traits are obtained by low-temperature screening. Compared with the wild-type Mangifera indica, the cold resistance of the plant of Mangifera indica with the target traits is respectively improved by 1.9□, 3.9□ and 2.5□.

### Embodiment 16: Obtaining Solanum tuberosum transforming AaICE1 gene of Ageratina adenophora of different degrees of methylation

The expression vectors of the AaICE1, *AaICE1*(DC)1 and *AaICE1*(DC)2 genes of different degrees of methylation constructed in embodiment 1 are subjected to genetic transformation of Solanum tuberosum and are introduced into Solanum tuberosum through the agrobacterium infection.

Individual colonies are picked, inoculated into a 50 ml of YEB liquid culture medium containing 25 mg/L chloramphenicol and 5 mg/L Bialaphos, and subjected to shaking culture overnight at 140 rpm and 28□. Thereafter, 5ml of bacterial solution is collected, inoculated into 50 ml of YEB liquid culture medium, and subjected to shaking culture for standby use until OD600 of the bacterial solution is 0.5. Virus-free test-tube seedlings of Solanum tuberosum having 4 to 5 stem nodes are collected, cut into single-node stems of about 0.5 cm under a sterile condition, and placed into a liquid culture medium (MS + 3 mg/L 6-BA + 8% sucrose). The virus-free test-tube seedlings that are subcultured for 30 d are cut into axillary bud-free stem fragments about 0.5 cm and pre-cultured for 2 d. The stem fragments are infected with a standby bacterial solution for 10 min, co-cultured for 2 d, then inoculated onto a callus inducing culture medium (MS + 0.1 mg/L 2,4-D + 1 mg/L BA + 5 mg/L Bialaphos + 400 mg/L cefotaxime sodium), and cultured for 10-20 d. Thereafter uncontaminated calluses are transferred into a bud inducing culture medium (MS + 1 mg/L ZT + 1 mg/L GAS + 5 mg/L Bialaphos + 400 mg/L cefotaxime sodium). The obtained regenerated plant is inoculated into a rooting medium, and subjected to the rooting culture. In the genetic transformation experiment, 15, 18 and 19 resistant plants of Solanum tuberosum with transformed AaICE1, AaICE1(DC)1 and AaICE1(DC)2 genes are obtained respectively. The transgenic plants with target traits are obtained by low-temperature screening. Compared with the wild-type plant of Solanum tuberosum, the cold resistance of the plant of Solanum tuberosum with the target traits is respectively improved by 1.5□, 3.4□ and 2.7□.

### Embodiment 17: Obtaining Capsicum annuum transforming AaICE1 gene of Ageratina adenophora of different degrees of methylation

The expression vectors of the AaICE1, *AaICE1*(DC)1 and *AaICE1*(DC)2 genes with different degrees of methylation constructed in embodiment 1 are subjected to genetic transformation of Capsicum annuum and are infected by agrobacterium.

Agrobacterium individual colonies are picked from a YEP plate by using a sterile toothpick, inoculated into a YEP liquid culture medium containing 5 mg/L Bialaphos and 25 mg/L Rifampicin, placed onto a constant-temperature table concentrator at 28□, and subjected to shaking culture at 220 rpm for 24 h, and then the bacterial solution is transferred into a new resistant YEP culture medium, and cultured to a logarithmic phase under the same conditions. The bacterial solution is centrifuged at 5000 rpm for 5 min to precipitate thalli and remove supernatant, and an MS liquid culture medium is added for suspending the thalli for standby use. Plump seeds of Capsicum annuum are selected, washed with water for several times, sterilized with 70% ethanol for 30 s, rinsed with sterile water for one time, immersed in 0.1% HgCl₂ for 5-8 min, and rinsed with sterile water for 3-4 times. The seeds of Capsicum annuum are then inoculated onto 1/2 MS culture medium, and cultured in dark for 14 h/d at 25□ and under an illumination intensity of 2000 1x to form aseptic seedlings. Hypocotyl explants of 12-day-old aseptic seedlings of Capsicum annuum is cut, used as a transformation receptor, pre-cultured for 2 d on a PD pre-culture medium (MB5+3 mg/L 6-BA+1 mg/L IAA+4 mg/L AgN03+30 g/L sucrose +5 g/L agar), placed into liquid MS, collectively transferred into a prepared agrobacterium bacterial solution, and immersed for 5-14 min. The explants are drained on sterilized filter paper to remove the bacterial solution, placed onto the PD culture medium, and cultured in dark. Each dish holds ten explants with front side up. After the explants are cultured in dark for 1-4 d at 25□ on the PD, the explants are transferred onto a PD selection culture medium (containing 500mg/L carbenicillin), subjected to selective culture for 1-4 d, finally transferred onto the PD culture medium (containing 5 mg/L Bialaphos and 500 mg/L carbenicillin), and subjected to the selective culture. The explants are transferred and inoculated every five days. After being cultured for 15 days, the explants having differentiated adventitious buds are inoculated onto an SE culture medium (MB5+3 mg/L 6-BA+1 mg/L IAA+2 mg/L GA3+4 mg/L AgN03+30 g/L sucrose +5 g/L agar +5 mg/L Bialaphos+500 mg/L Carb) to promote the elongation of the buds. When the adventitious buds grow to 2-3 cm, the elongated buds are cut off, transferred onto SR (1/2MS+0.2 mg/L IAA+0.1 mg/L NAA+5 mg/L Bialaphos +200 mg/L Cef), and rooted. Except for the co-culture, other culture phases are carried out at 25□ and under illumination condition of 14h/d light, and light intensity of 2000 lx °C. In the genetic transformation experiment, 9, 11 and 8 resistant plants of Capsicum annuum with transformed AaICE1, AaICE1(DC)1 and AaICE1(DC)2 genes are obtained respectively. The transgenic plants with the target traits are obtained by low-temperature screening. Compared with the wild-type plant, the cold resistance of the plant of Capsicum annuum with the target traits is respectively improved by 2.1□, 4.5□ and 3.6□.

### Embodiment 18: Obtaining Sorghum bicolor transforming AaICE1 gene of Ageratina adenophora of different degrees of methylation

The expression vectors of the AaICE1, *AaICE1*(DC)1 and *AaICE1*(DC)2 genes with different degrees of methylation constructed in embodiment 1 are subjected to genetic transformation of Sorghum bicolor by means of agrobacterium infection.

An appropriate amount of stored bacterial solution is inoculated into 20 ml of YEP liquid culture medium, and subjected to shaking culture at 200 rpm and 28□ for 24-30 h. An appropriate amount of first-activated bacterial solution is inoculated into 40 ml of YEP liquid culture medium, and subjected to shaking culture at 200 rpm and 28□ for 24-30 h until OD600 of the bacterial solution is 0.6. The bacterial solution is centrifuged at 6000 rpm for 10 min, and then thalli are collected. Every 50 ml of sterile water is mixed with 500 ul of acetosyringone and 10 ul of sillwet L-77 to prepare a solution. The solution is used to suspend the thalli obtained by centrifugation until the OD600 of the bacterial solution is about 0.6 for standby use. Seeds of Sorghum bicolor are immersed in 70% alcohol for 1 min, washed with sterile water for one time, transferred into 0.1% mercury bichloride, shaken at 150 rpm on a table concentrator, and sterilized for 10 min. Finally the seeds are rinsed with sterile water for more than 6 times. The sterilized seeds are placed in a 100 ml conical flask having 100-200 seeds in each flask, added with an appropriate amount of sterile water, shaken at 150 rpm on the table concentrator, and germinated in about 24 h, and buds about 1 cm come up. The buds are taken out from the conical flask containing the seeds, and embryonically streaked. The scratched seeds are immersed in standby agrobacterium infection solution, and subjected to suction filtering for 30 min, which facilitates the invasion of the agrobacterium into cells ongrowth points. The seeds after being subjected to the infection transformation are laid in a culture disk with filter paper, placed in a culture room at 25□, and cultured in dark for 48-96 h. Ticarcillin (160 mg/L) and cefotaxime sodium (150 mg/L) are added into the sterile water, and the seeds are placed into a 100 ml conical flask, and washed for more than 4 times and at least 4 min at each time. Then the seeds are placed in the culture dish, cultured in a culture room (14-h light/10-h dark) at 25□. After the seeds are rooted, the seeds are transferred into Hoagland culture liquid, subjected to liquid culture and strengthening, and cultured until a 2-leaf to 4-leaf period. The seedlings are transplanted into paper cups, cultured in a greenhouse, and are subjected to the conventional growth management. In the genetic transformation experiment, 15, 13 and 17 resistant plants of Sorghum bicolor with transformed AaICE1, AaICE1(DC)1 and AaICE1(DC)2 genes are obtained respectively. The transgenic plants with the target traits are obtained by low-temperature screening. Compared with the wild-type plant, the cold resistance of the plant of Sorghum bicolor with the target traits is respectively improved by 1.8□, 3.9□ and 3.0□.

### Embodiment 19: Obtaining Vitis vinifera transforming AaICE1 gene of Ageratina adenophora of different degrees of methylation

The expression vectors of the AaICE1, *AaICE*(DC)1 and *AaICE1*(DC)2 genes of different degrees of methylation constructed in embodiment 1 are subjected to genetic transformation of Vitis vinifera and are introduced into Vitis vinifera through the agrobacterium infection.

PEM of Vitis vinifera subjected to the inducing propagation is subcultured onto a fresh X6 culture medium and pre-cultured in dark at 26□ for one week for transgenic manipulation. 200 uL of bacterial solution is inoculated into 20 ml of liquid LB culture medium (containing 5 mg/L Bialaphos). After being cultured at 180rpm and 28□ for 24 h, 20 uL of bacterial solution is inoculated into 20 ml of liquid LB culture medium (containing 5 mg/L Bialaphos), and subjected to the secondary activation culture for 20 h under the same conditions. The bacterial solution is transferred into 50 ml of sterilized centrifugal tube, and centrifuged for 8 min at 5000*g to remove the supernatant, the bacterial solution is re-suspended and centrifuged in a re-suspension buffer solution (MS medium + 20 g/L sucrose + 100 uM AS) for two times. The bacterial solution is incubated at 180 rpm and 28□ for 1 h in the re-suspension buffer solution °C, the concentration of the bacterial solution is diluted and adjusted by the re-suspension solution until OD₆₀₀ of the solution is 0.3-0.4. The PEM of Vitis vinifera is put into in a triangular flask containing agrobacterium solution, and infected for 20 min, the surface bacterial solution is blotted up by using sterile filter paper, and the PEM of Vitis vinifera is subcultured into a sterile culture dish which has two layers of sterilized filter paper and contains about 5-6 m of X6 liquid culture medium (mixed with 10 uM of AS). After being co-cultured in dark at 26□ for 48 h, the PEM is degermed for 20 min by using a cephalosporin solution at the concentration of 200 mg/L and a carbenicillin solution at the concentration of 200 mg/L, and rinsed with sterile water for 3 times.The PEM of Vitis vinifera is inoculated into a culture medium (MS + 5 mg/L Bialaphos + 200 mg/L Cef + 200 mg/L Carb + 10 mg/L Hyg + 30 g/L sucrose + 3 g/L Phytagel). The PEM-induced resistant SE is inoculated to a culture medium (MS + 0.2mg/L KT + 0.1mg/L NOA + 10mg/L Hyg + 30 g/L sucrose + 3g/L Phytagel). The resistant SE is cultured in dark at 26□, and subcultured every three weeks until resistant embryos come up. Through the genetic transformation of Vitis vinifera, 17, 19 and 14 plants with transformed AaICE1, AaICE1(DC)1 and AaICE1(DC)2 genes are obtained, and screened at a low temperature to obtain transgenic plants with target traits. The cold resistance of the obtained resistant plants is respectively improved by 1.4□, 3.0□ and 2.1□.

### Embodiment 20: Obtaining Malus domestica transforming AaICE1 gene of Ageratina adenophoraof different degrees of methylation

The expression vectors of the AaICE1, *AaICE1*(DC)1 and *AaICE1*(DC)2 genes of different degrees of methylation constructed in embodiment 1 are subjected to genetic transformation of Malus domestica by using an ultrasonic transformation method.

Leaves of test-tube seedlings of Malus domestica are cut into fragments of about 4 mm². The leaf fragments are placed into a container containing 20 ml of ultrasonic buffer solution containing: 13.7 mmol/L NaCI, 2.7 mmol/L KCI, 10 mmol/L Na₂HPO₄, 2 mmol/L KH₂POP₄, 5%DMSO, and pH is 7.4. Every milliliter of buffer solution contains 5 ug of plasmids DNA. The leaf fragments are ultrasonically treated for 20 min under a sound intensity of 0.5-1.0 W/cm². The ultrasonically-treated leaves are transferred onto an MS regeneration culture medium containing 4 mg/L 6-BA and 0.2 mg/L NAA, subjected to recovery growth for 1 week, then transferred into a regeneration culture medium containing 5 mg/L Bialaphos, and subjected to dark culture screening for 2 weeks, transferred every 2 weeks, and transferred onto an MS subculture medium containing 0.5 mg/L 6-BA and 0.1 mg/L NAA in 2 months, and this course lasts for 3 months in total. Culture conditions: illumination is continued for 16 h each day, the illumination intensity is 40 umol m⁻² s⁻¹, and subculture and storage are conducted at a constant temperature of 25□. The subculture is conducted every 4 weeks. 12, 13 and 10 resistant plants with transformed AaICE1, AaICE1(DC)1 and AaICE1(DC)2 genes are respectively obtained from 300 leaves of explants. The resistant plants are screened at a low temperature to obtain transgenic plants with target traits. Compared with the wild-type plants, the cold resistance of the obtained resistant plants is respectively improved by 1.8□, 4.1□ and 2.5□.

### Embodiment 21: Obtaining Chorispora bungeana transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Chorispora bungeana and are introduced into Chorispora bungeana through the agrobacterium infection.

Seeds of Chorispora bungeana are disinfected (70% alcohol and 1% sodium hypochlorite), and then sowed in an MS culture medium. After one week, hypocotyl is cut, placed on a differentiation culture medium (MS + 2 mg/L 6-BA+ 0.2 mg/L NAA+ 3% sucrose + 0.6% agar), and pre-cultured for 2 d. The stored agrobacterium EHA105 is activated. 500 ul of activated bacterial solution is collected, inoculated into 50 ml of YEB liquid culture medium, subjected to shaking culture at 220 rpm and 28□ until OD600 of the bacterial solution is 0.5, centrifuged at 4□ (4000 r/min, 10 min) to remove supernatant, and re-suspended in 20 ml of pre-cooled MS liquid culture medium for standby use. The pre-cultured hypocotyl is infected by resuspension bacterial solution for 10 min, dried on filter paper, then transferred onto a differentiation culture medium, and co-cultured for 2 d. The co-cultured hypocotyl is transferred onto a screening culture medium (MS + 2 mg/L 6-BA + 0.2 mg/L NAA + 5 mg/L Bialaphos + 400 mg/L Cb + 3% sucrose + 0.6% agar), and subjected to the resistant screening, and the screening culture medium is changed every 10 d. Resistant buds obtained by multi-time screening are transferred onto a rooting culture medium (MS + 2 mg/L 6-BA +0.4 mg/L NAA + 5 mg/L Bialaphos + 400 mg/L Cb + 3% sucrose + 0.6% agar), and subjected to the rooting culture. After tissue cultured seedlings have strong root systems, the root systems are cleaned to remove residual culture medium, transplanted into nutritional soil, and cultured in aphytotron. In the genetic transformation experiment, 18, 15 and 21 resistant plants with transformed AaICE1, AaICE1(DC)1 and AaICE1(DC)2 genes are obtained. The transgenic plant with target traits is obtained by low-temperature screening. Compared with the wild-type plants, the cold resistance of the obtained resistant plants is respectively improved by 1.9□, 3.4□ and 2.4□.

### Embodiment 22: Obtaining Solanum capsicoides transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Solanum capsicoides and are introduced into Solanum capsicoides through the agrobacterium infection.

Seeds of Solanum capsicoides are immersed in 75% alcohol for 30 s, rinsed with sterile water for one time, immersed with 10% NaC10 solution for 20 min, rinsed with sterile water for 3 times, inoculated onto 1/2 MS culture medium, germinated, and cultured into aseptic seedlings in light/dark (16 h/18 h) at 26□, used as a tested material. An overexpression vector is constructed by Agrobacterium strain EHA105, plasmid vector pBI121 and the ScICE1 gene. Cotyledons of aseptic seedlings are selected with leaf tips removed, cut into pieces in a uniform size of 0.5 mm², inoculated into a pre-culture medium MS + NAA 0.2 mg/L + ZT 1.2 mg/L, pre-cultured for 2 d, and subsequently subjected to agrobacterium infection transformation. The agrobacterium bacterial solution with an OD value of 0.5 is collected. The infection time is 10 min. The infected explant is inoculated into a co-culture medium (1/2MS + NAA 0.2 mg/L + ZT 1.2 mg/L + 200 umol/L acetosyringone), cultured in dark for 2 d, and then cleaned with sterile water containing 150 mg/L Timentin, surface water is blotted up with sterile filter paper, then the explant is inoculated into a culture medium (MS + NAA 0.2 mg/L + ZT 1.2 mg/L + 150mg/L Timentin), subjected to recovery culture, and then placed into an induced callus culture medium (adding 0.1 mg/L NAA,, 3.0 mg/L ZT). The explants are cultured for 16 h at daytime and 8 h at night under an illumination intensity of 1600 1x. When the explants grow for about two weeks, small, tender, round and hard green calluses come up, and the explants are inoculated into a germination culture medium (mixed with 0.1 mg/L NAA, 4.0 mg/L ZT, 1.5 mg/L 6-BA, 12.0 mg/L AgN0₃) for inducing the germination. Seedlings formed by elongating and differentiating buds send forth 2-3 pieces of euphylla, are cut off from bases, and inoculated into 1/2 MS rooting culture medium for inducing the rooting. The illumination time is 16 h/d, the temperature is 24-27□, and the illumination intensity is 1600 1x. The regenerated seedling growing in the culture medium is poor in environmental adaptability. A small hole of the triangular flask shall be opened for ventilation. The ventilation volume shall be gradually increased according to the growth condition of the seedling so as to improve the environmental adaptability of the seedlings. After two weeks, when the seedling has 4-5 pieces of euphylla, three main roots and a plurality of lateral roots, a flask mouth is opened. At the same time, sufficient water of the seedling shall be guaranteed. The seedling can be transplanted after being continuously cultured for 5 d. In the genetic transformation experiment, 8, 10 and 7 resistant plants with transformed AaICE1, AaICE1(DC)1 and AaICE1(DC)2 genes are obtained. The transgenic plants with target traits are obtained by screening at low temperature. Compared with the wild-type plant, the cold resistance of the obtained resistant plants is respectively improved by 1.5□, 3.0□ and 2.2□.

### Embodiment 23: Obtaining Actinidia chinensis transforming Ageratina adenophora AaICE1 gene ofdifferent degrees of methylation

The expression vectors of the AaICE1, AaICE1(DC)1 and AaICE1(DC)2 genes of different degrees of methylation constructed in embodiment 1 are subjected to genetic transformation of Actinidia chinensis and are introduced into the Actinidia chinensis through the agrobacterium LBA4404.
The stored agrobacterium LBA4404 strain is collected and streaked, and thereafter, individual colonies of the agrobacterium are picked. The picked individual colonies are placed into 20 ml of liquid YEP culture medium, subjected to shaking culture at 200 rpm and 28□ for 16-24 h. 0.5 ml of bacterial solution is sucked into 50 ml of YEP culture liquid containing similar antibiotics, and subjected to the amplification culture under the same conditions. When the bacterial solution is cultured until OD600 of the bacterial solution is 0.5, the bacterial solution is centrifuged at 4000 rpm and 4□ for 10 min, precipitated, and suspended in an equal volume of liquid MS culture medium containing 100 uM of acetosyringone for standby use. Small and tender leaves of aseptic seedlings of Actinidia chinensis are cut into pieces of 0.5 cm² under a sterile condition, and inoculated onto a culture medium containing MS basic culture medium having 3.0 mg/L 6-BA and 1.0 mg/L NAA, pre-cultured for 3 d. After 3 d, the pre-cultured leaves are placed in the re-suspended bacterial solution, slightly shaken in the meanwhile to allow the agrobacterium to sufficiently contact the leaves, and infected for 10 min, then surplus bacterial solution is blotted up by using sterile filter paper, the leaves are placed with back side down on a regeneration culture medium containing 100 uM AS, and cultured in dark at 28□ for 2 d. A layer of sterile filter paper shall be put on the co-culture medium with to prevent the excessive growth of the agrobacterium. Co-cultured leaf explants are rinsed with sterile water for 4-5 times, the residual sterile water is removed by using sterile filter paper, the explants are inoculated onto a regeneration culture medium containing 500 mg/L Carb and 5 mg/L Bialaphos, subcultured once a month, and cut off after differentiated buds grow to 2-3 cm, and the differentiated buds are inserted into a rooting culture medium. Adventitious roots can be seen in 5-10 d. After the rooted seedlings grow to 5 cm, culture flasks are placed in a natural condition for culture for 3-5 d. The covers are gradually opened in a sequence of loosening the covers on the first two days, half opening the covers on the third and fourth days, removing the covers on the fifth and sixth days, covering the flasks with hard paperboard in a size half of the covers, and removing the hard paperboards on the seventh and eighth days, so that the tissue cultured seedlings are exposed in nature and exercised for 10 d. Thereafter, new seedlings are carefully taken out by using tweezers, cleaned with tap water to remove agar on roots, transplanted in the greenhouse soil, and cultured. Through the genetic transformation, a total of 8, 11 and 12 plants of Actinidia chinensis with transformed *AaICE1, AaICE1(DC)1* and *AaICE1(DC)2* genes are obtained. After the low-temperature screening, the cold resistance of the target plant is respectively improved by 2.0□, 3.6□ and 2.7□.

### Embodiment 24: Obtaining Prunus persica transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Prunus persica and are introduced into Prunus persica through the infection of agrobacterium GV3101.

Fruits of Prunus persica in normal shape and uniform size and without damage or worms are sequentially scrubbed with detergent water, washed with tap water, immersed in 0.2% mercury bichloride for 10 min, taken out, rinsed with sterile water for 5 times, and peeled under a sterile condition. Kernels are cut apart and young embryos are taken out. The young embryos are inoculated onto a callus inducing culture medium (MS + 2,4-D 0.5 mg/L + BA 0.75 mg/L + NAA 1.0 mg/L + 2% sucrose + 7.5g/L agar with pH 5.8), for inducing callusesat 26□ in dark. Individual colonies of agrobacterium tumefaciens GV3101 containing the plasmids are picked, and inoculated into 4 ml of YEP liquid culture medium containing 5 mg/L Bialaphos, Rif 20 mg/L and Gem 40 mg/L, cultured in dark at 180 rpm and 28□ for 36 h. 1 ml of the above bacterial solution is mixed with 50 ml of fresh YEP liquid culture medium and 200 umol/L acetosyringone, and cultured for 12 h under same conditions. Thereafter, 40 ml of the bacterial solution is collected and centrifuged at 5000 rpm and 4□ for 10 min. The precipitates are added into liquid MS, and the mixture is continuously subjected to shaking culture in dark at 180 rpm and 28□ for 2 h until a logarithmic phase for standby use. The pre-cultured explants are directly immersed in an agrobacterium suspension and taken out, surplus bacterial solution is removed by using sterile filter paper, the pre-cultured explants are placed into the culture medium (MS + 2,4-D 0.5 mg/L + BA 0.75 mg/L + NAA 1. 0 mg/L + 2% sucrose + 7.5g/L agar, pH5.8), and co-cultured in dark at 26□. The induced calluses are inoculated into a differentiation culture medium (MS + NAA 0.05 mg/L + BA 1.0 mg/L), cultured under a 16-h light/8-h dark cycle and illumination intensity of 2000-3000 lx at the same temperature above. Thereafter, adventitious buds are transferred onto a rooting culture medium (1/2 MS+IBA 1.0 mg/L), subjected to rooting culture, and take roots after 5 d. In the transgenic experiment of Prunus persica, 7, 9 and 9 resistant plants with transformed AaICE1, AaICE1(DC)1 and AaICE1(DC)2 genes are respectively obtained. The transgenic plants with target traits are obtained by screening at low temperature. Compared with the wild-type plant of Prunus persica, the cold resistance of the obtained resistant plants is respectively improved by 1.8□, 3.7□ and 2.0□.

### Embodiment 25: Obtaining Zea mays transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Zea mays and are introduced into Zea mays through the infection of agrobacterium GV3101.

Under a greenhouse condition, a receptor material is seeded at different stages by taking 7 d as a stage. A tested material of Zea mays is subjected to controlled pollination according to the test requirements. Young embryos of Zea mays are collected 10-13 d after the pollination, and used as a transformation receptor. After cobs are sterilized with sodium hypochlorite, young embryos are taken out, washed with an infection solution (mixed with AS) for 4 times, added into agrobacterium bacterial solution at a given concentration, kept still for 15 min, taken out, dried by using sterilized filter paper, placed on a co-culture medium, co-cultured in dark at 25□ for 2-5 d, transferred onto a tranquillization culture medium, and cultured in dark at 28□ for 7 d. In the infection and co-culture steps, the concentration OD500 of the bacterial solution is 0.3-0.5. The infection time is 10 min. The co-culture time is 3 d. The young embryos are transferred from the tranquillization culture medium into a resistant screening culture medium containing a marker gene, and cultured in dark. The browning calluses and water-soaking calluses shall be discarded during the subculture at each time. The calluses growing normally are broken with tweezers and separately selectively cultured. Meanwhile, frequent observation is required, the contaminated material or material with re-appearance of agrobacterium during the subculture screening shall be moved out immediately, while the uncontaminated material is continuously cultured. Furthermore, the tissue blocks having browning contamination are frequently picked out or the good or uncontaminated tissue blocks are transferred onto a similar new culture medium. After the tissue blocks are swelled, the large tissue blocks shall be broken into small pieces frequently. After the subculture screening is continuously carried out for 3 times, the selected resistant calluses are transferred onto a regeneration culture medium, subjected to recovery culture for 15 d or longer (dark culture). The resistant calluses are transferred onto a regeneration culture medium, germinated and cultured under a condition of 12 h light and an illumination intensity of 3000 lx at 28□. When the regenerated plant of Zea mays grows, and has three leaves, the seedlings can be transplanted into a flask containing a rooting culture medium, and cultured indoor. When the seedlings send forth strong roots, the seedlings are taken out from a can flask, rinsed with water to remove the culture medium, and transplanted into a small flowerpot containing a mixture of nutrition soil and vermiculite (1:3). When the seedling of Zea mays sends forth 2-3 new leaves, the seedlings can be transferred into a farmland or a large flowerpot. After three to four leaves emerge, leaf DNA can be extracted for PCR detection. The containing of the introduced *ZmICE1* gene is determined. In the experiment, 10, 13 and 12 resistant plants with transformed AaICE1, AaICE1(DC)1 and AaICE1(DC)2 genes are respectively obtained. The transgenic plants with target traits are obtained by screening at a low temperature of 6□. Compared with the wild-type plants, the cold resistance of the obtained resistant plants is respectively improved by 13□, 3.0□ and 1.7□.

### Embodiment 26: Obtaining Gossypium hirsutum transforming Ageratina adenophora AaICE1 gene ofdifferent degrees of methylation

The expression vectors of the AaICE1, AaICE1(DC)1 and AaICE1(DC)2 genes of different degrees of methylation constructed in embodiment 1 are subjected to genetic transformation of Gossypium hirsutum.

The constructed plant expression vector agrobacterium bacterial solution is collected for extracting a great amount of plant expression vector plasmids DNA. Flower buds that are blossomed in the next day are selected for selfing. Since Gossypium hirsutum is an often cross-pollinated plant, and the natural cross-pollination rate is about 10%, the foreign pollen often leads to the hybridization of different varieties. On the day before the blossoming, corolla may be elongated rapidly. Yellow or white corolla is protruded out of the flower bud like a finger and will be blossomed into flowers on the next day. The flower buds are selected and tied with a thread on the front end of the finger-shaped corolla, and the other end of the thread is tied to a boll handle to serve as marker when in harvesting. In about 20-24 h after the blossoming, i.e. the next day after the blossoming, young ovaries with good fruit branches and flower position are selected as a transformation object. Generally the flowers on a first and second fruit nodes of each fruit branch are selected for the transgenic manipulation. The cotton bolls on these fruit nodes generally have high boll formation rate, so that more seeds can be harvested. 50 mL of microsyringe is used as a micro injection tool. The micro injection tool shall be cleaned with light detergent before and after the use at each time and rinsed with distilled water. During injection, petals are removed or stripped, and styles are smoothened. When the petals are removed, an epidermal layer of the young ovary shall not be damaged to avoid the increase of falling rate. The micro injector is held by a right hand, the young ovary with the petals removed is gently supported by a left hand, and a needle is fed from a style smoothened position to about two thirds of the length of the ovary along the longitudinal axis direction of the ovary and withdrawn to about one third of the length of the ovary. The seeds of Gossypium hirsutum obtained by transformation of a pollen tube pathway method are harvested. Thereafter, seedlings are planted in pots in a greenhouse. At the stage of 2-3 leaves, the leaves of Gossypium hirsutum are sprayed with 5 mg/L Bialaphos. The growth condition of the plant is observed after one week, and the plant with chlorotic spots on leaf surfaces is removed. The plants with normal growth are considered as the plants acquiring the resistant genes. In the experiment, 15, 13 and 17 resistant plants with transformed AaICE1, AaICE1(DC)1 and AaICE1(DC)2 genes are respectively obtained. The transgenic plants with target traits are obtained by screening at a low temperature of 4□. Compared with the wild-type plant of Gossypium hirsutum, the cold resistance of the obtained resistant plants isrespectively improved by 1.7□, 4.2□ and 2.9□.

### Embodiment 27: Obtaining Arachis hypogaea transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Arachis hypogaea.

When plants of Arachis hypogaea enter a full-blossom stage, the transgenic manipulation is carried out. Prior to the full-blossom stage, flower buds are picked off at about 5:00 in the afternoon everyday to prevent the pollination and fruitage of the flowers not subjected to the transgenic manipulation from interfering the detection of the transgenic plants in offspring in the next season. A micropipettor and a glass needle formed by drawing a dropper are respectively used to introduce plasmids DNA containing target genes by a stigma smearing method and a flower tube injection method. Two DNA concentrations, i.e. 50 ug/mL and 100 ug/mL, are selected for treating two varieties, i.e. "Shanyou523" and "Shanyou21". After the plant of Arachis hypogaea enters the full-blossom stage, the manipulation begins about 6:30 in the morning everyday. For the smearing manipulation, the micropipettor is used to suck 5 uL of DNA solution, and the DNA solution is smeared onto a flower stigma. For the injection method, the glass needle is used to inject about 5 uL of DNA solution on the lower portion of calyx tube in a direction towards the ovaries. More than 500 flowers are treated for each variety. The seeds of Arachis hypogaea obtained by genetic transformation are harvested, and thereafter, seedlings are planted in a greenhouse pot. At the stage of 2-3 leaves, the leaves of Arachis hypogaea are sprayed with 5 mg/L Bialaphos. The growth condition of the plant is observed after one week, and the plant with chlorotic spots on leaf surfaces is removed. The plants with normal growth are considered as the plants acquiring the resistant genes, and the resistant plants are used for PCR verification. Through the genetic transformation, 8, 14 and 15 resistant plants with transformed AaICE1, AaICE1(DC)1 and AaICE1(DC)2 genes are obtained respectively. The transgenic plants with target traits are obtained by screening at a low temperature. Compared with the wild-type plants of Arachis hypogaea, the cold resistance of the obtained resistant plants is respectively improved by 1.3□, 2.8□ and 1.9□.

### Embodiment 28: Obtaining Brassica juncea transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Brassica juncea and are introduced into brassica juncea through the infection of agrobacterium GV3101.

Seeds of brassica juncea are disinfected (75% alcohol and 0.1% sodium hypochlorite), and then sowed in an MS culture medium. After 7 d, hypocotyl (0.5-0.8 cm) is cut, placed on a brassica juncea differentiation culture medium (MS + 2 mg/L 6-BA+ 0.2 mg/L NAA+ 3% sucrose + 0.6% agar), and pre-cultured for 2 d. The agrobacterium introduced with plasmids containing *BjICE1* genes is activated. During infection, 500 ul of activated bacterial solution is collected, inoculated into 50 ml of antibiotics-free YEB liquid culture medium, subjected to shaking culture at 225 rpm and 28□ until the growth vigorous period of agrobacterium (OD600 is about 0.5-0.6) on a table concentrator, centrifuged at 4□ (4000 r/min, 10 min) to remove supernatant, re-suspended in 20 ml of pre-cooled MS liquid medium for standby use. The pre-cultured hypocotyl is infected by resuspension bacterial solution for 10 min, dried on filter paper, then transferred onto a differentiation culture medium, and co-cultured for 2 d. The co-cultured hypocotyl is transferred onto a Brassica juncea screening culture medium (MS + 2 mg/L 6-BA + 0.2 mg/L NAA + 5 mg/L Bialaphos + 400 mg/L Cb + 3% sucrose + 0.6% agar), and subjected to resistant screening, and the screening medium is changed every 10 d. The resistant buds obtained by multi-time screening are transferred onto a Brassica juncea rooting culture medium (MS + 2 mg/L 6-BA + 0.4 mg/L NAA + 5 mg/L Bialaphos + 400 mg/L Cb + 3% sucrose + 0.6% agar), and subjected to the rooting culture. After tissue cultured seedlings have strong root systems, the root systems are cleaned, after residual culture medium is removed, transplanted into nutritional soil, and placed in a phytotron (temperature: 25+/-2□; illumination intensity: 2000 lx; relative humidity: 70%; light/dark=16/8 h), and cultured. Through the genetic transformation, 24 and 21 resistant plants with transformed AaICE1, AaICE1(DC)1 and AaICE1(DC)2 genes are obtained respectively. The transgenic plants with target traits are obtained at a low temperature. Compared with the wild-type plant of Brassica juncea, the cold resistance of the obtained resistant plants is respectively improved by 1.8□, 4.0□ and 2.9□. Embodiment 29: Obtaining Brassica rapa transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Brassica rapa, and carry out the transgenic experiment for Brassica rapa by means of a pollen mediation method.

After plants of Brassica rapa enter a full-blossom stage, 10-15 flower buds that will be blossomed in 1-2 d on a main stem or a first branch are selected and emasculated by hands. Other flower buds on the selected stem or branch are picked off. Then the selected flower buds are bagged. The flower buds that will be blossomed on the next day of the same variety are selected and bagged for pollen collection on the next day. About 0.4 g of pollen collected from the bagged plants that are blossomed in the next morning is suspended in 25 ml of 7.5% sucrose solution, and ultrasonically treated for the first time, and 20 ug of plasmid DNA containing different cold-resistant population *BrICE1* genes is added into the solution for second ultrasonic treatment. After the second treatment, 10 ul of 1/10000 (W/V) boric acid is added into the solution. The treated pollen is carried onto a Brassica rapa stigma that is emasculated the day before, and then the pollinated plants of Brassica rapa are bagged and hung with plates marked with the number of pollinated flower buds. The bags are removed 5-6 d after the pollination, so that the treated pollinated plants can develop sufficiently. Seeds are harvested. After the harvested seeds are planted, when two cotyledons develop, the cotyledons are sprayed with 5 mg/L Bialaphos solution. After being cultured in light for 7 d, the leaf edges of the non-transgenic plants turn yellow, while the transgenic plants grow normally. Through the genetic transformation, 33 and 37 resistant plants with transformed AaICE1, AaICE1(DC)1 and AaICE1(DC)2 genes are obtained respectively. After the low-temperature screening, compared with the wild-type plants of Brassica rapa, the cold resistance of the obtained resistant plants is respectively improved by 1.5□, 4.2□ and 2.6□ under a treatment condition of -10□.

### Embodiment 30: Obtaining Chrysanthemum dichrum transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Chrysanthemum dichrum and are introduced into Chrysanthemum dichrum through the agrobacterium infection.

The stored agrobacterium with pCAMBIA1301 plasmids of the target gene is collected, inoculated into a YEB liquid culture medium containing 50 mg/L Rifampicin, and cultured overnight. Then the agrobacterium bacterial solution is streaked, and respectively inoculated onto YEB plate culture media containing 5 mg/L Bialaphos and 50 mg/L rifampicin, the plate is inversely placed in a constant-temperature incubator at 28□, culture is carried out until individual colonies come up, and the plate in a refrigerator at 4□ for standby use. The agrobacterium individual colonies are respectively picked from the YEB plate culture media, inoculated into 20 ml of YEB liquid culture medium containing 5 mg/L Bialaphos and 50 mg/L rifampicin, and subjected to shaking culture at 180 rpm and 28□ until a logarithmic phase. After two-times activation, the bacterial solution is inoculated into the YEB liquid culture medium containing 100 pmol/L RS in a ratio of 1:100. After the bacterial solution is subjected to shaking culture for 4-6 h when the OD600 is about 0.4-0.6, the bacterial solution is used for genetic transformation. Leaves on the upper end of the cultured aseptic chrysanthemum plant are collected as explants, and cut into pieces of about 8mm^{∗}8mm by using sterilized scissors, the pieces are placed into the embryo callus inducing culture medium (Ms + 0.5 mg/L 6-BA + 1.5 mg/L NAA + sucrose 30 g/L + agar powder 5 g/L) for pre-culturing. After the pieces are pre-cultured for 3 d, the bacterial solution for infection is poured into a sterile triangular flask. The pre-cultured leaves are rinsed with sterile water for 1-2 times, dried with sterile filter paper, and placed into the triangular flask, and directly infected for 25 min. The inflected leaves are taken out, and the surplus bacterial solution is blotted up by using sterile filter paper. The infected leaves are inoculated onto the co-culture medium, andco-cultured at 25+/-2□ for 2 days. When colonies come up on the peripheries of the leaves, the leaves are taken out, rinsed with sterile water for 2-3 times and dried by using sterile filter paper. The leaves are transferred into an embryonic callus-inducing screening culture medium (300 mg/L Cef), and cultured until the resistant somatic embryos are completely differentiated. After the leaves are cultured on the screening differentiation medium for 15-20 d, a minority of chrysanthemum leaves begin to be differentiated at the incisions. When the regenerated plants in the culture medium grow to about 2 cm, the regenerated plants are stripped from a chrysanthemum parental body by using sterile scissors, transferred into an MS basic culture medium with no plant growth hormones for rooting, and cultured at 25□ under a light cycle of 16 h/8 h and under illumination intensity of 1200-2400 lx. Roots begin to be germinated from a root base on the sixth day. Through the genetic transformation, 9, 11 and 10 resistant plants with transformed AaICE1, AaICE1(DC)1 and AaICE1(DC)2 genes are obtained respectively. The plants with target traits are obtained by screening at a low temperature. Compared with the wild-type plants of Chrysanthemum dichrum, the cold resistance of the obtained resistant plants isrespectively improved by 2.2□, 4.4□ and 2.9□.

### Embodiment 31: Obtaining Daucus carota transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Daucus carota and are introduced into Daucus carota through the agrobacterium infection.

Individual colonies of agrobacterium tumefaciens engineering bacteria are picked and inoculated into 50 ml of LB liquid culture medium containing 5 mg/L Bialaphos, subjected to shaking culture in dark at 200 rpm and 28□ until an OD600 value of the bacterial solution is about 0.6. The agrobacterium tumefaciens culture solution is transferred into a 50 ml sterile centrifugal tube on a super clean bench, and centrifuged at 8000 rpm and 4□ for 5 min to remove supernatant, and thalli are suspended by using an equal volume of sterile MS liquid culture medium for standby use. The culture dish sterilized at high pressure is laid with 2 to 3 pieces of sterile filter paper wet by adding an appropriate amount of B5 liquid medium. Aseptic seedlings are placed on the filter paper. Hypocotyl is cut into segments in a length of 0.5 cm by using an aseptic and sharp scalpel for genetic transformation. The treated hypocotyl of aseptic seedlings of Daucus carota is placed into a prepared infection solution on a super clean bench for 15-20 min, and gently shaken in the meanwhile, allowing each hypocotyl segment can be sufficiently infected. The explants are taken out, and the surplus bacterial solution on the explants is blotted up by using sterile filter paper. The explants are placed on a co-culture medium, and cultured in dark at 26□ for 2-3 d. After being co-cultured, the explants are taken out, placed into an empty sterile triangular flask, rinsed with sterile water for 2-3 times, rinsed with sterile water containing 500 mg/L Carb for 1-2 times, dried by using sterile filter paper, transferred, inoculated onto a B5 bacteriostatic culture medium at a given concentration of antibiotics, subjected to callus culture at 26□ in light for 16 h each day, subcultured for 2-3 times, and transferred onto a differentiation culture medium after the calluses come up, and embryoids and buds are induced. After the induced buds grow for about 40 d, and when the seedlings grow to 2-3 cm, the seedlings are cut off, transferred onto a rooting culture medium for inducing rooting. When normal roots and 3-4 pieces of euphylla come up, the resistant seedlings are moved out, rinsed with tap water for removing culture medium on the roots, planted in a nutritional pot with vermiculite and peat (2:1), and covered with a plastic film under conditions of 26□ in light for 16 h each day, a vent port of plastic cloth is opened after 7 d for appropriate seedling hardening, conventional management is carried out in a greenhouse when the resistant seedlings are gradually adaptive to the external growth environment. In the transgenic experiment, 9, 12 and 10 resistant plants with transformed AaICE1, AaICE1(DC)1 and AaICE1(DC)2 genes are respectively obtained. After the low-temperature screening, compared with the wild-type plant of Daucus carota, the cold resistance of the obtained resistant plants is respectivelyimproved by 1.5□, 3.0□ and 2.0□.

### Embodiment 32: Obtaining Glycine max transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Glycine max and are introduced into Glycine max through the agrobacterium infection.

Sterilized seeds of Glycine max are poured into a sterile plastic jar added with a liquid germination medium (LGM) (sucrose 20 g/L, pH 5.8). The volume of the medium is about twice the seed volume so as to completely cover the seeds and provide sufficient water. After being covered with a lid, the sterile plastic jar is placed in an incubator for 16 h and dark culture is carried out at 23□ all day. 40 ml of agrobacterium resuspension solution is poured into a sterile culture dish. By examining LGM after the Glycine max is sowed, the contaminated LGM is discarded. The seeds of Glycine max are first obliquely cut along a seed embryo direction by using a scalpel, then cut apart along the axis, and peeled. The seeds are gently cut for three times at an embryo tip position parallel to the axis and immersed in a culture dish containing the re-suspended agrobacterium bacterial solution for 30 min. After the infection is finished, the explants are transferred into an iron box laid with sterile filter paper by using sterilized tweezers and placed horizontally with incisions up. LCCM (Bs salt powder 0.321 g/L, sucrose 30 g/L, MES 3.9 g/L, pH 5.4, BAP 1.67 mg/L, GA3 0.25 mg/L, Cys 400 mg/L, DTT 154.2 mg/L, and As 39.24 mg/L) is gently added into the iron box. The iron box after being closed is placed in an intelligent incubator for 3 days and dark culture is carried out at 23□ all day. After being co-cultured for 3 days, the explants are taken out by using the sterilized tweezers on a super clean bench, and cotyledons are cut off by using the scalpel, while hypocotyl is reserved. The cut explants are placed into a shoot inducing medium (SIM) (BS salt powder 3.21 g/L, sucrose 30 g/L, MES 0.59 g/L, Noble Agar 8 g/L, BAP 1.67 mg/L, Tic 250 mg/L, Cef 100 mg/L, PH 5.7). Each dish holds 7 explants. The top ends of the explants must be higher than a plane of the medium. The hypocotyl part must be inserted into the medium. Two disposable plastic culture dishes are overlapped on bottom and sealed with a micro ventilation-type medical adhesive tape. The novel culture dish is placed into a phytotron, subjected to recovery culture at 25□ all day long for 14 days °C in light/dark ratio of 16/8 h each day. The explants with shoots are transferred into SIM in a shoot elongation medium (SEM) (MS salt powder 4.43 g/L, sucrose 30 g/L, MES 0.59 g/L, Noble Agar 8 g/L, pH 5.7, Asp 50 mg/L, Glu 50 mg/L, IAA 0.1 mg/L, GA3 0.5 mg/L, ZR 1 mg/L, Tic 250 mg/L). The explants with no induced adventitious buds are discarded. The explants are still cultured in the phytoron under culture conditions of 25□ all day and cultured in a light/dark ratio of16/8 h each day. Fresh SEM is generally changed every two weeks. When the culture medium is changed at each time, the dead explant is discarded. The subculture of the present batch can be ended when SEM is carried out for 5 times. In the elongation process of the shoots, the shoots are cut off from the explants, placed into a culture dish, the bottom of the shoots is immersed with IBA (1 mg/ml) for 30 s, then the shoots are transferred into a rooting medium (RM) (MS salt powder 4.43 g/L, sucrose 20 g/L, MES 0.59 g/L, Phytagel 3 g/L, pH 5.6, Asp 50 mg/L, L-Glu 50 mg/L, Tic 125 mg/L, Cef 50 mg/L, IBA 1 mg/L), and continuously cultured. After 1-2 weeks, when a given number of roots come up from the bottom of the seedling, the seed lings are taken out from the RM. After the roots are washed with tap water, the seedlings are transplanted into soil. After the seedlings are transplanted in the soil, the seedlings need to first grow for about one week in the phytotron to adapt to the natural environment, then transferred into a greenhouse, and cultured. In the transgenic experiment, 6, 9 and 9 resistant plants with transformed AaICE1,AaICE1(DC)1 and AaICE1(DC)2 genes are respectively obtained. Compared with the wild-type plants of Glycine max, the cold resistance of the obtained resistant plants is respectively improved by 2.3□, 4.0□ and 2.8□.

### Embodiment 33: Obtaining Raphanus sativus transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Raphanus sativus and are introduced into Raphanus sativus through the agrobacterium infection.

The cotyledons-with-petiole explants of a four-day-old aseptic seedling of a Raphanus sativus material are cut, first pre-cultured for 2 days, infected with EHA105 agrobacterium bacterial solution the OD600 of which is 0.3-0.6 for 5-7 min, then co-cultured for 5 days, transferred, inoculated onto a culture medium (MS + 6-BA 6 mg·L⁻¹ + NAA 0.05 mg·L⁻¹+ Cef 500 mg·L⁻¹), subjected to the delayed screening for 7 d, then transferred, inoculated onto a culture medium (MS + 6-BA 6 mg·L⁻¹ + NAA 0.05 mg·L⁻¹+ 5 mg·L⁻¹Bialaphos Cef 500 mg·L⁻¹), and subjected to the resistant screening for 10 days. The explants are subjected to transferring and subculture every 10 days. By using the agrobacterium-mediated genetic transformation method, 11, 16 and 13 resistant plants with transformed AaICE1, AaICE1(DC)1 and AaICE1(DC)2 genes are obtained respectively. After the low-temperature screening, compared with the wild-type plant of Raphanus sativus, the cold resistance of the obtained resistant plants is respectively improved by 1.7□ ,4.1□.1rea2.9□.

### Embodiment 34: Obtaining Triticum aestivum transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Triticum aestivum and are introduced into Triticum aestivum through the agrobacterium infection.

Ears of Triticum aestivum on 12-14 d after the flowering are collected. Immature grains that are uniform in size are collected from the middle part of the young ears of Triticum aestivum. Embryos are inoculated to the surface of a callus inducing culture medium, subjected to dark culture at 25□, transferred into the same culture mediumin 20 d, and subcultured. The embryos are inoculated onto the MC culture medium,cultured for 15-20 d, placed into a refrigerator at 4□ after the calluses formed by the embryos are completely developed, subjected to cold treatment for about 2 months, and then subcultured at a normal temperature. The calluses are transferred into a culture medium containing MC and acetosyringone (AS) 0.1 mmol/L in the early to middle August, and began to be transformed after being cultured for 5-7 d. The embryo calluses are transferred into an engineering bacteria infection solution for 0.5-1.0 h, then transferred to an MD culture medium containing AS 0.1 mmol/L, co-cultured for 2 d, and rinsed with sterile water containing 0.5 g/L cefazolin sodium, surplus liquid is blotted up by using sterile toilet paper, and the calluses are transferred to a culture medium (MD + 500 mg/L Carb + 5 mg/L Bialaphos),and screened. The screened resistant calluses are transferred to an ME differentiation culture medium mixed with 0.5 g/L cefazolin sodium and 5 mg/L Bialaphos. After being subjected to the selection culture for 6-8 weeks in 5 mg/L Bialaphos, the calluses are transferred to the regeneration culture medium MD mixed with IAA 1 mg/L and ZT 1 mg/L, and regenerated into seedlings. By using the agrobacterium-mediated genetic transformation method, 19, 12 and 18 resistant plants with transformed AaICE1, AaICE1(DC)1 and AaICE1(DC)2 genes are obtained respectively. Plants with target traits are obtained by screening at a low temperature. Compared with the wild-type plants of Triticum aestivum, the cold resistance of the obtained resistant plants isrespectively improved by 2.2□,4.2□ and 3.3□ under a treatment condition of -10□.

### Embodiment 35: Obtaining Hordeum vulgare transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Hordeum vulgare and are introduced into Hordeum vulgare through the agrobacterium infection.

The size of immature embryos of ears of Hordeum vulgare is examined. The size of the young embryo is ensured to be between 1.5-2 mm generally at the time about 14 days after the pollination. The immature seeds are completely separated from the ears, placed into a conical flask, and placed in a super clean bench. All steps hereinafter are carried out in the super clean bench. The seeds are simply rinsed with 70% alcohol for 30 s, washed with sterile water for three times, treated with 50% sodium hypochlorite for 4 min, and rinsed with sterile water for 3-4 times, and the residue of sodium hypochlorite is reduced as far as possible. The lower part, at about one third position, of the seed is clamped by tweezers so that immature embryos upward. The middle parts of the seeds are pricked through ultrafine-tip tweezers, and part of the seed coat is raised and removed so as to expose the immature embryos. The hypocotyl is removed by tweezers as far as possible, and the completeness of scutella is kept. The immature embryos are separated, and placed on a BCI callus inducing culture medium with small scutella up. Each culture dish holds about 25 embryos. The to-be-inoculated bacterial solution is dried using a 200 ul gun head and dropped on the immature embryos. The bacterial solution can also be dipped by a blade or an inoculation ring to wet the surface of the embryo. If the immature embryos have excessive bacterial solution, the immature embryos need to be dragged back and forth on the surface of the culture medium to remove the excessive bacterial solution on the surface. If the immature embryos are just wet, the embryos are directly transferred onto a fresh BCI culture medium with small scutella down and cut surface up. The culture dish is sealed to perform the dark culture for 72 h under conditions of 24□. If there are immature embryos with excessive growth of agrobacterium, the immature embryos need to be placed into a small beaker, rinsed with sterile water and the sterile water containing 160 mg/L Timentin antibiotics for several times, and the solution on the surface of the embryo is blotted up by using filter paper. The immature embryos are transferred onto a BCS callus screening culture medium (containing 5 mg/L bialaphos and 160 mg/L Timentin) with scutella down, and cultured in dark at 24□ for 6-8 weeks. The fresh culture medium is changed every 2 weeks. When the calluses are transferred, the calluses formed by differentiating the immature embryos shall not be separated as far as possible. After the calluses grow to a certain size on the selection culture medium, the calluses are transferred to a BCT transition culture medium (containing 5 mg/L Bialaphos and 160 mg/L Timentin). The culture is carried out in low light at 24□. The illumination condition can be achieved by covering the culture dish with a piece of paper. After being subjected to the transitional culture for two weeks, the calluses may have the regeneration trend. If no regeneration occurs, the fresh culture medium is changed every two weeks. After having apparent regeneration trend, the calluses are transferred to the BCR callus regeneration culture medium (containing 5 mg/L Bialaphos and 160 mg/L Timentin). The calluses are cultured in full light at 24□. The fresh culture medium is changed every two weeks. A plant culture flask is selected to substitute a glass culture dish so as to increase the regeneration space of the plant. After the calluses grow to a certain size on the selection culture medium, the calluses are transferred to B13M, and cultured in low light at 24□. The culture medium is changed every two weeks. When growing bigger, the regenerated shoots are transferred into a test tube, and cultured. When the shoots develop to 2-3 cm, young seedlings are carefully transferred into a glass test tube in a length of 15 cm, and cultured. The glass test tube is sealed with a breathable bacteria filter plastic film. The culture medium in the test tube is a rooting culture medium. In the genetic transformation experiment, 14, 19 and 15 resistant plants with transformed AaICE1, AaICE1(DC)1 and AaICE1(DC)2 genes are obtained respectively. After the low-temperature screening, compared with the wild-type plants of Hordeum vulgare, the cold resistance of the obtained resistant plants is respectively improved by 1.8□,3.4□ and 2.7□.

### Embodiment 36: Obtaining Juglans regia transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Juglans regia and are introduced into Juglans regia through the agrobacterium infection.

Agrobacterium frozen at -80□ and carrying transformation plasmids is streaked on a solid YEB plate culture medium containing 5 mg/L Bialaphos and cultured overnight at 28□. Individual colonies on the plate are picked, inoculated into a liquid YEB culture medium containing 5 mg/L Bialaphos, and subjected to shaking culture (200 rpm) overnight at a constant temperature of 28□ on a table concentrator. A lightabsorption value of the bacterial solution under the wavelength of 600 nm is determined by using an ultraviolet visible spectrophotometer. When the OD600 of the bacterial solution is 0.5-0.8, the bacteria solution is poured into a 30 ml centrifugal tube, centrifuged for 5-6 min at 6000 rpm at the normal temperature to remove the supernatant, added with liquid DKW culture medium until the volume is 30 ml, and subjected to vortex mixing for 5 min, and then the bacterial solution is ready for infection. The well pre-cultured stem segments of Juglans regia are mixed with the obtained agrobacterium bacterial solution, and infected for 30 min. The infected stem segments are dried by sterile filter paper, inoculated into co-culture medium that is a combination of DKW + 6-BA 0.5 mg/L + IBA 0.1 mg/L + AS (acetosyringone) 200 umol/L + sucrose 30 g/L + agar 6.0 mg/L, regulated until pH is 5.8 prior to the high-pressure sterilization, and co-cultured in dark for 3 days. The culture condition: the culture temperature is 25+/-1□. After being co-cultured for 3 days, the stem segments are transferred into a screening culture medium that is a combination of DKW + 6-BA 0.5 mg/L+IBA 0.1 mg/L + 5 mg/L Bialaphos + Cef 500 mol/L + sucrose 30 g/L + agar 6.0 mg/L. Adventitious buds are germinated in 5 weeks. The culture conditions: the culture temperature is 25+/-1□, the illumination time is 16 h each day, and the illumination intensity is 2000-2500 1x. In the genetic transformation experiment, 11, 12 and 17 resistant plants with transformed AaICE1, AaICE1(DC)1 and AaICE1(DC)2 genes are obtained respectively. Compared with the wild-type plants of Juglans regia, the cold resistance of the obtained resistant plants isrespectively improved by 1.7□, 3.8□ and 2.7□.

### Embodiment 37: Obtaining Lactuca sativa transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Lactuca sativa and are introduced into Lactuca sativa through the agrobacterium infection.

Agrobacterium individual colonies carrying *LsICE1* gene of different degrees of methylation are picked from an LB plate containing Kam and Rif, inoculated into an LB liquid culture medium containing 5 mg/L Bialaphos, and cultured overnight at 150 rpm and 28□ on a table concentrator until a mid-log phase (OD600=0.4-0.8). 10 mlof bacterial solution is collected and sub-packaged in 10 ml sterilized centrifugal tubes, centrifuged for 3 min at 3000 rpm to remove supernatant, suspended with sterilized deionized water, and diluted until the OD600 of the bacterial solution is about 0.5, and the bacterial solution can be used for infection. Cotyledon explants of Lactuca sativa infected by agrobacterium are co-cultured on a differentiation culture medium (MS + 6-BA 0.5 mg/L + NAA 0.20 mg/L) together with the agrobacterium, then transferred to a screening culture medium (Sm8 + 5 mg/L Bialaphos + Cef 200 mg/L), subjected to the resistant screening, and subcultured every week. After the cotyledon is cultured for one week, a base part of the cotyledon can gradually send forth few compact calluses and gradually turn green. Budlet points can be formed in about one week and are gradually differentiated to form tender leaves, and budlets are formed. A plurality of budlets can be generally formed on one callus. When growing to 2-3 cm, the buds are cut off, inserted into a rooting culture medium (1/2Ms + NAA 0.05 mg/L + Cef 200 mg/L), subjected to the inducing rooting, and transplanted to a seedling pot after 2-3 weeks. By the method, 17, 18 and 20 resistant plants with transformed AaICE1, AaICE1(DC)1 and AaICE1(DC)2 genes are respectively obtained. After the low-temperature screening, compared with the wild-type plant of Lactuca sativa, the cold resistance of the obtained resistant plants is respectively improved by 2.0□, 3.8□ and 2.5□.

### Embodiment 38: Obtaining Cymbidium hybridum transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Cymbidium hybridum and are introduced into Cymbidium hybridum through the agrobacterium infection.

20 ul of bacterial solution of the engineering bacterial strain frozen by glycerin is picked and diluted to 200 ul, then smeared to a YEB solid culture medium containing 5 mg/L Bialaphos. Individual colonies are picked after 20 h, inoculated to a YEB liquid culture medium containing 5 mg/L Bialaphos, and subjected to shaking culture for about 16 h at 28□. The bacterial solution is diluted by using the YEB liquid culture medium with a pH value of 5.2 until the OD600 of the bacterial solution is 0.4. Tips and roots of the cultured aseptic seedlings are cut off, a base part with a height of about 0.5 cm remains, is longitudinally cut into 2-4 tissue blocks (0.3-0.5 cm), pre-cultured for 2 d (by adding AS solution with the concentration of 100 mmol/ml), immersed in the bacterial solution for 10 min, taken out, after the bacterial solution is blotted up by sterile filter paper, inoculated to the co-culture medium, and co-cultured for 3 d, theexpalnts are separated and selected. The explants are cleaned with an MS liquid culture medium containing 50 mg/L Cef, and continuously cultured on a PLB inducing culture medium containing 50 mg/L Cef, and the agrobacterium is eliminated. The explants are transferred to the PLB inducing culture medium containing 50 mg/L Cef, and subjected to continuous culture for 20-30 d. When the explants are generallyat the PLB formation and development phase, the explants are then transferred to the screening culture medium containing 5 mg/L Bialaphos,followed by primary screening of resistant positive transgenic plants. By this method, 11, 10 and 10 resistant plants with transformed AaICE1, AaICE1(DC)1 and AaICE1(DC)2 genes are respectively obtained. After the low-temperature screening, compared with the wild-type plant of Cymbidium hybridum, the cold resistance of the obtained resistant plants is respectively improved by 2.2□, 3.9□ and 2.8□.

### Embodiment 39: Obtaining Phalaenopsis aphrodite transforming Ageratina adenophora AaICE1 geneof different degrees of methylation

The expression vectors of the AaICE1, AaICE1(DC)1 and AaICE1(DC)2 genes of different degrees of methylation constructed in embodiment 1 are subjected to genetic transformation of Phalaenopsis aphrodite and are introduced into Phalaenopsis aphrodite through the agrobacterium infection.

Protocorms of Phalaenopsis aphrodite are pre-cultured in a pre-culture liquid culture medium (added with 6-BA1.0 mg/L + NAA5.0 mg/L + 2,4-D 2.0 mg/L + AS 100 µmol/L) for 3 d. The agrobacterium EHA105 for transformation is subjected to shaking culture in an LB (pH 7.0) culture medium containing 5 mg/L Bialaphos, rifampicin 50.0 mg/L and acetosyringone 100 umol/L at 150 r/min and 28□ for 24 h The LB culture medium is mixed with 20% (v/v) bacterial solution, after being activated for 5 h, and the OD600 of the bacterial solution is about 0.60 by measurement. The bacterial solution is centrifuged at 6000 r/min for 3 min, are re-suspended by using a sterilization liquid culture medium (mixed with AS 100 umol/L) and diluted to the original volume, and the bacterial solution can be directly used for the genetic transformation of the protocorms. The protocorms are infected for 15 min, and then inoculated to a solid culture medium (mixed with 6-BA 1.0 mg/L + NAA 5.0 mg/L + 2,4-D 2.0 mg/L + AS 100 µmol/L), and co-cultured for 3 d. The protocorms are rinsed with a liquid culture medium containing 5.0 mg/L meropenem for 3 times, subjected to the alternating culture respectively on the solid culture media (added with KT 3.0 mg/L) mixed with meropenem 5.0 mg/L and cefotaxime Na salt 100.0 mg/L, and transferred and inoculated every 3 d until the agrobacterium is completely removed. The bacteria-free protocorms are transferred onto the solid culture medium containing 5 mg/L Bialaphos and KT 3.0 mg/L, selectively cultured, transferred to the same culture medium every 2 weeks, and subcultured. After 45 d, the protocorms are cultured on a protocorm-like bodies (PLBs) inducing culture medium (mixed with 1.0 mg/L TDZ and 1.0 mg/L 2,4-D) containing 5 mg/L Bialaphos. New PLBs emerge on a section and are differentiated to form seedlings. The plants are transferred into a proliferation culture medium (6-BA 5.0 mg/L), and cultured. When the height of adventitious plantlets reaches 3-5 cm, the adventitious plantlets are cut apart and transferred to a rooting culture medium (Hyponex No.1 30.0 g/L+ NAA0.3 mg/L). When each plant sends forth 4-5 new roots having a length of 2-4 cm, the plants are taken out of the flasks, and transplanted. In the transgenic experiment, 15, 18 and 20 resistant plants with transformed AaICE1, AaICE1(DC)1 and AaICE1(DC)2 genes are respectively obtained. Compared with the wild-type plants of Phalaenopsis aphrodite, the cold resistance of the plant with target traits screened at the low temperature is respectively improved by 2.0□, 3.6□ and 2.7□.

### Embodiment 40: Obtaining Chimonanthus praecox transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Chimonanthus praecox and are introduced into Chimonanthus praecox through the agrobacterium infection.

The agrobacterium EHA105 stored in a refrigerator at -80□ is inoculated onto a YEB plate culture medium containing 5 mg/L Bialaphos, and cultured in dark for 2-3 d. After colonies come up, individual colonies are picked, inoculated into 30 ml of YEB liquid culture medium containing 5 mg/L Bialaphos, subjected to first activation, subjected to shaking culture at 28□ on a table concentrator for 1-2 d, and taken off from the table concentrator for standby use when the OD600 of the bacterial solution is 0.8-1.0. The first-activated 50ul bacterial solution is inoculated into 50 ml of YEB liquid culture medium containing no Bialaphos, subjected to the second activation, and subjected to shakingculture at 28□ on an ordinary table concentrator until the OD600 is 0.4. In this experiment, half-developed cotyledons of Chimonanthus praecox are adopted as explants and cut into square pieces in a size of 1 cm^{∗}1 cm, and pre-cultured for 3 d. The activated agrobacterium EHA105 bacterial solution is centrifuged at 6000 rpm and 4□ to collect thalli. The thalli are added into the liquid MS + AS 100 umol/L,and re-suspended until the OD600 is 0.4. Calluses are infected for 10 min, co-cultured for 2 d, degermed by using liquid MS + Cef 1000 umol/L, rinsed with sterile water for 5 times, and subjected to selection culture in a screening culture medium (Ms + BA 1.0 mg/L + NAA 0.5 mg/L + KT 0.5 mg/L + IBA 2.0 mg/L+ 2,4-D 0.2 mg/L + 5 mg/L Bialaphos + Cef 1000 umol/L). The regenerated plants are transferred onto MS + 6-BA 1.0 mg/L + NAA 0.1 mg/L, immediately transferred into 1/2MS + NAA 0.1 mg/L, and subjected to the rooting culture when the seedling grows to 3-4 cm. In the transgenic experiment, 8, 14 and 13 resistant plants with transformed AaICE1, AaICE1(DC)1 and AaICE1(DC)2 genes are respectively obtained. After the low-temperature screening, compared with the wild-type Chimonanthus praecox, the cold resistance of the obtained resistant plants is respectively improved by 1.7□, 4.2□ and 3.0□.

### Embodiment 41: Obtaining Camellia sinensis transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Camellia sinensis and are introduced into Camellia sinensis through the agrobacterium infection.

Immature tea fruits in August-September are selected, cleaned with distilled water, and peeled, and milky white or slightly-brown immature seeds are taken out. Outer seed coats are cut apart. The surface of the seeds are disinfected with 75% ethanol for 30 s under a sterile condition, the seeds are immersed with 0.1% HgCl₂ for 3 min and then rinsed with sterile water for 6-8 times. Cotyledons are cut into square pieces in a size of 0.5 cm. The cotyledon pieces are inoculated to a solid culture medium (improved ER + 0.1 mg/L + 1.0 mg/L 6-BA + 0.1 g/L chloramphenicol + 30 g/L sucrose). After the inoculation, the cotyledon pieces are cultured in a constant-temperature culture room at 25+/-2□ in artificial light for 13 h each day under the illumination intensity of 1500-2000 lx. After the seeds are cultured for 25 d, the calluses of green cotyledons are selected as explants. The stored agrobacterium tumefaciens EHA 105 are streaked on a prepared YEB solid culture medium plate, and cultured in dark at 28□ for 1-2 d. Individual colonies are picked by using an inoculation ring, inoculated into 5 ml of YEB liquid culture medium, and subjected to shaking culture at 220 rpm and 28□ for 24 h. 1 ml of bacterial solution is sucked under a sterile condition into the liquid culture medium containing 200 uml/L acetosyringone, subjected to shaking culture at 220 rpm and 28□ for 4-6 h, and used for explant infection when an OD value measured at 600 nm is about 0.6-0.8. On the super clean bench the calluses of the cotyledons that already turn green are selected, used as explants, inoculated into a pre-culture medium (improved ER + 0.2 mg/L IBA + 30 g/L sucrose), and pre-cultured in dark at 25□ for 3 d. The pre-cultured cotyledon calluses are placed in the standby agrobacterium tumefaciens bacterial solution. An experimental material is immersed by the bacterial solution, infected for 15 min, then after the surplus bacterial solution on the surface of the experimental material is blotted up by sterile filter paper, inoculated to a YEB co-culture medium with 150 umol/L AS, and cultured in dark at 25□ for 3 d. After being co-cultured, the material is rinsed with sterile water for 3-4 times. Water on the surface of the material is dried with sterile filter paper. The calluses are transferred into the pre-culture medium, then subjected to the delayed selection culture for 3 d, and transferred and inoculated to bacteriostatic culture medium with 100 umol/L Cef and 100 umol/L Spe, and cultured in dark. The material is transferred and inoculated every 3 d. After 15 d, the explants are transferred into a screening culture medium containing 100 umol/L Spe for screening resistant buds. After the explants are subjected to the screening culture for 20 d, the vigorous resistant buds are transferred into a novel differentiation culture medium containing 5 mg/L Bialaphos and 100 umol/L Spe, and cultured. After being cultured for 30 d in the differentiation culture medium, the cotyledon calluses are differentiated to form seedlings. The seedlings are transferred into the rooting culture medium, and after 2 weeks, a complete resistant plant can be obtained. Through the genetic transformation, 12, 16 and 17 resistant plants with transformed AaICE1, AaICE1(DC)1 and AaICE1(DC)2 genes are obtained respectively. After the low-temperature screening, compared with the wild-type plant, the cold resistance of the obtained resistant plants is respectively improved by 2.3□ ,4.3□ and 3.2□.

### Embodiment 42: Obtaining Eucalyptus camaldulensis transforming Ageratina adenophora AaICE1 gene of different degrees of methylation

The expression vectors of the AaICE1, AaICE1(DC)1 and AaICE1(DC)2 genes of different degrees of methylation constructed in embodiment 1 are subjected to genetic transformation of Eucalyptus camaldulensis and are introduced into Eucalyptus camaldulensis through the agrobacterium infection.

The GV3101 strain stored in a low-temperature refrigerator is taken out. The surface of the prepared culture medium is gently streaked with 5-6 lines in a super clean bench by adopting a streaking method. A culture dish is inversely placed, and subjected to the dark culture at 25-27□ for 24-26 h. Individual colonies on the surface of the culture medium in the culture dish are picked by using an inoculation needle, placed onto a liquid culture medium in 15 ml centrifugal tube, and subjected to suspension culture at 180 rpm and 27□ for 12-18 h on a constant-temperature table concentrator. The bacterial solution is placed into a 100 ml triangular flask according to a ratio of 1/40, and subjected to continuous culture for about 6 h. When the OD600 of the bacterial solution is 0.5, the bacterial solution is used for the infection treatment. Eucalyptus urophylla cultivated clones DH201-2 are used as a material. Stems collected from the upper part and middle part of a plant that is cultured for 30 d on the rooting culture medium are cultured on the pre-culture medium for 6 d. In the super clean bench, the explants of Eucalyptus camaldulensis are immersed in a bacterial solution in a triangular flask for 30 min, and then the plant material is taken out, inoculated onto a plant co-culture medium (100 uM acetosyringone, PH 5.8), and cultured for 3 d. Thereafter, the infected stems are transferred into an inducing regeneration culture medium (Ms + 0.05 mg/L TDZ + 0.5 mg/L NAA + 5 mg/L Bialaphos + 300mg/L cephalosporin, PH 5.8), and cultured for 15 d, thus forming calluses. The browning, loose and yellowing calluses are removed. The red-green calluses with good growth are transferred to the same culture medium for more than 3 times to remove the agrobacterium, and then transferred to the culture medium only containing the Bialaphos, and adventitious buds are obtained. The regenerated shoots are transferred onto a seedling strengthening culture medium, cultured for 20 days, then transferred into a rooting culture medium, and subjected to the rooting culture. In the transgenic experiment, 14, 19 and 11 resistant plants with transformed AaICE1,AaICE1(DC)1 and AaICE1(DC)2 genes are respectively obtained. Compared with the wild-type plants of Eucalyptus camaldulensis, the cold resistance of the obtained resistant plants is respectivelyimproved by 3.7□ and 2.3□.

### Embodiment 43: Obtaining Arabidopsis thaliana by insitu replacing AaICE1 gene of Ageratina adenophora of different degrees of methylation through a CRISPR/Cas9 technology

The sequence of Ageratina adenophora AaICE1 gene is SEQ ID NO: 1, and according to the degenerate feature of a codon, sites that are theoretically methylated on the *AaICE1* gene are replaced to obtain the AaICE1(DC)1 gene (SEQ ID NO: 3) on which all methylation sites are replaced and AaICE1(DC)2 gene on which part of methylation sites are replaced (SEQ ID NO: 3). The AaICE1 is divided into 3 segments for the CRISPR/Cas9 vector construction and transgenic manipulation, and each segment is about 500 bp. The sgRNA with a guiding function is artificially designed. The sgRNA sequences are respectively 5'-GCTCCTATTTCGATGGGGTT TGG-3', 5'-GATAAATGAGAGCGGTAAAG CGG-3' and 5'-GCAGTGCTTTTCGATACAGC AGG-3'. The Cas9 protein is guided to perform site-specific cleavage for DNA. The sgRNA generally has a length of 20 nt. Promoters can be selected from CaMV 35S, U6, T7 and YAO. In the present experiment, the promoter YAO with high efficiency is selected. The vector pHEE401E and the AaICE1, AaICE1(DC)1 and AaICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, and the pHEE401E and the Bar gene are subjected to enzyme digestion by SacII and AsiSI. The Bar gene is bonded into pHEE401E and used as a resistant screening gene for constructing a CRISPR/Cas9 expression vector that is introduced into Arabidopsis thaliana through a pollen tube mediation method.

The surface of a flower of Arabidopsis thaliana at a full-blossom stage is partially immersed in an agrobacterium suspension (OD=0.8) for 5 min and gently rotated simultaneously. An infected plant is bagged, kept in a highly moist state and cultured in a dark room for 24 hours. When seeds are mature and pods are naturally cracked, the seeds can be harvested. The collected seeds of Arabidopsis thaliana are vernalized at 4□ for 2 days, and cultured on a plate containing 5 mg/L Bialaphos after being disinfected. Seeds successfully introduced with recombinant plasmids can grow normally. The non-transgenic seeds cannot grow normally and can only send forth two cotyledons, and will die due to severely inhibited growth of the root, and will die. The screened plant is transplanted to a culture medium in which vermiculite, peat and perlite are in a ratio of 9: 3: 1 and covered with a film for 2-3 days. By means of molecular verification, 19, 18 and 26 plants of Arabidopsis thaliana with transformed *AaICE1,AaICE1*(DC)1 and *AaICE1*(DC)2 genes are obtained respectively.

The wild-type plant and in-situ replaced plant of Arabidopsis thaliana are normally cultured in an illumination culture room until the seedling age of 3 weeks, subjected to cold adaption at 4□ for 3 days, placed at 0□ for 1 hour, placed at -4□ for 4 hours, °C °Ctreated for 24 h under the condition of dropping the temperature by 2□ per hour until -8□, and then cultured at 23□ for 5 d. The low-temperature harm conditions of the wild-type and in-situ replaced plants of Arabidopsis thaliana are observed. Each treatment process is repeated for 3 times. The low-temperature harm indexes of the plants after being cultured for 5 d are counted. The low-temperature harm indexes of the Arabidopsis thaliana with in-situ replaced *AaICE1, AaICE1*(DC)1 and *AaICE1*(DC)2 genes are respectively 0.59, 0.14 and 0.36. Compared with the wild type Co1-0, the cold resistance of the obtained resistant plants is respectively improved by 1.8□, 3.5□ and 2.4□.

### Embodiment 44 Obtaining Manihot esculenta by insitu replacing MeICE1 genes through a CRISPR/Cas9 technology

The *MeICE1* gene of Manihot esculenta is cloned. The cDNA sequence of the obtained gene is SEQ ID NO: 9. According to the degenerate feature of a codon, sites that are theoretically methylated on the *MeICE1* gene are replaced. According to the requirements on target traits, the sites of different degrees of methylation on the *MeICE1* gene are quantitatively replaced. The *MeICE1* gene on which all methylation sites are replaced has a sequence of SEQ ID NO: 50 and is named *MeICE1(DC)1.* The *MeICE1* gene on which part of methylation sites are replaced has a sequence of SEQ ID NO: 91 and is named *MeICE1(DC)2.* The *MeICE1(DC)1* is divided into 3 segments for CRISPR/Cas9 vector construction and in-situ replacement of Manihot esculenta, and each segment is about 400 bp. The sgRNA with a guiding function is artificially designed, and has a length of 20 nt and sequences respectively shown as 5'-GCCTCACCTTTGCTAAATAGAGG-3', 5'-GTAAACCCTGGACCATTTTCAGG-3' and 5'-GCCTATCATTGAGCTTCTTCCGG-3'.The sgRNA sequence of the *MeICE1*(DC)2 gene is 5'-GGTTTTGAGTGCATAAAGTAGGG-3'. The Cas9 protein is guided to perform site-specific cleavage for DNA. The YAO promoter is selected. The vector pHEE401E and the pHEE401E, and *MeICE1(DC)1* and *MeICE1(DC)2* genes of different degrees of methylation are cleaved by restriction endonucleases BsaI and KpnI, and the target gene is bonded behind the zCas9 sequenc. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector that is introduced into Manihot esculenta through an agrobacterium-mediated method.

500 ul of agrobacterium GV3101 expression vector stored in an ultralow-temperature refrigerator is added into 50 ml of YEB (mixed with 5mg/L Bialaphos) liquid culture medium, and cultured at 240 rpm and 28□ for 18-24 h. Then the mixture is centrifuged at 4□ and 4000 rpm for 10 min and diluted by liquid MS until an OD value is 1.0 for standby use. Somatic embryo green cotyledons of Manihot esculenta that is mature for 10-15 d are chopped, then about 500 segments of the cotyledon embryos are placed in 30 ml of agrobacterium culture suspension and the suspension is shaken for several times in the meanwhile. The bacterial solution is removed by suction after 45 min. The cotyledon segments are placed in a super clean bench for 20 min or the surplus liquid is dried using sterilized filter paper, and the cotyledon segments are transferred onto a stem organ generation culture medium, and co-cultured in lightat 26□ for 3 d (16 h for each day). After the transformed material is co-cultured for 3 d, calluses are taken out, rinsed with sterile water for 3 times, then rinsed for two times by using the liquid MS (pH5.3) culture medium containing 500 mg/L carbenicillin, and placed in a super clean bench for 20 min or sterile filter paper is used to blot up the residual liquid on the transformed material. The calluses are transferred onto a stem organ generation culture medium containing 5 mg/L Bialaphos and 500 mg/L carbenicillin, and cultured in dark at 26□ for 1 week. The calluses are transferred onto the stem organ generation culture medium containing 5 mg/L Bialaphos and 500 mg/L carbenicillin, and cultured in light at 26□ for 2 weeks (16 h for each day)°C. Resistant adventitious buds are picked and placed onto a stem elongation culture medium. After being cultured in light at 26□ for 2-3 weeks (16 h for each day)°C, and the emerging stems are cut off, transferred onto an MS culture medium, and cultured. When the stems grow into plants, the plants are cut apart for subculture. The stem segments about 1 cm coming up from lateral buds are cut, and transferred onto a hormone-free culture medium containing 5 mg/L Bialaphos for rooting experiment to eliminate the false-positive plant, and the stem segments of the wild-type plant are used as references. After 1-2 weeks, the transgenic positive plant can be normally rooted, while the false-positive plant and the reference plant cannot send forth roots. A total of 15 and 13 plants with in-situ replaced MeICE1(DC)1 and MeICE1(DC)2 genes of Manihot esculenta are obtained. Compared with the wild-type plant of Manihot esculenta, the cold resistance of the obtained resistant plants is respectively improved by 4.0□ and 3.0□.

### Embodiment 45 Obtaining Actinidia chinensis by insitu replacing AcICE1 gene of different degrees of methylation through a CRISPR/Cas9 technology

The *AcICE1* gene cDNA of Actinidia chinensis is cloned and has a sequence of SEQ ID NO: 10. According to degenerate features of a codon, sites that can be theoretically methylated on the *AcICE1* gene are replaced. According to requirements on target traits, the sites of different degrees of methylation on the *AcICE1* gene are quantitatively replaced. The *AcICE1* gene on which all methylation sites are replaced has a sequence of SEQ ID NO: 51 and is named *AcICE1(DC)1;* and the *AcICE1* gene on which part of methylation sites are replaced has a sequence of SEQ ID NO: 92 and is named AcICE1(DC)2. The *AcICE1(DC)1* is divided into 3 segments for CRISPR/Cas9 vector construction and in-situ replacement of Actinidia chinensis, and each segment is about 500 bp. The sgRNA with a guiding function is artificially designed, and has a length of 20 nt and sequences respectively shown as 5'-GTGGTAGGAGGGCTCTTCCATGG-3', 5'-GTGGCTGCAGAGAAAATCCAAGG-3' and 5'-GATATCCACCATGTTACCGGTGG-3'. The sgRNA sequence of the *AcICE1(DC)2* gene is 5'-GATGGTTCAAATCTTGATGGTGG-3'. The Cas9 protein is guided to perform site-specific cleavage for DNA. The YAO promoter is selected. The vector pHEE401E and *AcICE1(DC)1* and *AcICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to the enzyme digestion by SacII and AsiSI, and the Bar gene is bonded into pHEE401E, and used as a resistant screening gene for constructing a CRISPR/Cas9 expression vector that is introduced into Actinidia chinensis through an agrobacterium LBA4404.

The stored agrobacterium strain LBA4404 is collected and streaked, and individual colonies of the agrobacterium are picked. The picked individual colonies are placed in 20 ml of liquid YEP culture medium containing 5 mg/L Bialaphos, subjected to shaking culture at 200 rpm and 28□ for 16-24 h. 0.5 ml of bacterial solution is collected by suction, inoculated in 50 ml of YEP culture liquid containing 5 mg/L Bialaphos, and subjected to amplification culture under the same conditions. When the bacterial solution is cultured until an OD600 value is 0.5, the bacterial solution is centrifuged at 4000 rpm and 4□ and precipitated, and then suspended in an equal volume of liquid MS culture medium with 100 uM of acetosyringone for standby use. Small and tender leaves of aseptic seedlings of Actinidia chinensis are cut into pieces of 0.5 cm² under a sterile condition, and inoculated onto a culture medium containing MS basic culture medium, 3.0 mg/L 6-BA and 1.0 mg/L NAA, and pre-cultured for 3 d. After 3 d, the pre-cultured leaves are placed in the re-suspended bacterial solution, gently shaken in the meanwhile to allow the agrobacterium to sufficiently contact the leaves, infected for 10 min, and then surplus bacterial solution is blotted up by using sterile filter paper, and the leaves are placed with back side down on a regeneration culture medium containing 100 uM AS, and cultured in dark at 28□ for 2 d. A layer of sterile filter papershould be placed on the co-cultured medium to prevent the excessively vigorous growth of the agrobacterium. Co-cultured cotyledon explants are rinsed with sterile water for 4-5 times, the residual sterile water is removed by using sterile filter paper, the co-cultured cotyledon explants are inoculated onto a regeneration culture medium containing 5 mg/L Bialaphos and 400 mg/L Carb, subcultured once a month, and cut off after differentiated buds grow to 2-3 cm, and the cut differentiated buds are inserted into a rooting culture medium. Adventitious roots can be seen after 5-10 d. When the rooted seedlings grow to 5 cm, culture flasks are placed under a natural condition for culturing for 3-5 d. The covers are gradually opened in a sequence of loosening the covers on the first two days, half opening the cover on the third and fourth days, replacing the covers with a hard paperboard which is half of the cover on the fifth and sixth days, removing the hard paperboard on the seventh and eighth days, so that the tissue cultured seedlings are exposed in nature and subjected to hardening for 10 d. Thereafter, new seedlings are carefully taken out by using tweezers, cleaned with tap water to remove agar on roots, transplanted in the greenhouse soil, and cultured. A total of 11 and 12 transgenic plants with in-situ replaced Actinidia chinensis *AcICE1(DC)1* and *AcICE1(DC)2* genes are obtained by means of genetic transformation. The cold resistance of the obtained resistant plants is respectively improved by 3.6□ and 2.4□.

### Embodiment 46 Obtaining Musa nana by in situ replacing MaICE1 gene of different degrees of methylation through a CRISPR/Cas9 technology

The *MaICE1* gene cDNA of Musa nana is cloned and has a sequence of SEQ ID NO: 11. According to degenerate features of a codon, sites that can be theoretically methylated on the *MaICE1* gene are replaced. According to requirements on target traits, the sites of different degrees of methylation on the *MaICE1* gene are quantitatively replaced. The MaICE1 gene on which all methylation sites are replaced has a sequence of SEQ ID NO: 52 and is named MaICE1(DC)1; and the *MaICE1* gene on which part of methylation sites are replaced has a sequence of SEQ ID NO: 93 and is named *MaICE1(DC)2.* The *MaICE1(DC)1* is divided into 3 segments for CRISPR/Cas9 vector construction and in-situ replacement of Musa nana, and each segment is about 400 bp. The sgRNA with a guiding function is artificially designed, and has a length of 20 nt and sequences respectively shown as 5'-GGCCTCGAAGGGGTGATGGG NGG-3', 5'-GCAGAAGGCGGTGCCAGAGC NGG-3' and 5'-GAGGTCGACGAGTTGGACGA NGG-3'. The sgRNA sequence of the *MaICE1(DC)2* gene is 5'-GGCCTCGAAGGGGTGATGGGCGG-3'. The Cas9 protein is guided to perform site-specific cleavage for DNA. The YAO promoter is selected. The vector pHEE401E and the *MaICE1(DC)1* and *MaICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector that is introduced into Musa nana through the agrobacterium GV3101.

An embryonic suspension cell system (ECS) of Musa nana is used as a transformation receptor. The ECS of Musa nana under the subculture for 10 d is collected, and centrifuged to removesupernatant. Every 1 mL of cell compact volume ECS is added with 40 ml of agrobacterium GV3101 containing *MaICE1* gene of different degrees of methylation, and is kept still in dark at 27□ for 1-2 h, and then the mixture is co-cultured. Co-culture conditions: the mixture is subjected to shaking culture in dark at 50 rpm and 27□ for 24 h. Then a culture is kept still to remove the supernatant, and added with 40 ml of liquid culture medium. The mixture is continuously co-cultured for 7 d by increasing the rotation speed to 110 rpm. The co-cultured solution is kept still to remove the supernatant, and added with 40 ml of Musa nana ECS liquid screening culture medium. The mixed solution is subjected to shaking culture at a rotation speed of 100 rpm at 27□, subcultured every 10-14 d and continuously subcultured for more than 3 generations. A subculture method includes: 0.1-0.5 ml of cell compact volume ECS of a previous generation is collected and continuously subjected to shaking culture in a fresh liquid screening culture medium. Meanwhile, the remaining ESC after the subculture of each generation and the ESC obtained after the original co-culture is completed are screened by a semi-solid screening culture medium, and the screened mixture is used as a reference. When semisolid screening is carried out, the ESC is washed with M2 culture medium containing 500 mg/L cephalosporin for 2-3 times, and the sterile paper is used to blot up surplus culture medium. Then the ECS is transferred onto the semisolid screening culture medium for embryonic induction. A somatic embryo inducing culture medium is changed every month until the maturity of the embryo. The ECS subjected to 3-generation liquid screening and culture is collected and is kept still, and the supernatant is removed. Then the ECS is uniformly laid on a somatic embryo inducing culture medium M3, and cultured in dark for 2-3 months. The somatic embryo inducing culture medium is changed every month until the maturity of the embryos. The mature resistant somatic embryo is transferred onto a somatic embryo germination culture medium, and cultured in light/in dark (for 12h/12h) until the somatic embryos are germinated into seedlings. Then the seedlings are transferred onto a rooting culture medium, and cultured in light/in dark (12h/12h), thus obtaining a complete transformed plant. A total of 21 and 15 plants with in-situ replaced Musa nana *MaICE1(DC)1* and *MaICE1(DC)2* are obtained in the present experiment. The cold resistance of the obtained resistant plants is respectively improved by 3.9□ and 2.9□.

### Embodiment 47 Obtaining Musa basjoo by insitu replacing MabICE1 gene through a CRISPR/Cas9 technology

The *MbICE1* gene cDNA of Musa basjoo is cloned and has a sequence of SEQ ID NO: 12. According to degenerate features of a codon, sites that can be theoretically methylated on the *MbICE1* gene are replaced. According to requirements on target traits, the sites of different degrees of methylation on the *MbICE1* gene are quantitatively replaced. The *MbICE1* gene on which all methylation sites are replaced has a sequence of SEQ ID NO: 53 and is named *MbICE1(DC)1;* and the *MbICE1* gene on which part of methylation sites are replaced has a sequence of SEQ ID NO: 94 and is named *MbICE1(DC)2.* The *MabICE1(DC)1* is divided into 3 segments for CRISPR/Cas9 vector construction and in-situ replacement of Musa basjoo, and each segment is about 500 bp. The sgRNA with a guiding function is artificially designed, and has a length of 20 nt and sequences respectively shown as 5'-GGCATTGAAGACAGACGAAACGG-3', 5'-GCAGAAAAAGTCGGCATCTTGGG-3' and 5'-GTAGGCAGTGAAGAACTTGAAGG-3'. The sgRNA sequence of the MbICE1(DC)2 gene is 5'-GCATGGACTTAAAGCCGGAG AGG-3'. The Cas9 protein is guided to perform site-specific cleavage for DNA. The YAO promoter is selected. The vector pHEE401E and the *MabICE1(DC)1* and *MabICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector that is introduced into Musa basjoo through an agrobacterium GV3101. The operation method of the genetic transformation experiment of Musa basjoo is similar to that of the genetic transformation experiment of Musa nana (embodiment 11). In the genetic transformation experiment, a total of 13 and 15 plants with in-situ replaced *MabICE1(DC)1* and *MabICE1(DC)2* genes are obtained. Compared with the wild-type plants of Musa basjoo, the cold resistance of the obtained resistant plants is respectively improved by 4.2□ and 2.6□.

### Embodiment 48 Obtaining Theobroma cacao by insitu replacing TcICE1 genes through a CRISPR/Cas9 technology

The *TcICE1* gene cDNA of Theobroma cacao is cloned and has a sequence of SEQ ID NO: 13. According to degenerate features of a codon, sites that can be theoretically methylated in the *TcICE1* gene are replaced. According to requirements on target traits, the sites of different degrees of methylation on the *TcICE1* gene are quantitatively replaced. The *TcICE1* gene on which all methylation sites are replaced has a sequence of SEQ ID NO: 54 and is named *TcICE1(DC)1;* and the *TcICE1* gene on which part of methylation sites are replaced has a sequence of SEQ ID NO: 95 and is named TcICE1(DC)2. The *TcICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GCAGCAAAATATTATCTTGATGG-3', 5'-GGAGGGAATTTCGGGTTGTTNGG-3' and 5'-GATAAAAACGAAAAGAGAAGGGG-3'. The sgRNA sequence of the *TcICE1(DC)2* gene is 5'-GGAGGGAATTTCGGGTTGTTCGG-3'. The Cas9 protein is guided to perform site-specific cleavage for DNA. The YAO promoter is selected. The vector pHEE401E and the *TcICE1(DC)1* and *TcICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector that is introduced into Theobroma cacao through an agrobacterium LBA4404.

Mature fruits of Theobroma cacao are collected from the field and peeled, and the fruits of Theobroma cacao without peel are immersed in 75% alcohol for several seconds, and then immersed in NaC10 solution with a volume percentage of 5% for 30 min. After the seed coats are removed, the seeds are transferred into a germination culture medium, and cotyledons are collected after 10 days later, and used as explants. Thalli of agrobacterium LBA4404/PBI121 cultured until an OD₆₀₀ value is about 1.37 are resuspended in a same volume of PCG liquid culture medium (DKW + 2,4-D 3.0 mg/L + KT 1.0 mg/L + TDZ 0.01 mg/L + Glucose 20 g/L + glutamine 250 mg/L) for infecting a cotyledon material of Theobroma cacaowhich that is cultured for 10 d in the germination culture medium, and the infected cotyledon material is co-cultured in dark at 28□ for 3 days. The co-cultured cotyledon material is transferred onto a PCG solid culture medium (1% agar powder) containing 500 mg/L Carb and 5 mg/L Bialaphos, subjected to bacteriostasis and resistant screening culture, subcultured for one time on the same culture medium after 10 d, cultured in the PCG culture medium for 20 days, then transferred into an SCG culture medium (KW + 2, 4-D 3 mg/L+ KT 1 mg/L + Glucose 20 g/L+10% CW + 1% agar powder) containing 500 mg/L Carb and 5 mg/L Bialaphos, cultured for 20 days, then transferred into an ED culture medium (DKW + Sucrose 30 g/L+ Glucose 1 g/L + 1% agar powder) containing Car 500 mg/L and 5 mg/L Bialaphos, and cultured until an embryoid comes up. Meanwhile, frequent observation is required, if there is contamination or re-appearance of agrobacterium, the uncontaminated material is moved out immediately for continuous culture. The mature embryos are transferred into an embryo development culture medium. The culture media are classified into the culture media at two phases, the culture medium at a first phase is PEC (1/2DKW + KNO₃ 0.3 g/L+AA 1000X stock solution 1 ml/L+ glucose 20 g/L + sucrose 10 g/L + 1% agar powder). During the culture at the first phase, the culture condition is that the daily illumination period is 16h light/8-h dark. The culture medium is changed every 30 days until a bud with 1-2 pieces of euphylla come up. The mature embryos are transferred into an RD medium at the second phase (1/2DKW + KN0₃ 0.3g/L + Glucose 10g/L+ Sucrose 5g/L + IBA at different concentrations 1.0 mg/L + IAA atdifferent concentrations 1.0 mg/L). The culture conditions at the second phase are consistent with that at the first phase. The embryos are transferred to a new culture medium every 30 days until the seedlings are formed. A total of 10 and 9 plants with in-situ replaced *TcICE1(DC)1* and *TcICE(DC)2* genes are obtained in the present experiment. The cold resistance of the obtained resistant plants is respectively improved by 3.9□ and 2.2□.

### Embodiment 49 Obtaining Citrus trifoliata by insitu replacing CtICE1 genes through a CRISPR/Cas9 technology

The *CtICE1* gene cDNA of Citrus trifoliata is cloned and has a sequence of SEQ ID NO: 14. According to degenerate features of a codon, sites that can be theoretically methylated on the *CtICE1* gene are replaced. According to requirements on target traits, the sites of different degrees of methylation on the *CtICE1* gene are quantitatively replaced. The *CtICE1* gene on which all methylation sites are replaced has a sequence of SEQ ID NO: 55 and is named *CtICE1(DC)1;* and the *CtICE1* gene on which part of methylation sites are replaced has a sequence of SEQ ID NO: 96 and is named *CtICE1(DC)2.* The *CtICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GCTTTGATTTGGGTGAAATGGGG -3', 5'-GCAATTAGCACTATCACTGGTGG-3' and 5'-GCTGTCATCAGTTGTTTCAATGG-3'. The sgRNA sequence of the *CtICE1(DC)2* gene is 5'-TGCTTGAAGTGGAAGATGAC TGG-3'. The Cas9 protein is guided to perform site-specific cleavage for DNA. The YAO promoter is selected. The vector pHEE401E and *TcICE1(DC)1* and *TcICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector that is introduced into Citrus trifoliata through an agrobacterium LBA4404.

Fruits of Citrus trifoliata are cut apart to take out seeds. The seeds are cleaned with tap water, and then pectin is removed by using 1 mol/L NaOH. Then the seeds after the pectin is removed are rinsed, subjected to shaking cleaning by using 70% alcohol for about 1 min in a super clean bench, immersed with 2% sodium hypochlorite for 18 min for sterilization, and shaken for several times in the meanwhile. Thereafter, the sodium hypochlorite is discarded. The seeds are rinsed with sterile water for three times, 5-6 min for each time. The sterilized seeds are peeled off under a sterile condition, placed in a test tube containing a solid culture medium, and cultured in dark at 24□. After being cultured for 15-30 d, the seeds are transferred to the illumination culture (at 24□, and under a light cycle of 14-h/10-h), and then epicotyl is collected for transformation. The epicotyl or stems are obliquely cut into stem segments in a length of about 0.5-1 cm, and nodes should be avoided as far as possible. The shaken agrobacterium bacterial solution is diluted with an LM suspension culture medium containing 20 mg/L AS to adjust the OD₆₀₀ value of the bacterial solution to 0.8. The cut explant material is immersed in the prepared agrobacterium bacterial solution, infected for 20 min, and shaken for several times during the time. After the infection, the bacterial solution on the surface of the explant is blotted up by using sterile filter paper, and the explant is inoculated to a co-culture medium (MT + BA1.0 mg/L + IAA 0.5 mg/L + AS 20 mg/L), and cultured in dark at 21-23□ for 3 d. The co-culture medium can be covered with a layer of sterile filter paper, so as to reduce the contamination probability of the agrobacterium. After being co-cultured for three days, the transformed explant is washed with 400 mg/L cephalosporin for one time, rinsed with sterile water for 3-5 times until the agrobacterium on the surface of the explant is cleaned, after being dried by using sterile water absorption paper, the explant is transferred into a screening culture medium containing 5 mg/L Bialaphos and 400 mg/L cephalosporin (MT + BA 1.0 mg/L + IAA 0.5 mg/L + 5 mg/L Bialaphos + Cef 400 mg/L), cultured in dark at 25□ for two weeks, and then transferred to light/dark culture (16/8h). The explant is subcultured every 20 d, and regenerated buds at non-incisions are removed during the subculture. When the resistant buds grow to about 0.5 cm, the resistant buds are transferred to an elongation culture medium (MT + BA 0.5 m/L + AA 0.5 mg/L + GA 0.5 mg/L + 5 mg/L Bialaphos + Cef 400 mg/L) for promoting the elongation. When the resistant buds grow to 1-2 cm, the resistant buds are transferred to a rooting culture medium (MT + BA 0.5 mg/L+ IAA 0.5 mg/L + activated carbon 0.5 g/L) for rooting induction. A total of 20 and 20 plants with in-situ replaced *CtICE1(DC)1* and *CtICE(DC)2* genes are obtained by means of genetic transformation. The cold resistance of the obtained resistant plants is respectively improved by 3.8□ and 1.7□. Embodiment 50 Obtaining Vitis vinifera by insitu replacing VvICE1 genes through a CRISPR/Cas9 technology

The *VvICE1* gene cDNA of Vitis vinifera is cloned and has a sequence of SEQ ID NO: 15. According to degenerate features of a codon, sites that can be theoretically methylated on the *VvICE1* gene are replaced. According to requirements on target traits, the sites of different degrees of methylation on the *VvICE1* gene are quantitatively replaced. The *VvICE1* gene on which all methylation sites are replaced has a sequence of SEQ ID NO: 56 and is named *VvICE1(DC)1;* and the *VvICE1* gene on which part of methylation sites are replaced has a sequence of SEQ ID NO: 97 and is named *VvICE1(DC)2.* The *VvICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GAGGAGGAGTGGTTGGTGAGCGG-3' and 5'-GTGGAGGAGACTGCAAATGATGG-3'. The sgRNA sequence of the *VvICE1(DC)2* gene is 5'-GTGAGCGGTGTCGAAATGAA AGG-3'. The Cas9 protein is guided to perform site-specific cleavage for DNA. The YAO promoter is selected. The vector pHEE401E and TcICE1(DC)1 and TcICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI,and the Bar gene is bonded into pHEE401E and used as a resistant screening gene for constructing a CRISPR/Cas9 expression vector that is introduced into Vitis vinifera through an agrobacterium.

PEM of Vitis vinifera after being subjected to the inducing propagation is subcultured onto a fresh X6 culture medium and pre-cultured in dark at 26□ for one week for transgenic manipulation. 20 uL of bacterial solution is collected and inoculated into 20 ml of liquid LB culture medium (containing 5 mg/L Bialaphos). After being cultured at 180 rpm and 28□ for 24 h, 20 uL of the bacterial solution is inoculated into 20 ml of liquid LB culture medium (containing 5 mg/L Bialaphos), and subjected to the secondary activation culture for 20 h under the same conditions. The bacterial solution is transferred into a 50 ml sterilized centrifugal tube and centrifuged for 8 min at 5000*g to removesupernatant, the bacterial solution is re-suspended and centrifuged in a re-suspension buffer solution (MS culture medium + 20 g/L sucrose + 100 uM AS) for two times. The bacterial solution is incubated at 180 rpm and 28□ for 1 h in the re-suspension buffer solution, and the concentration of the bacterial solution is adjusted by the adding re-suspension solution until an OD₆₀₀ value is 0.3-0.4. The PEM of Vitis vinifera is put into a triangular flask containing an agrobacterium bacterial solution and infected for 20 min, the surface bacterial solution is blotted up by using sterile filter paper, and the PEM of Vitis vinifera is subcultured into a sterile culture dish which has two layers of sterilized filter paper and contains about 5-6 m of X6 liquid culture medium (mixed with 100 uM AS). After being co-cultured in dark at 26□ for 48 h, the PEM is degermed for 20 min in a cephalosporin solution at the concentration of 200 mg/L and a carbenicillin solution at the concentration of 200 mg/L, and rinsed with sterile water for 3 times. The PEM of Vitis vinifera is inoculated into the culture medium (MS + 5 mg/L Bialaphos + 200 mg/L Cef + 200 mg/L Carb + 10 mg/L Hyg + 30 g/L sucrose + 3 g/L Phytagel). The PEM-induced resistant SE is inoculated to the culture medium (MS + 0.2mg/L KT + 0.1mg/L NOA + 10mg/L Hyg + 30 g/L sucrose + 3g/L Phytagel). The resistant SE is cultured in dark at 26□, and subcultured every three weeks until resistant somatic embryos come up. A total of 10 and 9 plants with in-situ replaced WwICE1(DC)1 and WwICE(DC)2 genes are obtained by means of genetic transformation of Vitis vinifera. The cold resistance of the obtained resistant plants is respectively improved by 3.3□ and 1.7□.

### Embodiment 51 Obtaining Malus domestica by insitu replacing MdICE1 gene through a CRISPR/Cas9 technology

The *MdICE1* gene cDNA of Malus domestica is cloned and has a sequence of SEQ ID NO: 16. According to degenerate features of a codon, sites that can be theoretically methylated on the *MdICE1* gene are replaced. According to requirements on target traits, the sites of different degrees of methylation on the *MdICE1* gene are quantitatively replaced. The *MdICE1* gene on which all methylation sites are replaced has a sequence of SEQ ID NO: 57 and is named *MdICE1(DC)1;* and the *MdICE1* gene on which part of methylation sites are replaced has a sequence of SEQ ID NO: 98 and is named *MdICE1(DC)2.* The *MdICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GAAGACAAGTTGGGATTTTGAGG-3', 5'-GCACGGTTACCGGTGGAGGAGGG-3' and 5'-GCAGCTTGGCCATTTGGGCTAGG-3'. The sgRNA sequence of the *MdICE1(DC)2* gene is 5'- GGTTGAGCAGCAAGCTTTCT GGG-3'. The Cas9 protein is guided to perform site-specific cleavage for DNA. The YAO promoter is selected. The vector pHEE401E and *MdICE1(DC)1* and *MdICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The Malus domestica is subjected to the genetic transformation by means of ultrasonic transformation method. Leaves of test-tube plantlets of Malus domestica are cut into fragments of about 4 mm². The leaf fragments are placed into a container containing 20 ml of ultrasonic buffer solution (13.7 mmol/L NaCI, 2.7 mmol/L KCI, 10 mmol/L Na₂HPO₄, 2 mmol/L KH₂POP₄, 5%DMSO, pH 7.4). Every microliter of buffer solution contains 5 ug of plasmid DNA. The leaf fragments are ultrasonically treated for 20 min under a sound intensity of 0.5-1.0 W/cm². The ultrasonically-treated leaves are transferred onto an MS regeneration culture medium containing 4 mg/L 6-BA and 0.2 mg/L NAA for growth recovery for 1 week, then transferred into a regeneration culture medium containing 5 mg/L Bialaphos for dark culture screening for 2 weeks, transferred every two weeks, and transferred onto an MS subculture medium containing 0.5 mg/L 6-BA and 0.1 mg/L NAA after 2 months, and this course lasts for 3 months in total. Culture conditions: illumination is continued for 16 h per day, the illumination intensity is 40 umol m⁻² s⁻¹, and subculture and storage are carried out at a constant temperature of 25□. The subculture is carried out every 4 weeks. 16 and 17 resistant plants with in-situ replaced *MdICE1(DC)1* and *MdICE1(DC)2* genes are respectively obtained from 400 explants cotyledons. Compared with the wild-type plant, the cold resistance of the obtained resistant plants is respectively improved by 4.1□ and 2.5□.

### Embodiment 52 Obtaining Chorispora bungeana by insitu replacing CbICE1 gene of different degrees of methylation through a CRISPR/Cas9 technology

The *CbICE1* gene cDNA of Chorispora bungeana is cloned and has a sequence of SEQ ID NO: 17. According to degenerate features of a codon, sites that can be theoretically methylated on the *CbICE1* gene are replaced. According to requirements on target traits, the sites of different degrees of methylation on the *CbICE1* gene are quantitatively replaced. The *CbICE1* gene on which all methylation sites are replaced has a sequence of SEQ ID NO: 58 and is named *CbICE1(DC)1;* and the *CbICE1* gene on which part of methylation sites are replaced has a sequence of SEQ ID NO: 99 and is named *CbICE1(DC)2.* The *CbICE1(DC)1* is divided into 3 segments for CRISPR/Cas9 vector construction and in-situ replacement and each segment is about 500 bp. The sgRNA with a guiding function is artificially designed, and has a length of 20 nt and sequences respectively shown as 5'-GAAGATCTTGTGGATGTGGTGGG-3', 5'-GTTCAGATTGGAGGAGGGAAGGG-3' and 5'-GTGAACATTCATATGTTCTGTGG-3'. The sgRNA sequence of the *CbICE1(DC)2* gene is 5'-GAAGATCTTGTGGATGTGGTGGG-3'. The Cas9 protein is guided to perform site-specific cleavage for DNA. The YAO promoter is selected. The vector pHEE401E and *CbICE1(DC)1* and *CbICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Seeds of Chorispora bungeana are disinfected (70% alcohol and 1% sodium hypochlorite), and then sowed in an MS culture medium. After one week, hypocotyl is cut, placed on a differentiation culture medium (MS + 2 mg/L 6-BA+ 0.2 mg/L NAA+ 3% sucrose + 0.6% agar), and pre-cultured for 2 d. The stored agrobacterium EHA105 is activated. 500 ul of activated bacterial solution is collected, inoculated into 50 ml of YEB liquid culture medium, subjected to shaking culture at 220 rpm and 28□ until an OD600 value of the bacterial solution is 0.5, centrifuged at 4□ (4000 r/min, 10 min) to remove supernatant, and re-suspended in 20 ml of pre-cooled MS liquid culture medium for standby use. The pre-cultured hypocotyl is infected by using re-suspension bacterial solution for 10 min, dried on filter paper, then transferred onto a differentiation culture medium, and co-cultured for 2 d. The co-cultured hypocotyl is transferred onto a screening culture medium (MS + 2 mg/L 6-BA + 0.2 mg/L NAA + 5 mg/L Bialaphos + 400 mg/L Cb + 3% sucrose + 0.6% agar), and subjected to resistant screening, and the screening culture medium is changed every 10 d. Resistant buds obtained by multi-times screening are transferred onto a rooting culture medium (MS + 2 mg/L 6-BA + 0.4 mg/L NAA + 5 mg/L Bialaphos + 400 mg/L Cb + 3% sucrose + 0.6% agar) for rooting culture. After tissue cultured seedlings have strong root systems, the root systems are cleaned to remove residual medium, transferred into nutritional soil, and cultured in an artificial weather room. 22 and 25 resistant plants with in-situ replaced MdICE1(DC)1 and CbICE1(DC)2 genes are respectively obtained by means of genetic transformation experiments. Compared with the wild-type plant, the cold resistance of the obtained resistant plants is respectively improved by 3.4□ and 2.4□.

### Embodiment 53 Obtaining Solanum capsicoides by insitu replacing ScICE1 gene of different degrees of methylation through a CRISPR/Cas9 technology

The *ScICE1* gene cDNA of Solanum capsicoides is cloned and has a sequence of SEQ ID NO: 18. According to degenerate features of a codon, sites that can be theoretically methylated on the *ScICE1* gene are replaced. According to requirements on target traits, the sites of different degrees of methylation on the *ScICE1* gene are quantitatively replaced. The *ScICE1* gene on which all methylation sites are replaced has a sequence of SEQ ID NO: 59 and is named *ScICE1(DC)1;* and the *ScICE1* gene on which part of methylation sites are replaced has a sequence of SEQ ID NO: 100 and is named *SeICE1(DC)2.* The *SeICE1(DC)1* is divided into 2 segments for CRISPR/Cas9 vector construction and in-situ replacement, and each segment is about 500 bp. The sgRNA with a guiding function is artificially designed, and has a length of 20 nt and sequences respectively shown as 5'-GTACAGTTCGATGGGATTCGGGG-3' and 5'-GGTTGTCCCAAAGATAACCAAGG-3'. The sgRNA sequence of the *ScICE1(DC)2* gene is 5'-GTAATCTGACTGATAGAAAA AGG-3'. The Cas9 protein is guided to perform site-specific cleavage for DNA. The YAO promoter is selected. The vector pHEE401E and ScICE1(DC)1 and ScICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Solanum melongna L. is used as a receptor for the genetic transformation. Seeds are immersed in 75% alcohol for 30 s, rinsed with sterile water for one time, immersed in 10% NaC10 solution for 20 min, rinsed with sterile water for 3 times, inoculated onto 1/2 MS culture medium, germinated, and cultured into aseptic seedlings in light/dark (16 h/18 h) at 26□, and the aseptic seedlings are used as a tested material. An overexpression vector is constructed by Agrobacterium tumefaciens strain EHA105, plasmid vector pBI121 and *ScICE1* gene. Cotyledons of aseptic seedlings are selected, with leaf tips removed, cut into pieces in a uniform size of 0.5 mm², inoculated into a pre-culture medium (MS + NAA 0.2 mg/L + ZT 1.2 mg/L), pre-cultured for 2 d, and subjected to agrobacterium infection transformation. The agrobacterium bacterial solution with an OD value of 0.5 is collected. The infection time is 10 min. The infected explant is inoculated into a co-culture medium (1/2MS + NAA 0.2 mg/L + ZT 1.2 mg/L + 200 umol/L acetosyringone), cultured in dark for 2 d, and then cleaned by using sterile water containing 150 mg/L Timentin, after the surface water is blotted up by using sterile filter paper, the explant is inoculated into a culture medium (MS + NAA 0.2 mg/L + ZT 1.2 mg/L + 150mg/L Timentin) for recovery culture, and then placed into an induced callus culture medium (added with 0.1 mg/L NAA, 3.0 mg/L ZT). The explants are cultured in light for 16 h and in dark for 8 h under an illumination intensity of 1600 1x. When growing for about two weeks, the explants send forth small, tender, round and hard green calluses, and are inoculated into a germination culture medium (mixed with 0.1 mg/L NAA, 4.0 mg/L ZT, 1.5 mg/L 6-BA, 12.0 mg/L AgN0₃) for inducing germination. Seedlings formed by elongation and differentiation of buds send forth 2 to 3 pieces of euphylla, are cut off from bases, inoculated into 1/2 MS rooting culture medium, and subjected to inducing rooting. The illumination time is 16 h each day, the temperature is 24-27□, and the illumination intensity is 1600 1x. The regenerated seedling growing in the culture medium is poor in environmental adaptability. A small hole of the triangular flask shall be opened for ventilation. The ventilation volume shall be gradually increased according to the growth condition of the seedling so as to improve the environmental adaptability of the seedlings. After two weeks, when the seedling has 4 to 5 pieces of euphylla, three main roots and a plurality of side roots, the flask opening is opened. The sufficient water of the seedling should be guaranteed. The seedling can be transplanted after being continuously cultured for 5 d. In the genetic transformation experiment, 13 and 10 resistant plants with in-situ replaced *ScICE1(DC)1* and *SeICE1(DC)2* genes are respectively obtained. Compared with the wild-type plant, the cold resistance of the obtained resistant plants is respectively improved by 3.0□ and 2.2□.

### Embodiment 54 Obtaining Solanum lycopersicum by insitu replacing SlICE1 gene of different degrees of methylation through a CRISPR/Cas9 technology

The *SlICE1* gene cDNA of Solanum lycopersicum is cloned and has a sequence of SEQ ID NO: 19. According to degenerate features of a codon, sites that can be theoretically methylated on the *SlICE1* gene are replaced. According to requirements on target traits, the sites of different degrees of methylation on the *SlICE1* gene are quantitatively replaced. The *SlICE1* gene on which all methylation sites are replaced has a sequence of SEQ ID NO: 60 and is named *SlICE1(DC)1;* and the *SlICE1* gene on which part of methylation sites are replaced has a sequence of SEQ ID NO: 101 and is named *SlICE1(DC)2.* The *SlICE1(DC)1* is divided into 3 segments for CRISPR/Cas9 vector construction and in-situ replacement, and each segment is about 500 bp. The sgRNA with a guiding function is artificially designed, and has a length of 20 nt, and sequences respectively shown as 5'-GGTTGTTGTTAATAGCCTTTTGG-3', 5'-GTGGATGATACTGTTAAGAA TGG-3' and 5'-GCTGTTATCAGCTGCTTCAATGG-3'. The sgRNA sequence of the SlICE1(DC)2 gene is 5'-GTTGTTGTTAATAGCCTTTTGGG-3'. The Cas9 protein is guided to perform site-specific cleavage for DNA. The YAO promoter is selected. The vector pHEE401E and *SlICE1(DC)1* and *SlICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

A variety of Solanum lycopersicum with high germination rate on 1/2MS basic culture medium is selected as a material of the present experiment. When a seedling on the 1/2MS basic culture medium grows to a height of about 5-6 cm (about 18 days), cotyledons of aseptic seedlings are cut and used as a receptor material for transformation. A small amount of agrobacterium tumefaciens pBLGC bacterial solution containing a target expression vector is collected from a glycerin tube, subjected to streaking culture on a YEP solid culture medium (mixed with 5 mg/L Bialaphos and 20mg/L Rif) and cultured in dark at 28□ until individual colonies in a diameter of about 1 mm come up (about 36-48 h). The individual colonies are collected and transferred to a similar solid culture medium plate, and cultured until a vigorous growth period (about 36 h). Individual colonies of agrobacterium are picked from the YEP plate, inoculated into a YEP liquid medium containing 5 mg/L Bialaphos and 20 mg/L Rif, uniformly mixed, subjected to shaking culture overnight at 180-200 rpm at 28□ (about 16-18 h). On the next day, the mixture in the inoculation amount of 1%is inoculated to 20 mL of YEP liquid culture medium, and subjected to shaking culture at 180 rpm and 28□ for 3-4 h, and the agrobacterium is cultured to a logarithmic phase (an OD600 value is about 0.6). Then the mixture is centrifuged for 15 min at 4000 rpm and 4□ to collect thalli are collected and remove supernatant. The thalli are diluted to be 3 to 5 times of the original bacterial solution by using a YEP liquid culture medium,the mixture is added with 200 um of acetosyringone, and shaken for 1-2 h at 180-200 rpm and 28□. Fully-developed green cotyledon leaves are collected, scratched by using a sterile scalpel, and infected for about 1-20 min in a prepared agrobacterium suspension and then the leaves are taken out, a, dried by using sterile filter paper, transferred into a solid differentiation culture medium (Ms + 6-BA 2mg/L + IAA 0.2 mg/L), and co-cultured in dark at 23-25□ for 2-3 d (according to the growth condition of thalli). After being co-cultured for three days, the cotyledon leaves are transferred onto a screening culture medium (Ms + 6-BA 1 mg/L + IAA 0.2 mg/L + 5 mg/L Bialaphos + 500 mg/L carbenicillin). The culture medium is changed every three days until no agrobacterium presents. Calluses or regenerated cluster buds can be seen near some incisions after 14 days, and then the regenerated buds are separated into single plants as far as possible, and continuously screened under the pressure of 5 mg/L Bialaphos. After about 4 weeks, the single plants are transferred into a differentiation culture medium containing 5 mg/L Bialaphos and 500 mg/L carbenicillin, and screened for about 1 month. The obtained resistant buds (clusters) are further screened. During the screening, the carbenicillin is always kept at 500 mg/L. In about two months, the regenerated buds grow to 2-3 cm, and in the mean time, the survival seedlings can be transferred into a rooting culture medium (1/2MS, IBA lmg/L, sucrose 30g/L), and subjected to differentiation culture for 1-2 weeks. When the seedlings take roots, the carbenicillin is removed. Finally the plants with well-developed root systems are transplanted in potted soil. In the transgenic experiment, 22 and 21 resistant plants with in-situ replaced *SlICE1(DC)1* and *SlICE(DC)2* genes are respectively obtained. Compared with the wild-type plant, the cold resistance of the obtained resistant plants is respectively improved by 4.1□ and 2.4□.

### Embodiment 55 Obtaining Prunus persica by insitu replacing PpICE1 gene of different degrees of methylation through a CRISPR/Cas9 technology

The *PpICE1* gene cDNA of Prunus persica is cloned and has a sequence of SEQ ID NO: 20. According to degenerate features of a codon, sites that can be theoretically methylated on the *PpICE1* gene are replaced. According to requirements on target traits, the sites of different degrees of methylation on the *PpICE1* gene are quantitatively replaced. The *PpICE1* gene on which all methylation sites are replaced has a sequence of SEQ ID NO: 61 and is named *PpICE1(DC)1;* and the *PpICE1* gene on which part of methylation sites are replaced has a sequence of SEQ ID NO: 102 and is named *PpICE1(DC)2.* The *PpICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GTCAAGATTGTTGAAAACCGAGG-3', 5'-GGCAAAGTTCTTCCTTGATACGG-3' and 5'-GTACTGCTTTTATCTGATCCGGG-3'. The sgRNA sequence of the *PpICE1(DC)2* gene is 5'-GTCAAGATTGTTGAAAACCGAGG-3'. The Cas9 protein is guided to perform site-specific cleavage for DNA. The YAO promoter is selected. The vector pHEE401E and *SlICE1(DC)1* and *SlcICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401Eand used as a resistant screening gene for constructing a CRISPR/Cas9 expression vector.

Fruits of Prunus persica in normal shape and uniform size and without damage or worms are sequentially scrubbed with detergent water, washed with tap water, immersed in 0.2% mercury bichloride for 10 min, taken out, rinsed with sterile water for 5 times, and peeled under a sterile condition. Kernels are cut apart, and young embryos are taken out. The young embryos are inoculated onto a callus inducing culture medium (MS + 2,4-D 0.5 mg/L + BA 0.75 mg/L + NAA 1.0 mg/L + 2% sucrose + 7. 5 g/L agar, pH 5.8), and subjected to dark culture at 26□ for inducing calluses. Individual colonies of agrobacterium tumefaciens GV3101 containing the plasmids are picked, inoculated into 4 ml of YEP liquid culture medium containing 5 mg/L Bialaphos, Rif 20 mg/L and Gem 40 mg/L, and cultured in dark at 180 rpm and 28□ for 36 h. 1 ml of the above bacterial solution is added with 50 ml of fresh YEP liquid culture medium and 200 umol/L acetosyringone, and the mixture is cultured for 12 h under the same conditions. Then 40 ml of the bacterial solution is collected and centrifuged at 5000 rpm and 4□ for 10 min. The precipitates are added into liquid MS, and the mixture is continuously subjected to shaking culture in dark at 180 rpm and 28□ for 2 h until a logarithmic phase for standby use. The pre-cultured explant is directly immersed in an agrobacterium suspension and then taken out, surplus bacterial solution is blotted upby using sterile filter paper, and the pre-cultured explant is co-cultured in dark at 26□ on the culture medium (MS + 2,4-D 0.5 mg/L + BA 0.75 mg/L + NAA 1. 0 mg/L + 2% sucrose + 7. 5g/L agar, pH 5.8). The induced calluses are inoculated into a differentiation culture medium (MS + NAA 0.05 mg/L + BA 1.0 mg/L), and cultured under a cycle of 16-h light/8-h dark and illumination intensity of 2000-3000 lx at the same temperature above. Thereafter, adventitious buds are transferred onto a rooting culture medium (1/2 MS+IBA 1.0 mg/L), subjected to rooting culture, and take roots after 5 days. In transgenetic experiment of Prunus persica, 11 and 9 resistant plants with in-situ replaced *PpICE1(DC)1* and *PpICE(DC)2* genes are respectively obtained. Compared with the wild-type plant of Prunus persica, the cold resistance of the obtained resistant plants is respectively improved by 4.0□ and 2.1□.

### Embodiment 56 Obtaining Zea mays by insitu replacing ZmICE1 gene of different degrees of methylation through a CRISPR/Cas9 technology

The *ZmICE1* gene cDNA of Zea mays is cloned and has a sequence of SEQ ID NO: 21. According to degenerate features of a codon, sites that can be theoretically methylated on the *ZmICE1* gene are replaced. According to requirements on target traits, the sites of different degrees of methylation on the ZmICE1 gene are quantitatively replaced. The *ZmICE1* gene on which all methylation sites are replaced has a sequence of SEQ ID NO: 62 and is named *ZmICE1(DC)1;* and the *ZmICE1* gene on which part of methylation sites are replaced has a sequence of SEQ ID NO: 103 and is named *ZmICE1(DC)2.* The *ZmICE1(DC)1* is divided into 3 segments for CRISPR/Cas9 vector construction and in-situ replacement, and each segment is about 400 bp. The sgRNA with a guiding function is artificially designed, and has a length of 20 nt and sequences respectively shown as 5'-GGGGCTACCTGGGCTCCGACGGG-3', 5'-GAAGATCAGCAAGATGGATAGGG-3' and 5'-GCTGAGTGCGCGGATGGTCCTGG-3'. The sgRNA sequence of the *ZmICE1(DC)2* gene is 5'-GTGGCCGCGGCCGCGGAGGAGGG-3'. The Cas9 protein is guided to perform site-specific cleavage for DNA. The YAO promoter is selected. The vector pHEE401E and *ZmICE1(DC)1* and *ZmICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Under a greenhouse condition, a receptor material is seeded at different stages by taking 7 days as a stage. A tested material of Zea mays is subjected to controlled pollination according to the test requirements. Young embryos of Zea mays are collected as a transformation receptor 10-13 d after the pollination. After cobs are disinfected by using sodium hypochlorite, young embryos are taken out by stripping (the young embryos shall be carefully taken out by stripping and damage to the young embryos should be avoided), washed with an infection solution (added with AS) for 4 times, added into agrobacterium bacterial solution at a given concentration, kept still for 10-30 min, taken out, then dried by using sterilized filter paper, placed on a co-culture medium, co-cultured in dark at 25□ for 2-5 d, then transferred onto a tranquillization culture medium, and cultured in dark at 28□ for 7 d. In the infection and co-culture steps, the concentration OD500 of the bacterial solution is 0.3-0.5. The infection time is 10 min. The co-culture time is 3 d. The young embryos are transferred from the tranquillization medium into a resistant screening medium containing a marker gene, and cultured in dark. The browning calluses and water-soaking calluses shall be discarded during the subculture at each time. The calluses growing normally are broken with tweezers and separately subjected to selection culture. During the subculture screening, frequent observation is required, the contaminated material or material with re-appearance of agrobacterium shall be moved out immediately, while the uncontaminated material is continuously cultured. Furthermore, the tissue blocks having browning contamination are frequently picked out or the good or uncontaminated tissue blocks are transferred onto a similar new culture medium. After the tissue blocks are swelled, the large tissue blocks shall be broken into small pieces frequently. After the subculture screening is continuously carried out for 3 times, the selected resistant calluses are transferred onto a regeneration culture medium, and subjected to recovery culture for 15 d or for a longer time (dark culture). The resistant calluses are transferred onto the regeneration culture medium, germinated, and cultured in light at 28□ under an illumination intensity of 3000 lx for 12 h each day. When the regenerated plant grows and has 3 leaves, the seedlings can be transplanted into a flask containing a rooting culture medium, and cultured indoor. When the seedlings send forth strong roots, the seedlings are taken out from a can flask, rinsed with water for removing the culture medium, and transplanted into a small flowerpot containing a mixture of nutrition soil and vermiculite (1: 3). When the seedlings of Zea mays send forth another 2 to 3 new leaves, the seedlings of Zea mays can be transferred into a farmland or a large flowerpot. After three to four leaves emerge, leaf DNA can be extracted for PCR detection. The containing of the introduced *ZmICE1* gene is determined. In the present experiment, 14 and 12 resistant plants with in-situ replaced *ZmICE1(DC)1* and *ZmICE(DC)2* genes are respectively obtained. Under a low-temperature treatment condition of 6□, compared with the wild-type plant, the cold resistance of the obtained resistant plants is respectively improved by 3.3□ and 1.8□.

### Embodiment 57 Obtaining Gossypium hirsutum by insitu replacing GhICE1 gene of different degrees of methylation through a CRISPR/Cas9 technology

The *GhICE1* gene cDNA of Gossypium hirsutum is cloned and has a sequence of SEQ ID NO: 22. According to degenerate features of a codon, sites that can be theoretically methylated on the *GhICE1* gene are replaced. According to requirements on target traits, the sites of different degrees of methylation on the *GhICE1* gene are quantitatively replaced. The *GhICE1* gene on which all methylation sites are replaced has a sequence of SEQ ID NO: 63 and is named *GhICE1(DC)1;* and the *GhICE1* gene on which part of methylation sites are replaced has a sequence of SEQ ID NO: 104 and is named GhICE1(DC)2. The GhICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GTCAAGATGGTTGAAAACGGAGG-3', 5'-GCCCTTTCCTTTTACCCTTTTGG-3' and 5'-GTAAACATCCACATGTTCTGTGG-3'. The sgRNA sequence of the GhICE1(DC)2 gene is 5'-GTCAAGATGGTTGAAAACGGAGG-3'. The Cas9 protein is guided to perform site-specific cleavage for DNA. The YAO promoter is selected. The vector pHEE401E and GhlICE1(DC)1 and GhICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The constructed plant expression vector agrobacterium bacterial solution is collected, and a great number of plant expression vector plasmids DNA are extracted. Flower buds that will be blossomed on the next day are selected for selfing. Since the Gossypium hirsutum is an often cross-pollinated plant, and the natural cross-pollination rate is about 10%, the foreign pollen often leads to the hybridization of different varieties. On the day before the blossoming, corolla may be elongated rapidly. Yellow or milky white corolla protrudes out of the flower bud like a finger and will be blossomed into flowers on the next day. The flower buds are selected and tied with a thread at the front end of the finger-shaped corolla, and the other end of the thread is tied to a boll handle, and used as a marker when in harvesting. In about 20-24 h after the blossoming, i.e. the next day, young ovaries with good fruit branches and flower position are selected as a transformation object. Generally the flowers on a first and second fruit nodes of each fruit branch are selected for the transgenic manipulation. The cotton bolls on these fruit nodes generally have high boll formation rate, so that more seeds can be harvested. 50 mL of microsyringe is used as a tool for microinjection. The microsyringe shall be cleaned with light detergent before and after the use at each time and rinsed with distilled water. During the injection, petals are removed or stripped, and styles are smoothened. When the petals are removed, an epidermal layer of the ovary shall not be damaged so as to avoid the increase of falling rate. The microsyringe is held by a right hand, the young ovary with the petals removed is gently supported by a left hand, and a needle is inserted from a style smoothened position to about two thirds of the length of the ovary along the longitudinal axis direction of the ovary and withdrawn to about one third of the length of the ovary. The cotton seeds obtained by the transformation of a pollen tube pathway method are harvested, and thereafter, seedlings are planted in pots in a greenhouse. At the stage of 2-3 leaves, the cotton leaves are sprayed with 5 mg/L Bialaphos. The growth condition of the plant is observed after one week, and the plant with chlorotic spots on the leaf surfaces is removed. The plants in normal growth are considered as the plants acquiring the resistant genes. In the present experiment, 24 and 25 resistant plants with in-situ replaced *GhICE1(DC)1* and *GhICE(DC)2* genes are respectively obtained. Under a low-temperature treatment condition of 4□, compared with the wild-type plant of Gossypium hirsutum, the cold resistance of the obtained resistant plants is respectively improved by 4.6□ and 2.8□.

### Embodiment 58 Obtaining Arachis hypogaea by insitu replacing AhICE1 gene of different degrees of methylation through a CRISPR/Cas9 technology

The *AhICE1* gene cDNA of Arachis hypogaea is cloned and has a sequence of SEQ ID NO: 23. According to degenerate features of a codon, sites that can be theoretically methylated in the *AhICE1* gene are replaced. According to requirements on target traits, the sites of different degrees of methylation on the *AhICE1* gene are quantitatively replaced. The *AhICE1* gene on which all methylation sites are replaced has a sequence of SEQ ID NO: 64 and is named *AhICE1(DC)1;* and the *AhICE1* gene on which part of methylation sites are replaced has a sequence of SEQ ID NO: 105 and is named *AhICE1(DC)2.* The sgRNA with a guiding function is artificially designed and has a length of 20 nt. The sgRNA sequences of the *AhICE1(DC)1* and *AhICE2(DC)* genes are both 5'-GCCGTCCAAAAATCTTATGGCGG-3'. The Cas9 protein is guided to perform site-specific cleavage for DNA. The YAO promoter is selected. The vector pHEE401E and GhICE1(DC)1 and GhICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

When plants of Arachis hypogaea enter a full-blossom stage, transgenosis is carried out. Prior to entering the full-blossom stage, flower buds are picked off at about 5:00 in the afternoon every day to prevent the pollination and fruitage of the flowers not subjected to the transgenosis from interfering the detection of the transgenic plants in offspring in next season. A micropipettor and a glass needle formed by drawing a dropper are respectively used to introduce plasmids DNA containing target genes by adopting a stigma smearing method and a flower tube injection method. Two DNA concentrations, 50 ug/mL and 100 ug/mL, are selected for treating two varieties, i.e. "Shanyou523" and "Shanyou21". After the plant of Arachis hypogaea enters the full-blossom stage, the manipulation begins at about 6: 30 in the morning every day. For the smearing manipulation, the micropipettor is used to suck 5 uL of DNA solution, and the DNA solution is smeared onto a flower stigma. For the injection method, the glass needle is used to inject about 5 uL of DNA solution on the lower portion of a calyx tube in a direction towards the varies. More than 500 flowers are treated for each variety. The seeds of Arachis hypogaea obtained by genetic transformation are harvested, and then seedlings are planted in pots in a greenhouse. At the stage of 2-3 leaves, the leaves of Arachis hypogaea are sprayed with 5 mg/L Bialaphos. The growth condition of the plant is observed after one week, and the plant with chlorotic spots on leaf surfaces is removed. The plants in normal growth are considered as the plants acquiring the resistant genes, and the resistant plants are used for PCR verification. By means of genetic transformation, 12 and 15 resistant plants with in-situ replaced *AhICE1(DC)1* and *AhICE(DC)2* genes are respectively obtained. Compared with the wild-type plant of Arachis hypogaea, the cold resistance of the obtained resistant plants is respectively improved by 2.0□ and 1.3□.

### Embodiment 59 Obtaining Brassica juncea by insitu replacing BjICE1 gene of different degrees of methylation through a CRISPR/Cas9 technology

The *BjICE1* gene cDNA of Brassica juncea is cloned and has a sequence of SEQ ID NO: 24. According to degenerate features of a codon, sites that can be theoretically methylated on the *BjICE1* gene are replaced. According to requirements on target traits, the sites of different degrees of methylation on the *BjICE1* gene are quantitatively replaced. The *BjICE1* gene on which all methylation sites are replaced has a sequence of SEQ ID NO: 65 and is named *BjICE1(DC)1;* and the *BjICE1* gene on which part of methylation sites are replaced has a sequence of SEQ ID NO: 106 and is named *BjICE1(DC)2.* The *BjICE1(DC)1* is divided into 2 segments for CRISPR/Cas9 vector construction and in-situ replacement, and each segment is about 500 bp. The sgRNA with a guiding function is artificially designed, and has a length of 20 nt and sequences respectively shown as 5'-GTTCGGTTCGTTGACGCAGCTGG-3' and 5'-GTTCAGAACGGAGGAGGTAAAGG-3'. The sgRNA sequence of the *BjICE1(DC)2* gene is 5'-GCTCCGGTTTCGATGGGGTTCGG-3'. The Cas9 protein is guided to perform site-specific cleavage for DNA. The YAO promoter is selected. The vector pHEE401E and *BjICE1(DC)1* and *BjICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Seeds of Brassica juncea are disinfected (75% alcohol and 0.1% sodium hypochlorite), and sowed in an MS culture medium. After 7 d, hypocotyl (0.5-0.8 cm) is cut, and placed on a Brassica juncea differentiation culture medium (MS + 2 mg/L 6-BA+ 0.2 mg/L NAA+ 3% sucrose + 0.6% agar), and pre-cultured for 2 d. The agrobacterium introduced with plasmids containing the *BjICE1* gene is activated. When the infection is carried out, 500 uL of activated bacterial solution is collected, inoculated into 50 ml of antibiotics-free YEB liquid culture medium, subjected to shaking culture at 225 rpm and 28□ until the growth vigorous period of the agrobacterium (an OD600 value is about 0.5-0.6) on a table concentrator, centrifuged at 4□ (4000 r/min, 10 min) to remove supernatant, and re-suspended in 20 ml of precooled MS liquid culture medium for standby use. The pre-cultured hypocotyl is infected by re-suspension bacterial solution for 10 min, dried on filter paper, then transferred onto a differentiation culture medium, and co-cultured for 2 d. The co-cultured hypocotyl is transferred onto a Brassica juncea screening culture medium (MS + 2 mg/L 6-BA + 0.2 mg/L NAA + 5 mg/L Bialaphos + 400 mg/L Cb + 3% sucrose + 0.6% agar), and subjected to resistant screening, and the screening culture medium is changed every 10 d. The resistant buds obtained by multi-time screening are transferred onto a Brassica juncea rooting culture medium (MS + 2 mg/L 6-BA + 0.4 mg/L NAA + 5 mg/L Bialaphos + 400 mg/L Cb + 3% sucrose + 0.6% agar) for rooting culture. After tissue cultured seedlings have strong root systems, the root systems are cleaned for removing residual culture medium, transplanted to nutritional soil, and cultured in an phytotron (temperature: 25+/-2□; illumination intensity: 2000 lx; relative humidity: 70%; light/dark=16/8 h). By means of genetic transformation, 24 and 21 resistant plants with in-situ replaced *BjICE1(DC)1* and *BjICE(DC)2* genes are respectively obtained. Compared with the wild-type plant of Brassica juncea, the cold resistance of the obtained resistant plants is respectively improved by 4.6□ and 2.7□.

### Embodiment 60 Obtaining Brassica rapaby insitu replacing BrICE1 gene of different degrees of methylation through a CRISPR/Cas9 technology

The *BrICE1* gene cDNA of Brassica rapa is cloned and has a sequence of SEQ ID NO: 25. According to degenerate features of a codon, sites that can be theoretically methylated on the BrICE1 gene are replaced. According to requirements on target traits, the sites of different degrees of methylation on the *BrICE1* gene are quantitatively replaced. The *BrICE1* gene on which all methylation sites are replaced has a sequence of SEQ ID NO: 66 and is named *BrICE1(DC)1;* and the *BrICE1* gene on which part of methylation sites are replaced has a sequence of SEQ ID NO: 107 and is named *BrICE1(DC)2.* The *BrICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GTTCGGTTCGTTGACGCAGCTGG-3', 5'--GACTCTTTCTTGCCGTGTCAAGG-3' and 5'-GCCGTCATCAGCTGTTTCAACGG-3'. The sgRNA sequence of the *BrICE1(DC)2* gene is 5'-GTCGAGGCTAAAAGCCTGAGAGG-3'. The Cas9 protein is guided to perform site-specific cleavage for DNA. The YAO promoter is selected. The vector pHEE401E and *BrICE1(DC)1* and *BrICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to the enzyme digestion by SacII and AsiSI, and the Bar gene is bonded into pHEE401E, and used as a resistant screening gene for constructing a CRISPR/Cas9 expression vector.

The transgenic experiment for Brassica rapa is carried out by a pollen-mediated method. When a plant of Brassica rapa enters a full-blossom stage, 10-15 flower buds that will be blossomed in 1-2 d on a main stem or a first branch are emasculated by hands, other flower buds on the selected stem or branch are removed, and the flower buds are bagged. Inflorescences that will be blossomed on the next day of the same variety are selected and bagged for pollen collection on the next day. About 0.4 g of pollen collected from the bagged plants that are blossomed in the very morning of the next day is suspended in 25 ml of 7.5% sucrose solution, ultrasonically treated for the first time, and added with 20 ug of plasmid DNA containing different cold-resistant population *BrICE1* genes for second ultrasonic treatment. After the second treatment, 10 uL of 1/10 000 (W/V) boric acid is added into the solution. The treated pollen is given to stigmas of Brassica rapa that are emasculated the day before, then the pollinated plants of Brassica rapa are bagged and hung with plates marked with the number of pollinated flower buds. The bags are removed 5-6 d after the pollination, so that the treated pollinated plants can develop sufficiently. Seeds are harvested. After the harvested seeds are planted, when two cotyledons develop, the cotyledons are sprayed with 5 mg/L Bialaphos solution. After the plants are cultured in light for 7 d, the leaf edges of the non-transgenic plants turn yellow, while the transgenic plants grow normally. By means of genetic transformation, 33 and 37 resistant plants with in-situ replaced *BrICE1(DC)1* and *BrICE(DC)2* genes are respectively obtained. Compared with the wild-type plant of Brassica rapa, the cold resistance of the obtained resistant plants is respectively improved by 4.4□ and 2.7□.

### Embodiment 61 Obtaining Chrysanthemum dichrum by insitu replacing CdICE1 gene of different degrees of methylation through a CRISPR/Cas9 technology

The *CdICE1* gene cDNA of Chrysanthemum dichrum is cloned and has a sequence of SEQ ID NO: 26. According to degenerate features of a codon, sites that can be theoretically methylated on the *CdICE1* gene are replaced. According to requirements on target traits, the sites of different degrees of methylation on the *CdICE1* gene are quantitatively replaced. The *CdICE1* gene on which all methylation sites are replaced has a sequence of SEQ ID NO: 67 and is named *CdICE1(DC)1;* and the *CdICE1* gene on which part of methylation sites are replaced has a sequence of SEQ ID NO: 108 and is named *CdICE1(DC)2.* The *CdICE1(DC)1* is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GGGTTGTTGGAAATGAAGCTAGG-3', 5'-GTTGGTGGTGTAGGCGTCAATGG-3' and 5'-GCAGTGCTTCTAGAGACTGCAGG-3'. The sgRNA sequence of the *CdICE1(DC)2* gene is 5'-GTAACTAGTCTCCGTATACTCGG-3'. The Cas9 protein is guided to perform site-specific cleavage for DNA. The YAO promoter is selected. The vector pHEE401E and *CdICE1(DC)1* and *CdICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The stored agrobacterium carrying pCAMBIA1301 plasmids of the target gene is collected, inoculated into a YEB liquid culture medium containing 50 mg/L Rifampicin, and cultured overnight. Thereafter, the bacterial solution is subjected to streaking inoculation onto YEB plate culture media containing 5 mg/L Bialaphos and 50 mg/L Rifampicin, the plate is invertedly placed in a constant-temperature incubator at 28□, and culture is carried out until individual colonies come up. The plate is stored in a refrigerator at 4□ for standby use. The agrobacterium individual colonies are respectively picked from the YEB plate culture medium, inoculated into 20 ml of YEB liquid culture medium containing 5 mg/L Bialaphos and 50 mg/L rifampicin, and subjected to shaking culture at 180 rpm and 28□ until a logarithmic phase. After two-times activation, the bacterial solution is inoculated into the YEB liquid culture medium only containing 100 pmol/L RS in a ratio of 1: 100. After the bacterial solution is subjected to shaking culture for 4-6 h, when an OD600 value of the bacterial solution is about 0.4-0.6, the bacterial solution is used for genetic transformation. Leaves on the upper end of the cultured aseptic plant of Chrysanthemum dichrum are collected as explants, and cut into pieces of about 8mm^{∗}8mm by using sterilized scissors, the pieces are placed into an embryo callus inducing culture medium (Ms + 0.5 mg/L 6-BA + 1.5 mg/L NAA + sucrose 30 g/L + agar powder 5 g/L) for pre-culturing. After the pieces are pre-cultured for 3 d, the bacterial solution for infection is poured into a sterile triangular flask. The pre-cultured leaves are rinsed with sterile water for 1-2 times, dried by using sterile filter paper, and directly infected in the triangular flask for 25 min. The inflected leaves are taken out, and the excessive bacterial solution on the infected leaves are blotted up by using the sterile filter paper. The infected leaves are inoculated onto the co-culture medium, and co-cultured at 25+/-2□ for 2 days. When colonies come up on the peripheries of the leaves, the leaves are taken out, rinsed with sterile water for 2-3 times, and dried by using sterile filter paper. The leaves are transferred into an embryonic callus-induced screening culture medium (added with 300 mg/L Cef), and cultured until the resistant embryonic somatic embryos are completely differentiated. After the leaves are cultured on the screening differentiation medium for 15-20 d, a minority of chrysanthemum leaves begin to be differentiated at incisions. When the regenerated plants in the culture medium grow to about 2 cm, the regenerated plants are stripped from a chrysanthemum parental body by using sterile scissors, transferred into the MS basic culture medium with no plant growth hormones for rooting, and cultured at 25□ in a light cycle of 16-h/8-h, and under the illumination intensity of 1200-2400 lx. Roots begin to be germinated from a root base on the sixth day. By means of genetic transformation, 8 and 10 resistant plants with in-situ replaced *CdICE1(DC)1* and *CdICE(DC)2* genes are respectively obtained. Compared with the wild-type plant of Chrysanthemum dichrum, the cold resistance of the obtained resistant plants is respectively improved by 5.0□ and 2.6□.

### Embodiment 62 Obtaining Thellungiella halophila by insitu replacing ThICE1 gene of different degrees of methylation through a CRISPR/Cas9 technology

The *ThICE1* gene cDNA of Thellungiella halophila is cloned and has a sequence of SEQ ID NO: 27. According to degenerate features of a codon, sites that can be theoretically methylated on the *ThICE1* gene are replaced. According to requirements on target traits, the sites of different degrees of methylation on the *ThICE1* gene are quantitatively replaced. The *ThICE1* gene on which all methylation sites are replaced has a sequence of SEQ ID NO: 68 and is named *ThICE1(DC)1;* and the *ThICE1* gene on which part of methylation sites are replaced has a sequence of SEQ ID NO: 109 and is named *ThICE1(DC)2.* The *ThICE1(DC)1* is divided into 2 segments for CRISPR/Cas9 vector construction and in-situ replacement, and each segment is about 500 bp. The sgRNA with a guiding function is artificially designed, and has a length of 20 nt and sequences respectively shown as 5'-GCTAGAAACGAAGGCTGAGA AGG-3' and 5'-GGAGGAAGAAGCTTAATGAT AGG-3'. The sgRNA sequence of the *ThICE1(DC)2* gene is 5'-GGGTTTGGGAGTCCTGCAAATGG-3'. The Cas9 protein is guided to perform site-specific cleavage for DNA. The YAO promoter is selected. The vector pHEE401E and *ThICE1(DC)1* and *ThICE1(DC)2* genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Seeds of Thellungiella halophila are placed in a test tube, immersed in sterile water, and placed in a refrigerator at 4□ for one month. The seeds of Thellungiella halophila are uniformly dot sowed in a culture medium (nutrition soil: vermiculite: perlite=4: 3: 1). Then the seeds are placed in a culture room at 25□ in light for 16 h each day under illumination intensity of 2000 lx and the relative humidity of 70% for one month. Petioles are cut off, cleaned with clean water, disinfected with 70% ethanol, disinfected with 2% (V/V) sodium hypochlorite for 10 min, rinsed with sterile water for 4 times, and then used as explants. The explants are inoculated onto a pre-culture medium (MS + 2.5 mg/L 6-BA + 0.1 mg/L NAA (PH 5.8)), and cultured in dark at 22□ under the relative humidity of 70% for 3 d. Individual colonies are picked from a plate, inoculated into 5 ml of LB liquid culture medium containing 5 mg/L Bialaphos, and subjected to shaking culture at 180 rpm and 28□ until an OD600 value of the bacterial solution is 0.6. The bacterial solution of which the OD600 value is 0.6 is transferred into a liquid culture medium according to a ratio of 1%, cultured at 180 rpm and 28□ until the OD600 value of the bacterial solution is 0.3, and the bacterial solution can be used for transformation. The explants are immersed in the bacterial solution for 5 min, and then placed on the sterile filter paper for removing the bacterial solution attached on the explants. The explants are inoculated onto a co-culture medium (MS + 2.5 mg/L 6-BA + 0.1 mg/L NAA (PH 5.8)), and cultured in dark at 25□. The co-cultured explants is transferred onto a selection culture medium (MS + 2.5 mg/L 6-BA + 0.1 mg/L NAA + 5 mg/L Bialaphos + 300 mg/L Cef(PH5.8)), and cultured for 2 weeks. After being subjected to selection culture for 4 d, the explants are rinsed with sterile water every 3 d, and a fresh selection culture medium is changed. After the explants are subjected to the selection cultured for 2 weeks, resistant calluses generated by the transformation cells of the explants are transferred onto the selection culture medium for subculture propagation culture. When adventitious buds grow to 1 cm, the adventitious buds are cut off, inoculated onto a rooting culture medium (1/2MS + 5 mg/L Bialaphos + 300 mg/L Cef(PH5.8)), and adventitious roots come up after 15 d. In the transgenic experiment, 13 and 12 resistant plants with in-situ replaced ThICE1(DC)1 and ThICE(DC)2 genes are respectively obtained. Compared with the wild-type plant of Thellungiella halophila, the cold resistance of the obtained resistant plants is respectively improved by 3.2□ and 1.7□.

### Embodiment 63 Obtaining Daucus carota by insitu replacing DcICE1 gene of different degrees of methylation through a CRISPR/Cas9 technology

The DcICE1 gene cDNA of Daucus carota is cloned and has a sequence of SEQ ID NO: 28. According to degenerate features of a codon, sites that can be theoretically methylated on the DcICE1 gene are replaced. According to requirements on target traits, the sites of different degrees of methylation on the DcICE1 gene are quantitatively replaced. The DcICE1 gene on which all methylation sites are replaced has a sequence of SEQ ID NO: 69 and is named DcICE1(DC)1; and the DcICE1 gene on which part of methylation sites are replaced has a sequence of SEQ ID NO: 110 and is named DcICE1(DC)2. The DcICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GGTTCTCTAATAATTATCTTGGG-3', 5'-GTCTCTTACTTTCCACCATGAGG-3'. The sgRNA sequence of the DcICE1(DC)2 gene is 5'-GGTTGGTGTCGGGTTTAACCGGG-3'. The Cas9 protein is guided to perform site-specific cleavage for DNA. The YAO promoter is selected. The vector pHEE401E and DcICE1(DC)1 and DcICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Individual colonies of agrobacterium tumefaciens engineering bacteria are picked, inoculated into 50 ml of LB liquid culture medium containing 5 mg/L Bialaphos, and subjected to shaking culture in dark at 200 rpm and 28□ until OD600 of the bacterial solution is about 0.6. The agrobacterium tumefaciens culture solution is transferred into a 50 ml sterile centrifugal tube on a super clean bench, and centrifuged at 800 rpm and 4□ for 5 min to remove supernatant, and thalli are suspended in an equal volume of sterile MS liquid culture medium for standby use. A culture dish sterilized at high pressure is paved with 2-3 pieces of sterile filter paper wetted by adding an appropriate amount of B5 liquid culture medium. Aseptic seedlings are placed on the filter paper. Hypocotyl is cut into segments in a length of 0.5 cm by using aseptic and sharp scalpel for genetic transformation. The treated hypocotyl of the aseptic seedling of Daucus carota is placed into a prepared infection solution on a super clean bench for 15-20 min, and gently shaken in the meanwhile to ensure that each hypocotyl segment can be sufficiently infected. The explants are taken out, and surplus liquid is blotted up by using sterilized filter paper. The explants are placed on a co-culture medium, and cultured in dark at 26□ for 2-3 d. After being co-cultured, the explants are taken out, placed into an empty sterile triangular flask, rinsed with sterile water for 2-3 times, rinsed with sterile water containing 500 mg/L Carb for 1-2 times, dried by using sterile filter paper, transferred, inoculated onto B5 bacteriostatic culture medium at a given concentration of antibiotics, subjected to callus culture in light at 26□ for 16 h each day, subcultured for 2-3 times, and transferred onto the differentiation culture medium after the calluses come up, and embryoids and buds are induced. After the induced buds grow for about 40 d, and when the seedling grow to 2-3 m, the seedlings are cut off, and transferred onto the rooting medium for inducing rooting. When normal roots and 3 to 4 pieces of euphylla come up, the resistant seedlings are moved out, rinsed with tap water for removing culture medium on the roots, planted in a nutritional pot with vermiculite and peat (2:1), covered with a plastic film, cultured at 26□ in light for 16 h each day, a vent port of plastic cloth is opened after 7 d for appropriate seedling hardening, conventional management is carried out in a greenhouse when the resistant seedlings are gradually adaptive to the external growth environment. In the transgenic experiment, 7 and 10 resistant plants with in-situ replaced DcICE1(DC)1 and DcICE(DC)2 genes are respectively obtained. Compared with the wild-type plant of Daucus carota, the cold resistance of the obtained resistant plants is respectively improved by 3.1□ and 2.1□.

### Embodiment 64 Obtaining Glycine max by insitu replacing GmICE1 of different degrees of methylation through a CRISPR/Cas9 technology

The GmICE1 gene cDNA of Glycine max is cloned and has a sequence of SEQ ID NO: 29. According to degenerate features of a codon, sites that can be theoretically methylated on the GmICE1 gene are replaced. According to requirements on target traits, the sites of different degrees of methylation on the GmICE1 gene are quantitatively replaced. The GmICE1 gene on which all methylation sites are replaced has a sequence of SEQ ID NO: 70 and is named GmICE1(DC)1; and the GmICE1 gene on which part of methylation sites are replaced has a sequence of SEQ ID NO: 111 and is named GmICE1(DC)2. The GmICE1(DC)1 is divided into 2 segments for CRISPR/Cas9 vector construction and in-situ replacement, and each segment is about 500 bp. The sgRNA with a guiding function is artificially designed,and has a length of 20 nt and sequences respectively shown as 5'-GAAAAGGGTGGGTTGGGCCCCGG-3' and 5'-GTCTGTCATTGAGCTTCTTCCGG-3'. The sgRNA sequence of the GmICE1(DC)2 gene is 5'-GAGGTGAGAGGGGGAGCAGGAGG-3'. The Cas9 protein is guided to perform site-specific cleavage for DNA. The YAO promoter is selected. The vector pHEE401E and GmICE1(DC)1 and GmICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Disinfected soybean seeds are poured into a sterile plastic jar added with a liquid germination medium (LGM) (sucrose 20 g/L, pH 5.8). The volume of the culture medium is about twice the seed volume so as to completely cover the seeds and to provide sufficient water. After being covered with a lid, the sterile plastic jar is placed in an incubator for 16 h, and darkculture is carried out at 23□ all day long. 40 ml of agrobacterium re-suspension solution is poured into a sterile culture dish. By examining the LGM after the soybean is sowed, the contaminated LGM is discarded. The soybean seed is obliquely cut along a seed embryo direction by using a scalpel, then cut apart along the axis, and peeled. The soybean seed is gently cut for three times at an embryo tip position parallel to the axis and immersed in a culture dish by using the re-suspended agrobacterium bacterial solution for 30 min. After the infection is finished, the explants are transferred into an iron box with sterile filter paper by using sterilized tweezers and placed horizontally with an incision up. LCCM (Bs salt powder 0.321 g/L, sucrose 30 g/L, MES 3.9 g/L, pH 5.4, BAP 1.67 mg/L, GA3 0.25 mg/L, Cys 400 mg/L, DTT 154.2 mg/L, and As 39.24 mg/L) is gently added into the iron box. After being closed, the iron box is placed in an intelligent incubator for 3 days, and dark culture is carried out at 23□ all day long. After being co-cultured for 3 days, the explants are taken out by using sterilized tweezers on a super clean bench, cotyledons are cut off by using a scalpel, and hypocotyl is reserved. The cut explants are placed into a bud inducing culture medium (SIM) (BS salt powder 3.21 g/L, sucrose 30 g/L, MES 0.59 g/L, Noble Agar 8 g/L, BAP 1.67 mg/L, Tic 250 mg/L, Cef 100 mg/L, PH 5.7). Each dish holds 7 explants. The top ends of the explants must be higher than a plane of the culture medium. The hypocotyl part must be inserted into the culture medium. Two disposable plastic culture dishes are overlapped on bottom and sealed with a micro ventilation-type medical adhesive tape. The new culture dish is placed into a phytotron, subjected to recovery culture at 25□ all day long for 14 days in a light/dark ratio of 16 h/8 h each day. The explants with shoots are transferred into SIM in a shoots elongation culture medium (SEM) (MS salt powder 4.43 g/L, sucrose 30 g/L, MES 0.59 g/L, Noble Agar 8 g/L, pH 5.7, Asp 50 mg/L, Glu 50 mg/L, IAA 0.1 mg/L, GA3, 0.5 mg/L, ZR 1 mg/L, Tic 250 mg/L). The explants with no induced cluster buds are discarded. The explants are still cultured in the phytotron at 25□ all day long in a light/dark ratio of 16 h/8 h each day. Fresh SEM is generally changed every two weeks. When the culture medium is changed each time, the dead explant is discarded. The subculture of the present batch can be ended when SEM is conducted for 5 times. During the elongation of the shoots, the shoots are cut off from the explants and placed into a culture dish, the bottom of the shoots is immersed with IBA (1 mg/ml) for 30 s, then the shoots are transferred into a rooting culture medium (RM) (MS salt powder 4.43 g/L, sucrose 20 g/L, MES 0.59 g/L, Phytagel 3 g/L, pH 5.6, Asp 50 mg/L, L-Glu 50 mg/L, Tic 125 mg/L, Cef 50 mg/L, IBA 1 mg/L), and continuously cultured. After 1-2 weeks, when a given number of roots come up from the bottom of the seedling, the seedling is taken out from the RM culture medium. After the roots of the seedlings are washed with tap water, the seedlings are transplanted into soil. After the seedlings are transplanted into the soil, the seedlings need to grow for about one week in the phytotron to adapt to the natural environment, and are then transferred into a greenhouse, and cultured. In the transgenic experiment, 11 and 9 resistant plants with in-situ replaced GmICE1(DC)1 and GmICE(DC)2 genes are respectively obtained. Compared with the wild-type plant of Glycine max, the cold resistance of the obtained resistant plants is respectively improved by 4.2□ and 3.0□.

### Embodiment 65 Obtaining Raphanus sativus by insitu replacing RsICE1 gene of different degrees of methylation through a CRISPR/Cas9 technology

The RsICE1 gene cDNA of Raphanus sativus is cloned and has a sequence of SEQ ID NO: 30. According to degenerate features of a codon, sites that can be theoretically methylated on the RsICE1 gene are replaced. According to requirements on target traits, the sites of different degrees of methylation on the RsICE1 gene are quantitatively replaced. The RsICE1 gene on which all methylation sites are replaced has a sequence of SEQ ID NO: 71 and is named RsICE1(DC)1; and the RsICE1 gene on which part of methylation sites are replaced has a sequence of SEQ ID NO: 112 and is named RsICE1(DC)2. The RsICE1(DC)1 is divided into 3 segments for CRISPR/Cas9 vector construction and in-situ replacement, and each segment is about 400 bp. The sgRNA with a guiding function is artificially designed, and has a length of 20 nt and sequences respectively shown as 5'-GATTTCAGATTCCTCGGTGGTGG -3', 5'-GGAGGAAGAAGCTTAATGATAGG-3' and 5'-GCTGGCTACCATGAAAGCTTTGG-3'. The sgRNA sequence of the RsICE1(DC)2 gene is 5'-GATTTCAGATTCCTCGGTGGAGG-3'. The Cas9 protein is guided to perform site-specific cleavage for DNA. The YAO promoter is selected. The vector pHEE401E and RsICE1(DC)1 and RsICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E, and used as a resistant screening gene for constructing a CRISPR/Cas9 expression vector.

The cotyledons-with-petiole explants of 4-day-old aseptic seedlings of Raphanus sativus material are cut first, pre-cultured for 2 days, infected by using EHA105 agrobacterium bacterial solution of which OD600 is 0.3-0.6 for 5-7 min, co-cultured for 5 days, transferred and inoculated onto a culture medium (MS + 6-BA 6 mg·L⁻¹ + NAA 0.05 mg·L⁻¹+ Cef 500 mg·L⁻¹), subjected to the delayed screening for 7 d, then transferred and inoculated onto a culture medium (MS + 6-BA 6 mg·L⁻¹ + NAA 0.05 mg·L⁻¹ + 5 mg·L⁻¹ Bialaphos Cef 500 mg·L⁻¹), and subjected to resistant screening for 10 days. The explants are transferred and subcultured every 10 days. By using the agrobacterium-mediated genetic transformation method, 6 and 5 resistant plants with in-situ replaced RsICE1(DC)1 and RsICE1(DC)2 genes are respectively acquired. Compared with the wild-type plant of Raphanus sativus, the cold resistance of the obtained resistant plants is respectively improved by 4.5□ and 2.4□.

### Embodiment 66 Obtaining Triticum aestivum by insitu replacing TaICE1 gene of different degrees of methylation through a CRISPR/Cas9 technology

The TaICE1 gene cDNA of Triticum aestivu is cloned and has a sequence of SEQ ID NO: 31. According to degenerate features of a codon, sites that can be theoretically methylated on the TaICE1 gene are replaced. According to requirements on target traits, the sites of different degrees of methylation on the TaICE1 gene are quantitatively replaced. The TaICE1 gene on which all methylation sites are replaced has a sequence of SEQ ID NO: 72 and is named TaICE1(DC)1; and the TaICE1 gene on which part of methylation sites are replaced has a sequence of SEQ ID NO: 113 and is named TaICE1(DC)2. The TaICE1(DC)1 is divided into 3 segments for CRISPR/Cas9 vector construction and in-situ replacement, and each segment is about 400 bp. The sgRNA with a guiding function is artificially designed, and has a length of 20 nt and sequences respectively shown as 5'-GCGTGGACGCCTCCTCCTTGGGG-3', 5'-GCTGCAGGGTGGGCGTCGACGGG-3' and 5'-GCCGTCATCAGCTGCTTCGATGG-3'. The sgRNA sequence of the TaICE1(DC)2 gene is 5'-GTAGGCCGGGGGCGCGGCGGCGG-3'. The Cas9 protein is guided to perform site-specific cleavage for DNA. The YAO promoter is selected. The vector pHEE401E and TaICE1(DC)1 and TaICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Ears of Triticum aestivum on 12-14 d after the flowering are collected. Immature grains that are consistent in size are collected from the middle part of the ears of Triticum aestivum. Embryos are inoculated to the surface of a callus inducing culture medium, cultured in dark at 25□, transferred into the same culture medium after 20 d, and subcultured. The embryos are inoculated onto an MC culture medium, cultured for 15-20 d, placed into a refrigerator at 4□ after the calluses formed by the embryos are completely developed, cold treated for about 2 months, and subcultured at a normal temperature. The calluses are transferred into a culture medium containing MC and acetosyringone (AS) 0.1 mmol/L in the early to middle August, and begin to be transformed after being cultured for 5-7 d. The embryo calluses are transferred into an engineering bacteria infection solution for 0.5-1.0 h, then transferred to an MD culture medium containing AS 0.1 mmol/L, co-cultured for 2 d, and rinsed by usingsterile water containing 0.5 g/L cefazolin sodium, surplus liquid is blotted up by using sterile toilet paper, the calluses are transferred to a culture medium (MD + 500 mg/L Carb + 5 mg/L Bialaphos), and screened. The screened resistant calluses are transferred into an ME differentiation culture medium containing 0.5 g/L cefazolin sodium and 5 mg/L Bialaphos. After being subjected to selection culture in 5 mg/L Bialaphos for 6-8 weeks, the calluses are transferred to a regeneration culture medium (MD + IAA 1 mg/L + ZT 1 mg/L),and regenerated into seedlings. By using the agrobacterium-mediated genetic transformation method, 23 and 25 resistant plants with in-situ replaced TaICE1(DC)1 and TaICE1(DC)2 genes are respectively obtained. Compared with the wild-type plant of Triticum aestivum, the cold resistance of the obtained resistant plants is respectively improved by 4.8□ and 3.3□.

### Embodiment 67 Obtaining Hordeum vulgare by insitu replacing HvICE1 gene of different degrees of methylation through a CRISPR/Cas9 technology

The HvICE1 gene cDNA of Hordeum vulgare is cloned and has a sequence of SEQ ID NO:32. According to degenerate features of a codon, sites that can be theoretically methylated on the HvICE1 gene are replaced. According to requirements on target traits, the sites of different degrees of methylation on the HvICE1 gene are quantitatively replaced. The HvICE1 gene on which all methylation sites are replaced has a sequence of SEQ ID NO: 73 and is named HvICE1(DC)1; and the HvICE1 gene on which part of methylation sites are replaced has a sequence of SEQ ID NO: 114 and is named HvICE1(DC)2. The sgRNA with a guiding function is artificially designed and has a length of 20 nt. The sgRNA sequences of the HvICE(DC)1 gene and the HvICE1(DC)2 gene are both 5'- GATGCCGGCCAAGAACCTGATGG-3'. The Cas9 protein is guided to perform site-specific cleavage for DNA. The YAO promoter is selected. The vector pHEE401E and HvICE1(DC)1 and HvICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E, and used as a resistant screening gene for constructing a CRISPR/Cas9 expression vector.

The size of immature embryos of ears of Hordeum vulgare is examined. The size of the embryo is ensured to be between 1.5 mm and 2 mm generally at the time about 14 days after the pollination. The immature seeds are completely separated from the ears, placed into a conical flask, and placed in a super clean bench. All steps hereinafter are carried out in the super clean bench. The seeds are simply rinsed with 70% alcohol for 30 s, washed with sterile water for three times, treated with 50% sodium hypochlorite for 4 min, and rinsed with sterile water for 3-4 times, and the residue of sodium hypochlorite is reduced as far as possible. The lower part, at about one third position, of the seed is clamped by tweezers so that immature embryos face upward. The middle parts of the seeds are pricked through ultrafine-tip tweezers, and part of the seed coat is raised and is removed so as to expose the immature embryos. The hypocotyl is removed by tweezers as far as possible, and the completeness of scutella is kept. The immature embryos are separated, and placed on a BCI callus inducing culture medium with small scutella up. Each culture dish holds about 25 embryos. The to-be-inoculated bacterial solution is dried a 200 ul gun head and dripped on the immature embryos. The bacterial solution can also be dipped by a blade or an inoculation ring, and used for wetting the surface of the embryo. If the immature embryos have excessive bacterial solution, the immature embryos need to be dragged back and forth on the surface of the culture medium to remove the excessive bacterial solution on the surface. If the immature embryos are just wet, the embryos are directly transferred onto a fresh BCI culture medium with small scutella down and cut surface up. The culture dish is sealed to perform the culture in dark at 24□ for 72 h. If there are immature embryos with excessive growth of agrobacterium, the immature embryos need to be placed into a small beaker, and rinsed with sterile water and sterile water containing 160 mg/L Timentin antibiotics for several times, and the surface liquid is blotted up with filter paper. The immature embryos are transferred onto the BCS callus screening medium (containing 5 mg/L Bialaphos and 160 mg/L Timentin) with scutella down, and cultured in dark at 24□ for 6-8 weeks. The fresh culture medium is changed every 2 weeks. When the calluses are transferred, the calluses formed by differentiating the immature embryos shall not be separated as far as possible. After the calluses grow to a certain size on the selection culture medium, the calluses are transferred to a BCT transition culture medium (containing 5 mg/L Bialaphos and 160 mg/L Timentin). The culture is carried out in low light at 24□. The illumination condition can be achieved by covering the culture dish with a piece of paper. After being subjected to transitional culture for two weeks, the calluses may have the regeneration trend. If no regeneration occurs, the fresh culture medium is changed every two weeks. After having apparent regeneration trend, the calluses are transferred to a BCR callus regeneration culture medium (containing 5 mg/L Bialaphos and 160 mg/L Timentin). The calluses are cultured in full light at 24□. The fresh culture medium is changed every two weeks. A plant culture flask is selected to substitute a glass culture dish so as to increase the regeneration space of the plant. After the calluses grow to a certain size on the selection culture medium, the calluses are transferred to B13M, and cultured in low light at 24□. The culture medium is changed every two weeks. When growing bigger, the regenerated shoots are transferred into a test tube, and cultured. When the shoots develop to 2-3 cm, the young seedlings are carefully transferred into a glass test tube in a length of 15 cm, and cultured. The glass test tube is sealed with a breathable bacteria filter plastic film. The culture medium in the test tube is a rooting culture medium. In the genetic transformation experiment, 9 and 7 resistant plants with in-situ replaced HvICE1(DC)1 and HvICE(DC)2 are respectively acquired. Compared with the wild-type plant of Hordeum vulgare, the cold resistance of the obtained resistant plants is respectively improved by 2.4□ and 1.7□.

### Embodiment 68 Obtaining Hevea brasiliensis by insitu replacing HbICE1 gene of different degrees of methylation through a CRISPR/Cas9 technology

The HbICE1 gene cDNA of Hevea brasiliensis is cloned and has a sequence of SEQ ID NO:33. According to degenerate features of a codon, sites that can be theoretically methylated on the HbICE1 gene are replaced. According to requirements on target traits, the sites of different degrees of methylation on the HbICE1 gene are quantitatively replaced. The HbICE1 gene on which all methylation sites are replaced has a sequence of SEQ ID NO: 74 and is named HbICE1(DC)1; and the HbICE1 gene on which part of methylation sites are replaced has a sequence of SEQ ID NO: 115 and is named HbICE1(DC)2. The HbICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GAATCAGGCTTCAAATTTGATGG-3', 5'-GGATGAATACTTGTAGATTGAGG-3' and 5'-GTTTATTGCTCTCCACTATGAGG-3'. The sgRNA sequence of the HbICE1(DC)2 gene is 5'-GAATCAGGCTTCAAATTTGATGG-3'. The Cas9 protein is guided to perform site-specific cleavage for DNA. The YAO promoter is selected. The vector pHEE401E and HbICE1(DC)1 and HbICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Anthers at a late uninucleate stage of Hevea brasiliensis are inoculated onto an embryonic callus inducing medium, cultured for 30 d, and used for agrobacterium infection. After being infected in EHA105 suspension for 5 min, calluses are transferred onto a co-culture medium containing 5uM of acetosyringone and 10 mg/L silver nitrate, and cultured in dark at 22□ for 2 d. Each treatment is repeated for 3 times. According to the bacteria generation condition, the co-cultured calluses are washed with sterile water, then transferred into 50 mg/L Timentin, immersed for 1 min, dried by using sterile filter paper, and transferred onto a callus inducing culture medium containing 10 mg/L silver nitrate and 500 mg/L Timentin, subjected to recovery culture, and cultured in dark at 25□ for 7 d. The calluses recovering the growth are transferred into a callus inducing culture medium containing 10 mg/L silver nitrate, 5 mg/L Bialaphos and 50 mg/L Timentin for induction of the somatic embryogenesis. Finally, the proliferated somatic embryos are transferred into a plant regeneration culture medium containing 5 mg/L Bialaphos for induction of the plant regeneration. In the genetic transformation experiment, 6 and 9 resistant plants with in-situ replaced HbICE1(DC)1 and HbICE(DC)2 genes are respectively acquired. Compared with the wild-type plant of Hevea brasiliensis, the cold resistance of the obtained resistant plants is respectively improved by 2.9□ and 1.8□.

### Embodiment 69 Obtaining Juglans regia by insitu replacing JrICE1 gene of different degrees of methylation through a CRISPR/CasI technology

The JrICE1 gene cDNA of Juglans regia is cloned and has a sequence of SEQ ID NO: 34. According to degenerate features of a codon, sites that can be theoretically methylated on the JrICE1 gene are replaced. According to requirements on target traits, the sites of different degrees of methylation on the JrICE1 gene are quantitatively replaced. The JrICE1 gene on which all methylation sites are replaced has a sequence of SEQ ID NO: 75 and is named JrICE1(DC)1; and the JrICE1 gene on which part of methylation sites are replaced has a sequence of SEQ ID NO: 116 and is named JrICE1(DC)2. The JrICE1(DC)1 is divided into 3 segments for CRISPR/Cas9 vector construction and in-situ replacement, and each segment is about 400 bp. The sgRNA with a guiding function is artificially designed, and has a length of 20 nt and sequences respectively shown as 5'-GCTAACACCAGCAACAGTTGCGG-3', 5'-GGAATGCCCTGTTTATGAACAGG-3' and 5'-GTTCATGGAGAACGATAAGGCGG-3'. The sgRNA sequence of the JrICE1(DC)2 gene is 5'-GAGGACAAAGTGGGCTTGGGAGG-3'. The Cas9 protein is guided to perform site-specific cleavage for DNA. The YAO promoter is selected. The vector pHEE401E and JrICE1(DC)1 and JrICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Agrobacterium frozen at -80□ and carrying transformation plasmids is streaked on a solid YEB plate culture medium containing 5 mg/L Bialaphos, and cultured overnight at 28□. Individual colonies on the plate are picked, inoculated into the liquid YEB culture medium containing 5 mg/L Bialaphos, and subjected to shaking culture (200 rpm) overnight at 28□ on a constant-temperature table concentrator. A light absorption value of the bacterial solution under the wavelength of 600 nm is determined by using an ultraviolet visible spectrophotometer. When an OD600 value of the bacterial solution is 0.5-0.8, the bacteria solution is poured into a 30 ml centrifugal tube, centrifuged at 6000 rpm and at the normal temperature for 5-6 min to remove the supernatant, added with liquid DKW culture medium until the volume is 30 ml, and subjected to vortex mixing for 5 min for standby use of infection. The well pre-cultured stems of Juglans regia are mixed with the obtained agrobacterium bacterial solution, and infected for 30 min. The infected stems are dried by using sterile filter paper, inoculated into a co-culture medium that is a combination of DKW + 6-BA 0.5 mg/L + IBA 0.1 mg/L + AS (acetosyringone) 200 umol/L + sucrose 30 g/L + agar 6.0 mg/L, regulated until pH is 5.8 prior to the high-pressure sterilization, and co-cultured in dark for 3 days. The culture condition: the culture temperature is 25+/-1□. After being co-cultured for 3 days, the stems are transferred into a screening culture medium that is a combination of DKW + 6-BA 0.5 mg/L+IBA 0.1 mg/L + 5 mg/L Bialaphos + Cef 500 mol/L + sucrose 30 g/L + agar 6.0 mg/L. Adventitious buds are germinated after 5 weeks. The culture conditions: the culture temperature is 25+/-1□, the illumination time is 16 h/d, and the illumination intensity is 2000-2500 lx. In the genetic transformation experiment, 12 and 10 resistant plants with in-situ replaced JrICE1(DC)1 and JrICE(DC)2 genes are respectively obtained. Compared with the wild-type plant of Juglans regia, the cold resistance of the obtained resistant plants is respectively improved by 3.4□ and 2.1□.

### Embodiment 70 Obtaining Lactuca sativa by insitu replacing LsICE1 gene of different degrees of methylation through a CRISPR/Cas9 technology

The LsICE1 gene cDNA of Lactuca sativa is cloned and has a sequence of SEQ ID NO: 35. According to degenerate features of a codon, sites that can be theoretically methylated on the LsICE1 gene are replaced. According to requirements on target traits, the sites of different degrees of methylation on the LsICE1 gene are quantitatively replaced. The LsICE1 gene on which all methylation sites are replaced has a sequence of SEQ ID NO: 76 and is named LsICE1(DC)1; and the LsICE1 gene on which part of methylation sites are replaced has a sequence of SEQ ID NO: 117 and is named LsICE1(DC)2. The LsICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GGGAATTGGGAGGTACAAATTGG-3', 5'-GTTGGGCTAGGGATCGCGGTTGG -3' and 5'-GCCTCCTACTGCAAAACATATGG-3'. The sgRNA sequence of the LsICE1(DC)2 gene is 5'- GGAGAAAGAAGCTAAACGACCGG-3". The Cas9 protein is guided to perform site-specific cleavage for DNA. The YAO promoter is selected. The vector pHEE401E and LsICE1(DC)1 and LsICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E, and used as a resistant screening gene for constructing a CRISPR/Cas9 expression vector.

Agrobacterium individual colonies carrying the *LsICE1* gene of different degrees of methylation are picked from an LB plate containing Kam and Rif, inoculated into an LB liquid culture medium containing 5 mg/L Bialaphos, and cultured overnight at 150 rpm and 28□ on a table concentrator until a mid-log phase (OD600=0.4 -0.8). 10 ml of bacterial solution is collected and sub-packaged in 10 ml sterilized centrifugal tubes, centrifuged at 3000 rpm for 3 min to removesupernatant, then suspended with sterilized deionized water, and diluted until an OD600 value of the bacterial solution is about 0.5 for infection. The cotyledon explant of Lactuca sativa infected by the agrobacterium is co-cultured with agrobacterium on a differentiation culture medium (MS + 6-BA 0.5 mg/L + NAA 0.20 mg/L), then transferred into a screening culture medium (Sm8 + 5 mg/L Bialaphos + Cef 200 mg/L), and subjected to the resistant screening. The explants are subcultured every week. After the explants are cultured for one week, a base part of the cotyledon can gradually send forth few compact calluses and gradually turn green. Budlet points can be formed in about one week and are gradually differentiated to form tender leaves to form budlets. A plurality of budlets can be generally formed on one callus. When growing to 2-3 cm, the budlets are cut off, inserted into a rooting culture medium (1/2Ms + NAA 0.05 mg/L + Cef 200 mg/L), subjected to the inducing rooting, and transplanted to a seedling pot after 2-3 weeks. By means of the method, 28 and 24 resistant plants with in-situ replaced LsICE1(DC)1 and LsICE1(DC)2 genes are respectively acquired. Compared with the wild-type plant of Lactuca sativa, the cold resistance of the obtained resistant plants is respectively improved by 3.2□ and 1.5□.

### Embodiment 71 Obtaining Oryza sativa by insitu replaced OsICE1 gene of different degrees of methylation through a CRISPR/Cas9 technology

The OsICE1 gene cDNA of Oryza sativa is cloned and has a sequence of SEQ ID NO: 36. According to degenerate features of a codon, sites that can be theoretically methylated on the OsICE1 gene are replaced. According to requirements on target traits, the sites of different degrees of methylation on the OsICE1 gene are quantitatively replaced. The OsICE1 gene on which all methylation sites are replaced has a sequence of SEQ ID NO: 77 and is named OsICE1(DC)1; and the OsICE1 gene on which part of methylation sites are replaced has a sequence of SEQ ID NO: 118 and is named OsICE1(DC)2. The OsICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GTGGTGGTGGTGGCGCGCATGGG-3', 5'-GGCGGTCGTTGAGCTTCTTCCGG-3' AND 5'-GCCCCACTGGACAACAGCCAAGG-3'. The sgRNA sequence of the OsICE1(DC)2 gene is 5'-GCGTCCCAAATGCCGGAGTTCGG-3'. The Cas9 protein is guided to perform site-specific cleavage for DNA. The YAO promoter is selected. The vector pHEE401E and OsICE1(DC)1 and OsICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E, and used as a resistant screening gene for constructing a CRISPR/Cas9 expression vector.

(Husked) mature seeds of Oryza sativa cv Nipponbare are selected and disinfected by using a mercury bichloride method (immersed in 0.1% mercury bichloride for 15 min, and cleaned with sterilized water), and air-dried on a super clean bench. Thereafter, embryos are placed in a callus-inducing culture medium (4.4 g/L MS + 2.5 mg/L 2,4-D + 600 mg/L casein + 30g/l sucrose + 5g/L Phytagel, regulated with KOH until pH is 5.8, and sterilized with high pressure), and subjected to callus culture at 28□ for 2 weeks. The agrobacterium GV3101 carrying plasmids of a target gene is inoculated into 5 ml of YEB liquid culture medium containing 5 mg/L Bialaphos, subjected to shaking culture until a later period of a logarithmic phase,and then subjected to amplification culture in a volume ratio of 1: 100. The agrobacterium thalli are collected when OD600 of the bacterial solution is 0.10 and re-suspended in an infection culture medium. Calluses of Oryza sativa are infected by a conventional method, placed onto a subculture medium containing 5 mg/L Bialaphos after the co-culture infection, cultured in dark at 28□ for 12-16 d, and differentiated on a re-differentiation culture medium, and a Bialaphos-resistant positive seedling can be obtained. In the transgenic experiment, 17 and 14 resistant plants with in-situ replaced OsICE1(DC)1 and OscICE(DC)2 genes are respectively obtained. Under a cold treatment condition at 5□, compared with the wild-type plant of Nipponbare, the cold resistance of the obtained resistant plants is respectively improved by 3.1□ and 2.0□.

### Embodiment 72 Obtaining Phalaenopsis aphrodite by insitu replacing PaICE1 gene of different degrees of methylation through a CRISPR/Cas9 technology

The PaICE1 gene cDNA of Phalaenopsis aphrodite is cloned and has a sequence of SEQ ID NO: 37. According to degenerate features of a codon, sites that can be theoretically methylated on the PaICE1 gene are replaced. According to requirements on target traits, the sites of different degrees of methylation on the PaICE1 gene are quantitatively replaced. The PaICE1 gene on which all methylation sites are replaced has a sequence of SEQ ID NO: 78 and is named PaICE1(DC)1; and the PaICE1 gene on which part of methylation sites are replaced has a sequence of SEQ ID NO: 119 and is named PaICE1(DC)2. The PaICE1(DC)1 is divided into 3 segments for CRISPR/Cas9 vector construction and in-situ replacement, and each segment is about 400 bp. The sgRNA with a guiding function is artificially designed, and has a length of 20 nt and sequences respectively shown as 5'-GTTTGTGGGGGAAGAAGGACTGG-3', 5'-GGGATGGTGAGTTTGAGAAGCGG-3' and 5'-GAATAGTAGTCTTACCGGAGGGG-3'. The sgRNA sequence of the PaICE1(DC)2 gene is 5'-GCCGGGAGCTCCTTAAACCAGGG-3'. The Cas9 protein is guided to perform site-specific cleavage for DNA. The YAO promoter is selected. The vector pHEE401E and PaICE1(DC)1 and PaICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E to be used as a resistant screening gene for constructing a CRISPR/Cas9 expression vector.

Protocorms of Phalaenopsis aphrodite are pre-cultured in a pre-culture liquid culture medium (added with 6-BA1.0 mg/L + NAA5.0 mg/L + 2,4-D 2.0 mg/L + AS 100 µmol/L) for 3 d. Agrobacterium EHA105 for transformation is subjected to shaking culture in an LB (pH 7.0) culture medium containing 5 mg/L Bialaphos and 100 umol/L acetosyringone at 150 r/min and 28□ for 24 h. 20% bacterial solution is added into the above LB culture medium, activated for 5 h, and the OD600 of the bacterial solution is about 0.60. The bacterial solution is centrifuged at 6000 r/min for 3 min, re-suspended with a sterilization liquid culture medium (added with 100 umol/L AS), and diluted to the original volume directly for the genetic transformation of protocorms. The protocorms are infected for 15 min, then inoculated to a solid culture medium (6-BA 1.0 mg/L + NAA 5.0 mg/L + 2,4-D 2.0 mg/L + AS 100 µmol/L), and co-cultured for 3 d. The protocorms are rinsed with a liquid culture medium containing 5.0 mg/L meropenem for 3 times, and separately alternatively cultured on the solid culture medium (added with KT 3.0 mg/L) mixed with meropenem 5.0 mg/L and cefotaxime Na salt 100.0 mg/L. The protocorms are transferred and inoculated every 3 d until the agrobacterium is completely removed. The bacteria-free protocorms are transferred onto the solid culture medium containing 5 mg/L Bialaphos and KT 3.0 mg/L, selectively cultured, transferred to the same culture medium every 2 weeks, and subcultured. After 45 d, the protocorms are cultured on a proto-bulbous body (PLBs) inducing culture medium (mixed with 1.0 mg/L TDZ and 1.0 mg/L 2,4-D) containing 5 mg/L Bialaphos. New PLBs come up on a section and are differentiated to form seedlings. The plants are transferred into a proliferation culture medium (6-BA 5.0 mg/L), and cultured. When the height of adventitious plantlets reaches 3-5 cm, the adventitious plantlets are cut apart and transferred to a rooting culture medium (Hyponex No.1 30.0 g/L+ NAA0.3 mg/L). When each plant sends forth 4 to 5 new roots in a length of 2-4 cm, the plants are taken out of flasks, and transplanted. In the transgenic experiment, 19 and 20 resistant plants with in-situ replaced PaICE1(DC)1 and PacICE(DC)2 genes are respectively obtained. Compared with the wild-type plant of Phalaenopsis aphrodite, the cold resistance of the obtained resistant plants is respectively improved by 3.6□ and 2.1□. Embodiment 73 Obtaining Prunus mume by insitu replacing PmICE1 gene of different degrees of methylation ta CRISPR/Cas9 technology

The PmICE1 gene cDNA of the Prunus mume is cloned and has a sequence of SEQ ID NO: 38. According to degenerate features of a codon, sites that can be theoretically methylated in the PmICE1 gene are replaced. According to requirements on target traits, the sites of different degrees of methylation in the PmICE1 gene are quantitatively replaced. The PmICE1 gene on which all methylation sites are replaced has a sequence of SEQ ID NO: 79 and is named as PmICE1(DC)1; and the PaICE1 gene on which part of methylation sites are replaced has a sequence of SEQ ID NO: 120 and is named as PmICE1(DC)2. The sgRNA with a guiding function is artificially designed and is in a length of 20 nt. The sgRNA sequences of the PmICE(DC)1 gene and the PmICE1(DC)2 gene are both 5'- GCGTCGAGTTCCCAACGACTCGG-3'. The Cas9 protein is guided to perform site-specific cleavage for DNA. The YAO promoter is selected. The vector pHEE401E and PmICE1(DC)1 and PmICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are enzyme digested by SacII and AsiSI, and the Bar gene is bonded into pHEE401E to be used as a resistant screening gene for constructing a CRISPR/Cas9 expression vector.

The agrobacterium EHA105 stored in a refrigerator at -80□ is inoculated onto a YEB plate culture medium containing 5 mg/L Bialaphos and cultured in dark for 2-3 d. After colonies come up, individual colonies are picked, inoculated into 30 ml of YEB liquid culture medium containing 5 mg/L Bialaphos, activated for the first time, subjected to shaking culture at 28□ on a table concentrator for 1-2 d, and taken off from the table concentrator for standby use when the OD600 value is 0.8-1. The 50 ul bacterial solution subjected to first activation is inoculated into 50 ml of YEB liquid culture medium without Bialaphos and activated for the second time, and subjected to shaking culture at 28□ on an ordinary table concentrator until the OD600 value is 0.4. In this experiment, half-developed cotyledons of Prunus mume are adopted as explants and cut into square pieces in a size of 1 cm^{∗}1 cm and pre-cultured for 3 d. The activated agrobacterium EHA105 bacterial solution is centrifuged at 6000 rpm and 4□ to collect thalli. The thalli are added into the liquid MS + AS 100 umol/L and re-suspension is carried out until OD600 value is 0.4. Calluses are infected for 10 min, co-cultured for 2 d, subjected to bacteria removal with liquid MS + Cef 1000 umol/L, rinsed with sterile water for 5 times, and selectively cultured in a screening culture medium Ms + BA 1.0 mg/L + NAA 0.5 mg/L + KT 0.5 mg/L + IBA 2.0 mg/L+ 2,4-D 0.2 mg/L + 5 mg/L Bialaphos + Cef 1000 umol/L. The regenerated plants are transferred onto MS + 6-BA 1.0 mg/L + NAA 0.1 mg/L, and immediately transferred into 1/2MS + NAA 0.1 mg/L for rooting culture when the seedling grows to a height of 3-4 cm. In the transgenic experiment, 11 and 13 resistant plants with in-situ replaced PmICE1(DC)1 and PmICE(DC)2 genes are respectively obtained. Compared with the wild-type plant of Prunus mume, the cold resistance of the obtained resistant plants is respectively improved by 2.2□ and 1.5□.

### Embodiment 74 Obtaining Camellia sinensis by insitu replacing CsICE1 gene of different degrees of methylation of methylation through a CRISPR/Cas9 technology

The cDNA of the CsICE1 gene of Camellia sinensis is cloned and has a sequence of SEQ ID NO: 39. According to degenerate features of a codon, sites that can be theoretically methylated on the CsICE1 gene are replaced. According to requirements on target traits, the sites of different degrees of methylation on the CsICE1 gene are quantitatively replaced. The CsICE1 gene on which all methylation sites are replaced has a sequence of SEQ ID NO: 80 and is named CsICE1(DC)1; and the CsICE1 gene on which part of methylation sites are replaced has a sequence of SEQ ID NO: 121 and is named CsICE1(DC)2. The CsICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GCAATGTACAAGGGTATAGTGGG -3', 5'-GTTTATGGAGAACAATAAGGTGG -3' and 5'-GCGGTACTCTTGGATTCAGCTGG-3'. The sgRNA sequence of the CsICE1(DC)2 gene is 5'-GGGATTGAGGACGGAAGAAATGG-3'. The Cas9 protein is guided to perform site-specific cleavage for DNA. The YAO promoter is selected. The vector pHEE401E and CsICE1(DC)1 and CsICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Immature tea fruits in August-September are selected, cleaned with distilled water, and peeled, and milky white or slightly-brown seeds are taken out. After the outer seed coats are cut apart, the surface of the seeds are disinfected with 75% ethanol for 30 s under a sterile condition, the seeds are immersed with 0.1% HgCl₂ for 3 min and then rinsed with sterile water for 6-8 times. Cotyledons are cut into square pieces in a size of 0.5 cm, and inoculated to a solid culture medium (improved ER + 0.1 mg/L + 1.0 mg/L 6-BA + 0.1 g/L chloramphenicol + 30 g/L sucrose). After the inoculation, the cotyledon square pieces are cultured in a constant-temperature culture room at 25+/-2□ in artificial light for 13 h each day under the illumination intensity of 1500-2000 lx. After the seeds are cultured for 25 d, cotyledon calluses that turn green are selected as explants. The stored agrobacterium tumefaciens EHA105 are streaked on a prepared YEB solid culture medium plate, and cultured in dark at 28□ for 1-2 d. Individual colonies are picked by using an inoculation ring, inoculated into 5 ml of YEB liquid culture medium, and subjected to shaking culture at 220 rpm and 28□ for 24 h. 1 ml of bacterial solution is sucked under a sterile condition into the liquid culture medium containing 200 uml/L acetosyringone, subjected to shaking culture at 220 rpm and 28□ for 4-6 h, and used for explant infection when an OD value measured at 600nm is 0.6-0.8. The cotyledon calluses that already turn green are selected as explants from the super clean bench, inoculated into a pre-culture medium (improved ER + 0.2 mg/L IBA + 30 g/L sucrose), and pre-cultured in dark at 25□ for 3 d. The pre-cultured cytoledon calluses are placed in the standby agrobacterium tumefaciens bacterial solution. An experimental material is immersed by the bacterial solution, and infected for 15 min, then the surplus bacterial solution on the surfaceis blotted up with sterile filter paper, the experimental material is inoculated to a YEB co-culture medium added with 150 umol/L AS, and cultured in dark at 25□ for 3 d. After being co-cultured, the material is rinsed with sterile water for 3-4 times. Water on the surface of the material is blotted up with sterile filter paper. The calluses are transferred and inoculated into the pre-culture medium,subjected to delayed selection culture for 3 d, and then transferred and inoculated to a bacteriostatic culture medium containing 100 umol/L Cef and 100 umol/L Spe, and cultured in dark. The material is transferred and inoculated every 3 d. After 15 d, the explants are transferred into a screening culture medium containing 100 umol/L Spe for screening the resistant buds. After the explants are subjected to screening culture for 20 d, the vigorous resistant buds are transferred into a new differentiation culture medium added with 100 umol/L Spe for culturing. After being cultured for 30 d in the differentiation culture medium, the cotyledon calluses are differentiated to form seedlings. After the seedlings are transferred into a rooting culture medium, and cultured for 2 weeks, a complete resistant plant can be obtained. By means of genetic transformation, 6 and 7 resistant plants with in-situ replaced CsICE1(DC)1 and CsICE(DC)2 genes are respectively obtained. Compared with the wild-type plant of Camellia sinensis, the cold resistance of the obtained resistant plants is respectively improved by 3.3□ and 2.0□.

### Embodiment 75 Obtaining Arabidopsis thaliana by insitu replacing AtICE1 gene of different degrees of methylation of different degrees of methylationthrough a CRISPR/Cas9 technology

The AtICE1 gene cDNA of Arabidopsis thaliana is cloned and has a sequence of SEQ ID NO: 40. According to degenerate features of a codon, sites that can be theoretically methylated on the AtICE1 gene are replaced. According to requirements on target traits, the sites of different degrees of methylation on the AtICE1 gene are quantitatively replaced. The AtICE1 gene on which all methylationsites are replaced has a sequence of SEQ ID NO: 81 and is named AtICE1(DC)1; and the AtICE1 gene on which part of methylationsites are replaced has a sequence of SEQ ID NO: 122 and is named AtICE1(DC)2. The AtICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GCTCCTATTTCGATGGGGTTTGG-3', 5'-GATAAATGAGAGCGGTAAAGCGG-3' and 5'-GCAGTGCTTTTCGATACAGCAGG-3'. The sgRNA sequence of the BrICE1(DC)2 gene is 5'-GAAGATCTTGTGGATGTGGTTGG-3'. The Cas9 protein is guided to perform site-specific cleavage for DNA. The YAO promoter is selected. The vector pHEE401E and AtICE1(DC)1 and AtICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The stored agrobacterium tumefaciens GV3101 are streaked on a prepared YEB solid culture medium plate, and are subjected to dark culture at 28□ for 1-2 d. Individual colonies are picked by using an inoculation ring,and are inoculated into 5 ml of YEB liquid culture medium, and subjected to shaking culture at 220 rpm and 28□ for 24 h. 1 ml of bacterial solution is sucked under a sterile condition into YEB liquid culture medium, and subjected to shaking culture at 220 rpm and 28□ for 4-6 h, and the bacterial solution can be used for infection on Arabidopsis thaliana when an OD value of the bacterial solution measured at 600nm is 0.6-0.8. The surface of a flower of Arabidopsis thaliana at a full-blossom stage is partially immersed in an agrobacterium suspension (OD=0.8) for 5 min and gentlyrotatedat the same time. An infected plant is bagged, kept in a highly moist state and cultured in a dark room for 24 hours. When seeds are mature and pods are naturally cracked, the seeds can be harvested. The collected seeds of Arabidopsis thaliana are vernalizated for 2 days, and then cultured on a plate containing 5 mg/L Bialaphos after being sterilized. The seeds that are successfully introduced with recombinant plasmids can normally send forth more than 4 pieces of euphylla on the resistant culture medium. The non-transgenic seeds cannot grow normally and can only send forth two cytoledons, root growth is severely inhibited and non-transgenic seeds will generally die after 10 days of germination. The screened plant is transplanted to a matrix in which vermiculite, peat and perlite are in a ratio of 9: 3: 1 and covered with a film for 2-3 days. By means of genetic transformation, 26 and 27 resistant plants with in-situ replaced AtICE1(DC)1 and AtICE(DC)2 genes are respectively obtained. Compared with the wild-type Arabidopsis thaliana plant Col-0, the cold resistance of the obtained resistant plants is respectively improvedby 2.4□ and 1.5□.

### Embodiment 76 Obtaining Brassica rapabyinsitu replacing BrrICE1 gene of different degrees of methylationthrougha CRISPR/Cas9 technology

The BrrICE1 gene cDNAof the Brassica rapais cloned and has a sequence of SEQ ID NO: 41. According to degenerate features of a codon, sites that can be theoretically methylated onthe BrrICE1 gene are replaced. According to requirements on target traits, the sites of different degrees of methylation on the BrrICE1 gene are quantitatively replaced. The BrrICE1 gene on whichallmethylationsites are replaced has a sequence of SEQ ID NO: 82 and is named BrrICE1(DC)1; and the BrrICE1 gene on whichpartof methylationsites are replaced has a sequence of SEQ ID NO: 123 and is named BrrICE1(DC)2. The BrrICE1(DC)1 is divided into 3 segments for CRISPR/Cas9 vector construction and in-situ replacement, and each segment is about 500 bp. The sgRNA with a guiding function is artificially designed, and has a length of 20 nt and sequences respectively shown as 5'-GTCGAGGCTAAAAGCCTGAGAGG -3',5'-GTTCAGAACGGAGGAGGTAAAGG -3' and 5'-GCCAGAGGGAGAGATCCATTTGG-3'. The sgRNA sequence of the BrrICE1(DC)2 gene is 5'-GCAACACCCTTATGCGGAGGTGG-3'. The Cas9 protein is guided to perform site-specific cleavage for DNA. The YAO promoter is selected. The vector pHEE401E and BrrICE1(DC)1 and BrrICE1(DC)2 genes of differentdegrees of methylation are cleaved by restriction endonucleases BsaI and KpnI, and the target gene is bonded behind the zCas9 sequence. Rrestriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Seeds of Brassica rapa are sterilized with 1% sodium hypochlorite for 20 min, and then washed with sterile water for 4-5 times. Thereafter, the seeds are placed onto a germination culture medium (Mₛ culture medium containing 20 g/L sucrose, PH5.8) and cultured in light at 24□ under the illumination intensity of 60-80 uEm⁻²s⁻¹°C for 5 days (16 h/8 h for each day). The stored agrobacterium tumefaciens EHA101 are streaked on a prepared YEB solid culture medium plate, and subjected to dark culture at 28□ for 1-2 d. Individual colonies are picked by an inoculation ring, inoculated into 5 ml of YEB liquid culture medium, and subjected to shaking culture at 220 rpm and 28□ for 24 h. 1 ml of bacterial solution is sucked under a sterile condition into YEB liquid culture medium and subjected to shaking culture at 220 rpm and 28□ for 4-6 h,and then the bacterial solution can be used for explant infection when an OD value of the bacterial solution measured at 600nm is about 2.0. The bacterial suspension (0.5 -1.0 ul) is injected to roots of cotyledonary petioles by using anagrobacterium inoculation injector and then co-cultured on an Mₛ culture medium for 3 d. Then the bacterial solution is transferred to a callus inducing culture medium (Ms + 20uM 6-BA + 3% sucrose + 500mg/L carbenicillin + 5 mg/L Bialaphos,PH 5.8) and cultured for 2-3 weeks under the same culture conditions. After being cultured in the above way, sections of cotyledonary petioles of Brassica rapa are developed into calluses from exposed vascular tissues in a short period of time. The calluses are developed into stem meristem 4 weeks later, and the stem meristem is transferred onto an elongation culture medium (Ms + 3% sucrose + 500mg/L carbenicillin + 5 mg/L Bialaphos,PH 5.8) after 1-2 weeks, and cultured. Seedlings are transferred into a greenhouse after 2-3 weeks, and cultured. By means of genetic transformation, 14 and 17 resistant plants with in-situ replaced BrrICE1(DC)1 and BrrICE1(DC)2 genes are respectively obtained. Compared with the wild-type plant of Brassica rapa, the cold resistance of the obtained resistant plantsis respectively improvedby 3.8□ and 2.1□. Embodiment 77 Obtaining Isatistinctoria by insitu replacing ItICE1 gene of different degrees of methylationthrougha CRISPR/Cas9 technology

The ItICE1 gene cDNAof Isatistinctoriais cloned and has a sequence of SEQ ID NO: 42. According to degenerate features of a codon, sites that can be theoretically methylated on the ItICE1 gene are replaced. According to requirements on target traits, the sites of different degrees of methylationon the ItICE1 gene are quantitatively replaced. The ItICE1 gene on which all methylation sites are replaced has a sequence of SEQ ID NO: 83 and is named ItICE1(DC)1; and the ItICE1 gene on which part of methylation sites are replaced has a sequence of SEQ ID NO: 124 and is named ItICE1(DC)2. The ItICE1(DC)1 is divided into 3 segments for CRISPR/Cas9 vector construction and in-situ replacement, and each segment is about 500 bp. The sgRNA with a guiding function is artificially designed, and has a length of 20 nt, and sequences respectively shown as 5'-GAGCCGAACCCCATGGAGATCGG-3',5'-GCTGAGCGATGATGGAGAGATG G -3' and 5'-GCTGGCTACCATGAAAGCTTTGG-3'. The sgRNA sequence of the ItICE1(DC)2 gene is 5'-GATGGACGAGACGGGGATTGAGG-3'. The Cas9 protein is guided to perform site-specific cleavage for DNA. The YAO promoter is selected. The vector pHEE401E and ItICE1(DC)1 and ItICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

After being immersed and sterilized with 70% alcohol for 45 s-1 min, seeds of Isatistinctoria are immersed and disinfected with 0.1% HgCl₂ for 15 min, washed with sterile water for 3-5 times, inoculated onto an MS basic culture medium, and cultured in light at 25□ under an illumination intensity of 2000 lx for 12-15 d (16 h for each day). Individual colonies are picked from an activated and stored agrobacterium plate containing a target expression vector, inoculated in 25 ml of LB liquid culture medium (containing 5 mg/L Bialaphos), subjected toshakingculture in dark at 28□ until OD₆₀₀ of the bacterial solution is 0.1-0.3, and centrifuged at 4000 r/min for 10 min, then thalli are collected, and the supernatant is removed. The thalli are re-suspended in an MS liquid culture medium of the same volume,and added with 100 umol/L acetosyringone, and the bacterial solution can be used for explant infection. Seedlings that are germinated from aseptic seeds and grow to 4-5 cm are taken out. Cotyledonary petioles of Isatistinctoria (with growth points removed) are cut, and used as explants. The cotyledonary petioles are downwards inserted into a pre-culture medium (MS basic culture medium + 3 rng/L 6-BA + 0.2 mg/L NAA + 2 mg/L ZT, PH 5.8), pre-cultured in light at 25+/-1□ for 1 d. The cotyledonary petioles are immersed in the prepared agrobacterium infection bacterial solution, shaken at 10 rpm for 10 min on a table concentrator, taken out, dried with sterile filter paper after the bacterial solution is removed, inserted into a co-culture medium (MS basic culture medium + 3mg/L 6-BA + 0.2 mg/L NAA + 2 mg/L ZT + 100 umol/L acetosyringone, PH 5.8), and cultured in dark at 28□ for 2-3 d. When agrobacterium colonies can be seen in the co-culture medium, the cotyledon petioles are taken out for bacteria removal, first washed with sterile water for one time, then subjected to shaking immersion in sterile water containing Cef 500 mg/L for 20 min, washed with sterile water for 3-5 times, and placed on sterilized filter paper to sufficiently blot up the residual water. The cotyledonary petioles are transferred onto a differentiation culture medium (MS basic culture medium + 3mg/L 6-BA + 0.2 mg/L NAA + 2mg/L ZT + 0.5 g/L AgN0₃ + 5 mg/L Bialaphos + 200 mg/L Cef + 300 mg/L Carb, PH 5.8), and subjected to differentiation screening culture in light at 25□ for 16 h per day°C. The cotyledonary petioles begin to be differentiated into buds after 25 days. The differentiated green buds of Isatistinctoria are cut off, and placed in a propagation culture medium (MS basic culture medium + 3 mg/L 6-BA + 0.2mg/L NAA + 6 mg/L + 500 mg/L Cef, PH 5.8), and subcultured for 3-4 times (subcultured every two weeks). Seedlings obtained by propagation are rooted for 25 d, after the seedlings send forth an appropriate number of roots, a cover of an aseptic plant culture jar is opened, and the seedlings are placed in a room for 2 days. Nutritional soil, sandy soil and field soil are mixed in a ratio of 1: 1: 1, and used as a culture medium for transforming the seedlings. Tissue cultured seedlings are carefully taken out,and washed with water to remove the culture medium attached thereon. The plant is transplanted into a seeding pot and cultured in a greenhouse. By means of genetic transformation, 12 and 10 resistant plants with genetically-transformed ItICE1(DC)1 and ItICE(DC)2 genes are respectively obtained. Compared with the wild-type plant of Isatistinctoria, the cold resistance of the obtained resistant plants is respectively improvedby 3.3□ and 1.9□.

### Embodiment 78 Obtaining Eucalyptus camaldulensiby insitu replacing EcICE1 gene of different degrees of methylationthrougha CRISPR/Cas9 technology

The EcICE1 gene cDNA of Eucalyptus camaldulensiis cloned and has a sequence of SEQ ID NO: 43. According to degenerate features of a codon, sites that can be theoretically methylated on the EcICE1 gene are replaced. According to requirements on target traits, the sites of different degrees of methylationon the EcICE1 gene are quantitatively replaced. The EcICE1 gene on which all methylation sites are replaced has a sequence of SEQ ID NO: 84 and is named EcICE1(DC)1; and the EcICE1 gene on which part of methylation sites are replaced has a sequence of SEQ ID NO: 125 and is named EcICE1(DC)2. The EcICE1(DC)1 is divided into 3 segments for CRISPR/Cas9 vector construction and in-situ replacement, and each segment is about 500 bp. The sgRNA with a guiding function is artificially designed, and has a length of 20 nt, and sequences respectively shown as 5'-GGCGGTTCCGAGCTGGGCTCCGG -3', 5'--GGATGAAAATTAGTCGAAGGGGG -3' and 5'-GTCTGCGTGCACAAAACATGCGG-3'. The sgRNA sequence of the EcICE1(DC)2 gene is 5'-GGGCTTTTTGGCTCGGTGCAGGG-3'. The Cas9 protein is guided to perform site-specific cleavage for DNA. The YAO promoter is selected. The vector pHEE401E and EcICE1(DC)1 and EcICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The GV3101 strain stored in a low-temperature refrigerator is taken out. The surface of the prepared culture medium is gently streaked with 5-6 lines in a super clean bench by a streaking method. A culture dish is invertedly placed at 25-27□, and subjected to the dark culture for 24-36 h. Individual colonies on the surface of the culture medium in the culture dish are picked by using an inoculation needle, placed ona 15 ml of centrifugal tube liquid culture medium, and subjected to suspension cultureon a constant-temperature table concentrator at 180 rpm and 27□ for 12-18 h. The bacterial solution is collected,placed into a 100 ml triangular flask according to a ratio of 1/40, and continuously cultured for about 6 h. When OD600 of the bacterial solution is 0.5, the bacterial solution is used for infection. Cultivated clones DH201-2 of Eucalyptus urophylla are used as a material. Stems collected from the upper part and middle part of a plant that is cultured for 30 d on the rooting culture medium are cultured on a pre-culture medium for 6 d. In the super clean bench, explants of Eucalyptus camaldulensis are immersed in the bacterial solution in the triangular flask for 30 min, and thenthe plant material is taken out, inoculated onto a plant co-culture medium (100 uMacetosyringone, PH 5.8), and cultured for 3 d. Thereafter, the infected stems are transferred into an inducing regeneration culture medium of (Ms + 0.05 mg/L TDZ + 0.5 mg/L NAA + 5 mg/L Bialaphos + 300mg/L cephalosporin, PH 5.8), and cultured for 15 d to form calluses. The browning, loose and yellowing calluses are removed. The red-green well-grown calluses are transferred to the same culture medium for more than 3 times to remove the agrobacterium, and then transferred to the culture medium only having the Bialaphos, thus obtaining regenerated buds. The regenerated buds are transferred onto a seedling strengthening culture medium, cultured for 20 days, then transferred into a rooting culture medium for rooting culture. In the transgenic experiment, 9 and 11 resistant plants with in-situ replaced EcICE1(DC)1 and EcICE(DC)2 genes are respectively obtained. Compared with the wild-type plant of Eucalyptus camaldulensi, the cold resistance of the obtained resistant plants is respectively improvedby 3.7□ and 2.3□.

### Embodiment 79 ObtainingJatrophacurcasinsitu replacing JcICE1 gene of different degrees of methylationthrough a CRISPR/Cas9 technology

The JcICE1 gene cDNA of Jatrophacurcasis cloned and has a sequence of SEQ ID NO: 44. According to degenerate features of a codon, sites that can be theoretically methylated on the JcICE1 gene are replaced. According to requirements on target traits, the sites of different degrees of methylationon the JcICE1 gene are quantitatively replaced. The JcICE1 gene on which all methylation sites are replaced has a sequence of SEQ ID NO: 85 and is named JcICE1(DC)1; and the JcICE1 gene on which part of methylation sites are replaced has a sequence of SEQ ID NO: 126 and is named JcICE1(DC)2. The JcICE1(DC)1 is divided into 3 segments for CRISPR/Cas9 vector construction and in-situ replacement, and each segment is about 500 bp. The sgRNA with a guiding function is artificially designed, and has a length of 20 nt, and sequences respectively shown as 5'-GGTTCGATTTGGGTGACATTGGG -3', 5'-GGCAAAGCTTATCCTTGATACGG-3' and 5'-GTCTCCTGCTCTCCACTATGAGG-3'. The sgRNA sequence of the JcICE1(DC)2 gene is 5'-GAAACGTGTCCTGGGTTTGCAGG-3'. The Cas9 protein is guided to perform site-specific cleavage for DNA. The YAO promoter is selected. The vector pHEE401E and JcICE1(DC)1 and JcICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Tender leaves (third to fifth leaves, counted from a growth point) of two-year-old potted seedlings ofJatrophacurcas in a culture room are collected, disinfected, inoculated to a callus inducing or direct adventitious bud inducing culture medium, pre-cultured for 3 days, cut into square pieces of 3 mm^{∗}3 mm, infected by using a bacterial solution (containing 100 uM AS) at the concentration of OD600 of 1.0 for 10 min, and subsequently co-cultured for 3 days (the co-culture medium is mixed with 100 uM AS, pH 5.3). After being co-cultured for 3 days, the leaf pieces are washed with a liquid MS culture medium containing 500 mg/L Cb to remove bacteria for 3-5 times. The transformed leaves of Jatrophacurcas shall be germinated in dark, transferred to light cultureafter about 2 weeks,the adventitious bud direct inducing culture medium is SIO-I: MS+TDZ 1.0 mg/L + 5 mg/L Bialaphos + GA3 0.5 mg/I, and the inducing culture medium is mixed with Cb 500 mg/L. When more bud points are formed, the leaves are transferred to an adventitious bud propagation elongation culture medium (MS + BA 0.3 mg/L + IBA 0.01 mg/L) for light culture. The culture medium is mixed with 5 mg/L Bialaphos and Cb 500 mg/L. After 20-30 days, the well-elongated adventitious buds are transferred into a rooting culture medium 1/2 MS + IBA 2.0 mg/L, and rooted in light. The culture medium is free from any antibiotics. After about30 days, a rooted plant can be obtained. Seedlings that are well rooted are collected, and gradually exercised step by step. Then a flask cover is gradually opened. The rooted seedlings are taken out after the flask cover is completely opened for 2-3 days, carefully washedto remove the culture medium, and planted in sterilized vermiculite matrix after old leaves and dead leaves are removed. At the beginning, a ground part is covered with a film to prevent the excessively high evaporation effect, avoid the direct sunlight and improve the survival rate. Meanwhile, attention should be paid to the humidity in the film, so that the rotting and death of seedlings caused by excessively high humidity can be prevented. By means of genetic transformation, 7 and 8 resistant plants with in-situ replaced JcICE1(DC)1 and JcICE(DC)2 genes are respectively obtained. Compared with the wild-type plant of Jatrophacurcas, the cold resistance of the obtained resistant plants is respectively improvedby 3.2□ and 1.8□.

### Embodiment 80 ObtainingPiceaabiesbyinsitu replacing PiaICE1 gene of different degrees of methylationthrougha CRISPR/Cas9 technology

The PiaICE1 gene cDNAof Piceaabiesis cloned and has a sequence of SEQ ID NO: 45. According to degenerate features of a codon, sites that can be theoretically methylated onthe PiaICE1 gene are replaced. According to requirements on target traits, the sites of different degrees of methylationonthe PiaICE1 gene are quantitatively replaced. The PiaICE1 gene on which all methylation sites are replaced has a sequence of SEQ ID NO: 86 and is named PiaICE1(DC)1; and the PiaICE1 gene on which part of methylation sites are replaced has a sequence of SEQ ID NO: 127 and is named PiaICE1(DC)2. The PiaICE1(DC)1 is divided into 2 segments for CRISPR/Cas9 vector construction and in-situ replacement, and each segment is about 500 bp. The sgRNA with a guiding function is artificially designed, and has a length of 20 nt, and sequences respectively shown as 5'-GGAGTTGGCCTGCGAGGGGTTGG-3' and 5'-GGACTCGAACCTGCGAAGCTGGG-3'. The sgRNA sequence of the BrICE1(DC)2 gene is 5'-GTTCAAGGCAATGCTGGACGCGG-3'. The Cas9 protein is guided to perform site-specific cleavage for DNA. The YAO promoter is selected. The vector pHEE401E and PiaICE1(DC)1 and PiaICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Stem tips ingood physiological status of the test-tube seedling of Piceaabies that is subcultured for 1 month are selected, and needle-point leaves are gently stripped. Stems in a length of about 0.5 cm that are close to the stem tips are cut, and precultured for 3 d on an inducing culture medium (DCR + 6-BA 1. 0 mg/L + TDZ 0. 001 mg/L + NAA 0. 1 mg/L, PH 5.8) prior to the agrobacterium infection. Individual colonies of agrobacterium EHA105 are picked, inoculated into a liquid culture medium of 5 ml YEB + 5 mg/L Bialaphos, and shaken overnight at 220 rpm and 27□. 0.5 ml of bacterial solution is collected,transferred into a liquid culture medium containing25 m1 YEB and5 mg/L Bialaphos, and subjected to amplification culture for 6 h. When an OD600 value of the bacerial solution is 0.4, the bacterial solution is centrifuged at 5000 rpm for 10 min, and thalli are collected. The thalli are suspended by an equal volume of liquid inducing culture medium. The pre-cultured stem is immersed in the prepared infection bacterial solution for 15 min and gently shaken. The stem is taken out, placed on the sterile filter paper to remove surplus bacterial solution, and then placed back to the inducing culture medium for co-culturing (80 uM AS is added). After being co-cultured in dark for a period of time, the stem is washed with liquid inducing culture medium for 3 times to remove surplus agrobacterium. The stem is transferred to a primary screening culture medium (DCR + 6-BA 1.0 mg/L + TDZ 0.001 mg/L + NAA 0.1 mg/L + Cef 200 mg/L + Bialaphos 5 mg/L, PH 5.8), and cultured. The washed stem is transferred, inoculated to the primary screening culture medium, cultured about 20 d, and thentransferred into a secondary screening culture medium (DCR + Cef 200 mg/L + Bialaphos 5 mg/L, PH 5.8) when few stems begin to be sprout. The stem is sub-cultured for 25 d on the secondary screening culture medium. A total of 186 Km resistant buds are obtained on the primary screening culture medium. The primarily obtained Km resistant buds are subcultured for two times on the secondary screening culture medium. When a great number of buds are whitened during the subculture, the buds that are finally kept green are considered as the Km resistant buds. A total of 39 Km resistant buds are obtained in the present research. The adventitious buds in a height of about 1-5 cm are transferred into a rooting culture medium (1/4MS basic culture medium added with IBA 0. 2 mg/L, NAA 0. 1 mg/L, sucrose 296, agar powder 0.6%, pH 5.8). After the adventitious buds are inoculated for 7 d, root primordium begins to come up on bases of the adventitious buds and can be elongated to 1-2 cm after 25 d. By means of genetic transformation, 10 and 13 resistant plants with in-situ replaced PiaICE1(DC)1 and PiaICE(DC)2 genes are respectively obtained. Compared with the wild-type plant of Piceaabies, the cold resistance of the obtained resistant plants is respectively improvedby 3.9□ and 2.2□.

### Embodiment 81 ObtainingPopulussuaveolensby insitu replacing PsICE1 gene of different degrees of methylation through a CRISPR/Cas9 technology

The PsICE1 gene cDNAof Populussuaveolensis cloned and has a sequence of SEQ ID NO: 46. According to degenerate features of a codon, sites that can be theoretically methylated onthe PsICE1 gene are replaced. According to requirements on target traits, the sites of different degrees of methylationonthe PsICE1 gene are quantitatively replaced. The PsICE1 gene on which all methylation sites are replaced has a sequence of SEQ ID NO: 87 and is named PsICE1(DC)1; and the PsICE1 gene on which part of methylation sites are replaced has a sequence of SEQ ID NO: 128 and is named PsICE1(DC)2. The PsICE1(DC)1 is divided into 3 segments for CRISPR/Cas9 vector construction and in-situ replacement, and each segment is about 500 bp. The sgRNA with a guiding function is artificially designed, and has a length of 20 nt, and sequences respectively shown as 5'-GGTTTCTTCCAACCCTTCAAAGG -3', 5'-GGTCGTGGTGAGGATCAAAAGGG -3' and 5'-GCAGTCCTACTGGATTCAGCTGG-3'. The sgRNA sequence of the PsICE1(DC)2 gene is 5'-GCAGCTAGAGGAAAGAACTTGGG-3'. The Cas9 protein is guided to perform site-specific cleavage for DNA. The YAO promoter is selected. The vector pHEE401E and PsICE1(DC)1 and PsICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

Agrobacterium GV3101 strain stored at -70□ is collected and molten on ice. An appropriate amount of bacterial solution is collected,and smeared onto an LB culture medium plate containing corresponding antibioticsinverting culture is performed at 28□ for about 2 d until individual colonies grow. Individual colonies are picked from the plate, inoculated into 5 ml of LB liquid culture medium containing 100 mg/L spc and 50 mg/L GM, and subjected to shakingculture at 220 rpm and 28□ for 24 h, 0.4 ml of bacteria solution is taken out, transferred,inoculated into 20 ml of LB culture medium containing the corresponding antibiotics, and cultured for about 6 h until an OD600value of the bacterial solution is 0.5. The bacterial solution is centrifuged at 5000 rpm for 10 min, and thalli are collected. The thalli is re-suspended by using an equal volume of 1/2 MS liquid culture medium. The bacterial solution is diluted until the OD600value of the bacterial solution is 0.5, and used for infection. The bacterial solution is mixed with 20 uM acetosyringone and gently shaken at 90 rpm and 25□ for 30 min. Tissue cultured seedlings of Populussuaveolens that are basically consistent in growth (4-6 weeks) are selected, 2 to 3 stems are taken, and leaves and axillary buds are trimmed. Then the stems are rapidly cut into stem segments in a length of 1-1.5 m by using a blade. The stem segments are transferred into the prepared infection solution, and taken out after the infection solution is gently shaken at 25□ (250 ml triangular flask, 90 rpm) for 30 min. The residual bacterial solution is blotted up with filter paper. Then the stem fragments are transferred to an antibiotic-free screening culture medium, and cultured in dark. The co-culture medium is a differentiation culture medium. A dark co-culture method is adopted. After being co-cultured for 2 d, the stem segments are placed on a screening culture medium (callus inducing culture medium + cef 200 mg/L + 5 mg/L Bialaphos, PH 5.6), and screened, so that green resistant calluses can come up on both end edges of the stem segments. After the screened stem segments completely send forth budlets, the stem segments are transferred onto a bud elongation culture medium (MS + 6-BA 0.2 mg/L + TDZ 0.01 mg/L + cef 200 mg/L + 5 mg/L Bialaphos, PH 5.6). The bud elongation culture medium has a low hormones concentration, which is conducive to the rapid growth of the resistant buds. When the buds grow to 1-2 cm, the stem segments are transferred to a rooting culture medium (1/2MS + cef 200 mg/L + 5 mg/L Bialaphos,PH 5.6) for inducing the rooting. A complete transgenic plant can be formed when the stem segments are cultured over a period of time after they have taken root. By means of genetic transformation, 20 and 15 resistant plants with in-situ replaced PsICE1(DC)1 and PsICE(DC)2 genes are respectively obtained. Compared with the wild-type plant of Populussuaveolens, the cold resistance of the obtained resistant plants is respectivelyimproved by 3.6□ and 2.1□.

### Embodiment 82 Obtaining Populustomentosa by insitu replacing PtICE1 gene of different degrees of methylation through a CRISPR/Cas9 technology

The PtICE1 gene cDNA of Populustomentosa is cloned and has a sequence of SEQ ID NO: 47. According to degenerate features of a codon, sites that can be theoretically methylated on the PtICE1 gene are replaced. According to requirements on target traits, the sites of different degrees of methylationonthe PtICE1 gene are quantitatively replaced. The PtICE1 gene on which all methylation sites are replaced has a sequence of SEQ ID NO: 88 and is named PtICE1(DC)1; and the PtICE1 gene on which part of methylation sites are replaced has a sequence of SEQ ID NO: 129 and is named PtICE1(DC)2. The PtICE1(DC)1 is divided into 3 segments for CRISPR/Cas9 vector construction and in-situ replacement, and each segment is about 400 bp. The sgRNA with a guiding function is artificially designed, and has a length of 20 nt, and sequences respectively shown as 5'-GTTTCATCTCTGTTAAACAGGGG-3',5'-GATGTTAGCTTTGATGGGTCGGG -3' and 5'-GCTTCCAGCTAAGAATTTGATGG-3'. The sgRNA sequence of the PtICE1(DC)2 gene is 5'- GTTTCATCTCTGTTAAACAGGGG-3'. The Cas9 protein is guided to perform site-specific cleavage for DNA. The YAO promoter is selected. The vector pHEE401E and PtICE1(DC)1 and PtICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The operation method of the genetic transformation experiment for Populustomentosais similar to that of the genetic transformation experiment for Populussuaveolens (embodiment 39). In the genetic transformation experiment, a total of 18 and 22 plants of Populustomentosa with in-situ replaced PtICE1(DC)1 and PtICE1(DC)2 genes are obtained. Compared with the wild-type plant of Populustomentosa, the cold resistance of the obtained resistant plants is respectively improvedby 3.7□ and 2.2□.

### Embodiment 83 Obtaining Arabidopsis thaliana by insitu replacing PopICE1 gene of Potamogeton perfoliatus of different degrees of methylation through a CRISPR/Cas9 technology

The PopICE1 gene cDNA of Potamogeton perfoliatus is cloned and has a sequence of SEQ ID NO: 48. According to degenerate features of a codon, sites that can be theoretically methylated onthe PopICE1 gene are replaced. According to requirements on target traits, the sites of different degrees of methylationonthe PopICE1 gene are quantitatively replaced. The PopICE1 gene on which all methylation sites are replaced has a sequence of SEQ ID NO: 89 and is named PopICE1(DC)1; and the PopICE1 gene on which part of methylation sites are replaced has a sequence of SEQ ID NO: 130 and is named PopICE1(DC)2. The sgRNA with a guiding function is artificially designed and has a length of 20 nt. The sgRNA sequences of the *PopICE1*(DC)1 and *PopICE1*(DC)2 genes are both 5'-GCTCCTATTTCGATGGGGTT NGG-3'. The Cas9 protein is guided to perform site-specific cleavage for DNA. The YAO promoter is selected. The vector pHEE401E and PtICE1(DC)1 and PtICE1(DC)2 genes of different degrees of methylation are cleaved by restriction endonucleases BsaI and KpnI, and the target gene is bonded behind the zCas9 sequence. Restriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The stored agrobacterium tumefaciens GV3101 are collected, streaked on a prepared YEB solid culture medium plate, and cultured in dark at 28□ for 1-2 d. Individual colonies are picked by using an inoculation ring, inoculated into 5 ml of YEB liquid culture medium, and subjected toshakingculture at 220 rpm and 28□ for 24 h. 1 ml of bacterial solution is sucked into YEB liquid culture medium under a sterile condition, subjected to shaking culture at 220 rpm and 28□ for 4-6 h, and used for explant infection when an OD value measured at 600nm is 0.6-0.8. The surface of a flower of Arabidopsis thaliana at a full-blossom stage is partially immersed in an agrobacterium suspension (OD=0.8) for 5 min and gently rotated simultaneously. An infected plant is bagged, kept in a highly moist state and cultured in a dark room for 24 hours. When seeds are mature and pods are naturally cracked, the seeds can be harvested. The collected seeds of Arabidopsis thaliana are vemalizated for 2 days, and cultured on a plate containing 5 mg/L Bialaphos after being disinfected. The seeds that are successfully introduced with recombinant plasmids can normally send forth more than 4 pieces of euphylla on the resistant culture medium. The non-transgenic seeds cannot grow normally and can only send forth two cytoledons, and rooth growth is severely inhibited. The screened plant is transplanted to a medium in which vermiculite, peat and perlite are in a ratio of 9: 3: 1, and covered with a film for 2-3 days. By means of genetic transformation, 14, 19 and 17 resistant plantes with in-situ replaced PopICE1, PopICE1(DC)1, PopICE1(DC)2 genes are respectively obtained. Compared with the wild-type plant Co1-0 of Arabidopsis thaliana, the cold resistance of the obtained resistant plants is respectively improved by 0.8□, 2.0□ and 1.5□. Embodiment 84 Obtaining Arabidopsis thaliana by insitu replacing PwICE1 gene of Potamogetonwrightii of different degrees of methylation through a CRISPR/Cas9 technology

The PwICE1 gene cDNAof Potamogetonwrightiiis cloned and has a sequence of SEQ ID NO: 49. According to degenerate features of a codon, sites that can be theoretically methylated onthe PwICE1 gene are replaced. According to requirements on target traits, the sites of different degrees of methylationonthe PwICE1 gene are quantitatively replaced. The PwICE1 gene on which all methylation sites are replaced has a sequence of SEQ ID NO: 90 and is named PwICE1(DC)1; and the PwICE1 gene on which part of methylation sites are replaced has a sequence of SEQ ID NO: 131 and is named PwICE1(DC)2. The sgRNA with a guiding function is artificially designed and has a length of 20 nt. The sgRNA sequences of the PwICE1(DC)1 and PwICE1(DC)2 genes are both 5'-GCTCCTATTTCGATGGGGTT NGG-3'. The Cas9 protein is guided to perform site-specific cleavage for DNA. The YAO promoter is selected. The vector pHEE401E and PtICE1(DC)1 and PtICE1(DC)2 genes of different degrees of methylation are cleaved by restriction endonucleases BsaI and KpnI, and the target gene is bonded behind the zCas9 sequence. Rrestriction enzyme cutting sites SacII and AsiSI are added to two ends of a Bar gene, the pHEE401E and the Bar gene are subjected to enzyme digestion by SacII and AsiSI, and the Bar gene is bonded into pHEE401E and used as a resistant screening gene for constructing a CRISPR/Cas9 expression vector.

The operation method of an experiment for genetically transformating Arabidopsis thalian by using PwICE1 gene of different degrees of methylation of Potamogetonwrightii is similar to that of an experimentin embodiment 47. In the genetic transformation experiment, a total of 18, 15 and 19 plants with in-situ replaced PwICE1, PwICE1(DC)₁ and PwICE1(DC)₂ genes of Potamogetonwrightii are respectively obtained. Compared with the wild-type plant of Arabidopsis thaliana, the cold resistance of the obtained resistant plants is respectively improved by 0.7□, 1.9□ and 1.2□.

The present invention is apparently not limited to the above embodiments and may have numorous variations. Sorghum bicolor and Saccharum officinarum are in a same grass family with Oryza sativa, Zea mays, Triticum aestivum and Hordeum vulgare in the above embodiments; Capsicum annuum and Solanum tuberosum are in a same Solanaceae family with Nicotiana tabacum, Solanum capsicoides and Solanum lycopersicum in the above embodiments; other vegetables are in a same cruciferous family with Brassica campestris, Brassica rapa and Raphanus sativus; and Hevea brasiliensis,Manihot esculenta, Dimocarpus longa, Theobroma cacao and other tropical crops such as Chaenomeles sinensis and the like are included.All variations that can be directly derived or associated by those of ordinary skills in the prior art from the disclosure of the present invention should fall within the protection scope of the present invention.

## Claims

1. A method for epigenetically manipulating phenotypic plasticity traits of plants, **characterized by** comprising: quantitatively replacing a methylation site of a target gene by means of a degenerate codon, and carrying out a transgenic manipulation by designing a synthetic gene; or constructing a plant expression vector by using CRISPR/Cas9 editing technology to quantitatively replace the methylation site with an in-situ in-vivo gene degenerate codon and to quantitatively regulate a methylation degree and an expression level of an exogenous or in-vivo target gene in a receptor to attain quantitative regulation of a plasticity trait of a plant.

2. The method according to claim 1, **characterized by** comprising: designing a synthetic geneafter a methylation cytimidine site of the target gene is quantitatively replaced by the degenerate codon,and then carrying out the transgenic genetic manipulation to quantitatively regulate the methylation degree and gene expression level of the exogenous target gene in the receptor so as to attain quantitative regulation of the plasticity trait of the plant.

3. The method according to claim 1, **characterized by** comprising: constructing the plant expression vector by using CRISPR/Cas9 technology to replace the methylation cytimidine site with the in-situ in-vivo gene degenerate codon, and then quantitatively regulating the methylation degree and gene expression level of the in-vivo target gene to attain quantitative regulation of the plasticity trait of the plant.

4. The method according to any one of claims 1-3, **characterized in that** the plasticity trait is cold resistance.

5. The method according to claim 4, **characterized in that** the target gene is ICE1 gene.

6. The method according to claim 4, **characterized in that** the *ICE1* gene is selected from Ageratina adenophora *AaICE1* gene, Manihot esculenta *MeICE1* gene, Actinidia chinensis *AcICE1* gene, Musa nana *MaICE1* gene, Musa basjoo *MabICE1* gene, Theobroma cacao *TcICE1* gene, Citrus trifoliata *CtICE1* gene, Vitis vinifera *VvICE1* gene, Malus domestica *MdICE1* gene, Chorispora bungeana *CbICE1* gene, Solanum capsicoides *ScICE1* gene, Solanum lycopersicum *SlICE1* gene, *Prunus persica PpICE1* gene, Zea mays *ZmICE1* gene, Gossypium hirsutum *GhICE1* gene, Arachis hypogaea *AhICE1* gene, Brassica juncea *BjICE1* gene, Brassica rapa *BrICE1* gene, Chrysanthemum dichrum *CdICE1* gene, Thellungiella halophila *ThICE1* gene, Daucus carota *DcICE1* gene, Glycine max *GmICE1* gene, Raphanus sativus *RsICE1* gene, Triticum aestivum *TaICE1* gene, Hordeum vulgare *HvICE1* gene, Hevea brasiliensis *HbICE1* gene, Juglans regia *JrICE1* gene, Lactuca sativa *LsICE1* gene, Oryza sativa *OsICE1* gene, Phalaenops is aphrodite *PaICE1* gene, Prunus mume *PmICE1* gene, Camellia sinensis *CsICE1* gene, Arabidopsis thaliana *AtICE1* gene, Brassica rapa *BrrICE1* gene, Isatis tinctoria *ItICE1* gene, Eucalyptus camaldulensis *EcICE1* gene, Jatropha curcas *JcICE1* gene, Picea abies *PiaICE1* gene, Populus suaveolens *PsICE1* gene, Populus tomentosa *PtICE1* gene, Potamogeton perfoliatus *PopICE1* gene, Potamogeton wrightii *PwICE1* gene, wherein a nucleotide sequence of Ageratina adenophora AaICE1 gene is SEQ ID NO: 1, a nucleotide sequence of Manihot esculenta MeICE1 gene is SEQ ID NO: 9, a nucleotide sequence of Actinidia chinensis AcICE1 gene is SEQ ID NO: 10, a nucleotide sequence of Musa nana MaICE1 gene is SEQ ID NO: 11, a nucleotide sequence of Musa basjoo MabICE1 gene is SEQ ID NO: 12, a nucleotide sequence of Theobroma cacao TcICE1 gene is SEQ ID NO: 13, a nucleotide sequence of Citrus trifoliata CtICE1 gene is SEQ ID NO: 14, a nucleotide sequence of Vitis vinifera VvICE1 gene is SEQ ID NO: 15, a nucleotide sequence of Malus domestica MdICE1 gene is SEQ ID NO: 16, a nucleotide sequence of Chorispora bungeana CbICE1 gene is SEQ ID NO: 17, a nucleotide sequence of Solanum capsicoides ScICE1 gene is SEQ ID NO: 18, a nucleotide sequence of Solanum lycopersicum SlICE1 gene is SEQ ID NO: 19, a nucleotide sequence of Prunus persica PpICE1 gene is SEQ ID NO: 20, a nucleotide sequence of Zea mays ZmICE1 gene is SEQ ID NO: 21, a nucleotide sequence of Gossypium hirsutum GhICE1 gene is SEQ ID NO: 22, a nucleotide sequence of Arachis hypogaea AhICE1 gene is SEQ ID NO: 23, a nucleotide sequence of Brassica juncea BjICE1 gene is SEQ ID NO: 24, a nucleotide sequence of Brassica rapa BrICE1 gene is SEQ ID NO: 25, a nucleotide sequence of Chrysanthemum dichrum CdICE1 gene is SEQ ID NO: 26, a nucleotide sequence of Thellungiella halophila ThICE1 gene is SEQ ID NO: 27, a nucleotide sequence of Daucus carota DcICE1 gene is SEQ ID NO: 28, a nucleotide sequence of Glycine max GmICE1 gene is SEQ ID NO: 29, a nucleotide sequence of Raphanus sativus RsICE1 gene is SEQ ID NO: 30, a nucleotide sequence of Triticum aestivum TaICE1 gene is SEQ ID NO: 31, a nucleotide sequence of Hordeum vulgare HvICE1 gene is SEQ ID NO: 32, a nucleotide sequence of Hevea brasiliensis HbICE1 gene is SEQ ID NO: 33, a nucleotide sequence of Juglans regia JrICE1 gene is SEQ ID NO: 34, a nucleotide sequence of Lactuca sativa LsICE1 gene is SEQ ID NO: 35, a nucleotide sequence of Oryza sativa OsICE1 gene is SEQ ID NO: 36, a nucleotide sequence of Phalaenopsis aphrodite PaICE1 gene is SEQ ID NO: 37, a nucleotide sequence of Prunus mume PmICE1 gene is SEQ ID NO: 38, a nucleotide sequence of Camellia sinensis CsICE1 gene is SEQ ID NO: 39, a nucleotide sequence of Arabidopsis thaliana AtICE1 gene is SEQ ID NO: 40, a nucleotide sequence of Brassica rapa BrrICE1 gene is SEQ ID NO: 41, a nucleotide sequence of Isatis tinctoria ItICE1 gene is SEQ ID NO: 42, a nucleotide sequence of Eucalyptus camaldulensis EcICE1 gene is SEQ ID NO: 43, a nucleotide sequence of Jatropha curcas JcICE1 gene is SEQ ID NO: 44, a nucleotide sequence of Picea abies PiaICE1 gene is SEQ ID NO: 45, a nucleotide sequence of Populus suaveolens PsICE1 gene is SEQ ID NO: 46, a nucleotide sequence of Populus tomentosa PtICE1 gene is SEQ ID NO: 47, a nucleotide sequence of Potamogeton perfoliatus PopICE1 gene is SEQ ID NO: 48, and a nucleotide sequence of Potamogeton wrightii PwICE1 gene is SEQ ID NO: 49.

7. The method according to claim 6, **characterized in that** the nucleotide sequences of the ICE1 genes after methylation cytimidine is all replaced by the degenerate codon are: Ageratina adenophora: SEQ ID NO: 3, Manihot esculenta: SEQ ID NO: 50, Actinidia chinensis: SEQ ID NO: 51, Musa nana: SEQ ID NO: 52, Musa basjoo: SEQ ID NO: 53, Theobroma cacao: SEQ ID NO: 54,Citrus trifoliata: SEQ ID NO: 55,Vitis vinifera: SEQ ID NO: 56, Malus domestica: SEQ ID NO: 57, Chorispora bungeana: SEQ ID NO: 58, Solanum capsicoides : SEQ ID NO: 59, Solanum lycopersicum: SEQ ID NO: 60, Prunus persica: SEQ ID NO: 61, Zea mays: SEQ ID NO: 62, Gossypium hirsutum: SEQ ID NO: 63, Arachis hypogaea: SEQ ID NO: 64, Brassica juncea: SEQ ID NO: 65, Brassica rapa: SEQ ID NO: 66, Chrysanthemum dichrum: SEQ ID NO: 67, Thellungiella halophila: SEQ ID NO: 68, Daucus carota: SEQ ID NO: 69, Glycine max: SEQ ID NO: 70, Raphanus sativus: SEQ ID NO: 71, Triticum aestivum: SEQ ID NO: 72, Hordeum vulgare: SEQ ID NO: 73, Heveabrasiliensis: SEQ ID NO: 74,Juglans regia: SEQ ID NO: 75, Lactuca sativa: SEQ ID NO: 76, Oryza sativa: SEQ ID NO: 77, Phalaenopsisaphrodite: SEQ ID NO: 78, Prunus mume: SEQ ID NO: 79, Camellia sinensis: SEQ ID NO: 80, Arabidopsis thaliana: SEQ ID NO: 81, Brassica rapa: SEQ ID NO: 82, Isatis tinctoria: SEQ ID NO: 83,Eucalyptus camaldulensis: SEQ ID NO: 84, Jatrophacurcas: SEQ ID NO: 85, Piceaabies: SEQ ID NO: 86, Populus suaveolens: SEQ ID NO: 87, Populus tomentosa: SEQ ID NO: 88, Potamogeton perfoliatus: SEQ ID NO: 89, Potamogetonwrightii: SEQ ID NO: 90; and nucleotide sequences of the ICE1 genes after methylation cytimidine is partially replaced by the degenerate codon are: Ageratina adenophora: SEQ ID NO: 4, Manihot esculentaManihot esculenta: SEQ ID NO: 91, Actinidia chinensis: SEQ ID NO: 92, Musa nana: SEQ ID NO: 93, Musa basjoo: SEQ ID NO: 94, Theobroma cacao: SEQ ID NO: 95,Citrus trifoliata: SEQ ID NO: 96,Vitis vinifera: SEQ ID NO: 97, Malus domestica: SEQ ID NO: 98, Chorispora bungeana: SEQ ID NO: 99, Solanum capsicoides : SEQ ID NO: 100 Solanum lycopersicum: SEQ ID NO: 101, Prunus persica: SEQ ID NO: 102, Zea mays: SEQ ID NO: 103, Gossypium hirsutum: SEQ ID NO: 104, Arachis hypogaea: SEQ ID NO: 105, Brassica juncea: SEQ ID NO: 106, Brassica rapa: SEQ ID NO: 107, Chrysanthemum dichrum: SEQ ID NO: 108, Thellungiella halophila: SEQ ID NO: 109, Daucus carota: SEQ ID NO: 110, Glycine max: SEQ ID NO: 111, Raphanus sativus: SEQ ID NO: 112, Triticum aestivum: SEQ ID NO: 113, Hordeum vulgare: SEQ ID NO: 114, Heveabrasiliensis: SEQ ID NO: 115,Juglans regia: SEQ ID NO: 116, Lactuca sativa: SEQ ID NO: 117, Oryza sativa: SEQ ID NO: 118, Phalaenopsisaphrodite: SEQ ID NO: 119, Prunusmume: SEQ ID NO: 120, Camellia sinensis: SEQ ID NO: 121, Arabidopsis thaliana: SEQ ID NO: 122, Brassica rapa: SEQ ID NO: 123, Isatis tinctoria: SEQ ID NO: 124,Eucalyptus camaldulensis: SEQ ID NO: 125, Jatropha curcas: SEQ ID NO: 126, Picea abies: SEQ ID NO: 127, Populus suaveolens: SEQ ID NO: 128, Populus tomentosa: SEQ ID NO: 129, Potamogeton perfoliatus: SEQ ID NO: 130, Potamogetonwrightii: SEQ ID NO: 131.

8. The method according to claim 6, **characterized by** comprising: introducing the synthetic AaICE1 gene or the AaICE1 gene after the methylation cytimidine is all or partially insitu replaced with the degenerate codon by means of CRISRP/Cas9 into plants of Oryza, Manihot esculentaManihot esculenta, Musa nana, Solanum lycopersicum, Theobroma cacao, Heveabrasiliensis, Citrus limon, Citrus sinensis, Citrus reticulata, Saccharumofficinarum, Carica papaya, Eriobotrya japonica, Litchi chinensis, Dimocarpuslongan, Mangiferaindica, Solanumtuberosum, Capsicum annuum, Sorghum bicolor, Vitis vinifera, Malus domestica, Chorispora bungeana, Solanum capsicoides , Actinidia chinensis, Prunus persica, Zea mays, Gossypium hirsutumspp, Arachis hypogaea, Brassica juncea,Brassica rapa, Chrysanthemum dichrum, Daucus carota, Glycine max, Raphanus sativus, Triticum aestivum, Hordeum vulgare, Juglans regia, Lactuca sativa, Cymbidium hybridum, Phalaenopsisaphrodite, Chimonanthus praecox, Camellia sinensis and Eucalyptus camaldulensi to obtain transgenetic plants with enhanced cold resistance, wherein the AaICE1 gene after the methylation cytimidine is all replaced has a sequence of SEQ ID NO: 3, and the AaICE1 gene after the methylation cytimidine is partially replaced has a sequence of SEQ ID NO: 4.

9. A cold-resistant gene, **characterized by** being selected from an ICE1 gene after the methylation cytimidine is all or partially replaced.

10. The gene according to claim 9, **characterized in that** thewild-type ICE1 gene is selected from Ageratina adenophora AaICE1 gene, Manihot esculentaManihot esculenta MeICE1 gene, Actinidia chinensis AcICE1 gene, Musa nana MaICE1 gene, Musa basjoo MabICE1 gene, Theobroma cacao TcICE1 gene, Citrus trifoliata CtICE1 gene, Vitis vinifera VvICE1 gene, Malus domestica MdICE1 gene, Chorispora bungeana CbICE1 gene, Solanum capsicoides ScICE1 gene, Solanum lycopersicum SlICE1 gene, Prunus persica PpICE1 gene, Zea mays ZmICE1 gene, Gossypium hirsutum GhICE1 GENE, Arachis hypogaea AhICE1 gene, Brassica juncea BjICE1 gene, Brassica rapa BrICE1 gene, Chrysanthemum dichrum CdICE1 gene, Thellungiella halophila ThICE1 gene, Daucus carota DcICE1 gene, Glycine max GmICE1 gene, Raphanus sativus RsICE1 gene, Triticum aestivum TaICE1 gene, Hordeum vulgare HvICE1 gene, Heveabrasiliensis HbICE1 gene, Juglans regia JrICE1 gene, Lactuca sativa LsICE1 gene, Oryza sativa OsICE1 gene, Phalaenopsisaphrodite PaICE1 gene, Prunusmume PmICE1 gene, Camellia sinensis CsICE1 gene, Arabidopsis thaliana AtICE1 gene, Brassica rapaBrrICE1 gene, Isatis tinctoria ItICE1 gene, Eucalyptus camaldulensis EcICE1 gene, Jatrophacurcas JcICE1 gene, Piceaabies PiaICE1 gene, Populus suaveolens PsICE1 gene, Populus tomentosa PtICE1 gene, Potamogeton perfoliatus PopICE1 gene, Potamogetonwrightii PwICE1 gene, wherein a nucleotide sequence of Ageratina adenophora AaICE1 gene is SEQ ID NO: 1, a nucleotide sequence of Manihot esculentaManihot esculenta MeICE1 gene is SEQ ID NO: 9, a nucleotide sequence of Actinidia chinensis AcICE1 gene is SEQ ID NO: 10, a nucleotide sequence of Musa nana MaICE1 gene is SEQ ID NO: 11, a nucleotide sequence of Musa basjoo MabICE1 gene is SEQ ID NO: 12, a nucleotide sequence of Theobroma cacao TcICE1 gene is SEQ ID NO: 13, a nucleotide sequence of Citrus trifoliata CtICE1 gene is SEQ ID NO: 14, a nucleotide sequence of Vitis vinifera VvICE1 gene is SEQ ID NO: 15, a nucleotide sequence of Malus domestica MdICE1 gene is SEQ ID NO: 16, a nucleotide sequence of Chorispora bungeana CbICE1 gene is SEQ ID NO: 17,a nucleotide sequence of Solanum capsicoides ScICE1 gene is SEQ ID NO: 18, a nucleotide sequence of Solanum lycopersicum SlICE1 gene is SEQ ID NO: 19, a nucleotide sequence of Prunus persica PpICE1 gene is SEQ ID NO: 20, a nucleotide sequence of Zea mays ZmICE1 gene is SEQ ID NO: 21, a nucleotide sequence of Gossypium hirsutum GhICE1 gene is SEQ ID NO: 22, a nucleotide sequence of Arachis hypogaea AhICE1 gene is SEQ ID NO: 23, a nucleotide sequence of Brassica juncea BjICE1 gene is SEQ ID NO: 24, a nucleotide sequence of Brassica rapa BrICE1 gene is SEQ ID NO: 25, a nucleotide sequence of Chrysanthemum dichrum CdICE1 gene is SEQ ID NO: 26, a nucleotide sequence of Thellungiella halophila ThICE1 gene is SEQ ID NO: 27, a nucleotide sequence of Daucus carota DcICE1 gene is SEQ ID NO: 28, a nucleotide sequence of Glycine max GmICE1 gene is SEQ ID NO: 29, a nucleotide sequence of Raphanus sativus RsICE1 gene is SEQ ID NO: 30, a nucleotide sequence of Triticum aestivum TaICE1 gene is SEQ ID NO: 31, a nucleotide sequence of Hordeum vulgare HvICE1 gene is SEQ ID NO: 32, a nucleotide sequence of Heveabrasiliensis HbICE1 gene is SEQ ID NO: 33, a nucleotide sequence of Juglans regia JrICE1 gene is SEQ ID NO: 34, a nucleotide sequence of Lactuca sativa LsICE1 gene is SEQ ID NO: 35, a nucleotide sequence of Oryza sativa OsICE1 gene is SEQ ID NO: 36, a nucleotide sequence of Phalaenopsis aphrodite PaICE1 gene is SEQ ID NO: 37, a nucleotide sequence of Prunusmume PmICE1 gene is SEQ ID NO: 38, a nucleotide sequence of Camellia sinensis CsICE1 gene is SEQ ID NO: 39, a nucleotide sequence ofArabidopsis thaliana AtICE1 gene is SEQ ID NO: 40, a nucleotide sequence of Brassica rapa BrrICE1 gene is SEQ ID NO: 41, a nucleotide sequence of Isatis tinctoria ItICE1 gene is SEQ ID NO: 42, a nucleotide sequence of Eucalyptus camaldulensis EcICE1 gene is SEQ ID NO: 43, a nucleotide sequence of Jatropha curcas JcICE1 gene is SEQ ID NO: 44, a nucleotide sequence of Picea abies PiaICE1 gene is SEQ ID NO: 45, a nucleotide sequence of Populus suaveolens PsICE1 gene is SEQ ID NO: 46, a nucleotide sequence of Populus tomentosa PtICE1 gene is SEQ ID NO: 47, a nucleotide sequence of Potamogeton perfoliatus PopICE1 gene is SEQ ID NO: 48, and a nucleotide sequence of Potamogeton wrightii PwICE1 gene is SEQ ID NO: 49;
the nucleotide sequences of the ICE1 genes after the methylation cytimidine is all replaced by the degenerate codon are: Ageratina adenophora: SEQ ID NO: 3, Manihot esculentaManihot esculenta: SEQ ID NO: 50, Actinidia chinensis: SEQ ID NO: 51, Musa nana: SEQ ID NO: 52, Musa basjoo: SEQ ID NO: 53, Theobroma cacao: SEQ ID NO: 54,Citrus trifoliata: SEQ ID NO: 55,Vitis vinifera: SEQ ID NO: 56, Malus domestica: SEQ ID NO: 57, Chorispora bungeana: SEQ ID NO: 58, Solanum capsicoides : SEQ ID NO: 59, Solanum lycopersicum: SEQ ID NO: 60, Prunus persica: SEQ ID NO: 61, Zea mays: SEQ ID NO: 62, Gossypium hirsutum: SEQ ID NO: 63, Arachis hypogaea: SEQ ID NO: 64, Brassica juncea: SEQ ID NO: 65, Brassica rapa: SEQ ID NO: 66, Chrysanthemum dichrum: SEQ ID NO: 67, Thellungiella halophila: SEQ ID NO: 68, Daucus carota: SEQ ID NO: 69, Glycine max: SEQ ID NO: 70, Raphanus sativus: SEQ ID NO: 71, Triticum aestivum: SEQ ID NO: 72, Hordeum vulgare: SEQ ID NO: 73, Heveabrasiliensis: SEQ ID NO: 74,Juglans regia: SEQ ID NO: 75, Lactuca sativa: SEQ ID NO: 76, Oryza sativa: SEQ ID NO: 77, Phalaenopsisaphrodite: SEQ ID NO: 78, Prunusmume: SEQ ID NO: 79, Camellia sinensis: SEQ ID NO: 80, Arabidopsis thaliana: SEQ ID NO: 81, Brassica rapa: SEQ ID NO: 82, Isatis tinctoria: SEQ ID NO: 83,Eucalyptus camaldulensis: SEQ ID NO: 84, Jatropha curcas: SEQ ID NO: 85, Picea abies: SEQ ID NO: 86, Populus suaveolens: SEQ ID NO: 87, Populus tomentosa: SEQ ID NO: 88, Potamogeton perfoliatus: SEQ ID NO: 89, Potamogeton wrightii: SEQ ID NO: 90; and
the nucleotide sequences of the ICE1 genes after the methylation cytimidine is partially replaced by the degenerate codon are: Ageratina adenophora: SEQ ID NO: 4, Manihot esculenta: SEQ ID NO: 91, Actinidia chinensis: SEQ ID NO: 92, Musa nana: SEQ ID NO: 93, Musa basjoo: SEQ ID NO: 94, Theobroma cacao: SEQ ID NO: 95,Citrus trifoliata: SEQ ID NO: 96,Vitis vinifera: SEQ ID NO: 97, Malus domestica: SEQ ID NO: 98, Chorispora bungeana: SEQ ID NO: 99, Solanum capsicoides : SEQ ID NO: 100 Solanum lycopersicum: SEQ ID NO: 101, Prunus persica: SEQ ID NO: 102, Zea mays: SEQ ID NO: 103, Gossypium hirsutum: SEQ ID NO: 104, Arachis hypogaea: SEQ ID NO: 105, Brassica juncea: SEQ ID NO: 106, Brassica rapa: SEQ ID NO: 107, Chrysanthemum dichrum: SEQ ID NO: 108, Thellungiella halophila: SEQ ID NO: 109, Daucus carota: SEQ ID NO: 110, Glycine max: SEQ ID NO: 111, Raphanus sativus: SEQ ID NO: 112, Triticum aestivum: SEQ ID NO: 113, Hordeum vulgare: SEQ ID NO: 114, Heveabrasiliensis: SEQ ID NO: 115,Juglans regia: SEQ ID NO: 116, Lactuca sativa: SEQ ID NO: 117, Oryza sativa: SEQ ID NO: 118, Phalaenopsisaphrodite: SEQ ID NO: 119, Prunusmume: SEQ ID NO: 120, Camellia sinensis: SEQ ID NO: 121, Arabidopsis thaliana: SEQ ID NO: 122, Brassica rapa: SEQ ID NO: 123, Isatis tinctoria: SEQ ID NO: 124,Eucalyptus camaldulensis: SEQ ID NO: 125, Jatropha curcas: SEQ ID NO: 126, Picea abies: SEQ ID NO: 127, Populus suaveolens: SEQ ID NO: 128, Populus tomentosa: SEQ ID NO: 129, Potamogeton perfoliatus: SEQ ID NO: 130, Potamogeton wrightii: SEQ ID NO: 131.

11. An expression vector comprising ICE1 gene after methylation cytimidine is all or partially replaced according to claim 10.

12. An application of the ICE1 gene according to claim 10 in cultivating cold-resistant plants.

13. The application according to claim 12, **characterized by** comprising the following steps:
(1) constructing a plant transformation plasmid comprising the ICE1 gene by using DNA recombination technology; and
(2)introducing the plant transformation plasmid constructed in step (1) into plant tissue by a gene gun or an agrobacterium infection method or pollen-mediated transformation, or carrying out the in-situ replacement for a receptor plant by using the CRISPR/Cas9 Technology to obtain a transgenic or gene-edited plant comprising the ICE1 genes of different degrees of methylation.

14. The application according to claim 12 or 13, **characterized in that** the plant is selected from Oryza, Manihot esculenta, Musa nana, Solanum lycopersicum, Theobroma cacao, Heveabrasiliensis, Citrus limon, Citrus sinensis, Citrus reticulata, Saccharumofficinarum, Carica papaya, Eriobotrya japonica, Litchi chinensis, Dimocarpuslongan, Mangiferaindica, Solanumtuberosum, Capsicum annuum, Sorghum bicolor, Vitis vinifera, Malus domestica, Chorispora bungeana, Solanum capsicoides , Actinidia chinensis, Prunus persica, Zea mays, Gossypium hirsutumspp, Arachis hypogaea, Brassica juncea,Brassica rapa, Chrysanthemum dichrum, Daucus carota, Glycine max, Raphanus sativus, Triticum aestivum, Hordeum vulgare, Juglans regia, Lactuca sativa, Cymbidium hybridum, Phalaenopsisaphrodite, Chimonanthus praecox, Camellia sinensis and Eucalyptus camaldulensi.


<110> NANJING AGRICULTURAL UNIVERSITY
<120> Method for Epigenetically Manipulating Phenotypic Plasticity Traits of
Plants

<130>  U30946WOEP

<140> EP18764709.4
<141> 2018-03-07

<150> PCT/CN2017/075976
<151> 2017-03-08

<160>131

<210> 1
<211> 1506
<212> DNA
<213> Ageratina adenophorum Spreng.
<220>
<400> 1
atgcttcccg ccggcggttc tatctggatg gccggagaac caagccaaga cgaaacatct      60
tcatggtcac ccacaaccca caacatcacc ggaattgaac ccactgcaac atcatgcaac     120
gacgctgacg accaccaccg catcaccacc aacgccacca gttattcatc tctcaccacc     180
ttgaaatcaa tgctggaaac cgaatggtat caccaccaca acaatctgaa tctcccttcc     240
gacggtaact ctctgttctt acccatggat tcatcgtctt cgtgttctcc gtcgcaatct     300
cataatcagt ttacccaatc ttcttacccc ttttttccac ccaaattcaa taacaatttg     360
aacaacccgt atgatctgcc gtttgatttc ggatgcgaat ctgctttctt accgaatcac     420
acttccaatt tgatgagttt tgctggttta tcttctcaaa atcacatccc catgccggag     480
atttcgtcca gttccgactt tccggcgacc aacaataaca acagcaacga caccggcgac     540
attgtaggac ccggtttcaa cccgaattta actgggttcg acggatttca cggcactgta     600
ccccaaactt cattgtttcc ggcgaggtct aaggttctcc ggccgcttga gatttctcca     660
ccggtcggag ctcagcctac actgtttcaa aaacgggcgg cgttacggca aagctcaggc     720
aaattgggtc cggttgaaaa tgaaaagaaa cggaagagaa gtgaggaaga tgaattcgat     780
gaaacgggta cgattgatgt ttctggattc aattacgatt ccgatgaaat tgaaccgatt     840
ggtgagcttg ctaacggaaa tggtgaaagc agttttgtta ccattggagg tgagaatcaa     900
agaggtaaga aaaaggggtt acctgcaaag aatttaatgg cggagagacg acggaggaag     960
aagctcaatg acagactgta tatgcttaga tcagttgtcc ccaaaattag caagatggat    1020
agagcttcga ttcttggtga cgcgattgat tacttgaagg aactactaca aaaaatcaat    1080
gatcttcata atgaacttga ggcaacacca caagggtctt tgatgcaaac ttcatcaagc    1140
atgcatcccc taacacccac ctcgccggcc cttctgcaac atgtcaaaga agaactatgt    1200
ccaaccgcca ccaacaacat tctcggcccc aaaaaccatc ttgcaaaggt cgaagtatat    1260
gcaaaagaag cgaggggtgt gaacatccac atgctgtgcg ggcgcagacc gggtctctta    1320
ctttctacat tgagggctct ggacaacctt gggctggaca ttcaacaagc tgtcataagc    1380
tgtttcaatg ggtttgcttt ggatgtattt cgagcccagc aatgtaggga agggcaggat    1440
atgttgcctg agcaaataaa agcagtgctt ctagagacag ctggatatca tcatggtgcc    1500
atttaa
1506

<210> 2
<211> 501
<212> PRT
<213> Ageratina adenophorum Spreng.
<220>
<400> 2
Met Leu Pro Ala Gly Gly Ser Ile Trp Met Ala Gly Glu Pro Ser Gln
1               5                   10                  15
Asp Glu Thr Ser Ser Trp Ser Pro Thr Thr His Asn Ile Thr Gly Ile
            20                  25                  30
Glu Pro Thr Ala Thr Ser Cys Asn Asp Ala Asp Asp His His Arg Ile
        35                  40                  45
Thr Thr Asn Ala Thr Ser Tyr Ser Ser Leu Thr Thr Leu Lys Ser Met
    50                  55                  60
Leu Glu Thr Glu Trp Tyr His His His Asn Asn Leu Asn Leu Pro Ser

65                  70                  75                  80
Asp Gly Asn Ser Leu Phe Leu Pro Met Asp Ser Ser Ser Ser Cys Ser
                85                  90                  95
Pro Ser Gln Ser His Asn Gln Phe Thr Gln Ser Ser Tyr Pro Phe Phe
            100                 105                 110
Pro Pro Lys Phe Asn Asn Asn Leu Asn Asn Pro Tyr Asp Leu Pro Phe
        115                 120                 125
Asp Phe Gly Cys Glu Ser Ala Phe Leu Pro Asn His Thr Ser Asn Leu
    130                 135                 140
Met Ser Phe Ala Gly Leu Ser Ser Gln Asn His Ile Pro Met Pro Glu
145                 150                 155                 160
Ile Ser Ser Ser Ser Asp Phe Pro Ala Thr Asn Asn Asn Asn Ser Asn
                165                 170                 175
Asp Thr Gly Asp Ile Val Gly Pro Gly Phe Asn Pro Asn Leu Thr Gly
            180                 185                 190
Phe Asp Gly Phe His Gly Thr Val Pro Gln Thr Ser Leu Phe Pro Ala
        195                 200                 205
Arg Ser Lys Val Leu Arg Pro Leu Glu Ile Ser Pro Pro Val Gly Ala
    210                 215                 220
Gln Pro Thr Leu Phe Gln Lys Arg Ala Ala Leu Arg Gln Ser Ser Gly
225                 230                 235                 240
Lys Leu Gly Pro Val Glu Asn Glu Lys Lys Arg Lys Arg Ser Glu Glu
                245                 250                 255
Asp Glu Phe Asp Glu Thr Gly Thr Ile Asp Val Ser Gly Phe Asn Tyr
            260                 265                 270
Asp Ser Asp Glu Ile Glu Pro Ile Gly Glu Leu Ala Asn Gly Asn Gly
        275                 280                 285
Glu Ser Ser Phe Val Thr Ile Gly Gly Glu Asn Gln Arg Gly Lys Lys
    290                 295                 300
Lys Gly Leu Pro Ala Lys Asn Leu Met Ala Glu Arg Arg Arg Arg Lys
305                 310                 315                 320
Lys Leu Asn Asp Arg Leu Tyr Met Leu Arg Ser Val Val Pro Lys Ile
                325                 330                 335
Ser Lys Met Asp Arg Ala Ser Ile Leu Gly Asp Ala Ile Asp Tyr Leu
            340                 345                 350
Lys Glu Leu Leu Gln Lys Ile Asn Asp Leu His Asn Glu Leu Glu Ala
        355                 360                 365
Thr Pro Gln Gly Ser Leu Met Gln Thr Ser Ser Ser Met His Pro Leu
    370                 375                 380
Thr Pro Thr Ser Pro Ala Leu Leu Gln His Val Lys Glu Glu Leu Cys
385                 390                 395                 400
Pro Thr Ala Thr Asn Asn Ile Leu Gly Pro Lys Asn His Leu Ala Lys
                405                 410                 415
Val Glu Val Tyr Ala Lys Glu Ala Arg Gly Val Asn Ile His Met Leu
            420                 425                 430
Cys Gly Arg Arg Pro Gly Leu Leu Leu Ser Thr Leu Arg Ala Leu Asp
        435                 440                 445
Asn Leu Gly Leu Asp Ile Gln Gln Ala Val Ile Ser Cys Phe Asn Gly
    450                 455                 460
Phe Ala Leu Asp Val Phe Arg Ala Gln Gln Cys Arg Glu Gly Gln Asp
465                 470                 475                 480
Met Leu Pro Glu Gln Ile Lys Ala Val Leu Leu Glu Thr Ala Gly Tyr
                485                 490                 495
His His Gly Ala Ile
            500

<210> 3
<211> 1506
<212> DNA
<213> Ageratina adenophorum Spreng.
<220>
<400> 3
atgctacctg ctggaggttc tatttggatg gctggagaac ctagtcaaga tgaaacatca        60

tcatggtcac ctactacaca taacatcact ggaattgaac ctactgctac atcatgtaat 120
gatgctgatg atcaccatcg tatcaccact aatgctacta gttattcatc tctcaccaca 180
ttaaaatcta tgctagaaac tgaatggtat caccaccaca ataatctaaa tctaccatct 240
gatggtaact ctctattttt acctatggat tcatcatcat catgttctcc atctcaatct 300
cataatcagt ttactcaatc atcatatcca ttttttccac ctaaatttaa taataatttg 360
aataatccat atgatctacc atttgatttt ggatgtgaat ctgcattttt acctaatcac 420
acatctaatt tgatgagttt tgctggttta tcatctcaaa atcacatacc tatgcctgag 480
atttcatcta gttctgattt tcctgctact aacaataata atagtaatga tactggagat 540
attgtaggac ctggttttaa tcctaattta actgggtttg atggatttca tggaactgta 600
cctcaaactt cattgtttcc tgctaggtct aaggttctac gacctctaga gatttctcca 660
cctgtaggag ctcagcctac actatttcaa aaacgtgctg cattacgtca aagttcagga 720
aaattgggtc ctgttgaaaa tgaaaagaaa cgtaagagaa gtgaggaaga tgaatttgat 780
gaaactggta ctattgatgt ttctggattt aattatgatt ctgatgaaat tgaacctatt 840
ggtgaactag ctaatggaaa tggtgaaagt agttttgtta ccataggagg tgagaatcaa 900
agaggtaaaa aaaaagggtt acctgcaaaa aatttaatgg ctgagagacg acgtaggaag 960
aaacttaatg atagacttta tatgctaaga tcagttgtac ctaaaattag taagatggat 1020
agagcttcta ttctaggtga tgctattgat tatttgaagg aactactaca aaaaataaat 1080
gatctacata atgaactaga ggctacacca caagggtcat tgatgcaaac atcatctagt 1140
atgcatcctc taacacctac ctctcctgct ctactacaac atgttaaaga agaactatgt 1200
cctactgcca ctaataacat tctaggacct aaaaaccatc tagcaaaggt agaagtatat 1260
gctaaagaag ctaggggtgt gaacatccac atgctatgtg ggcgtagacc tggtctatta 1320
ctatctacat tgagggctct agataaccta gggctagaca ttcaacaagc tgtcataagt 1380
tgttttaatg ggtttgcatt ggatgtattt cgagctcagc aatgtaggga agggcaggat 1440
atgttgcctg agcaaataaa agcagtgcta ctagagacag ctggatatca tcatggtgcc 1500
atttaa
1506

<210> 4
<211> 1506
<212> DNA
<213> Ageratina adenophorum Spreng.
<220>
<400> 4
atgctacctg ctggaggttc tatttggatg gctggagaac ctagtcaaga tgaaacatca 60
tcatggtcac ctactacaca taacatcact ggaattgaac ctactgctac atcatgtaat 120
gatgctgatg atcaccatcg tatcaccact aatgctacta gttattcatc tctcaccaca 180
ttaaaatcta tgctagaaac tgaatggtat caccaccaca ataatctaaa tctaccatct 240
gatggtaact ctctattttt acctatggat tcatcatcat catgttctcc atctcaatct 300
cataatcagt ttactcaatc atcatatcca ttttttccac ctaaatttaa taataatttg 360
aataatccat atgatctacc atttgatttt ggatgtgaat ctgcattttt acctaatcac 420
acatctaatt tgatgagttt tgctggttta tcatctcaaa atcacatacc tatgcctgag 480
atttcatcta gttctgattt tccggcgacc aacaataaca acagcaacga caccggcgac 540
attgtaggac ccggtttcaa cccgaattta actgggttcg acggatttca cggcactgta 600
ccccaaactt cattgtttcc ggcgaggtct aaggttctcc ggccgcttga gatttctcca 660
ccggtcggag ctcagcctac actgtttcaa aaacgggcgg cgttacggca aagctcaggc 720
aaattgggtc cggttgaaaa tgaaaagaaa cggaagagaa gtgaggaaga tgaattcgat 780
gaaacgggta cgattgatgt ttctggattc aattacgatt ccgatgaaat tgaaccgatt 840
ggtgagcttg ctaacggaaa tggtgaaagc agttttgtta ccattggagg tgagaatcaa 900
agaggtaaga aaaaggggtt acctgcaaag aatttaatgg cggagagacg acggaggaag 960
aagctcaatg acagactgta tatgcttaga tcagttgtcc ccaaaattag caagatggat 1020
agagcttcga ttcttggtga cgcgattgat tacttgaagg aactactaca aaaaatcaat 1080
gatcttcata atgaacttga ggcaacacca caagggtctt tgatgcaaac ttcatcaagc 1140
atgcatcccc taacacccac ctcgccggcc cttctgcaac atgtcaaaga agaactatgt 1200
ccaaccgcca ccaacaacat tctcggcccc aaaaaccatc ttgcaaaggt cgaagtatat 1260
gcaaaagaag cgaggggtgt gaacatccac atgctgtgcg ggcgcagacc gggtctctta 1320
ctttctacat tgagggctct ggacaacctt gggctggaca ttcaacaagc tgtcataagc 1380
tgtttcaatg ggtttgcttt ggatgtattt cgagcccagc aatgtaggga agggcaggat 1440
atgttgcctg agcaaataaa agcagtgctt ctagagacag ctggatatca tcatggtgcc 1500
atttaa
1506

<210> 5
<211> 1506

<212> DNA
<213> Ageratina adenophorum Spreng.
<220>
<221> modified_base
<222>(12,33,51,120,123,139,222,239,253,282,330,368,485,506,537,552,582,619,62
0,623,677,682,688,701,702,751,777,816,817,821,822,847,855,856,925,942,943,953
,954,994,1011,1033,1047,1174,1202,1224,1272,1304,1310,1370)
<223> m5c
<400> 5
atgcttcccg ccggcggttc tatctggatg gccggagaac caagccaaga cgaaacatct      60
tcatggtcac ccacaaccca caacatcacc ggaattgaac ccactgcaac atcatgcaac     120
gacgctgacg accaccaccg catcaccacc aacgccacca gttattcatc tctcaccacc     180
ttgaaatcaa tgctggaaac cgaatggtat caccaccaca acaatctgaa tctcccttcc     240
gacggtaact ctctgttctt acccatggat tcatcgtctt cgtgttctcc gtcgcaatct     300
cataatcagt ttacccaatc ttcttacccc ttttttccac ccaaattcaa taacaatttg     360
aacaacccgt atgatctgcc gtttgatttc ggatgcgaat ctgctttctt accgaatcac     420
acttccaatt tgatgagttt tgctggttta tcttctcaaa atcacatccc catgccggag     480
atttcgtcca gttccgactt tccggcgacc aacaataaca acagcaacga caccggcgac     540
attgtaggac ccggtttcaa cccgaattta actgggttcg acggatttca cggcactgta     600
ccccaaactt cattgtttcc ggcgaggtct aaggttctcc ggccgcttga gatttctcca     660
ccggtcggag ctcagcctac actgtttcaa aaacgggcgg cgttacggca aagctcaggc     720
aaattgggtc cggttgaaaa tgaaaagaaa cggaagagaa gtgaggaaga tgaattcgat     780
gaaacgggta cgattgatgt ttctggattc aattacgatt ccgatgaaat tgaaccgatt     840
ggtgagcttg ctaacggaaa tggtgaaagc agttttgtta ccattggagg tgagaatcaa     900
agaggtaaga aaaaggggtt acctgcaaag aatttaatgg cggagagacg acggaggaag     960
aagctcaatg acagactgta tatgcttaga tcagttgtcc ccaaaattag caagatggat    1020
agagcttcga ttcttggtga cgcgattgat tacttgaagg aactactaca aaaaatcaat    1080
gatcttcata atgaacttga ggcaacacca caagggtctt tgatgcaaac ttcatcaagc    1140
atgcatcccc taacacccac ctcgccggcc cttctgcaac atgtcaaaga agaactatgt    1200
ccaaccgcca ccaacaacat tctcggcccc aaaaaccatc ttgcaaaggt cgaagtatat    1260
gcaaaagaag cgaggggtgt gaacatccac atgctgtgcg ggcgcagacc gggtctctta    1320
ctttctacat tgagggctct ggacaacctt gggctggaca ttcaacaagc tgtcataagc    1380
tgtttcaatg ggtttgcttt ggatgtattt cgagcccagc aatgtaggga agggcaggat    1440
atgttgcctg agcaaataaa agcagtgctt ctagagacag ctggatatca tcatggtgcc    1500
atttaa
1506
<210> 6
<211> 1506
<212> DNA
<213> Arabidopsis thaliana overexpression AaICE1
<220>
<221> modified_base
<222>(12,74,117,275,295,307,367,368,376,390,401,412,413,426,489,495,572,579,5
96,604,620,677,680,821,822,836,855,923,941,949,952,966,972,976,992,1028,1041,
1126,1163,1165,1166,1272,1298,1302,1304,1307,1310,1311,1341,1351,1363,1370,14
11,1435)
<223> m5c
<400> 6
atgcttcccg ccggcggttc tatctggatg gccggagaac caagccaaga cgaaacatct      60
tcatggtcac ccacaaccca caacatcacc ggaattgaac ccactgcaac atcatgcaac     120
gacgctgacg accaccaccg catcaccacc aacgccacca gttattcatc tctcaccacc     180
ttgaaatcaa tgctggaaac cgaatggtat caccaccaca acaatctgaa tctcccttcc     240
gacggtaact ctctgttctt acccatggat tcatcgtctt cgtgttctcc gtcgcaatct     300
cataatcagt ttacccaatc ttcttacccc ttttttccac ccaaattcaa taacaatttg     360
aacaacccgt atgatctgcc gtttgatttc ggatgcgaat ctgctttctt accgaatcac     420
acttccaatt tgatgagttt tgctggttta tcttctcaaa atcacatccc catgccggag     480
atttcgtcca gttccgactt tccggcgacc aacaataaca acagcaacga caccggcgac     540
attgtaggac ccggtttcaa cccgaattta actgggttcg acggatttca cggcactgta     600
ccccaaactt cattgtttcc ggcgaggtct aaggttctcc ggccgcttga gatttctcca     660
ccggtcggag ctcagcctac actgtttcaa aaacgggcgg cgttacggca aagctcaggc     720
aaattgggtc cggttgaaaa tgaaaagaaa cggaagagaa gtgaggaaga tgaattcgat     780
gaaacgggta cgattgatgt ttctggattc aattacgatt ccgatgaaat tgaaccgatt     840
ggtgagcttg ctaacggaaa tggtgaaagc agttttgtta ccattggagg tgagaatcaa     900

agaggtaaga aaaaggggtt acctgcaaag aatttaatgg cggagagacg acggaggaag 960
aagctcaatg acagactgta tatgcttaga tcagttgtcc ccaaaattag caagatggat 1020
agagcttcga ttcttggtga cgcgattgat tacttgaagg aactactaca aaaaatcaat 1080
gatcttcata atgaacttga ggcaacacca caagggtctt tgatgcaaac ttcatcaagc 1140
atgcatcccc taacacccac ctcgccggcc cttctgcaac atgtcaaaga agaactatgt 1200
ccaaccgcca ccaacaacat tctcggcccc aaaaaccatc ttgcaaaggt cgaagtatat 1260
gcaaaagaag cgaggggtgt gaacatccac atgctgtgcg ggcgcagacc gggtctctta 1320
ctttctacat tgagggctct ggacaacctt gggctggaca ttcaacaagc tgtcataagc 1380
tgtttcaatg ggtttgcttt ggatgtattt cgagcccagc aatgtaggga agggcaggat 1440
atgttgcctg agcaaataaa agcagtgctt ctagagacag ctggatatca tcatggtgcc 1500
atttaa
1506

<210> 7
<211> 1506
<212> DNA
<213> Arabidopsis thaliana overexpression AaICE1(DC)1
<220>
<221> modified_base
<222>(89,104,125,139,785,941,949,952,964,976,992,1111,1126)
<223> m5c
<400> 7
atgctacctg ctggaggttc tatttggatg gctggagaac ctagtcaaga tgaaacatca 60
tcatggtcac ctactacaca taacatcact ggaattgaac ctactgctac atcatgtaat 120
gatgctgatg atcaccatcg tatcaccact aatgctacta gttattcatc tctcaccaca 180
ttaaaatcta tgctagaaac tgaatggtat caccaccaca ataatctaaa tctaccatct 240
gatggtaact ctctattttt acctatggat tcatcatcat catgttctcc atctcaatct 300
cataatcagt ttactcaatc atcatatcca ttttttccac ctaaatttaa taataatttg 360
aataatccat atgatctacc atttgatttt ggatgtgaat ctgcattttt acctaatcac 420
acatctaatt tgatgagttt tgctggttta tcatctcaaa atcacatacc tatgcctgag 480
atttcatcta gttctgattt tcctgctact aacaataata atagtaatga tactggagat 540
attgtaggac ctggttttaa tcctaattta actgggtttg atggatttca tggaactgta 600
cctcaaactt cattgtttcc tgctaggtct aaggttctac gacctctaga gatttctcca 660
cctgtaggag ctcagcctac actatttcaa aaacgtgctg cattacgtca aagttcagga 720
aaattgggtc ctgttgaaaa tgaaaagaaa cgtaagagaa gtgaggaaga tgaatttgat 780
gaaactggta ctattgatgt ttctggattt aattatgatt ctgatgaaat tgaacctatt 840
ggtgaactag ctaatggaaa tggtgaaagt agttttgtta ccataggagg tgagaatcaa 900
agaggtaaaa aaaaagggtt acctgcaaaa aatttaatgg ctgagagacg acgtaggaag 960
aaacttaatg atagacttta tatgctaaga tcagttgtac ctaaaattag taagatggat 1020
agagcttcta ttctaggtga tgctattgat tatttgaagg aactactaca aaaaataaat 1080
gatctacata atgaactaga ggctacacca caagggtcat tgatgcaaac atcatctagt 1140
atgcatcctc taacacctac ctctcctgct ctactacaac atgttaaaga agaactatgt 1200
cctactgcca ctaataacat tctaggacct aaaaaccatc tagcaaaggt agaagtatat 1260
gctaaagaag ctaggggtgt gaacatccac atgctatgtg ggcgtagacc tggtctatta 1320
ctatctacat tgagggctct agataaccta gggctagaca ttcaacaagc tgtcataagt 1380
tgttttaatg ggtttgcatt ggatgtattt cgagctcagc aatgtaggga agggcaggat 1440
atgttgcctg agcaaataaa agcagtgcta ctagagacag ctggatatca tcatggtgcc 1500
atttaa
1506
<210> 8
<211> 1506
<212> DNA
<213> Arabidopsis thaliana overexpression AaICE1(DC)2
<220>
<221> modified_base
<222>(89,104,125,139,604,620,677,680,821,822,836,855,923,941,949,952,966,972,
976,992,1028,1041,1126,1163,1165,1166,1272,1298,1302,1304,1307,1310,1311,1341
,1351,1363,1370,1411,1435)
<223> m5c
<400> 8
atgctacctg ctggaggttc tatttggatg gctggagaac ctagtcaaga tgaaacatca 60
tcatggtcac ctactacaca taacatcact ggaattgaac ctactgctac atcatgtaat 120
gatgctgatg atcaccatcg tatcaccact aatgctacta gttattcatc tctcaccaca 180

ttaaaatcta tgctagaaac tgaatggtat caccaccaca ataatctaaa tctaccatct 240
gatggtaact ctctattttt acctatggat tcatcatcat catgttctcc atctcaatct 300
cataatcagt ttactcaatc atcatatcca ttttttccac ctaaatttaa taataatttg 360
aataatccat atgatctacc atttgatttt ggatgtgaat ctgcattttt acctaatcac 420
acatctaatt tgatgagttt tgctggttta tcatctcaaa atcacatacc tatgcctgag 480
atttcatcta gttctgattt tcctgctact aacaataata atagtaatga tactggagat 540
attgtaggac ctggttttaa tcctaattta actgggtttg atggatttca tggaactgta 600
cctcaaactt cattgtttcc tgctaggtct aaggttctac gacctctaga gatttctcca 660
cctgtaggag ctcagcctac actatttcaa aaacgtgctg cattacgtca aagttcagga 720
aaattgggtc ctgttgaaaa tgaaaagaaa cgtaagagaa gtgaggaaga tgaatttgat 780
gaaactggta ctattgatgt ttctggattt aattatgatt ctgatgaaat tgaacctatt 840
ggtgaactag ctaatggaaa tggtgaaagt agttttgtta ccataggagg tgagaatcaa 900
agaggtaaaa aaaaagggtt acctgcaaaa aatttaatgg ctgagagacg acgtaggaag 960
aaacttaatg atagacttta tatgctaaga tcagttgtac ctaaaattag taagatggat 1020
agagcttcta ttctaggtga tgctattgat tatttgaagg aactactaca aaaaataaat 1080
gatctacata atgaactaga ggctacacca caagggtcat tgatgcaaac atcatctagt 1140
atgcatcctc taacacctac ctctcctgct ctactacaac atgttaaaga agaactatgt 1200
cctactgcca ctaataacat tctaggacct aaaaaccatc tagcaaaggt agaagtatat 1260
gctaaagaag ctaggggtgt gaacatccac atgctatgtg ggcgtagacc tggtctatta 1320
ctatctacat tgagggctct agataaccta gggctagaca ttcaacaagc tgtcataagt 1380
tgttttaatg ggtttgcatt ggatgtattt cgagctcagc aatgtaggga agggcaggat 1440
atgttgcctg agcaaataaa agcagtgcta ctagagacag ctggatatca tcatggtgcc 1500
atttaa
1506
<210> 9
<211> 1147
<212> DNA
<213> Manihot esculenta
<220>
<400> 9
atgttgtctg gattaaatgg cactgtctgg atggaagata aagaagcgca acactccacc 60
acagacaaca acattcacaa tagctgtgat gtgatagaga gcaaggaaga tatgggttcc 120
ctttctacat tcaaatccat gctcgaagta gaagaagaag actggtatat tgctaatagt 180
agtatccatc atcatcaaga taccatcaga gatattacat tttcaccaaa tcttgccgaa 240
acagataatc tgttgcttca ccctgcggat tcctcctctt cttgctcacc agcttcctct 300
gtttttaaca atatagaccc atctcaggtg ccctacttta tgcactcaaa acctacctta 360
tcttcacttc ttaacgctgt ctctaataac cctttagagc atgacattga gttgggcgaa 420
attgggttcc ttgacaacca gtcaactact actactacaa caaatgcctc acctttgcta 480
aatagaggag gcggagtttt aagcaactac acagatttga gctcaaataa tctgtttagc 540
tcttctaatt tatcccctga tccacctttt tcaactactc gtgcgcttca gttacccgaa 600
aatggtccag ggtttacagg gtttaatggt tttgttgaga gctcagggaa taggctgttc 660
tgtaataggt ccaagttgtt gaggcctctt gaaacatttc cttcgatggg tgcacaacca 720
actttgtttc aaaagagagc agctttgaag aagaatttga ctgataatgg aagcaatttg 780
ggggttctga gtggtattga gattgataaa ggaaagagag aaatcactca gttgatcgaa 840
gagaatgaga agaagagcaa attgggtaag ttagatgaca ctgtagagga tgtaagcatt 900
gacgggtcag gtttgaatta tgattctgat gaggtaactg aaaatattaa aatggaggag 960
attggtaaaa atggagggaa tagctcaaat gcaaatagca gcgtgactgg cggtggtgtt 1020
ggggatcaga aggggaagaa gaaggggctc cctgctaaga atttgatggc agaaaggaga 1080
cgccggaaga agctcaatga taggctgtac atgctaaggt ctgtagtccc aaaaataagc 1140
aaggtga
1147
<210> 10
<211> 1416
<212> DNA
<213> Actinidia chinensis
<220>
<400> 10
atgtatgaag agagtgaatg ttttgatcca aattctatgc aaggactgtc taaagatggg 60
ttttcacaaa atccaagttt ctccatggaa gagccctcct accaccaaaa cccctcaccg 120
gaatcagccg cgggtggtgg tgccgctgcc accgtggaag tagaactcca ccagcaaatg 180
ggtttggaca tggcaaatat ggaaccttgt tacaatatca acaacagcaa cagtctcatt 240
gatagtacct atttgatgca agaagttgtt cttgactcta accatgtttt gccctatgac 300
cactcaaatt gggacccaaa tctgaaccac aaccaccatc aagatttgaa ccatcatgtt 360

gaaattgcaa atgggcatta cccaaatttg attagcaaca attcctcaat cccagacctt 420
ctaaatctgc tccacttgcc aacgtgttca ccctcctctg tcctgcccaa ttcatcactt 480
tcctttttga gtcaagccca gaaatcgacg aattttcctc cctcgtttct cggggatttc 540
gcgtcaaacg agtccggaat ggtttcgaat cttgtgcacg actctctatt tcatctgaat 600
ctcccatcac ggccaccttt gcttagggag atgtttcagt ctctaccaaa ttgttcattg 660
tttggtggtg gaggtagtgg tggggtggtg gatgagaaag atgggagtga aggagggagg 720
cagtttgata atggggtctt gcagttttgt agagagtggg acatgaattg tatggccaaa 780
gaaggtaatg gtaagggcat tactaccaaa cactttgcca ctgagaggca aaggagagag 840
cacttgaatg gtaagttcac tgctttgagg agcttggtcc ccaaccccac caagactgat 900
agagcatcaa taattgggga tgcaattgag tacatcaaag agctactaag aacagtcaat 960
gagctgaaaa tcctagtcga gaagaagaga tgtggtaaag acagaagtaa gaggcataag 1020
acagaagaag atgggacgac tggagattta gaaaatgtca acaagaaacc cgaccctgac 1080
caatcctaca acgggccatc gcttagaagc tcgtggctgc agagaaaatc caaggatacg 1140
gaggtcgatg ttcggattat tgatgatgag gtgaccataa aactcgcaca gcgcaagaaa 1200
atcaattgcc ttctgattgt gtcgaaggtg atcgatgagc ttcagcttga tatccaccat 1260
gttaccggtg ggcttgttgg ggacaactac agttttctgt tcaacagcaa gatatgtgaa 1320
gggtcttctg tgtatgcaag tgcaatagcc aacaaactca ttgaggttgt gaacagacag 1380
tatgcagcaa tcccaccatc aaccagtagt tattag
1416
<210> 11
<211> 1145
<212> DNA
<213> Musa acuminata
<220>
<400> 11
atgttttcga gaattgatgg cggcttctgg atggatgacg gtggagacga cgacgaccct 60
gtcgcttgga caagaggaaa cgcctccagc tctagcggag ggggaatggg ggagaccaag 120
gaggacgagc tcggcctctc cggctttaag tccatgctcg tggacgactg gtacgtcggc 180
gccgccgccc atcacccctt cgaggccttc cagactcagc aggacctcaa agacgtcgcc 240
tttctcccga atccgtcgcc ccacgaggcc ctgttgctgc cccccgtcga tagcctcgac 300
caaagccaac cctttttctc gtcgagacct gccgtttcgt ctgtcttcaa tgcctcgaac 360
catttcggcg ttggggttga tctcaggagc gacgcgccgg ggtttcttcc ggcagccccg 420
gttttaaatc cccctgactt gatgaacggg ggaggtggcg ccatcgatgg agtgtttggc 480
ttcgtcggaa ttggaaccag cggacaactt ggttgtccgg agctgaactc cgcaagccaa 540
ttctccggcg cccttctgct acctcctgcc gagaactgct ccgtttccag ctctggcacc 600
gccttctgcc ctgttggctt cgacagcttg gagaattcac cgtttctcaa ccggtccaag 660
gtgttgaggc ctttggagat gttccctaca gcgggctcgc agcccaccct gttccagaag 720
cgggcggccg ccgctctact gcagaactcg gcggctgctg cagaaaaagt cggcatcttg 780
ggtccttggg cgtcgaaagg cagctgccaa gggagcagca gtgtggctgc attggaggaa 840
gagaaagaga ggaagaggaa agggaatgag gtcgacgagt tggacgaggg tagcattgac 900
aggtccggct tacattatga ttctgatgac tttgccggag agtgtgccaa agttgaggag 960
aatgcaggga atgtagcagg tggaggaggg aacagttcga gtgctgatgt catggtggcc 1020
gacaggggga agaagaaggg tctcccggcg aagaatctga tggcggagag gaggaggaga 1080
aagaaactca acgacaggct ttacatgctt agatctgttg tccccaagat aagcaagatg 1140
gatag
1145
<210> 12
<211> 1644
<212> DNA
<213> Musa AB Group
<220>
<400> 12
atgttttcga gaattgatgg cggcttctgg atggatgacg gtggagacga cgacgaccct 60
gtcgcttgga caagaggaaa cgcctccagc tctagcggag ggggaatggg ggagaccaag 120
gaggacgagc tcggcctctc cggctttaag tccatgctcg tggacgactg gtacgtcggc 180
gccgccgccc atcacccctt cgaggccttc cagactcagc aggacctcaa agacgtcgcc 240
tttctcccga atccgtcgcc ccacgaggcc ctgttgctgc cccccgtcga tagcctcgac 300
caaagccaac cctttttctc gtcgagacct gccgtttcgt ctgtcttcaa tgcctcgaac 360
catttcggcg ttggggttga tctcaggagc gacgcgccgg ggtttcttcc ggcagccccg 420
gttttaaatc cccctgactt gatgaacggg ggaggtggcg ccatcgatgg agtgtttggc 480
ttcgtcggaa ttggaaccag cggacaactt ggttgtccgg agctgaactc cgcaagccaa 540
ttctccggcg cccttctgct acctcctgcc gagaactgct ccgtttccag ctctggcacc 600
gccttctgcc ctgttggctt cgacagcttg gagaattcac cgtttctcaa ccggtccaag 660

gtgttgaggc ctttggagat gttccctaca gcgggctcgc agcccaccct gttccagaag 720
cgggcggccg ccgctctact gcagaactcg gcggctgctg cagaaaaagt cggcatcttg 780
ggtccttggg cgtcgaaagg cagctgccaa gggagcagca gtgtggctgc attggaggaa 840
gagaaagaga ggaagaggaa agggaatgag gtcgacgagt tggacgaggg tagcattgac 900
aggtccggct tacattatga ttctgatgac tttgccggag agtgtgccaa agttgaggag 960
aatgcaggga atgtagcagg tggaggaggg aacagttcga gtgctgatgt catggtggcc 1020
gacaggggga agaagaaggg tctcccggcg aagaatctga tggcggagag gaggaggaga 1080
aagaaactca acgacaggct ttacatgctt agatctgttg tccccaagat aagcaagatg 1140
gatagagctt ccattcttgg cgatgcgatt gagtacttga aagaacttct ccaaaggatc 1200
aacgaccttc aaaatgagct tgaatcaaca ccttcaagtt cttcactgcc tacaacaaat 1260
gcaacgagct tgcacccctt aactcccaca ctaccaactt tgccatgccg tttgaaggat 1320
gaactcaagc actgctcgag ttctttacca agtccaaata gtcagccagc aagggtcgaa 1380
gttaaggcga gggaaggccg agccgtcgac atacacatgt tctgtgcccg taggcctggt 1440
ctgctgcttt cagctttgcg atcacttgac agccttgggc ttgacattca gcaggcagtc 1500
atcagttgtt tcaatggatt cgcgctagac attttccaag ctgagcaatg caaagacccg 1560
ggtgttctcc tgaagaaatc aaggctgtgc tcttgcactc tgcaggtttg ctcaacacaa 1620
tgtagtagtc agcaatcaag gtag
1644
<210> 13
<211> 1344
<212> DNA
<213> Theobroma cacao
<220>
<400> 13
   atgctttcga gggtgaacgg cggtgtcgtt tggatggaag ataaagaaga tgaagaatct 60
   gcttcatgga acaggaccaa caacagcaac aacaacaata acaatagtgt tataatggag 120
   aacaaagaag agatgggttc gttgtctaca ttcaagtcca tgctagaaga agaatggtac 180
   gtagcaaaca acagtatttc aagccatcaa gatattagag acctttcatt ttcaccaaat 240
   cttgctgacc atcaagataa tattttgctg catcatcatc atcatcagca gcagcatcat 300
   ccagtggatt cttcctcttc ctgttcaccg tcttcctctg ttttcaacaa tcttgacccc 360
   tctcaggtac actacttttt gcaaccaaaa ccaaccttat cttcactcct taacgttgtt 420
   tctaataacc ctttagagca tgggtttgat ttgagtgaaa ttgggttcct tgacaaccaa 480
   gcaacaaatg ctactactct gttgaacagg ggaaatgctg ggttttgggg tagtttcgct 540
   gatttaagcc atggtaatca gatagacact gctaatttgt gtcctgagac tcagttttcg 600
   agttctagga tggttcagct accggaaaat ggtgcggggt tggcgggttt tcaaggattt 660
   gatgagaatc ctggaaatgc tctgttttta aataggtcga aggtgttaag gccactggaa 720
   agttttcctt cagtcggagc acaaccaact cttttccaaa aaagagcagc tttgaggaag 780
   aatttagctg atagtggagg gaatttcggg ttgttcggtg gaggaaaggt taatgctttg 840
   aatggaattg agggagataa agggaaaaaa gaaatggatg aagagaatga gaagaggaaa 900
   attatctata gagatgatct agaagatgtg agtattgatg ggtctgcttt aaattatgat 960
   tcagatgagt ttactgagaa taacaaggtg gaggaaactc ttaaaaatgg tgggaatact 1020
   tcgaatgcaa acagtactgt cactggagga gatcaaaggg gtaaaaagaa ggggcttcct 1080
   gctaagaatt tgatggctga gaggcgccgt cgaaagaaac tcaatgatag gctttacatg 1140
   ttgcggtctg ttgttccgaa gattagcaaa gtaagaattt ccttcacgtt tctctttttc 1200
   tttttttttc cccttctctt ttcgttttta tctcgtgaat tcaagtatga attattgagt 1260
   tattttagat cctcagaaat gtcaaatatt ctgacttttt atctttgttt tcatggttgg 1320
   tgtttttgta ttatgtgtga atga
   1344
<210> 14
<211> 1682
<212> DNA
<213> Citrus trifoliata
<220>
<400> 14
atgctttcta gactaaacgg tgtggtctgg atggacggga aagaagagga aggctcagct 60
gcgtgggcca gaaacaacat aaacaacaac agcagcaaca gcatcaacaa caattgcaac 120
actaacaaca tgagctgcag taacaacaac gacaacaaca atggtgtcat agagaatgaa 180
gaagaaatgg gttcacttcc tggattcaag tccatgcttg aagtggaaga tgactggtat 240
gttactggta atactagttt aaataaccat caagatatta catttccacc aaaccttggt 300
gacccaacaa ctgataatct gttgcttaac gctgtggatt cttcatcttc ttgttcacca 360
tcttcctctg ttttcaacaa ctttgatgca tctcaagttc actacttttt gcctcaaaaa 420
aactcctttt cttcattcat gaatgttgtg tccaacaaca actctttaga gcatggcttt 480
gatttgggtg aaatggggtt tcttgacaca caagcaactc atgctttgaa tagggggaat 540

ggtgggattt tgaatggttt caatgatttg agtgccaata atcagatgaa tgctactaat 600
ttatgttctg atccgcaatt tggaactaac cgtacgcttc aattcccgga aaatggtagc 660
agtagtttcg cgggttttcg gggttttgat gagaataatg ggaattctct gtttttgaat 720
aggtctaagt tgttgaggcc acttgaaact ttcccttcga cgggagcaca acccactctt 780
ttccaaaaaa gagctgcttt gaggaaaaat ttaggtggta atgaggccag tttaggggtt 840
ttaggtacac aaaatagcca acttttgagt gggattgaga gtgataaagg taagaaagaa 900
ctgactgaag ataatgagaa aaagaggaaa ttgagtatta gtgatgattt ggaggatgtg 960
agtgttgatg ggtcgggtct caactatgat tctgatgatt ttttagaaaa taataaggtg 1020
gaggaaatgg gtaaaaatgg tggtagtagc tccaatgcaa ttagcactat cactggtgga 1080
gatcagaagg gcaagaagaa gggtttgcca gcaaagaatc tgatggcaga gaggagacgc 1140
aggaagaagc tcaatgatag gctctacatg ttgcggtcag ttgttccaaa gattagcaaa 1200
atggataggg cttcaatttt gggggatgca attgagtatt tgaaggaact tctccaaagg 1260
atcaatgacc tccacaatga attggagtca acccctcctg gttcagcatt gactccttcc 1320
acaagcttct atcctttgac accgactcca cctgccttgc acagccgtat caaggatgaa 1380
ctttgcccca gctcactgcc aagcccaaat ggccaacctg caaggttgga ggtcagggtg 1440
agggaaggaa gagccgtgaa tatccacatg ttttgcagcc gtagaccagg tcttttgctc 1500
tccacgatga gggcattgga taatcttgga ctagatatcc agcaagctgt catcagttgt 1560
ttcaatggct ttgccatgga tgttttccga gctgagcaat gcaaggaagg ccaggacgtt 1620
catccggagc aaatcaaggc ggtactcttg gattcagccg gcttccatgg catgatgtag 1680
tt
1682
<210> 15
<211> 1092
<212> DNA
<213> Vitis vinifera
<220>
<400> 15
atgttaccca ggtcgaacga cgtcgtttgg acgcgaagcg acgccgtttc ctggtcgaag 60
aacagcaacg acaacaggtt tggtgatggc ggcggtgatg gtgagaggac gagttcggtg 120
tctgcgttga aggccattgt tgaggaggag tggttggtga gcggtgtcga aatgaaagga 180
aatttggtgt ctcagagtca gagcggagcg gtgaattcac tgttgcatcc aatggactgc 240
tcgtcgtcgt gctcgccggc gtcggcgttt ggtctggacc cggcctttca gggcaaatct 300
tgcttgtcct cgatggtttg tgggtaccca tttgatggct cttttgattt ggtatgtgac 360
ccggggtttc ttgagccgat acccgaaatt caggctccga gttcgagttc tttgatgggt 420
ttcaacgatt tggcttcgca gcccctgatg ggtgcttcaa atctgggctc gaattcgcag 480
tttccggcta cccatttggc gggaaacggc gggagtgcgg ccaccggtgg gtttaatcca 540
tcgggttttg agggttttgt ggggagttct ctgtttgtgg acaggtgtaa ggtgttgaaa 600
ccgctcgaga acttcccttc agttgggtca cagcccacgc tgttccagaa gagagcgatt 660
ctccggcgaa attcgacaga aagagcgggg aattatggag tttcgggcca agaaggcagt 720
gcaattccgg ttagagttgc cggagaagat aagggtaaga ggccggtggt ggaagaagaa 780
atggacaaga tgagaaagga caagagcaat gatgaggatg atatggacga agccagcatt 840
gctcggtccg gtttgatata cgattctgat gatgcaattg agaaccacaa ggtggaggag 900
actgcaaatg atggtggaga caactcaaat ttgaatggat cttccattgg aggagataga 960
aaagggaaga aaaaggggct gccggcgaag aatttgatgg ctgagaggcg ccggagaaag 1020
aagctgaacg ataggctcta catgctgagg tctgttgttc caaagatcag caaggtgaga 1080
attttctggt ga
1092
<210> 16
<211> 1596
<212> DNA
<213> Malus x domestica
<220>
<400> 16
   atggacgaca gggaggatag agactctgtt tcctggacta gaacctccgc caccgccacc 60
   gccggcaaca ccgagaacaa ggacgagatg ggttcttctc tgtctacctt caagtcgatg 120
   ctggaggttg aagacgactg gtacatggcg gcgaataaca gcatccaggg ccattcagat 180
   gtcggagaca tcagcttctc gccgagcttt gccgacccag aaagcttgct gctcaacccg 240
   gtggactctt cctcctcctg ctcaccgtcg tcgtcggttt tcaacaatct cgacccgaat 300
   caggttcatt actacatgcc tcaaaatccc aacttgtctt cgctgcttaa tgttgttcct 360
   aacaaccctt tggatcacgg cttcgatttg ggctgtgaca ttgggtttct tgacactcaa 420
   gcttcgggtg gcggttcgag tatgatgaac aggggaggtg gggctttatc tggattcaat 480
   gatttaagct cgaatagcca ggtgaatgct cagaatttgg gttctaattt acagttttcg 540
   actacccgca tgcctcaagc tcttgaaaac agttcaaatt tttcgggttt tcgaggtgtg 600
   gatgatggtt cggcaaatgc tttgtttccc aataggccta agttactgag accacttgaa 660
   tccttccctt ctgtgggagt ccagccaact cttttccaga aaagagcagc gcttcggaaa 720
   aacttatgcg atggtggagg gaatttgggt gttttggtct cacaaggggg cctggttttg 780
   aatgaagggg atgagaggaa gagggagatg ggtgtgcaga atgagaagaa gaggaaaatg 840
   agcggcggag atgatgtcga tgatctgagt ttcgatggtt cgggtttgaa ctatgattcg 900
   gatgagttta ctgagaacac taaggttgat gacggtgcca agaatggtgg gaacagctcg 960
   aatgcaaata gcacggttac cggtggagga gggggtcata aggggaagaa gaaagggttg 1020
   cctgccaaga atctgatggc tgagaggcgc cgccggaaga agctcaatga taggctgtac 1080
   atgctgagat ccgttgttcc gaagattagc aaaatggaca gggcctcaat ccttggggat 1140
   gcaattgagt acttgaagga acttctgcag aggatcaaca acctccacaa tgaactggag 1200
   tcaatccctc ctggatctgc attgacacct accggaaata ctttccaccc tttgacaccc 1260
   actccggcta ctctgccaaa tcgtatcaag gaagaacttt gcctcagctc attacctagc 1320
   ccaaatggcc aagctgcaag ggttgaagtt aggctgcgag aaggacgagc agttaacatc 1380
   cacatgttct gcggccgaag gccaggtctc ttgctctcaa ccatgagaac tttggataac 1440
   cttgggttag acatccagca agccgtcatc agctgtttca atggttttgc tatggatgtc 1500
   ttccgtgccg agcaatgcaa ggaaggccaa gacgtccacc cggatcagat aaaggcggta 1560
   cttctggatt cgatcgggtt ccatggcatg atgtag
   1596
<210> 17
<211> 1479
<212> DNA
<213> Capsella bursa-pastoris
<220>
<400> 17
atggttcttg acggaaacaa cggtggtgcg gtttggttag gcggtggtgg agaacgggaa 60
gagaacgagg aaggttcgtg gggaaggaat caagaagatg ggtctcagtt taaaccaatg 120
cttgaaggtg attggtttag caacccacca catccacaag atcttcagat gttacagaat 180
catcaagatt gcagatttct tggtggtttc cctttcaatc ctaatgacaa tcttcttctt 240
cagcactcga ttgattcgtc ttcttcttgt tctccttctc aagcttttag ccttgaccct 300
tctcagcaaa accagttctt gtccactaac aacaagtctt gtctcatcaa tgttccttct 360
tctgcaaacc cttttgacaa tgcttttgaa ttcggctctg aatctgggtt ccttagccaa 420
atacatgctc ccatgtcgat gggttttggt tctttgacac agttggggaa ccgggatttg 480
agttctgttc ctgatttctt gtctgcaagg tcgcttcttg cacaggatca caacagcagc 540
aattcagtgt tatgtggtgg aggaggtggg ttcacagctc cactggaatt agaaggtttt 600
gggagtcctg ccaatggtgg ttttgttggg aacagagcca aggttctgaa gcctttagag 660
gtgttagctt cgtctggtgc gcagcctact ctgttccaga aacgtgctgc tatgcgtcag 720
agctctagaa gcaaaatggg aaactctgag agctcgggaa tgaggaggtt gagtgatgat 780
ggggatatgg atgagactgg gattggggtt tcagggttga attatgagtc tgatgagcca 840
aacgagagtg ggaaagcagc tgagagtgtt cagattggag gagggaaggg taagaagaaa 900
ggaatgcctg ctaagaatct gatggctgag aggagaagga ggaagaagct taatgatagg 960
ctttatatgc ttagatcagt tgtccccaag atcagcaaaa tggatagagc atcaatactt 1020
ggagatgcaa ttgattatct gaaggaactt ttacaaagga tcaatgatct tcacaacgaa 1080
cttgagtcaa ctcctcctgg atctttgcct ccaacttcat caagcttcca tccattgacg 1140
cctacaccgc agactctttc ttgtcgtgtc aaggaagagt tatgtccctc ttctttgcca 1200
agtcctaaag gccagcaagc aagagttgag gttagattaa gggaaggaag agcagtgaac 1260
attcatatgt tctgtggtcg tagaccgggt cttttacttg ctaccatgaa agctttggat 1320
aatcttggac tggatgttca gcaagccgtg attagctgtt tcaacgggtt tgccttggat 1380
gttttccgcg ctgagcaatg tcaagaagga caggagatac tacctgatca aatcaaagca 1440
gtgctttttg atacagcagg ctatgctggt atgatctga
1479
<210> 18
<211> 981
<212> DNA
<213> Solanum chilense
<220>
<400> 18
atggttgatg atggaggtgt ggattggttc atgggtggtg gtgatagtaa caatcatcac 60
atcaacaaca acaacaacaa tggtggtggt ggtggtggtg gtggtagcaa catgcagagc 120
cacatttctt actctacaag tttcactgaa gctgaaaaca gtttgcttct gcaacctgtt 180
gattcttcag cttcctgctc gcctgtttct ggtaacgttt tcaacaacat tgacccttct 240
caggtgaact tcttcatgcc ccaaaaatct actattcctt cttcactcac tgggttatcc 300
aacaacccaa tggataacag cttcaatttg ggtatgttga atcaagctgg gaacgggatg 360
atgaatactg ggtatcatca tctgggttca ccaaaccaaa tggggactaa caatctgagt 420

tcttacactc agttttcttc tcccaatctg ctccagctac ctcaggtagc tggtgggtac 480
agttcgatgg gattcggggc taacaattct gctaatggaa acactttgtt tctcaatcgg 540
tctagaactc ataaaccttt agacaatttt gcttccattg gagcacagcc aactcttttc 600
cagaaaagga ttgcaaagaa cttggttagt aatggtgaaa atttaggtac agaaatcggt 660
caatcttcca gtaatctgac tgatagaaaa aggaagtcca gcatcaatga tgagtttgaa 720
gatgttagca tggatggaac attgaattac gactcggatg agttcatgga tatctctaac 780
aaaatggaag atgggatcaa gattggggac agctctaatg cagccagcac tgtcagtggt 840
gctgatcaaa agggaaaaaa gaagggacct ccagctaaga acctgatggc ggagcgacgc 900
cgcagaaaga agctgaatga caggctttac atgctgcgat cggttgtccc aaagataacc 960
aaggtaatga aattctttta a
981
<210> 19
<211> 1596
<212> DNA
<213> Solanum lycopersicum
<220>
<400> 19
atgctctcaa gagttaacag tatgcaggtt tgggatatgg aaggcaaaca agaagaagaa 60
aaagaagaaa attttagcaa caaggagaat accactaatg ttgagcttga aaacaaacaa 120
gatatggaat tgggtgcact ttctacattc aaatctatgt tagatggtac agatgtagat 180
tggtatcaca acaacatgca aaaccatact gaaaatatct gtttcactca gaatttcact 240
gaattagctg aaaacagcat gtttctacag ccagtagtac cagtggattc ttcatcttct 300
tgttcaccat cttcagtttc tgttttcaac aatctagacc catctcaggt acattacttg 360
ttagcccaaa aggctattaa caacaaccct ttggattata gttttaattt gggttgtgaa 420
aatggttttc ttgaagctca aggtatgggt ggtttgaata aagggggatt tcttcttgct 480
ggtggtgggt ttcatgattt gagctcacag aaccaaatgg gtaaccccaa tttgaattca 540
tttactcaat acccatcatc ccatttgccc caaaatacta ctactactgg gtttagtcca 600
ctagggtttg ttgatggttc tgcaaatgag aactctctgt ttctcaatag gtccaagttg 660
ttaaagccac ttgataattt tgcttcaaat ggagcacaac caaccctttt tcagaaaagg 720
gctgctctta ggaagaactt agctaatact actggtggca gtttggggga tttcggaggt 780
gaaattggcc agaattccat gaatggagag aatgaaagga agagaaagtg ggtagtgggg 840
gaagaacttg atgatgtgag ctttgatggg tgcacattga gttatgattc agatgacctt 900
actgaaaatg ttactaataa ggtggatgat actgttaaga atggtggaaa tagctcaaat 960
gcaactagca ctgtcacctg tgggaatcag aaaggaaaga aaaagggact tccggctaag 1020
aatttgatgg ctgaacgacg acgtaggaag aagctaaacg ataggcttta catgttgagg 1080
tctgttgtac caaggattag caagatggac agggcttcaa tcttagggga tgcaattgag 1140
tacttaaagg agcttttaca gaaaatcaat gacctgcata atgaactgga gtccacgcca 1200
cctagctctt cactgaccca aacaacaagc ttctatcctc taacaccaac tggaccagct 1260
ctgccaggcc gcatcaagga agaactctat ccgagttcat ttgcaagccc actgtctagc 1320
ccgactggac aacctgcaag ggttgaagta aaggcaagag aaggaagagc tgtgaatatc 1380
catatgtttt gcagccgcag accaggcctc ttactctcaa caatgagggc actcgacaac 1440
ctcggactgg acatccaaca agctgttatc agctgcttca atggatttgc cttagatatc 1500
tttagagctg agcaatgcaa agaaggtcaa gactttcatc ctgatcaaat caaagcagta 1560
ctgctggatt ccgctggttg ccatgggatg atatga
1596
<210> 20
<211> 1443
<212> DNA
<213> Prunus persica cultivar
<220>
<400> 20
atgcttgatg ttgatgatga ttggtatctg cccaataata gcattcagag ccattcagat 60
gtccgagaca tagccttctc gccgagcttt gctgacccag aaagcttgct gctcaaccca 120
gtggactctt cctcttcttg ctcaccgtct tcctcggttt tcaacaatct tgacccaaat 180
caggttcatt actatttgcc tcaaaattcc accttgtctt cgctccttaa tgttgttcct 240
aataaccctt tggagcatgg cttcgatttg ggctgtgata ttgggtttct tgacactcaa 300
gcatcgagtg gttcaagttt aatgaacagg ggaggtgggg ttttaactgg gttcagtggt 360
ttgagctcaa ataacccgat gaatgctcag gatttgtgtt caaatttgca attttcaacc 420
acccgtctgc ctcaattgct tgaaaatagt tcaaattttt cgggctttcg gggttttgag 480
gatacttcag cgaatgcttt gtttcccaat aggcctaaat tattgagacc ccttgactcc 540
gtccctgatg tgggagcacc gcccactctt tttcagaaaa gagttcagaa gaacttagct 600
gataatggag gaaatttggg tgctttaggc tcagaagggg gcctggcttt gaatcatatg 660
ggtgaggaga atgagaggaa gaggaaattg agcgggggag atgattttga agatgtgagt 720

atcgatggtt cgggtttgaa ctatgattca gatgacttta ctgaaaacac taagatggat 780
gacggtgcta agaatggtgg taatagctcg aatgcaaata gtactgttac tggcggaggg 840
gatcataagg ggaagaagaa agggttgcct gctaagaatt tgatggctga gaggcgccgc 900
cggaagaagc tcaatgatag gctgtatatg cttagatccg ttgttccaaa gatcagcaaa 960
atggacaggg catcaatcct tggggatgca attgagtact tgaaggaact tctgcaaaag 1020
atcaacaacc tccataatga attggagtca atccctccgg ggtcttcatt gacgcctacc 1080
ggaagtacat tccacccttt gacacccact ccggctaccc tgccaagccg tatcaaggaa 1140
gaactttgcc ccagctcatt acctagccca aacggccaac ccgcaagggt tgaagttagg 1200
ctacgagaag gaagagcagt gaatatccac atgttttgtg ggcgcagacc tggtctcttg 1260
ctctcgacca tgagaacctt agatagcctt gggctagaca tccagcaagc tgtcattagc 1320
tgtttcaatg gttttgctat ggatgtcttc cgtgctgggc aatgcaagga aggtcaagac 1380
gtccacccgg atcagataaa agcagtactt ttggagtcag tcgggttcca tggcttgata 1440
tag
1443
<210> 21
<211> 1131
<212> DNA
<213> Zea mays
<220>
<400> 21
atggacgact cggcggaggt gaagctggtg gacgagatca cgggagaggg aggggcggcg 60
ggggactggg gctacctggg ctccgacggg atgggctccg gctcctaccc ggcgttcccc 120
ttctccagag acgtcctatc cacgccgacc tccgcgtcgt tgctcctctc catggacccc 180
gcggcgctct tcgacttcaa cggcaccttc cctccctcct ccgcggccgc ggccaccgcc 240
ggctcctcgc tgtccgcctt ccacgacttc agctgcatca accccttcga cgacgccggc 300
cacttcctcg gcgctccccc gccggtgccg gcggcggcgg cgcctcagca gcaggggcag 360
aaaggtgggt tctttgcgcc gctgccggcc tcggacttca acgacgccgg gatgagctgg 420
gacgacgagg acgagatcga ccaaagcgtc gacgcctcct cgatggccat ctcggcctcc 480
atggagaatg ctgcgggcgc ggtggcgggg gcaagtggcg ccggcggtgg cagcgggaga 540
ggcaagaaga aagggatgcc ggccaagaac ctcatggcgg agcgccggcg tcgcaagaag 600
ctcaatgacc gcctctacat gctgcgctcg gtggtgccga agatcagcaa gatggatagg 660
gcttctatcc ttggcgacgc aattgagtat ctgaaggagc tcctgcagag gatcagcgat 720
cttcacaatg aacttgagtc agctccgagc tcttcgcttg ttggaccaac atcagctagc 780
ttcaacccat caacaccgac attgcagaca tttcctggcc aggtcaagga agaactttgc 840
ccgggctcat tcccaagccc tactggacaa caagccacag tcgaagtcag gatgagggaa 900
gggcatgcag tcaatatcca catgttctgt gcgcgtaggc caggcatcct gctgtcgaca 960
atgacggccc tcgacagcct aggcctcgac atcgagcagg cggtcatcag ctgcttcaat 1020
ggctttgcga tggacgtgtt ccgagctgag tgcgcggatg gtcctggaat ggtgcctgaa 1080
gaaattaagg ccgtgctgat gcacaccgcc ggcctccaca acgcaatgta g 1131
<210> 22
<211> 1539
<212> DNA
<213> Gossypium hirsutum Ls
<220>
<400> 22
atggagaaca aagaagatat gggtactttt tcaacattca aatccatgtt agatgatgaa 60
tggtacgtag gaaacaacag catttcaagc catcaagata ttagagacct ttcctttact 120
tcaaatcttg gtgaccatca tcaagataat cttttgctcc atcatcattc tcttccttca 180
gtggattctt catcttcatg ttcaccatct tcctccgttt tcaaccatct tgacccatct 240
caggtacagt acttttgcca accaaaacca aacttatctt caatccttaa ttttgtttct 300
aatagccctt tagaccatgg ttttgatttg agtgaaattg ggttccttga caaccaagca 360
acaaatggta ctactttgtt gaacagggga aatgctgggg ttttgggtag tttaactgat 420
ctaggtcatg ataatcattt agaccctgct aatttgtgtc ctgaggctca gttttcgagt 480
tctcggatag ttcagctacc agaaaatggt accgggtttg ctggttttga agggtttgat 540
gagaatcctg gaaatgctct gtttttaaag agatctaagt tgttaaggcc actagaaagt 600
tgtccttccg ttggtgcaca acctacttta ttccaaaaaa gagcagctat gaggaagaat 660
tcagctgata gtggaccaaa tttcggggtg ttagatggag gaaaggttag tgttcaaagt 720
ggtactgagg gagataaagg gaagaaagaa atgggtaaag aagagactga gaagaggaaa 780
attaacaaca gagatgatgt agaagatgtg agcattgatg ggtcagctct gaactatgat 840
tcagatgagt ttactgagaa taccaaggtg gaggaaactc ttaaaaatgg gggaaatacc 900
ataaatgcaa acactagtgc tactggagga gaccaaaacc aaaagggtaa aaggaaaggg 960
cttcctgcta agaatttgat ggctgagagg cgccgtcgaa agaaactcaa tgataggctt 1020
tacatgttgc gctctgttgt tccaaagatt agcaaaatgg atagagcttc aattctcggg 1080

gatgctatcg agtacctgaa ggaacttcta cagagaatca atgacctcca caatgaattg 1140
gagtcaaccc ttccaagctc ctcactgaca cctacaacca gcagtttcca tccattgaca 1200
cccactccag ctacctttcc tagccgtatc aaggatgaac ttggccccag ttcatcacca 1260
ggtctgaatg gacagccagc aagagttgaa gtaaggctga gagaaggaaa agctgtaaac 1320
atccacatgt tctgtgggcg gagaccaggt cttttgctct ccataatcag ggccttggac 1380
aaccttggac ttgacgccca gcaagcagtc attagctgtt tcaacggttt ttccatggat 1440
atctttcgag ctgagcaatg caaggaaggg caggacatca atcctgagaa tatcaaagct 1500
gtacttttgg attcaactgg cttccccaat atgatatag 1539
<210> 23
<211> 306
<212> DNA
<213> Arachis hypogaea
<220>
<400> 23
atggaggcag ttggtggttc tggaaacgct ctgttttcgg gcaagggaaa tggattggaa 60
gctttggtag tgagtaattt gaagagggtg caggaagttg aagaagctag caatgaaggt 120
tcaatgtgga attctgaaga ggatgatgag agaactgatg aggtggtggt tgaggagagt 180
gggaagaaga agaagaagaa ggggatgccg tccaaaaatc ttatggcgga gaggcggcga 240
aggaagaagc tcaatgatag gttgtacatg cttaggtctg ttgtgccaaa tattagcaag 300
gtataa
306
<210> 24
<211> 1029
<212> DNA
<213> Brassica juncea
<220>
<400> 24
atggttctcg acggaaacgg cggtggtgta tggttaggcg gcggcggcgg cgggggagga 60
gaacgggttc aggaggagga gaacgaggaa gcttcgtggg ggaggaatca agaagacggt 120
ggtcagttca agcctatgct tgaaggtggt ggtgattggt ttactagtag ccaacctcat 180
ccacaagatc tccagatgct gcagagccag caagacttca gattcctcgg tgggtttggt 240
ttcaacccca acgacaatct tcttcttctt caacactcca tggactcgtc ttcttcctgc 300
tctccctctc aggcttttag cctcgacccc tctcaagctt cgttcttggc tgctgctaac 360
aacaagtctt gtctcctcaa cgttgttcct tcctccgcaa acccttttga caatgccttt 420
gagttcggtt cggattctgg tttccttaac caaatccaag ctccggtttc gatggggttc 480
ggttcgttga cgcagctggg aagctctgtc cctgacttct tatctgctcg gtcgcttctc 540
ccgccggaaa acaacaacac agcgccctta tgcggcggag gaggaggtgg aggtgggttc 600
acagcgttgg agctggaagg gtttgggagt ccagcgagtt ttgttgggag cagaccgaaa 660
gttctgaagc ctttagaggt gttggcgtcg tctggcgcac agcctactct gttccagaag 720
cgtgcagcga tgcgtcagag ctctggaagc aagatgggga actcggagag ctcggggatg 780
aggaggctga gcgatgatgg agatatggat gagactgggg ttgaggtttc agggctgaac 840
tatgngtctg atgagctgaa cgagagcggt aaagcttctg agagtgttca gaacggagga 900
ggtaaaggga agaagaaagg gatgcctgct aagaatctga tggctgagag gaggaggagg 960
aagaagctta acgataggct ttacatgctt agatcagttg tccccaagat cagcaaagta 1020
aactattaa
1029
<210> 25
<211> 1494
<212> DNA
<213> Brassica rapa subsp.
<220>
<400> 25
atggttctcg acggaaacgg cggtggtgta tggttaggcg gcggcggcgg cggcggagga 60
ggagaacggg tgcaggagga ggagaacgag gaagcttcgt gggggaggaa tcaagaagac 120
ggtggtcagt tcaagcctat gcttgaaggt ggtggtgatt ggtttactag taaccaacca 180
catccacaag atctccagat gctacagagc cagcaagact tcagattcct cggtgggttt 240
ggtttcaacc ccaacgacaa tcttcttctt cttcaacact ccatggactc gtcttcttcc 300
tgctctccct ctcaggcttt tagcctcgac ccctctcaag cttcgttctt ggctgctgct 360
aacaacaagt cttgtctcct caacgttgtt ccttcctccg caaacccttt tgacaatgcc 420
ttcgagttcg ggtcggattc tggtttcctt aaccaaatcc aagctccggt ttcgatgggg 480
ttcggttcgt tgacgcagct ggggagctct gtccctgact tcttatctgc ccggtcgctt 540
ctcccgccgg aaaacaacaa cgcaacaccc ttatgcggag gtggaggagg tgggttcacg 600
ccgttggagc tggaagggtt tgggagtcca gcgagctttg tcgggagcag accgaaagtt 660

ctgaagcctt tagaggtgtt ggcgtcgtct ggtgcgcagc ctactttgtt ccagaaacgt 720
gcagccatgc gtcagagctc tggaagcaag atggggaact cggagagctc ggggatgagg 780
aggctgagcg atgatggaga tatggatgag actggggttg aggtttcagg gctcaactat 840
gagtctgatg agctgaacga gagcggtaaa gcttctgaga gtgttcagaa cggaggaggt 900
aaagggaaga agaaagggat gcctgctaag aatctgatgg ctgagaggag gaggaggaag 960
aagcttaatg atagactcta tatgcttaga tctgttgtcc ccaagatcag caaaatggat 1020
agagcatcaa tacttggaga tgcaattgat tacctcaagg agcttttaca aagaatcaat 1080
gaccttcaca acgagcttga gtcaactcca aatggatctc tccctctggc ttcatcaagc 1140
ttccatccat taacgcctac gccgcagact ctttcttgcc gtgtcaagga agagttgtgt 1200
ccctcttctt tgccaagccc taaaggccag caagcgagag ttgaggttag attaagggaa 1260
ggaagagcag tgaacattca catgttctgt ggtggtagac ctggtctttt actagctacc 1320
atgaaagctt tggataatct tggattggat gttcagcaag ccgtcatcag ctgtttcaac 1380
gggtttgcct tggatgtttt ccgcgctgag caatgccaag aaggacaaga gatattgcct 1440
gatcaaatca aagcagtgct tttcgataca gcgggatacg ctggtatgat ctga 1494
<210> 26
<211> 1416
<212> DNA
<213> Chrysanthemum dichrum
<220>
<400> 26
atgctaccgg aaaacgacac cgcttggatg gacaaccacc aagatgaaac atcttcatgg 60
acccctacaa ccaccccaaa ccaccaccac caaccaccat gcaccaacaa ccaaaacacc 120
tacacttcct taacaacctt aaaaaacatg ctagaaacag aatggttcca aaacaacaac 180
aacaacaacc ttaacctcac ctcaaccgac actaatccca tgtttttacc actagattca 240
tcatcctcat gttcaccttc acaatcacac tcacactcac atttcactca gtttccattc 300
atacccacta aatataacaa taatactaat cttaacttaa ctgctttcga cttgcctttc 360
gaattcgggt gtgaccctag cttcatttcc aacaaccctc ccactaactt tttgaatttc 420
cctacccaac aaatggcaac tagctccgag tatacggaga ctagttacca tgatgactta 480
accggaactg gtacagggtt cactggcttt gagccagtga atcctgtttt tccggttagg 540
tccaaagttt tgaggccttt ggaagtttct ccacctgttg gagcgcagcc cactttgttt 600
cagaagcggg ctgcgctgag acagagtgga gagaagttgg atgggttgga aattgtgaaa 660
aaaaggaaaa ggggtagtgg gagtgggagt ggggcccatg aggatgagat tgatgaaatg 720
ggtaatgggg atggttcggg gtttaattat gattcggatg aagttgagct tactagtgag 780
gttaatggga ataattcggg ttcggttatg gttggggata gtaaagggaa gaggaagggt 840
ttgcctgcta agaatttaat ggcggaaagg cgaaggagga agaagttgaa tgataggttg 900
tatatgctta gatctgttgt ccctaaaatt agcaagatgg acagggcttc aatacttggt 960
gatgctattg attacttgaa ggagttgttg caaaggatta atgatttaca taatgaactt 1020
gaggcaacac cacaaggttc tttgatgcaa gcttcgtcga gtatccatcc attgacgcct 1080
acaccaccaa cgcttccgca acatgttaag gaagagttgt gtccaagtac tcttcctagc 1140
ccaaaaaacc atccttcaaa ggtggaggtg catgcaagag aagggagggg tgtgaatatc 1200
cacatggtgt gtgggcgcag accaggtctg ttgctttcaa cgttgagggc cctagagaac 1260
cttggcctgg acattcagca agctgtcata agttgtttca atgggtttgc tttggatgta 1320
tttcgagccc agcaatgtag agaagggcag gagatgttac cggagcagat aaaagcagtg 1380
cttctagaga ctgcaggtta tcatggtgcc atttag
1416
<210> 27
<211> 1089
<212> DNA
<213> Thellungiella halophila
<220>
<400> 27
atggttctcg acggaaacgg cggtgcggtt tggttaggcg gcggagaacg gattcaggag 60
gaggagaacg aggaagcttc gtggggtagg aatcaagaag aaggtggagc ttcactttct 120
catttcaagc ctatgcttga aggtgattgg ttcagtaacc caccacatcc acaagatctc 180
caaatgttac agaatcagca agatttcaga ttcctcggtg gtttcccttt taatcccagc 240
gacaatcttc ttcttcacca gtcgattgat tcgtcttcgt cttgctctcc gtctcaagct 300
tttagcctcg acccttctca gccttcgttt ctagccgcca acaacaaatc ttgtctcctc 360
aatgttcctt cctccacaaa ccctttcgac aatgcgttcg agttcggctc tgattctggt 420
ttcctcggcc aaatccaagc cccgatttcg atggggtttg gttcgttgac acaactggga 480
aaccgggatt tgagctccgt ccctgatttc ttatctgctc ggtcgctact cccgccggaa 540
aacaacaact ccacgccgtt atgtggcgga ggaggcggtg gtggtggtgg attcactcct 600
ctggagctgg aagggtttgg gagtcctgca aatggcggtt ttgtagggaa cagagcgaaa 660
gttctgaaac ctttagaggt cttagcatcg tctggtgcac agcctactct gttccagaaa 720

cgtgcagcta tgcgtcagag ctctggaagc aagattggga actcggaagg ctcggggatg 780
aggaagctga gcgatgatgg agagatggat gagactggga ttgaggtttc tgggttgaat 840
tatgagtctg atgagctgaa tgagagtggt aaagcggcgg agagtgttca gaatggagga 900
ggaggatgta aaggtaagaa gaaaggaatg cctgctaaga atctgatggc tgagaggagg 960
aggaggaaga agcttaatga taggctttat atgctaagat cagttgtgcc caagatcagc 1020
aaagtaaaca attacttctc ttttatctct ttaagggctt tgtcttcatc gtttacttat 1080
cgccgttag
1089
<210> 28
<211> 1458
<212> DNA
<213> Daucus carota
<220>
<400> 28
atgctcccca cttccaacgg cgtcgtttgg atgccacctc aagataccac caacattgaa 60
caacagcaat tgttattaga tacatgtgta gatacaaaaa ttagcccttt tgcttcttct 120
tcttcgctca ctagttttga atcacttctg gaaaatgatt ggttctctaa taattatctt 180
gggaatggta atttagattt ttcagcttct ggttctcagt cacagccgtt cactcatgac 240
ccgtcacagt cacattctca gttttttcag cccaaatctt gcttctctcc aatgcttaat 300
gggttgtatg ataatgggtt tcagttgggt tctgacccga atttcacaaa cgggtcggat 360
tctcatgggt tcatgtctta taatgggttg aattctgtga ctaatttgga tatgggtttg 420
gtttcggatt ttggagggac ccggtttgag agtgacccgg ttggtgtcgg gtttaacccg 480
gttgggtttg gtgggtcatg tttagttgat gataatgttc atgataatga tgataatgct 540
ttgttttgta ataggagtaa tggtaatcag attttaaggc cacttgaggt ttcaccaagt 600
gtgggtgcag agccaactct ttttcagaag agggctgctt tacgacaagg ctccggtgag 660
ggcgaaatag ggaattcagg aaagagtgag ggtttgggga ggaagaggat gagaaatgcg 720
agtgaagatg gtgatgaatt tgatgatggc agtatgggtg tttcgggttc ttttaattat 780
gattcggatg actttgatac taaaaacgag aatgggggct ctggggatgt ggatggttcg 840
aattctgtga aggttggaga tcggaaggga aagaagaagg gatttcctgc taagaatttg 900
atggctgaga gacggcggag gaagaagctc aatgataggc tttacatgct gaggtctgtt 960
gttcccaaga tcagcaagat ggatcgagct tccatacttg gcgatgctat tgattattta 1020
aaggatcttc tgcagaggat aaatgatctt cataatgaac tcgaggcaat tccaccaggc 1080
tcctcagtaa cggcaacgtc aagccacaat ccattgactc acacttctac caccctccca 1140
catctcgtga aggaagaaat atactcaagt tcaatgccta gtccacaaag cacagctgca 1200
agggtcgagg taagggcaag agaagggagg gctgtcaaca tccacatgtt ttgtgcacgc 1260
agacctggtc tcttactttc caccatgagg gcccttgaca acctagggtt agacattcag 1320
caagctgtca tcagttgttt caatgggttt gctatggatg tatttcgagc cgagcaatgt 1380
ggtgaaaccg aggacattct gcctgatcag atcaaagcag ttcttctaga ttcggctggt 1440
tttcatggag caatttag
1458
<210> 29
<211> 1050
<212> DNA
<213> Glycine max
<220>
<400> 29
   atgatgtcga gaataaacaa cagccttgtc tggatggacg aaccagaagc cgaaaacact 60
   gcctccgctt cctgggccag aaactccaag cctaacaaca acaacatcat cattaccacc 120
   ggcacaacca caaccacagg tttcgtcgaa acctctaacg acttaggttc tctctgcgcc 180
   ttcaagccca tgctcgatgt tgacgacgaa tggtacaaca acatcaccaa ccaccaccac 240
   caccaccaag atatggcctt cgaccccgac aacctcttac tccacccctc cgccgtggac 300
   tcctcctcct cctgctcccc ctctcacctc cagttcttcc tccctcccaa ccccaaactc 360
   aaccccttcg atatgggctt tcttgaccct caagcttctt cttccttacc ccttctcgcc 420
   tccgaaacaa cacacttcac accccctcag accagcacca ccaccgcttt cgccggcttc 480
   cagagcctcc aagagggttc ctccaatcct ctctttctca accgttccaa cattctgaga 540
   cctctcgagt cgctgccccc ctccgggccc caacccaccc ttttccagaa gagagccgcg 600
   ctcaggaaga acatgaacat gagtgacaat aagaagagga aggagaagga tgaggtggtg 660
   gttgaggatg tgagtttcga cgggtctggc ttgaactacg attctgacga cttaactgag 720
   agtaattata atgacgctaa ggagaagaat ggtggtgggg gggttagttc caatgccaac 780
   agcaccgtca ccggtttgga tcagaaaggg aagaagaagg gaatgcctgc taagaatttg 840
   atggccgaac gacgccgccg gaagaagctc aatgacagac tctacatgct gaggtccgtt 900
   gttcccaaga ttagcaaagt aagatttctt ttcttttctt ttcttttcct ctcaccctct 960
   tgccaaattt tgaatctgca gttacttttc aagttcttgg agttaaattc atttgaaagg 1020
   tcaatacttt gtggtgtgtg tttggattag
   1050
<210> 30
<211> 1266
<212> DNA
<213> Raphanus sativus
<220>
<400> 30
atggttctcg acgggaatgg cggcgctgtt tggttaggcg gcggcggagc aggagaaagg 60
tttcatgagg acgaggagga ggagaacggt gcagctcact tcaaagctat gcttgaaggt 120
ggtgactggt tctttaacca accacaagat ctccacatgt tacagagcca tcatcatcaa 180
gatttcagat tcctcggtgg tggttgtggt ggtttcgctt ttaatcctaa cgacaatctt 240
cttcttgatc cgttcttggc cgctgctaac aacagcaagt cttgtctcct caacaacgtt 300
gcaaaccctt ttgataacaa taatgccttt gacttcgggt cgtctgattc ttgttttctt 360
ggagggtatg gttcgttgag ctcctcctcc gtccctgagt tcttatctgc tcgggagaac 420
aacaacaaca cctcaacgcc gttggagtca gaaggtttcg ggagcagagc gaaggttctg 480
aagcctttag aggtgttggc atcttccggt gcacagccta ctctgttcca gaaacgtgca 540
gccatgagga agctgagtga ggatggagag atggacgaga ctggggttga tgtttcaggg 600
ttgaactatg agtctgatga gctgaacgag aactgtaaag ttgttcagaa cggtggagga 660
ggaggaggta ataaaggtaa gaagaaaggg atgcctgcta agaatctgat ggctgagagg 720
agaaggagga agaagcttaa tgataggctt tatatgctta gatcagttgt ccccaagatc 780
agcaaaatgg atagagcatc aatacttgga gatgcaattg attacctcaa ggaactatta 840
caaaggatca atgatcttca caacgagctt gagtcaactc catctggttc tttgcctcca 900
acttcatcaa gcttccatcc gttaacgcct acaccgcaga ctctttcttg ccgtgtcaag 960
gaagagttgt gcccctcttc tttgccaagc cctaaagacc agcaagcaag agttgaggta 1020
agattaaggg aaggaagagc agtgaacatt cacatgtttt gtggtcgtag acctggtctt 1080
ttgctggcta ccatgaaagc tttggatagt cttggattgg atattcagca agctgtgatt 1140
agttgtttca acggttttgc cttggatgtt ttccgcgctg agcaatgcca agaaggacag 1200
gagataatgc ctgatcaaat caaagctgtg cttttcgata cagcaggcta tgctggtatg 1260
atctga
1266
<210> 31
<211> 1146
<212> DNA
<213> Triticum aestivum cultivar
<220>
<400> 31
atggagaact cggcggcggc ggtgggggtg gagaaggagg acgagctggt gggggcggc 60
gggggcgact gggggtacct cacgtcggag gcgatggcga cggccgggtt cccggcgttc 120
gggttcccct gcggcgccag gggcggcgtc acgcccgcgc cgacctcggc gtcgctgctc 180
atgtccatgg agcacgccgc gctgttcgac tacaacgccg ccttcccgtc gtcgtcgtcc 240
tccgccgccg ccgcgccccc ggcctaccac gacttcgggt ccggcggcaa ccccttcaac 300
gtcgacgccc cgccgttcct tctcgaggcc ccgccgccgc tgacggtggc gccgggaggg 360
cagaaagggg ggttcttggc gcccccgctg tcggcgttcg gcgatggcat ggggtgggac 420
gacgaggacg agctggatca gcagagcgtg gacgcctcct ccttgggggt ctcggcctcg 480
ctggagaatg ccgtggtcgg cgcgccgggg ggaggtggcg gcggcggcgg cggcaacggc 540
aagggcaaga agaaggggat gccggccaag aacctgatgg cggagcggcg gcgcaggaag 600
aagctcaacg accgcctcta catgctgcgc tccgtggtgc ccaagatcag caagatggat 660
agggcttcaa tccttggtga tgcaatcgac tacctgaagg agctcctgca gaggattagc 720
gatctccact ccgagctcga gtctgctccc agctctgccg cactcggtgg accatcgaca 780
gctaatagct tcctcccgtc gacgcccacc ctgcagccgt tccccggccg catcaaggag 840
gagcggtgcc cgccggcccc tttccctagc cccagcggcc agcaggcgac ggtggaggtg 900
aggatgaggg aggggcaggc ggtgaacatc cacatgttct gcgcgcgcag gccgggcatc 960
ctgctgtcca ccatgagggc gctggacagc ctcggcctcg acatcgagca ggccgtcatc 1020
agctgcttcg atggcttcgc catggacgtc ttccgcgccg agcaatgcag ggagggccct 1080
gggctcctgc cggaggaaat taaggccgtg ctcctgcact gcgccggtct ccagaacgcg 1140
atgtag
1146
<210> 32
<211> 315
<212> DNA
<213> Hordeum vulgare
<220>

<400> 32
atggggtggg acgacgagga cgagctggat cagcagagca tggacgcctc ctccttgggg 60
gtctccgcct cgctggagaa tgcggcggtc ggcgcgccgg ggggaggtgg cggcggcggc 120
aacggaaggg gcaagaagaa ggggatgccg gccaagaacc tgatggcgga gcggcggcgc 180
aggaagaagc tcaacgaccg cctctacatg ctgcgctccg tggtgcccaa gatcagcaag 240
gtgaggaacg ctcccccccg tcatggaaat cgatcaccta cttgttccca tctctactat 300
acatattatg gttga
315
<210> 33
<211> 1410
<212> DNA
<213> Hevea brasiliensis
<220>
<400> 33
   atgcttgata ccgactggta tgatatcaac ccatctcctc accatgacct ccatgctctc 60
   caaaatcacc atgaaatcag agaccttggt ttttgtgcta accccacacc tgaaaatctt 120
   ctcttacagc ctatggactc ttcctcttct tgctctccct cgcaggcctt tactctagac 180
   ccagcacagg cacaccagcc tttttttccc cccaaatcct gcttctcttc ccttctcaat 240
   gtcaccaacg cttttgacaa tggcttcgat ttgggttgcg aaaatgcttt tctcggccaa 300
   taccagccga atcaggcttc aaatttgatg ggttttagtg ggcccgagtt ccaagcttcc 360
   cagttgcttc cggtgtccga taatgctgcc gcaatcggag gtgctgggtt tagtaatgta 420
   ggacttgagg gttttgatgg ttctactgtg aatgctctgt ttttaagcag ggctaaggtt 480
   ttgaggccac ttgaggtttt gcctccagtg ggttcaccgc caactctatt tcagaagcga 540
   gccatgttaa ggcaaagtgg ggataagttg gggaatttgg agatatcagg gctgagatat 600
   ggaggagaaa ttggggggaa ttggggagaa atggaaagga aaagaaagaa gagtgaagaa 660
   ggtgaaattg aggaagcgag cttcgatgtt tcgggtttga attatgattc tgatgaacac 720
   aatggagatg gtaagattga agagagtgtg aagaataacg gtggaagcaa ttcgaatgcc 780
   aatagcaccg tcactggtgg aggtggtggt gatcaaaagg ggaagaagaa ggggatgccg 840
   gcaaagaatt tgatggctga gagaaggaga aggaagaggc ttaatgatag actctacatg 900
   cttagatctg ttgtccccaa gattagcaag atggatagag cttcaatact tggggatgcc 960
   attgattatt taaaggagct tctgcaaagg atcaatgatc ttcataataa attggagtca 1020
   accccacctg gtcctttgtt gcctcaatct acaagtattc atccggtgac tccaacacct 1080
   cccgccatac cttcccgcgt gaaggaagaa ctatgcccta gctcgttgtc aagccctaaa 1140
   aaccaagcta caagggtgga agttagggta agggaaggaa gggcagtcaa catccacatg 1200
   ttttgtgctc gcagaccagg tttattgctc tccactatga gggctctgga caaccttggg 1260
   ttagatattc agcaggctgt catcagttgt ttcaatgggt ttgcactcga tgtgtttcga 1320
   gctgagcaat gcagggaagg tcaggatgtc ttaccagagc aaatcaaagc agtacttctt 1380
   gattcagctg gctttcatgg aataatgtga
   1410
<210> 34
<211> 1158
<212> DNA
<213> Juglans regia
<220>
<400> 34
   atgctgtcca gagtaaacgg cgtctggatg gacgatagag aggaccatga tcctgcttcg 60
   tgggccagaa ccaccaacga cgctaacacc agcaacagtt gcggtgtggc agagaagaac 120
   gagcacgact tgggttcgct ctccaccttc aagtccatgc ttgaaatcga agacgactgg 180
   tataatatgg ccaataatac tctccaaagc cacactgata tcagagattt aactttctct 240
   cccaaccttg ctgaccaccc agacaatcta ttgctaaacc cagtggactc ctcctcttct 300
   tgctctccat cttcctctgt tttcaccaat ctcgacccct ctcaggtgaa ctactttttg 360
   cctcccaagc ccactttgtc ctcgctaatt aacggcctct cgagcaatcc tttagagaat 420
   ggatttgatt tgggatgcga ggttgggttt cttgaagccc aaacttccaa tgcttccact 480
   ctgctgaaca ggggaggtgg ggttttgagc aacttcaccg atttgagcac caattctcag 540
   attaatgctc ccagtatatg ctcggactcc caacttcgtc tgccaaattt tcccgaaaca 600
   ggtgctgggt tcgtggggtt taaggctttc gatgggggtt ctgggaatgc cctgtttatg 660
   aacaggtcta agattctgag acccctcgag tctttccctt caatgggcgc gcagcccact 720
   cttttccaaa agagagcggc tctaaggaaa aacatagcgg atagtggcgg caatttgtcg 780
   atttcgactt cggttgaggg agataagggg aagacagaaa cccatgaacc gggcgaggga 840
   aagaggaaac tgagcaatgg ggatgatgtg gaagatgcta gttttgatgg ctctggtctg 900
   aattacgatt cggatgagtt catggagaac gataaggcgg agggcaatgc caagaacacc 960
   ggcgggaata gctccaacgc cactagtacg gttaccggtg gaggagagca aaaggggaag 1020
   aagaaaggcc tccctgctaa gaatttgatg gccgagaggc gccgccggaa gaaacttaat 1080
   gatagattat acttgctgag gtccgtggtt cccaagatta gcaaagtaag cgtttttttc 1140
   gctgtgtttt tctcgtga
   1158
<210> 35
<211> 1497
<212> DNA
<213> Lactuca sativa
<220>
<400> 35
atgatgcccg acggtggtgc tgtttggatg gaaggaggag gaaaccagga tgaaattgga 60
acatcttcat ggacgccggc caccaccacc accacaaccg ccaatcacca tcatgttctt 120
ggaattgaag ctgctgcaac atcatgcaac gaagaaacca cagccgccaa cacttacgct 180
tctctcacca ccttgaaatc aatgcttgaa tccgagtggt atcaacacaa caatctcaat 240
ctctctacga atccttctga taacaattca atcttcttac ctatggattc ttcttcttcg 300
tgttcgccgt cgcattccca ccacccgttc tctctccccg acccttcgcc gtcacactca 360
tatcccttct ttccccccaa attcaacaac ccttatgaca ttgggtttga tttcggatgc 420
gaccctggct tcttacctaa tcacacttcc aatttgatga atttttctgg gttatgttcg 480
caaacccaca tcgccatccc ggagctttct tccagttccg actttccggc caccaacaat 540
agcaacgaca cgagcgacat tggaggacct gggttcaatc cgcagttaac ggcgtttgac 600
ggttttcatg gcgctgcacc catgaatcct ctgttcgcag ctaggtctaa ggttctacgg 660
ccacttgaca tttcgccacc ggtcggagct cagcctacac tgtttcaaaa acgggctgcg 720
ttacgacaaa gttcgctcaa attaggggaa ttgggaggta caaattgggg gaagagagca 780
gagagtaata gcggtatggg atcaaaatta acggtgggaa tagctctaat gcaaacagta 840
ctgtcactgg cggctatcgg gagcaatcaa aaggggaaaa agaaagggtt gccggcgaag 900
aatttaatgg cggaaagacg tcggagaaag aagctaaacg accggcttta catgctcaga 960
tctgtagtcc ccaaaatcag caagatggac agagcttcaa ttcttggcga tgcgatagag 1020
tacctgaaag agcttcttca gaagatcaac gaccttaact atgaactcga atcaacccca 1080
tcaacctcat cattgacacc caccaccacc ataaccaccc ccggcagcgg cacccccacc 1140
ggtttctacc ctctaacccc aactccgacc agtctccctt caagaatcaa ggaagaactt 1200
tgccccaccg cgatccctag cccaactggc cagcctgcaa gagttgaagt aagacagaga 1260
gaaggaagag cagtgaatat ccatatgttt tgcagtagga ggcctggttt gcttctatca 1320
accatgagag ctcttgataa tcttggttta gatattcaac aagctgtcat aagttgcttc 1380
aatggcttcg ctcttgatgt ttttcgagct gagcaatgca aagaaggtca agatgttcat 1440
cctgatcaag tgaaagcagt gctgctggag tccgctggtt accatggtgt cgcttga 1497
<210> 36
<211> 1575
<212> DNA
<213> Oryza sativa
<220>
<400> 36
atgctgccgc ggtttcacgg cgccatgtgg atgcaggacg acggcggcgg cgaccaagaa 60
cacgggcagg cggcgccgcc tgggcaggag cagcaccacc acgaccagca tctcatggcg 120
ttggcggccg cggccgcggg cggcgccggg ttcggcgcgg cgcaggcgcc ggcgccgctg 180
ctcgatgagg actggtactt cgacgcggcg ggtggtggtg gtggtggcgc gcatgggtcc 240
atgatgctgg gtttgtcgtc cgtccatggc gggattgggg cggggacgtc tggtggtggg 300
catgggcagc agttctcgct gctcaacatg ggcgccgcgg ccgcgccgtt cgacgtctcc 360
gggttcgacc tcgggatcgc ctgcggcggc gttggcggcg gcggcgacgt ggtgtcgttt 420
cttggcggcg ggaacgcgtc gaacaccgcg ctgctccccg tcgggaacgc ggggttcctc 480
ggcacgttcg gcgggttcgg caccgcggcg tcccaaatgc cggagttcgg cgggctcgcc 540
gggttcgaca tgttcgacgc gggcgccgtg aacaccgggg gcagctcctc ctcctcgtcg 600
gcggcggcgg cggcggcgtc cgcctcggcg cacgtgagca acaccgcgcc gttctccggg 660
cgcggcaagg cggcggtgct gcggccgctg gatatcgtcc cgcccgtggg cgcgcagccg 720
acgctgttcc agaagcgcgc gctccgccgc aacgccggcg aggacgacga cgacaagaag 780
cgcaaggccg ccgcgggcgc gggcgcgggc gcgctgtccg ccgacggcgc cgacatggtg 840
ctcgacgacg gcgacgacga cggcctcagc atcgacgcgt cgggcggcct caactacgac 900
tccgaggacg ccaggggcgg cgaggacagc ggcgccaaga aggagtcgaa cgccaacagc 960
acggtcaccg gcgacgggaa ggggaagaag aaggggatgc cggccaagaa cctcatggcg 1020
gagcgccgcc gccggaagaa gctcaacgac cgcctctaca tgctccgctc cgtcgtgccc 1080
aagatcagca agatggacag ggcttccatt ctcggcgacg cgattgagta cctgaaggag 1140
ctgctgcaga agatcaatga tcttcagaat gagctcgagt cgtcccccgc gacgtcgtca 1200
ttgcctccaa cacccacaag cttccatccc ctgacaccga cgctgcccac attgccgtcc 1260
cgcatcaagg aagagatctg cccaagtgca ttgccaagcc ccactggaca acagccaagg 1320
gttgaggtta ggctgaggga aggccgggct gtcaatatcc acatgttctg tgctcggagg 1380

cccggtctac tgctctctgc catgagggcc gtcgaaggcc ttggtctcga tgtccagcaa 1440
gctgtaatca gttgcttcaa tggctttacg ttggatattt ttaaggctga gcaatgcaag 1500
gacggccctg ggctgttgcc tgaagaaatc aaggccgttc tgatgcaatc cgccgggttc 1560
cataccatga tctag
1575
<210> 37
<211> 1152
<212> DNA
<213> Phalaenopsis aphrodite cultivar
<220>
<400> 37
atgctctcga ggctcagcgg cgccgtttgg agggacggag aagcggcaga ggaggaagac 60
gacaccgctt catggataag atccacctct tgcaccgccg ccatgggaga agtgagggac 120
gacatcgccc taccctgtct caaatctatt ctcgacgacg actggtacgt cgcagccacc 180
tcttccaacg ccggtaacct cgccgggagc tccttaaacc aggggtttgc cggcgactcc 240
tccgcgctcc tttcgccgcc ggtggattcc tcctgctcac cttcctccgt cttcacactg 300
gatccctctg cccaccagtc cttcttcccc cacaaacccg cactttcttc tttcctctct 360
gctgtctgct ccaacccttt tgattccggc ttcgattttg tctcggagaa cccgaccttc 420
ctcccctcgg cgaactccgc cgcctccacc tccgtttttg gtggtggaat cttgggtttc 480
cccaacgtgc agattaatag tcaagatttt ctgcctatga ccggaagctc ttctttgttg 540
aaccccatgg ccttcgattc gcttgaaaat tcgccatttt tgagcagagg gaaggttttg 600
aggcctctgg agatctttcc accagtaggt gctcagccta atctttttca gaaacgtgcg 660
gcggccgcac tgcgccagaa ttcaagttct attggtggtg agaaaggtat gcgtttgggg 720
gttttgggta tggaggatgc agggcagatg aggagctctt gggatggtga gtttgagaag 780
cggaggagaa tatatgatga ggatgagctt gatgatgcca gcattgatgg ctctggttta 840
aattatgatt ctgaggatat ctgtgaagtt gcagagactg taaagttgga tgaagagaag 900
aatgatggtg gaggagcacc tgccggagga ggaggaggag gtaatgtttc cattacgaat 960
agtagtctta ccggaggggg tggagagctg aagggtagga gaaaggggct tcctgctaag 1020
aatctgatgg ctgagaggcg taggaggaag aagctcaatg acaggcttta tatgctaaga 1080
tctgttgttc caaagatcag taaggtgaga accctcatct ccttcatttc attttcgctg 1140
gcttcttact ga
1152
<210> 38
<211> 537
<212> DNA
<213> Prunus mume
<220>
<400> 38
atgtccagca gcgctgtttg gatgttcccc tccaaatcct gcttctcttc tctcctcaac 60
gtcgtctcca ccaacccttt tgacaatggc tttgatttgg gctgcgacag tggctttctc 120
agctccttcc agggaaacca accttccaat tcctttgctt tgatgggttt cacagccatc 180
aattctcaat ctcagatggg tactcccgaa ttgagttcga gcgtcgagtt cccaacgact 240
cggttgctgt caacgcccaa caacgacact gagctcgatg gcaacttcag ccttacgggc 300
ttccaaggct tcgatggctc tgggggtgct cagctactca acagggctaa gccactgaga 360
ccccttcacg tattccctcc cgtgggtgct catcccactc tctttcagaa gggagcggaa 420
cttcgtcaaa acttcgccgg tgacggaccc gataaattgg ggaatttgga gatttcgggg 480
accgccacca ccactgccgt tggctccgcc gccgccgaga gaagaagaca gtgctga 537
<210> 39
<211> 1557
<212> DNA
<213> Camellia sinensis
<220>
<400> 39
atggaagata gaggagatga agaaacacct tcatgggctg ctcaaaacaa caacaatggc 60
tgtattgtag acaagaaaga cgaactgggt tcactctcta catttaaatc aatgcttgga 120
gttgaagacg aagactggta tgtaaacacc aacaacaaca acactatcct cactttccca 180
tcaaatttca ccgaatctga taacaatttg ctgttacaat cggtaggttc gtcttcgtct 240
tgttcaccat cttcagcttc agttttcacc aatctcgacc catctcagga acaatacttt 300
ctcccttcaa aaccctccat ttcttccgtc ctcaatccca tctccaacaa cccattagac 360
cacagcttcg atctgggttg cgaaactggg tttctccaga cccattcgtt gacttctctg 420
aacaggggag gtgggatttt gactggattc actgatttaa cctcacaatc cctgatgggt 480
actcacaatt tggccttaga tcctcaattt tcaaataccc attcgcttca attgaccggt 540
tcgacatcgt ttcggggttt tcaggaggag ggttttggga atcctttgtt tctaaatagg 600

tccaaaatgt tgaagccact tcaaaatttt acttcaattg gtgcacagcc aactctgttc 660
cagaaaagag ctgctctgcg caagaatttg gccaacaatg ggaccaattt ggggggtttg 720
ggtactgaaa gaggtgggat atcaagcaat gtacaagggt atagtgggaa gatggaagtg 780
agtgaagtga atgagaagaa gaagaggaag tacagcattg gggaagatgt tgaagatgtg 840
agccttgatg ggtctggatt gaattatgac tctgatgagt ttatggagaa caataaggtg 900
gaagagagtg gcaaaaatgg tgggaatagc tcgaatgcga atagtactgt cacgggcgga 960
gatcagaaag ggaagaagag agggcttcca gccaaaaatt tgatggcaga gcgacggcgg 1020
cggaagaagc tcaatgatag gctttacatg ctgaggtctg ttgttccaaa aattagcaag 1080
atggataggg cttcaattct tggggatgca attgaatact taaaggaact tctgcaaaag 1140
atcaaagacc ttcacagtga actggagtca aaccctcctg gctcttcatt gacacctacc 1200
tccacaagct tctacccttt gacacccacc ccacattccc tgccatgccg catcaaggaa 1260
gagctttgcc caagttcatt gccaagccca aatggactac ctgcaagggt agaagtaagg 1320
ttaagtgaac gtagagctgt taatatccac atgttttgta gccgcagacc aggtctcttg 1380
ctctctacta tgagggcttt ggagaacctt gggctagaca ttcaacaagc tgttatcagc 1440
tgcttcaatg ggtttgcgat ggatatcttc cgagcagagc aatgcagaga aggccaagat 1500
gtccatcctg accaaatcaa agcggtactc ttggattcag ctggcttcat gatgtag 1557
<210> 40
<211> 1485
<212> DNA
<213> Arabidopsis thaliana
<220>
<400> 40
atgggtcttg acggaaacaa tggtggaggg gtttggttaa acggtggtgg tggagaaagg 60
gaagagaacg aggaaggttc atggggaagg aatcaagaag atggttcttc tcagtttaag 120
cctatgcttg aaggtgattg gtttagtagt aaccaaccac atccacaaga tcttcagatg 180
ttacagaatc agccagattt cagatacttt ggtggttttc cttttaaccc taatgataat 240
cttcttcttc aacactctat tgattcttct tcttcttgtt ctccttctca agcttttagt 300
cttgaccctt ctcagcaaaa tcagttcttg tcaactaaca acaacaaggg ttgtcttctc 360
aatgttcctt cttctgcaaa cccttttgat aatgcttttg agtttggctc tgaatctggt 420
tttcttaacc aaatccatgc tcctatttcg atggggtttg gttctttgac acaattgggg 480
aacagggatt tgagttctgt tcctgatttc ttgtctgctc ggtcacttct tgcgccggaa 540
agcaacaaca acaacacaat gttgtgtggt ggtttcacag ctccgttgga gttggaaggt 600
tttggtagtc ctgctaatgg tggttttgtt gggaacagag cgaaagttct gaagccttta 660
gaggtgttag catcgtctgg tgcacagcct actctgttcc agaaacgtgc agctatgcgt 720
cagagctctg gaagcaaaat gggaaattcg gagagttcgg gaatgaggag gtttagtgat 780
gatggagata tggatgagac tgggattgag gtttctgggt tgaactatga gtctgatgag 840
ataaatgaga gcggtaaagc ggctgagagt gttcagattg gaggaggagg aaagggtaag 900
aagaaaggta tgcctgctaa gaatctgatg gctgagagga gaaggaggaa gaagcttaat 960
gataggcttt atatgcttag atcagttgtc cccaagatca gcaaaatgga tagagcatca 1020
atacttggag atgcaattga ttatctgaag gaacttctac aaaggatcaa tgatcttcac 1080
aatgaacttg agtcaactcc tcctggatct ttgcctccaa cttcatcaag cttccatccg 1140
ttgacaccta caccgcaaac tctttcttgt cgtgtcaagg aagagttgtg tccctcttct 1200
ttaccaagtc ctaaaggcca gcaagctaga gttgaggtta gattaaggga aggaagagca 1260
gtgaacattc atatgttctg tggtcgtaga ccgggtctgt tgctcgctac catgaaagct 1320
ttggataatc ttggattgga tgttcagcaa gctgtgatca gctgttttaa tgggtttgcc 1380
ttggatgttt tccgcgctga gcaatgccaa gaaggacaag agatactgcc tgatcaaatc 1440
aaagcagtgc ttttcgatac agcagggtat gctggtatga tctga 1485
<210> 41
<211> 1494
<212> DNA
<213> Brassica rapa
<220>
<400> 41
atggttctcg acggaaacgg cggtggtgta tggttaggcg gcggcggcgg cggcggagga 60
ggagaacggg tgcaggagga ggagaacgag gaagcttcgt gggggaggaa tcaagaagac 120
ggtggtcagt tcaagcctat gcttgaaggt ggtggtgatt ggtttactag taaccaacca 180
catccacaag atctccagat gctacagagc cagcaagact tcagattcct cggtgggttt 240
ggtttcaacc ccaacgacaa tcttcttctt cttcaacact ccatggactc gtcttcttcc 300
tgctctccct ctcaggcttt tagcctcgac ccctctcaag cttcgttctt ggctgctgct 360
aacaacaagt cttgtctcct caacgttgtt ccttcctccg caaacccttt tgacaatgcc 420
ttcgagttcg ggtcggattc tggtttcctt aaccaaatcc aagctccggt ttcgatgggg 480
ttcggttcgt tgacgcagct ggggagctct gtccctgact tcttatctgc ccggtcgctt 540
ctcccgccgg aaaacaacaa cgcaacaccc ttatgcggag gtggaggagg tgggttcacg 600

ccgttggagc tggaagggtt tgggagtcca gcgagctttg tcgggagcag accgaaagtt 660
ctgaagcctt tagaggtgtt ggcgtcgtct ggtgcgcagc ctactttgtt ccagaaacgt 720
gcagccatgc gtcagagctc tggaagcaag atggggaact cggagagctc ggggatgagg 780
aggctgagcg atgatggaga tatggatgag actggggttg aggtttcagg gctcaactat 840
gagtctgatg agctgaacga gagcggtaaa gcttctgaga gtgttcagaa cggaggaggt 900
aaagggaaga agaaagggat gcctgctaag aatctgatgg ctgagaggag gaggaggaag 960
aagcttaatg atagactcta tatgcttaga tctgttgtcc ccaagatcag caaaatggat 1020
agagcatcaa tacttggaga tgcaattgat tacctcaagg agcttttaca aagaatcaat 1080
gaccttcaca acgagcttga gtcaactcca aatggatctc tccctctggc ttcatcaagc 1140
ttccatccat taacgcctac gccgcagact ctttcttgcc gtgtcaagga agagttgtgt 1200
ccctcttctt tgccaagccc taaaggccag caagcgagag ttgaggttag attaagggaa 1260
ggaagagcag tgaacattca catgttctgt ggtggtagac ctggtctttt actagctacc 1320
atgaaagctt tggataatct tggattggat gttcagcaag ccgtcatcag ctgtttcaac 1380
gggtttgcct tggatgtttt ccgcgctgag caatgccaag aaggacaaga gatattgcct 1440
gatcaaatca aagcagtgct tttcgataca gcgggatacg ctggtatgat ctga 1494
<210> 42
<211> 1500
<212> DNA
<213> Isatis tinctoria cultivar
<220>
<400> 42
atgggtcttg acggaaacgg cggggcggta tggttaggcg gcggcggcgg cggaggagga 60
ggaggagaac ggattcagga ggaggagaac ggggaagctt cgtggggtag gaatcaagat 120
gacggtggag gttctctttc tcacttcaag cctatgcttg aaggtgactg gttcagtaac 180
acaccacacc cacaagatct ccagatgtta cagagccagc aagatttcag atttctcggt 240
ggtttccctt tcaaccccaa cgacagcctt cttcttcatc agtccattga ctcgtcttcc 300
tcttgctctc cctctcaagc ttttagcctc gacccatctc agccttcctt cttagcttcc 360
accaacaaat cttgtctcct caacgttcct tcctccgcaa accctttcga caatgccttc 420
gagttcgggt ctgattccgg tttcctcgcc caaatccaag ctccgatctc catggggttc 480
ggctctttga cgcagttggg gaaccgggaa ctgagctccg tccctgactt cttatctgct 540
cggtcgcttc ccccgccgga aaacaacaac acgactccgt tatacggcgg aggtgggttc 600
acgccgttgg agttggaagg ttttgggagt cctgcgagtg gcggtttcgc tgggaacaga 660
gcgaaagttc tgaagccttt agatgtcttg gcatcgtctg gtgcgcagcc tactctgttc 720
cagaaacgtg cagctatgcg tcagagctct ggaagcaaga ttgggaactc ggagagctca 780
gggatgagga agctgagcga tgatggagag atggacgaga cggggattga ggtttcaggg 840
ttgaattatg agtctgatga gctgaacgag ggaggtaaag cggcggagag tgttcagaat 900
ggaggaggag gtaaaggtaa gaagaaagga atgcctgcta agaatctgat ggctgagagg 960
aggaggagga agaagcttaa cgataggctt tatatgctta gatcagttgt ccccaagatc 1020
agcaaaatgg acagagcatc aatacttgga gatgcaattg attatctcaa ggaacttcta 1080
caaaggatca atgatcttca caacgaactt gagtcaactc ctcctggatc tttgcctcca 1140
acttcatcaa gcttccatcc attaacgcct acacctcaga ctctttcgtg ccgtgtcaag 1200
gaagagttat gtccctcttc tttgccaagc cctaaaggcc agcaagcgag agttgaggtt 1260
agattaaggg aaggaagagc agtgaacata cacatgtttt gtggtcgtag accgggtctt 1320
ttgctggcta ccatgaaagc tttggataat cttggattgg atgttcagca agccgtgatc 1380
agctgtttca acgggtttgc cttggatgtt ttccgagctg agcaatgcca agagggacag 1440
gagatattgc ctgatcaaat caaagcagtg ctttttgata cagcgggtac gctggtatga 1500
<210> 43
<211> 1572
<212> DNA
<213> Eucalyptus camaldulensis
<220>
<400> 43
atggttctgg gtggcgagga agacgaggtc tcctggaccc gggaccacca ccaccaccac 60
gacggcggcg gcggcggcaa cgacgacgaa gcggcgcggc acaaggaagg cgagctggcc 120
atgggagggg cctcgacctt caagtccatg ctcgacgccg actggtccta cttcgccacc 180
aacccgcctc accattccca gcagcagcag cagctgcagc ccgctccgga aatggccgac 240
ctttccttct cctccgccgc cccgattccc gataacgccc tcctcctcca ccattcgctc 300
gactcgtcct cctcgtgctc gccgtcgcag gccttcgtcc tcgacccctc gcagacccac 360
cagcccttcc tgcagcccaa gcactgcttc tcctccctcc tcaacgtcgc ctggaacaat 420
tcctttgaca atggattcga tttgggctgc gacgccgccg ggctttttgg ctcggtgcag 480
ggcaatcaga cctcgagcgc gccgcttctc atgggcggtt ccgagctggg ctccggttcc 540
gagctgctgc ccgtgcccag tcggctcgtc ccgctcggtg aaaactcaac ccaattgcca 600
ggcggcgggt tcgggctgac tgggttcgaa ggcttcgaga gccccggcaa cccactgttc 660

acgaacaggt ctaccaaggt cctaaggcct cttgaggttt tcccccagac gggcgcccag 720
ccgactctgt ttcagaagag agctgccctg agaaatgggt ctggcagtaa tggcggcggg 780
gctgagaaat tggggaattt ggagatcgac aagaagagaa agaagattga tgatggtatt 840
aatgacgaat tgagctttga tgggtccggt ttgaattatg ataccgatga gggtaatgag 900
agcgggaagg ctatggagga ctcgaagcac gaagggtgca attcgaatgc aaacagcacg 960
gtcacagtcg gcgatcagaa ggggaagaag aaggggctgc cggcgaagaa tctgatggct 1020
gagcggcgcc ggaggaagaa gctgaatgat aggctgtaca tgcttaggtc ggtagtgccc 1080
agatcagcaa gaatggatag agcttcaatt ttcggggagg ctattgatta tttgaaggag 1140
gtttgcaaaa ggatcaataa cctccataac gagctagatt cgaccccgcc tggaaccatg 1200
ttgccccctt cgactaattt tcatccattg acaccaactc cgcccaccct tccttgccgt 1260
gtgaaggaag aactgtgccc gagctccttg ccaagtccca aaggccagcc tgcaagagtg 1320
gaggttagag tgagggaagg gagagcagtg aacatccaca tgttttgtgc acgcagaccg 1380
gggctcttgc tctccaccat gagggctcta gacaaccttg ggttggacat tcaacaggct 1440
gtcatcagct gtttcaacgc attcgctatg gacattttcc gagctgagca atgtagggaa 1500
ggtcaagatg tcctcccaga acagatcaag gcacttctct tagagtcagc tggcttccat 1560
gggatggtgt ga
1572
<210> 44
<211> 1629
<212> DNA
<213> Jatropha curcas
<220>
<400> 44
atgctgtctg gactaaacgg cgctgtctgg atagatgata aagaaccgca ggactcgaca 60
acaaacacca ttaacattaa cgacagtagc tgtagtatta tacagagtaa acaagagatg 120
ggttcgcttt ctaccttcaa atccatgctt gatgtagaag acgactggta tgttgctaac 180
actagtatcc aaaatcatca agatggtatt agagatatca cattttcacc aaatctcggt 240
gacccagata atcttttgct tcattcagtg gattcttcct cttcttgctc accaacctcc 300
tctgttttca acaatctcga cccatctcaa gttcactact ttatgcatcc aaaacccacc 360
ttatcttcac ttcttaacgt tgtttctaat aaccctttag agcatgggtt cgatttgggt 420
gacattgggt ttcttgataa ccaggctact aatacaacta caacaacagc aaatgcctca 480
tctttactaa acaggggagg tggagtatta ggcaacttta ctgatatgag ctcaaacaat 540
cagattagca gttctaattt atgcactgac ccatcctttt ctactacccg gatgctccaa 600
ttgcctgaaa cgtgtcctgg gtttgcagga tttaggggtt ttgatgagag ttcaggaaat 660
gcactgtttt tgaataggtc taagttgtta aggcctcttg aaacattccc ttcaatgggt 720
gcacagccta ccttgtttca aaagagagca gcgttgagga aaaatttggc tgacaatggg 780
tctaatttgg gggctttaag tgggattgag ataaataaag ggaagagaga aatgagtcag 840
atgggtgaag agaatgagag aaagagaaag ttgggtaatg tagatgatac ttttgaagat 900
gtaagcattg atgggtcggg tttgaattat gactctgatg agttcacgga gaacactaaa 960
atggaggaga ttggcaaaaa tggagggaat agctctaatg caaatagcag tgtcactggc 1020
ggcggtggag atcagaaggg gaagaagaag gggcttcctg ctaaaaattt gatggcagaa 1080
aggaggcgcc ggaagaagct caatgatagg ttatatatgc ttaggtctgt agttccaaag 1140
attagcaaga tggacagggc atcaatactt ggggatgcaa ttgagtactt gaaggaactt 1200
ctgcaaagga tcaatgacct acataatgaa ttggagtcca ctcctcctgg ctcttctttg 1260
acacccacca caagttttca ccctttgacg cccactccat cagcccttcc cagccgtatc 1320
aaggataagc tttgcccaac ctcattgcca agtccaaatg gtcaaccggc aagggttgaa 1380
gtgagagtga gagaaggaag agctgtaaac atacacatgt tttgcagtcg aagagcaggt 1440
ctcctgctct ccactatgag ggctttggat aaccttggcc tagacatcca gcaagctgtc 1500
atcagttgtt tcaatggttt tgccatggat atctttcgag ctgagcaatg taaagaaggg 1560
caagatgttc atcctgagca aataaaagca gtgcttctgg attcagctgg cttccatgga 1620
gtgatgtag
1629
<210> 45
<211> 874
<212> DNA
<213> Picea abies
<220>
<400> 45
atgttgtcga gaatgaatct gaatggcggc gtgtggggtc tggaagatgc cacagacctc 60
cactgcaaca acgacgagaa tttgaatttg cccacgttca aggcaatgct ggacgcggaa 120
gaatggttta cttctcacag caatactagc aataacgata ttactactgc cagtattaat 180
aatgctaaca gccatcctga cggcccatgt ttcaatatcg atatgaagga ttttggatgc 240
tattcgtctc tggtgaatcc gaatcccgag cctcccaata tgctcttgca gcacgccatg 300

aattcttgtt cctcgtcgcc cgcttcgatt ttcagcctgg acccttcgca ggtccagtct 360
tttctagcgg gggggagcaa ccatcatcac caccaccatg tatctccgtt cccggacgtt 420
gcgggttcga gtcccttcca ggagttggcc tgcgaggggt tggtgagcag caacaatgcc 480
agcagtaaca gtagcagcaa tagctactta tatggggggc taccgggagg aattttcagg 540
cagctgagcc ccagcttcgc aggttcgagt ccccgcacga atactgttac ccagacgcct 600
aatctcagca gcccacacag ccagatgggt ggtagtagta cccccaatct tagccccaga 660
atgatgccca cgagcaatct caatctctcg gacaccggat tgttcgctag cagtaatcac 720
accaccacca acaacaacaa taatctgagc aatcttctgg gtttgagtag ccttatgagt 780
cctcgaatcc cagcgccatt aaattttagc agcccgaaga tgcttagacc ccttgagatt 840
tgcgccccag ttggtgctca gcctactttg tttc
874
<210> 46
<211> 1632
<212> DNA
<213> Populus suaveolens
<220>
<400> 46
atgctttgta gactcaatag ccacaatatt tgggttgatt atcatgccga agagcaggat 60
tccacatcgg accgccccca ccacaacaac attaccaata ctgcggctgg ttgtggcggg 120
gttatgttgg ggggaaaaca agaaatgggt tctctttcga ctttcaaatc tatgtttgaa 180
gtggaccacg agtggtacgt gactaacgcc tatagtgcta ttcctcgggg tgatcaagat 240
agtatcaaag acctcacctt ttcaccgcgt cttgtgcacc cttataatct tttgcttctg 300
ccagggcatt cgtcctcttc ttgttcccct tcttcttcag ttgtcaacaa tttggatgct 360
tccctgctac attcctttat gcctcccacg cccactttgt cctctctcct gcatgtggtt 420
tcttccaacc cttcaaagga tgggtttgat ttgagtgaaa ttggtttcct tgagaatcaa 480
ggaacaaatt caactacaac agcaaatgtt tcatctccgt taaacaggcc acgtggcgtg 540
ttggggaatt ttgggaattt cactgatttg agttcaacag tccgattctc attcctattt 600
gggttctcat ccccggacta caagtacccg catgcttccc ctgccgagct ccctcctgcg 660
ctctcctgtt ttacacggct tgatgagata tcggtgaatc ccttgttctc ttccaggact 720
aagttgctat cgcctcttga aacttaccct attcatgggc gctcagctac cctgtttcaa 780
agagagcagc tagaggaaag aacttgggga agtgagagag ataaagggaa gagagatatg 840
acacaaatta gagaagagaa tgacaagcag agaaactcta gcagtggcga tgattttctt 900
gaggaattag ctcagatggg tcgggtgaga attatgcatc tgggaattaa agagtatact 960
ttggtggaag agactggtaa gaatgttgga aacagttcaa aggtgaatag tggtgttact 1020
ggtcgtggtg aggatcaaaa ggggaagaaa aggggccttc cagctaagaa tttgatggca 1080
cagtggaggc gccggatgca gctcaatgat aggctgtaca cgatgaggtc tgttgttcca 1140
cagatcagca aaatggatag gccatcaatc ctcggtgacg caattgaata cttgaaagaa 1200
cttctgcaaa ggattaatga cctccataat gaattggagt caactcctcc cagctcttca 1260
ttgacgccaa ccactagttt tcatcctttg acaccgactc catcagcgga gcccagccgt 1320
atcatggacc aactttgccc cagctcattg ccaagcccaa atggccaacc tgcaagggtc 1380
gaggttagag tgagagaagc acgagccgta aacatccaca tgttttgtgg ccgcaaaaca 1440
ggtcttttgc tcttcactat gagagctttg gataaccttg ggctagacat ccagcaagct 1500
gtcattagct gtttcaatgg ttttcccatg gatatcttgc gaaatgagca acgcaaagaa 1560
gggcaggaca tgcatccaga tcaaattaaa gcagtcctac tggattcagc tggcttccat 1620
ggcacgatgt ag
1632
<210> 47
<211> 1194
<212> DNA
<213> Populus trichocarpa
<220>
<400> 47
atgctttata gactcaatag caacaatatt tggcttgaag atcataaaga agagcaagat 60
tcaacaacaa accatcatca ccacaacaac attaccaata ctgcagctgg ttgtggtggg 120
gttatgttgg agggaaaaga agaaatgggc tctctttcta ctttcaaatc tatgtttgaa 180
gtggaagatg agtggtacgt gactaacaac aacagtacta ttcatcaaaa ccatcaagat 240
agtatcaaag acctcacttt ttcaccaagt cttgtcgacc cagataatct tttgcttcac 300
caagtggatt cttcctcttc ttgttcacct tcttcttcag ttttcaacaa tttggatcct 360
tctcaggtac attactttat gcatccaaag cccactttgt cttcactcct caatgtggtt 420
tctaacaacc ctttagagca tgggtttgat ttgagtgaaa ttggtttcct tgagaatcaa 480
ggaacaaatt caactacaac agcaaatgtt tcatctctgt taaacagggg aagtggtgtt 540
ttggggaatt tggggaattt cactgatttg agctcaaaca gtcagattag tattcctaat 600
ttatgttctg atccacaatt ttcaagttcc cgcatgcttc agttgcctga aaatggtcct 660

gggtttaatg gttttagagg gcttgatgag atttcgggga atcagttgtt ttttaacagg 720
tctaaattgt taaggcctct tgaaacttac ccttcaatgg gcgcacagcc taccttgttt 780
caaaagagag cagctttgag gaagaacttg ggtgaagttg agagagataa agggaagaga 840
gaaatgactc aaattagtga agagaaagac aagaagagaa aatttagtag tggagatgat 900
tttcttgagg atgttagctt tgatgggtcg ggtttgaatt atgattctga tgaatttact 960
gagaatacta atttggagga gactggcaag aatggtggaa acagttcaaa ggcgaatagt 1020
ggtgttactg gtggtggtgt ggatcaaaag gggaagaaaa gggggcttcc agctaagaat 1080
ttgatggcag agaggaggcg ccggaagaaa ctcaatgata ggctgtacat gctgaggtct 1140
gttgttccaa agatcagcaa agtaagaatt ttcttcctca tcattttctt gtga 1194
<210> 48
<211> 519
<212> DNA
<213> Potamogeton perfoliatus
<220>
<400> 48
atgctcagat ctgttgttcc caaaatcagc aagatggata gagcttcaat cctgggcgat 60
gcaatagaat acctgaagga gcttcttcaa aggattaacg atctccacaa tgaacttgaa 120
tcatcacctt ccagttctgc tctccccaca cctacaggtt caagtctccg accgttgaca 180
ccgacaccac ttaccttgcc gtgccgggtc aaggaagagc tctgcccaag ttcgttaccg 240
agcccragcg gtcaaccagc cacggttgaa gtcaaggtga gagaaggagg agccgtcaat 300
atccacatgt tttgtgttcg caggcctggc ctcttgctct caacaatgcg rgctcttgat 360
agcatgggtc ttgatatcca gcaggcagtc ataagctgct tcaaygggtt tgctttagat 420
gttttccgag ctgagcaatg caaggatgrc cctggcgttc ctcctgatga tattaaaact 480
gtactgctgc attctgctgg tctacatggt tcgatgtaa
519
<210> 49
<211> 519
<212> DNA
<213> Potamogeton wrightii
<220>
<400> 49
atgctcagat ctgttgttcc caaaatcagc aagatggata gagcttcaat cctgggcgat 60
gcaatagaat acctgaagga gcttcttcaa aggattaatg atctccacaa tgaacttgaa 120
tcatcacctt ccagttctgc tctccccaca cctactggtt caagtctccg accgttgaca 180
ccgacaccac ttrccttgcc atgccgggtc aaggaagagc tctgcccaag ttcgttaccg 240
agcccaagca gtcaaccagc cacggttgaa gtcaaggtga gagaaggagg agccgtcaat 300
atccacatgt tttgtgttcg caggcctggc ctcttgctct caacaatgcg ggctcttgat 360
agcatgggtc ttgatatcca gcaggcagtc ataagctgct tcaacgggtt cgctttagat 420
gttttccgag ctgagcaatg caaggatggc cccggtgttc ctcctgatga tattaaaact 480
gtactgctgc attctgctgg tctacatggt tcgatgtaa
519
<210> 50
<211> 1147
<212> DNA
<213> Manihot esculenta
<220>
<400> 50
atgttgtctg gattaaatgg cactgtatgg atggaagata aagaagcaca acactccacc 60
acagataata acattcataa tagttgtgat gtgatagaga gtaaggaaga tatgggttca 120
ctatctacat ttaaatccat gctagaagta gaagaagaag attggtatat tgctaatagt 180
agtatccatc atcatcaaga taccataaga gatattacat tttcacctaa tctagcagaa 240
acagataatc tattgctaca tcctgcggat tcctcctcat catgctcacc agcatcctct 300
gtttttaata atatagatcc atctcaggtg ccatatttta tgcactctaa acctacatta 360
tcatcactac taaatgctgt ctctaataat ccattagagc atgacattga gttgggagaa 420
attgggttcc tagataacca gtctactact actactacta ctaatgcctc accattgcta 480
aatagaggag gtggagtttt aagtaactac acagatttga gctctaataa tctatttagc 540
tcatctaatt tatcacctga tccaccattt tctactactc gtgcactaca gttaccagaa 600
aatggtccag ggtttacagg gtttaatggt tttgttgaga gctcagggaa taggctattt 660
tgtaataggt ctaagttgtt gaggcctcta gaaacatttc catctatggg tgcacaacct 720
acattgtttc aaaagagagc agcattgaag aagaatttga ctgataatgg aagtaatttg 780
ggggttctaa gtggtattga gattgataaa ggaaagagag aaatcactca gttgatagaa 840
gagaatgaga agaagagtaa attgggtaag ttagatgaca ctgtagagga tgtaagcatt 900
gatgggtcag gtttgaatta tgattctgat gaggtaactg aaaatattaa aatggaggag 960

attggtaaaa atggagggaa tagctctaat gctaatagta gtgtgactgg tggtggtgtt 1020
ggggatcaga aggggaagaa gaaggggcta cctgctaaga atttgatggc agaaaggaga 1080
cgacggaaga agctaaatga taggctatac atgctaaggt ctgtagtacc taaaataagt 1140
aaggtga
1147
<210> 51
<211> 1416
<212> DNA
<213> Actinidia chinensis
<220>
<400> 51
atgtatgaag agagtgaatg ttttgatcct aattctatgc aaggactatc taaagatggg 60
ttttcacaaa atcctagttt ctccatggaa gagccatcct accaccaaaa tccatcacct 120
gaatcagcag ctggtggtgg tgcagctgcc actgtggaag tagaactcca ccagcaaatg 180
ggtttggaca tggctaatat ggaaccatgt tataatataa ataatagtaa tagtctcatt 240
gatagtacct atttgatgca agaagttgtt ctagactcta accatgtttt gccatatgac 300
cactctaatt gggatcctaa tctaaaccat aaccaccatc aagatttgaa ccatcatgtt 360
gaaattgcta atgggcatta tcctaatttg attagtaata attcctctat accagaccta 420
ctaaatctac tccatttgcc tacatgttca ccatcctctg tcctacctaa ttcatcacta 480
tcatttttga gtcaagcaca gaaatctact aattttcctc catcatttct aggggatttt 540
gcatctaatg agtcaggaat ggtttctaat ctagtgcatg actctctatt tcatctaaat 600
ctaccatcac ggccaccatt gctaagggag atgtttcagt ctctacctaa ttgttcattg 660
tttggtggtg gaggtagtgg tggggtggtg gatgagaaag atgggagtga aggagggagg 720
cagtttgata atggggtatt gcagttttgt agagagtggg acatgaattg tatggctaaa 780
gaaggtaatg gtaagggcat tactactaaa cattttgcca ctgagaggca aaggagagag 840
catttgaatg gtaagttcac tgctttgagg agtttggtac ctaatcctac taagactgat 900
agagcatcta taattgggga tgctattgag tacataaaag agctactaag aacagtaaat 960
gagctaaaaa tcctagtaga gaagaagaga tgtggtaaag atagaagtaa gaggcataag 1020
acagaagaag atgggactac tggagattta gaaaatgtta ataagaaacc agatcctgac 1080
caatcctata atgggccatc actaagaagc tcatggctac agagaaaatc caaggataca 1140
gaggtagatg ttcggattat tgatgatgag gtgaccataa aactagcaca gcgaaagaaa 1200
ataaattgcc tactaattgt gtctaaggtg atagatgagc tacagctaga tatccaccat 1260
gttactggtg ggctagttgg ggataactat agttttctat ttaatagtaa gatatgtgaa 1320
gggtcatctg tgtatgctag tgctatagct aataaactca ttgaggttgt gaatagacag 1380
tatgcagcta taccaccatc tactagtagt tattag
1416
<210> 52
<211> 1145
<212> DNA
<213> Musa acuminata
<220>
<400> 52
atgttttcta gaattgatgg aggattttgg atggatgatg gtggagatga tgatgatcct 60
gtagcatgga ctagaggaaa tgcctctagc tctagtggag gggaatgggg ggagactaag 120
gaggatgagc taggcctctc aggatttaag tccatgctag tggatgattg gtatgtagga 180
gctgctgctc atcatccatt tgaggcattc cagactcagc aggacctaaa agatgtagca 240
tttctaccta atccatcacc acacgaggca ctattgctac ctcctgtaga tagcctagac 300
caaagccaac catttttttc atctagacct gcagtttcat ctgtatttaa tgcctctaac 360
cattttggag ttggggttga tctaaggagt gatgcaccag ggtttctacc agcagcacca 420
gttttaaatc cacctgattt gatgaatggg ggaggtggag ccatagatgg agtgtttgga 480
tttgtaggaa ttggaactag tggacaacta ggttgtccag agctaaactc agctagccaa 540
ttctcaggag ccctactact acctcctgca gagaattgct ctgtttctag ctctggcaca 600
gcattttgtc ctgttggatt tgatagtttg gagaattcac catttctaaa tcgatctaag 660
gtgttgaggc cattggagat gtttcctaca gcaggctcac agccaactct attccagaag 720
cgagcagcag cagctctact acagaactca gcagctgctg cagaaaaagt aggcatattg 780
ggtccatggg catctaaagg aagttgccaa gggagtagta gtgtggctgc attggaggaa 840
gagaaagaga ggaagaggaa agggaatgag gtagatgagt tggatgaggg tagcattgat 900
aggtcaggat tacattatga ttctgatgat tttgcaggag agtgtgcaaa agttgaggag 960
aatgcaggga atgtagcagg tggaggaggg aatagttcta gtgctgatgt catggtggca 1020
gataggggga agaagaaggg tctaccagct aagaatctaa tggcagagag gaggaggaga 1080
aagaaactaa atgataggct atacatgcta agatctgttg tacctaagat aagtaagatg 1140
gatag
1145

<210> 53
<211> 1644
<212> DNA
<213> Musa AB Group
<220>
<400> 53
atgttttcta gaattgatgg aggattttgg atggatgatg gtggagatga tgatgatcct 60
gtagcatgga ctagaggaaa tgcctccagc tctagtggag ggggaatggg ggagactaag 120
gaggatgagc taggcctctc aggatttaag tccatgctag tggatgattg gtatgtagga 180
gcagcagcac atcatccatt tgaggcattc cagactcagc aggacctaaa agatgtagca 240
tttctaccta atccatcacc acatgaggca ctattgctac caccagtaga tagcctagac 300
caaagccaac catttttctc atctagacct gcagtttcat ctgtatttaa tgcctctaac 360
cattttggag ttggggttga tctaaggagt gatgcaccag ggtttctacc agcagcacca 420
gttttaaatc cacctgattt gatgaatggg ggaggtggag ccatagatgg agtgtttgga 480
tttgtaggaa ttggaactag tggacaacta ggttgtccag agctaaactc agctagccaa 540
ttctcaggag ccctactact acctcctgca gagaattgct cagtttctag ctctggcact 600
gcattttgtc ctgttggatt tgatagtttg gagaattcac catttctaaa tcggtctaag 660
gtgttgaggc cattggagat gtttcctaca gcaggctcac agcctactct attccagaag 720
cgggcagcag cagctctact acagaactca gcagctgctg cagaaaaagt aggcatattg 780
ggtccatggg catctaaagg aagttgccaa gggagtagta gtgtggctgc attggaggaa 840
gagaaagaga ggaagaggaa agggaatgag gtagatgagt tggatgaggg tagcattgat 900
aggtcaggat tacattatga ttctgatgat tttgcaggag agtgtgctaa agttgaggag 960
aatgcaggga atgtagcagg tggaggaggg aacagttcta gtgctgatgt catggtggca 1020
gataggggga agaagaaggg tctaccagct aagaatctaa tggcagagag gaggaggaga 1080
aagaaactaa atgataggct atacatgcta agatctgttg tacctaagat aagtaagatg 1140
gatagagcat ccattctagg agatgctatt gagtatttga aagaactact ccaaaggata 1200
aatgacctac aaaatgagct agaatctaca ccatcaagtt catcactacc tactactaat 1260
gctactagtt tgcatccatt aactcctaca ctacctacat tgccatgtcg tttgaaggat 1320
gaactaaagc attgctctag ttctttacct agtcctaata gtcagccagc tagggtagaa 1380
gttaaggcta gggaaggacg agcagtagac atacacatgt tttgtgcacg taggcctggt 1440
ctactactat cagcattgcg atcactagat agcctagggc tagacattca gcaggcagtc 1500
ataagttgtt ttaatggatt tgcactagac attttccaag ctgagcaatg taaagatcca 1560
ggtgttctcc taaagaaatc aaggctatgc tcatgcactc tacaggtttg ctctacacaa 1620
tgtagtagtc agcaatctag gtag
1644
<210> 54
<211> 1344
<212> DNA
<213> Theobroma cacao
<220>
<400> 54
atgctatcta gggtgaatgg tggtgtagtt tggatggaag ataaagaaga tgaagaatct 60
gcatcatgga ataggactaa taatagtaat aataataata ataatagtgt tataatggag 120
aataaagaag agatgggttc attgtctaca tttaagtcca tgctagaaga agaatggtat 180
gtagctaata atagtatttc tagccatcaa gatattagag acctatcatt ttcacctaat 240
ctagctgacc atcaagataa tattttgcta catcatcatc atcatcagca gcagcatcat 300
ccagtggatt catcctcatc atgttcacca tcatcctctg ttttcaataa tctagatcca 360
tctcaggtac actatttttt gcaacctaaa cctactttat catcactcct aaatgttgtt 420
tctaataatc ctttagagca tgggtttgat ttgagtgaaa ttgggttcct agataaccaa 480
gctactaatg ctactactct attgaatagg ggaaatgctg gggttttggg tagttttgct 540
gatttaagcc atggtaatca gatagacact gctaatttgt gtcctgagac tcagttttct 600
agttctagga tggttcagct accagaaaat ggtgcagggt tggcaggttt tcaaggattt 660
gatgagaatc ctggaaatgc tctattttta aataggtcta aggtgttaag gccactagaa 720
agttttccat cagtaggagc acaacctact ctattccaaa aaagagcagc attgaggaag 780
aatttagctg atagtggagg gaattttggg ttgtttggtg gaggaaaggt taatgcattg 840
aatggaattg agggagataa agggaaaaaa gaaatggatg aagagaatga gaagaggaaa 900
attatctata gagatgatct agaagatgtg agtattgatg ggtctgcttt aaattatgat 960
tcagatgagt ttactgagaa taataaggtg gaggaaactc taaaaaatgg tgggaatact 1020
tctaatgcta atagtactgt cactggagga gatcaaaagg gtaaaaagaa ggggctacct 1080
gctaagaatt tgatggctga gaggcgacgt cgaaagaaac taaatgatag gctatacatg 1140
ttgcggtctg ttgttcctaa gattagtaaa gtaagaattt cattcacatt tctatttttt 1200
tttttttttc cactactatt ttcattttta tctcgtgaat ttaagtatga attattgagt 1260
tattttagat cctcagaaat gtctaatatt ctaacatttt atctatgttt tcatggttgg 1320

tgtttttgta ttatgtgtga atga
1344
<210> 55
<211> 1682
<212> DNA
<213> Citrus trifoliata
<220>
<400> 55
atgctatcta gactaaatgg tgtggtatgg atggatggga aagaagagga aggctcagct 60
gcatgggcta gaaataacat aaataataat agtagtaata gcataaataa taattgtaac 120
actaataaca tgagttgtag taataataat gataataata atggtgtcat agagaatgaa 180
gaagaaatgg gttcactacc tggatttaag tccatgctag aagtggaaga tgattggtat 240
gttactggta atactagttt aaataaccat caagatatta catttccacc aaacctaggt 300
gatcctacta ctgataatct attgctaaat gctgtggatt catcatcatc atgttcacca 360
tcatcctctg tttttaataa ttttgatgca tctcaagttc actatttttt gcctcaaaaa 420
aactcatttt catcattcat gaatgttgtg tctaataata actcattaga gcatggattt 480
gatttgggtg aaatggggtt tctagacaca caagctactc atgcattgaa tagggggaat 540
ggtgggattt tgaatggttt taatgatttg agtgctaata atcagatgaa tgctactaat 600
ttatgttctg atccacaatt tggaactaat cgtacgctac aatttccaga aaatggtagt 660
agtagttttg caggttttcg aggttttgat gagaataatg ggaattctct atttttgaat 720
aggtctaagt tgttgaggcc actagaaact tttccatcta caggagcaca acctactcta 780
ttccaaaaaa gagctgcatt gaggaaaaat ttaggtggta atgaggctag tttaggggtt 840
ttaggtacac aaaatagcca actattgagt gggattgaga gtgataaagg taagaaagaa 900
ctaactgaag ataatgagaa aaagaggaaa ttgagtatta gtgatgattt ggaggatgtg 960
agtgttgatg ggtcaggtct aaactatgat tctgatgatt ttttagaaaa taataaggtg 1020
gaggaaatgg gtaaaaatgg tggtagtagc tctaatgcta ttagcactat cactggtgga 1080
gatcagaagg gtaagaagaa gggtttgcca gctaagaatc taatggcaga gaggagacga 1140
aggaagaagc taaatgatag gctctacatg ttgcgatcag ttgttcctaa gattagtaaa 1200
atggataggg catctatttt gggggatgct attgagtatt tgaaggaact actccaaagg 1260
atcaatgacc tccataatga attggagtct actcctcctg gttcagcatt gactccatcc 1320
actagtttct atccattgac acctactcca cctgcattgc acagtcgtat aaaggatgaa 1380
ctatgtccta gctcactacc tagtcctaat ggccaacctg ctagggttga ggtaagggtg 1440
agggaaggaa gagcagtgaa tatccacatg ttttgtagtc gtagaccagg tctattgctc 1500
tccactatga gggcattgga taatctagga ctagatatcc agcaagctgt cataagttgt 1560
tttaatggat ttgccatgga tgtttttcga gctgagcaat gtaaggaagg ccaggatgtt 1620
catcctgagc aaataaaggc agtactattg gattcagctg gattccatgg catgatgtag 1680
tt
1682
<210> 56
<211> 1092
<212> DNA
<213> Vitis vinifera
<220>
<400> 56
   atgttaccta ggtctaatga tgtagtttgg acacgaagtg atgctgtttc atggtctaag 60
   aatagtaatg ataataggtt tggtgatgga ggaggtgatg gtgagaggac tagttctgtg 120
   tctgcattga aggccattgt tgaggaggag tggttggtga gtggtgtaga aatgaaagga 180
   aatttggtgt ctcagagtca gagtggagca gtgaattcac tattgcatcc tatggattgc 240
   tcatcatcat gttcaccagc atctgcattt ggtctagatc cagcatttca gggaaaatct 300
   tgtttgtcct ctatggtttg tgggtatcca tttgatggct catttgattt ggtatgtgat 360
   ccagggtttc tagagcctat acctgaaatt caggctccta gttctagttc attgatgggt 420
   tttaatgatt tggcatcaca gcctctaatg ggtgcttcta atctaggctc taattctcag 480
   tttcctgcta cacatttggc aggaaatgga gggagtgctg ccactggtgg gtttaatcca 540
   tcaggttttg agggttttgt ggggagttct ctatttgtgg acaggtgtaa ggtgttgaaa 600
   ccactagaga attttccatc agttgggtca cagcctacac tattccagaa gagagctatt 660
   ctacgacgaa attctacaga aagagcaggg aattatggag tttctggcca agaaggaagt 720
   gctattccag ttagagttgc tggagaagat aagggtaaga ggccagtggt ggaagaagaa 780
   atggataaga tgagaaagga taagagtaat gatgaggatg atatggatga agctagcatt 840
   gctcgatctg gtttgatata tgattctgat gatgcaattg agaaccataa ggtggaggag 900
   actgctaatg atggtggaga taactctaat ttgaatggat catccattgg aggagataga 960
   aaagggaaga aaaaggggct accagctaag aatttgatgg ctgagaggcg acggagaaag 1020
   aagctaaatg ataggctcta catgctaagg tctgttgttc ctaagataag taaggtgaga 1080
   attttttggt ga
   1092
<210> 57
<211> 1596
<212> DNA
<213> Malus x domestica
<220>
<400> 57
atggatgata gggaggatag agactctgtt tcatggacta gaacctccgc cactgccact 60
gcaggaaaca cagagaataa ggatgagatg ggttcatctc tatctacatt taagtctatg 120
ctagaggttg aagatgattg gtacatggca gctaataata gcatccaggg ccattcagat 180
gtaggagaca taagtttctc accaagtttt gcagacccag aaagtttgct actaaatcca 240
gtggactcat cctcctcatg ctcaccatca tcatcagttt ttaataatct agatcctaat 300
caggttcatt actacatgcc tcaaaatcct aatttgtcat cactactaaa tgttgttcct 360
aataatccat tggatcatgg atttgatttg ggatgtgaca ttgggtttct agacactcaa 420
gcatcaggtg gtggttctag tatgatgaat aggggaggtg gggcattatc tggatttaat 480
gatttaagct ctaatagcca ggtgaatgct cagaatttgg gttctaattt acagttttca 540
actactcgca tgcctcaagc tctagaaaat agttctaatt tttctggttt tcgaggtgtg 600
gatgatggtt ctgctaatgc attgtttcct aataggccta agttactaag accactagaa 660
tcatttccat ctgtgggagt ccagcctact ctattccaga aaagagcagc actacggaaa 720
aatttatgtg atggtggagg gaatttgggt gttttggtct cacaaggggg cctagttttg 780
aatgaagggg atgagaggaa gagggagatg ggtgtgcaga atgagaagaa gaggaaaatg 840
agtggaggag atgatgtaga tgatctaagt tttgatggtt caggtttgaa ctatgattct 900
gatgagttta ctgagaacac taaggttgat gatggtgcta agaatggtgg gaatagctct 960
aatgctaata gcacagttac tggtggagga gggggtcata aggggaagaa gaaagggttg 1020
cctgctaaga atctaatggc tgagaggcga cgacgaaaga agctaaatga taggctatac 1080
atgctaagat cagttgttcc taagattagt aaaatggata gggcctcaat cctaggggat 1140
gctattgagt atttgaagga actactacag aggataaata acctccataa tgaactagag 1200
tctatacctc ctggatctgc attgacacct acaggaaata cattccatcc attgacacct 1260
actccagcta ctctacctaa tcgtataaag gaagaactat gcctcagctc attacctagt 1320
cctaatggcc aagctgctag ggttgaagtt aggctacgag aaggacgagc agttaacatc 1380
cacatgtttt gtggacgaag gccaggtcta ttgctctcta ccatgagaac tttggataac 1440
ctagggttag acatccagca agcagtcatc agttgtttta atggttttgc tatggatgta 1500
tttcgtgcag agcaatgtaa ggaaggccaa gatgtccatc cagatcagat aaaggcagta 1560
ctactagatt caatagggtt ccatggcatg atgtag
1596
<210> 58
<211> 1479
<212> DNA
<213> Capsella bursa-pastoris
<220>
<400> 58
atggttctag atggaaataa tggtggtgca gtttggttag gaggtggtgg agaacgagaa 60
gagaatgagg aaggttcatg gggaaggaat caagaagatg ggtctcagtt taaacctatg 120
ctagaaggtg attggtttag taatccacca catccacaag atctacagat gttacagaat 180
catcaagatt gtagatttct aggtggtttt ccatttaatc ctaatgataa tctactacta 240
cagcactcta ttgattcatc atcatcatgt tctccatctc aagcatttag cctagatcca 300
tctcagcaaa accagttttt gtccactaat aataagtcat gtctcataaa tgttccatca 360
tctgctaatc catttgataa tgcatttgaa tttggctctg aatctgggtt cctaagccaa 420
atacatgctc ctatgtctat gggttttggt tcattgacac agttggggaa tcgagatttg 480
agttctgttc ctgatttttt gtctgctagg tcactactag cacaggatca taatagtagt 540
aattctgtgt tatgtggtgg aggaggtggg ttcacagctc cactagaatt agaaggtttt 600
gggagtcctg ctaatggtgg ttttgttggg aatagagcta aggttctaaa gccattagag 660
gtgttagcat catctggtgc acagcctact ctattccaga aacgtgctgc tatgcgtcag 720
agctctagaa gtaaaatggg aaactctgag agctcaggaa tgaggaggtt gagtgatgat 780
ggggatatgg atgagactgg gattggggtt tcagggttga attatgagtc tgatgagcct 840
aatgagagtg ggaaagcagc tgagagtgtt cagattggag gagggaaggg taagaagaaa 900
ggaatgcctg ctaagaatct aatggctgag aggagaagga ggaagaagct taatgatagg 960
ctttatatgc taagatcagt tgtacctaag attagtaaaa tggatagagc atctatacta 1020
ggagatgcta ttgattatct aaaggaacta ttacaaagga taaatgatct acataatgaa 1080
ctagagtcta ctcctcctgg atcattgcct cctacatcat ctagtttcca tccattgaca 1140
cctacaccac agactctatc atgtcgtgta aaggaagagt tatgtccatc atcattgcct 1200
agtcctaaag gccagcaagc tagagttgag gttagattaa gggaaggaag agcagtgaac 1260
attcatatgt tttgtggtcg tagaccaggt ctattactag ctaccatgaa agcattggat 1320

aatctaggac tagatgttca gcaagcagtg attagttgtt ttaatgggtt tgcattggat 1380
gtttttcgag ctgagcaatg tcaagaagga caggagatac tacctgatca aataaaagca 1440
gtgctatttg atacagcagg ctatgctggt atgatatga
1479
<210> 59
<211> 981
<212> DNA
<213> Solanum chilense
<220>
<400> 59
atggttgatg atggaggtgt ggattggttt atgggtggtg gtgatagtaa taatcatcac 60
ataaataata ataataataa tggtggtggt ggtggtggtg gtggtagtaa catgcagagc 120
cacatttcat actctactag tttcactgaa gctgaaaata gtttgctact acaacctgtt 180
gattcatcag cttcatgctc acctgtttct ggtaatgttt ttaataacat tgatccatct 240
caggtgaatt ttttcatgcc acaaaaatct actattccat catcactcac tgggttatct 300
aataatccta tggataatag ttttaatttg ggtatgttga atcaagctgg gaatgggatg 360
atgaatactg ggtatcatca tctaggttca cctaaccaaa tggggactaa taatctaagt 420
tcatacactc agttttcatc tcctaatcta ctccagctac ctcaggtagc tggtgggtat 480
agttctatgg gatttggggc taataattct gctaatggaa acacattgtt tctaaatcga 540
tctagaactc ataaaccatt agataatttt gcatccattg gagcacagcc tactctattc 600
cagaaaagga ttgctaagaa tttggttagt aatggtgaaa atttaggtac agaaataggt 660
caatcatcta gtaatctaac tgatagaaaa aggaagtcta gcataaatga tgagtttgaa 720
gatgttagca tggatggaac attgaattat gactcagatg agttcatgga tatctctaat 780
aaaatggaag atgggataaa gattggggat agctctaatg cagctagcac tgtaagtggt 840
gctgatcaaa agggaaaaaa gaagggacct ccagctaaga acctaatggc agagcgacga 900
cgaagaaaga agctaaatga taggctatac atgctacgat cagttgtacc taagataact 960
aaggtaatga aattttttta a
981
<210> 60
<211> 1596
<212> DNA
<213> Solanum lycopersicum
<220>
<400> 60
atgctctcta gagttaatag tatgcaggtt tgggatatgg aaggaaaaca agaagaagaa 60
aaagaagaaa attttagtaa taaggagaat accactaatg ttgagctaga aaataaacaa 120
gatatggaat tgggtgcact atctacattt aaatctatgt tagatggtac agatgtagat 180
tggtatcata ataacatgca aaaccatact gaaaatatat gtttcactca gaatttcact 240
gaattagctg aaaatagcat gtttctacag ccagtagtac cagtggattc atcatcatca 300
tgttcaccat catcagtttc tgtttttaat aatctagatc catctcaggt acattatttg 360
ttagcacaaa aggctattaa taataatcca ttggattata gttttaattt gggttgtgaa 420
aatggttttc tagaagctca aggtatgggt ggtttgaata aagggggatt tctactagct 480
ggtggtgggt ttcatgattt gagctcacag aaccaaatgg gtaatcctaa tttgaattca 540
tttactcaat atccatcatc acatttgcca caaaatacta ctactactgg gtttagtcca 600
ctagggtttg ttgatggttc tgctaatgag aactctctat ttctaaatag gtctaagttg 660
ttaaagccac tagataattt tgcatctaat ggagcacaac ctactctatt tcagaaaagg 720
gctgctctaa ggaagaattt agctaatact actggtggaa gtttggggga ttttggaggt 780
gaaattggcc agaattccat gaatggagag aatgaaagga agagaaagtg ggtagtgggg 840
gaagaactag atgatgtgag ttttgatggg tgcacattga gttatgattc agatgaccta 900
actgaaaatg ttactaataa ggtggatgat actgttaaga atggtggaaa tagctctaat 960
gctactagca ctgtcacctg tgggaatcag aaaggaaaga aaaagggact accagctaag 1020
aatttgatgg ctgaacgacg acgtaggaag aagctaaatg ataggctata catgttgagg 1080
tctgttgtac ctaggattag taagatggat agggcttcta tattagggga tgctattgag 1140
tatttaaagg agctattaca gaaaataaat gacctacata atgaactaga gtccacacca 1200
cctagctcat cactaacaca aactactagt ttctatcctc taacaccaac tggaccagct 1260
ctaccaggac gcataaagga agaactctat cctagttcat ttgctagtcc actatctagt 1320
cctactggac aacctgctag ggttgaagta aaggctagag aaggaagagc tgtgaatatc 1380
catatgtttt gtagtcgtag accaggccta ttactctcta ctatgagggc actagataac 1440
ctaggactag acatccaaca agctgttata agttgtttta atggatttgc attagatata 1500
tttagagctg agcaatgtaa agaaggtcaa gattttcatc ctgatcaaat aaaagcagta 1560
ctactagatt cagctggttg ccatgggatg atatga
1596
<210> 61

<211> 1443
<212> DNA
<213> Prunus persica cultivar
<220>
<400> 61
atgctagatg ttgatgatga ttggtatcta cctaataata gcattcagag ccattcagat 60
gtacgagaca tagcattctc acctagtttt gctgatccag aaagtttgct actaaatcca 120
gtggactcat cctcatcatg ctcaccatca tcctcagttt ttaataatct agatcctaat 180
caggttcatt actatttgcc tcaaaattcc acattgtcat cactcctaaa tgttgttcct 240
aataatccat tggagcatgg ttttgatttg ggatgtgata ttgggtttct agacactcaa 300
gcatctagtg gttctagttt aatgaatagg ggaggtgggg ttttaactgg gtttagtggt 360
ttgagctcta ataatcctat gaatgctcag gatttgtgtt ctaatttgca attttcaacc 420
acacgtctac ctcaattgct agaaaatagt tctaattttt caggatttcg aggttttgag 480
gatacatcag ctaatgcatt gtttcctaat aggcctaaat tattgagacc actagactca 540
gtacctgatg tgggagcacc acctactcta tttcagaaaa gagttcagaa gaatttagct 600
gataatggag gaaatttggg tgcattaggc tcagaagggg gcctagcttt gaatcatatg 660
ggtgaggaga atgagaggaa gaggaaattg agtgggggag atgattttga agatgtgagt 720
atagatggtt caggtttgaa ctatgattca gatgatttta ctgaaaacac taagatggat 780
gatggtgcta agaatggtgg taatagctct aatgctaata gtactgttac tggaggaggg 840
gatcataagg ggaagaagaa agggttgcct gctaagaatt tgatggctga gaggcgacga 900
cggaagaagc taaatgatag gctatatatg ctaagatcag ttgttcctaa gataagtaaa 960
atggataggg catctatcct aggggatgct attgagtatt tgaaggaact actacaaaag 1020
ataaataacc tccataatga attggagtct atacctccag ggtcatcatt gacacctact 1080
ggaagtacat tccatccatt gacacctact ccagctactc tacctagtcg tattaaggaa 1140
gaactatgtc ctagctcatt acctagtcct aatggccaac cagctagggt tgaagttagg 1200
ctacgagaag gaagagcagt gaatatccac atgttttgtg ggcgaagacc tggtctattg 1260
ctctctacca tgagaacatt agatagccta gggctagaca tccagcaagc tgtcattagt 1320
tgttttaatg gttttgctat ggatgtattt cgtgctgggc aatgtaagga aggtcaagat 1380
gtccatccag atcagataaa agcagtacta ttggagtcag tagggtttca tggattgata 1440
tag
1443
<210> 62
<211> 1131
<212> DNA
<213> Zea mays
<220>
<400> 62
atggatgact cagcagaggt gaagctagtg gatgagatca caggagaggg aggggcagca 60
ggggattggg gctacctagg ctcagatggg atgggctcag gctcctatcc agcatttcca 120
ttctctagag atgtcctatc cacacctacc tcagcatcat tgctcctctc catggatcca 180
gcagcactat ttgattttaa tggcacattt cctccatcct cagcagcagc agccactgca 240
ggctcctcac tatcagcatt ccatgatttt agttgcataa atccatttga tgatgcaggc 300
catttcctag gagctccacc tcctgtgcca gcagcagctg ctcctcagca gcaggggcag 360
aaaggtgggt tttttgcacc actaccagcc tcagatttta atgatgcagg gatgagttgg 420
gatgatgagg atgagataga ccaaagtgta gatgcctcct caatggccat ctcagcctcc 480
atggagaatg ctgcaggagc agtggctggg gctagtggtg ctggaggtgg aagtgggaga 540
ggaaagaaga aagggatgcc agctaagaac ctcatggcag agcgacgacg tcgaaagaag 600
ctaaatgatc gcctctacat gctacgctca gtggtgccta agataagtaa gatggatagg 660
gcatctatcc taggagatgc tattgagtat ctaaaggagc tcctacagag gataagtgat 720
ctacataatg aactagagtc agctcctagc tcatcactag ttggacctac atcagctagt 780
tttaatccat ctacacctac attgcagaca tttcctggcc aggtaaagga agaactatgt 840
ccaggctcat ttcctagtcc tactggacaa caagccacag tagaagtaag gatgagggaa 900
gggcatgcag taaatatcca catgttttgt gctcgtaggc caggcatcct actatctact 960
atgacagcac tagatagcct aggcctagac atagagcagg cagtcataag ttgttttaat 1020
ggatttgcta tggatgtgtt tcgagctgag tgtgcagatg gtcctggaat ggtgcctgaa 1080
gaaattaagg cagtgctaat gcacactgca ggcctccata atgctatgta g 1131
<210> 63
<211> 1539
<212> DNA
<213> Gossypium hirsutum Ls
<220>
<400> 63
atggagaata aagaagatat gggtacattt tctacattta aatccatgtt agatgatgaa 60

tggtatgtag gaaataatag catttctagc catcaagata ttagagacct atcatttaca 120
tctaatctag gtgaccatca tcaagataat ctattgctcc atcatcattc tctaccatca 180
gtggattcat catcatcatg ttcaccatca tcctcagttt ttaaccatct agatccatct 240
caggtacagt atttttgcca acctaaacct aatttatcat ctatcctaaa ttttgtttct 300
aatagtccat tagaccatgg ttttgatttg agtgaaattg ggttcctaga taaccaagct 360
actaatggta ctacattgtt gaatagggga aatgctgggg ttttgggtag tttaactgat 420
ctaggtcatg ataatcattt agatcctgct aatttgtgtc ctgaggctca gttttctagt 480
tctcggatag ttcagctacc agaaaatggt actgggtttg ctggttttga agggtttgat 540
gagaatcctg gaaatgctct atttttaaag agatctaagt tgttaaggcc actagaaagt 600
tgtccatcag ttggtgcaca acctacatta ttccaaaaaa gagcagctat gaggaagaat 660
tcagctgata gtggacctaa ttttggggtg ttagatggag gaaaggttag tgttcaaagt 720
ggtactgagg gagataaagg gaagaaagaa atgggtaaag aagagactga gaagaggaaa 780
attaacaata gagatgatgt agaagatgtg agcattgatg ggtcagctct aaactatgat 840
tcagatgagt ttactgagaa tactaaggtg gaggaaactc taaaaaatgg gggaaatacc 900
ataaatgcta acactagtgc tactggagga gaccaaaacc aaaagggtaa aaggaaaggg 960
ctacctgcta agaatttgat ggctgagagg cgacgtcgaa agaaactaaa tgataggcta 1020
tacatgttgc gctctgttgt tcctaagatt agtaaaatgg atagagcatc tattctaggg 1080
gatgctatag agtacctaaa ggaactacta cagagaataa atgacctcca taatgaattg 1140
gagtctactc tacctagctc ctcactaaca cctactacta gtagtttcca tccattgaca 1200
cctactccag ctacatttcc tagtcgtata aaggatgaac taggacctag ttcatcacca 1260
ggtctaaatg gacagccagc tagagttgaa gtaaggctaa gagaaggaaa agctgtaaac 1320
atccacatgt tttgtgggcg gagaccaggt ctattgctct ccataataag ggcattggat 1380
aatctaggac tagatgcaca gcaagcagtc attagttgtt ttaatggttt ttccatggat 1440
atatttcgag ctgagcaatg taaggaaggg caggacataa atcctgagaa tataaaagct 1500
gtactattgg attctactgg atttcctaat atgatatag 1539
<210> 64
<211> 306
<212> DNA
<213> Arachis hypogaea
<220>
<400> 64
atggaggcag ttggtggttc tggaaatgct ctattttcag gtaagggaaa tggattggaa 60
gcattggtag tgagtaattt gaagagggtg caggaagttg aagaagctag taatgaaggt 120
tctatgtgga attctgaaga ggatgatgag agaactgatg aggtggtggt tgaggagagt 180
gggaagaaga agaagaagaa ggggatgcca tctaaaaatc taatggctga gaggcgacga 240
aggaagaagc taaatgatag gttgtacatg ctaaggtctg ttgtgcctaa tattagtaag 300
gtataa
306
<210> 65
<211> 1029
<212> DNA
<213> Brassica juncea
<220>
<400> 65
atggttctag atggaaatgg tggtggtgta tggttaggtg gtggaggagg aggggaggga 60
gaacgtgttc aggaggagga gaatgaggaa gcatcatggg ggaggaatca agaagatggt 120
ggtcagttta agcctatgct agaaggtggt ggtgattggt ttactagtag ccaacctcat 180
ccacaagatc tccagatgct acagagccag caagatttta gatttctagg tgggtttggt 240
tttaatccta atgataatct actactacta caacactcca tggactcatc atcatcatgc 300
tctccatctc aggcatttag cctagatcca tctcaagcat catttttggc tgctgctaac 360
aacaagtcat gtctcctaaa tgttgttcca tcctcagcta atccatttga taatgcattt 420
gagtttggtt cagattctgg tttcctaaac caaatccaag ctccagtttc tatggggttt 480
ggttcattga cacagctagg aagctctgta cctgattttt tatctgctcg atcactacta 540
cctcctgaaa ataataacac agcaccatta tgtggaggag gaggaggtgg aggtgggttc 600
acagcattgg agctagaagg gtttgggagt cctgctagtt ttgttgggag tagacctaaa 660
gttctaaagc cattagaggt gttggcatca tctggagcac agcctactct attccagaag 720
cgtgcagcta tgcgtcagag ctctggaagt aagatgggga actcagagag ctcagggatg 780
aggaggctaa gcgatgatgg agatatggat gagactgggg ttgaggtttc agggctaaac 840
tatgngtctg atgagctaaa tgagagtggt aaagcatctg agagtgttca gaatggagga 900
ggtaaaggga agaagaaagg gatgcctgct aagaatctaa tggctgagag gaggaggagg 960
aagaagctaa atgataggct atacatgcta agatcagttg tacctaagat aagtaaagta 1020
aactattaa
1029

<210> 66
<211> 1494
<212> DNA
<213> Brassica rapa subsp.
<220>
<400> 66
atggttctag atggaaatgg tggtggtgta tggttaggtg gtggaggagg tggaggagga 60
ggagaacggg tgcaggagga ggagaatgag gaagcttcat gggggaggaa tcaagaagat 120
ggtggtcagt ttaagcctat gctagaaggt ggtggtgatt ggtttactag taaccaacca 180
catccacaag atctccagat gctacagagc cagcaagatt ttagattcct aggtgggttt 240
ggttttaatc ctaatgataa tctactacta ctacaacact ccatggactc atcatcatca 300
tgctctccat ctcaggcatt tagcctagat ccatctcaag catcattttt ggctgctgct 360
aataataagt catgtctcct aaatgttgtt ccatcctcag ctaatccatt tgataatgca 420
tttgagtttg ggtcagattc tggtttccta aaccaaatcc aagctccagt ttctatgggg 480
tttggttcat tgacacagct agggagctct gtacctgatt ttttatctgc acggtcacta 540
ctaccaccag aaaataataa tgcaacacca ttatgtggag gtggaggagg tgggttcaca 600
ccattggagc tagaagggtt tgggagtcca gctagttttg tagggagtag acctaaagtt 660
ctaaagccat tagaggtgtt ggcatcatct ggtgcacagc ctacattgtt ccagaaacgt 720
gcagccatgc gtcagagctc tggaagtaag atggggaact cagagagctc agggatgagg 780
aggctaagtg atgatggaga tatggatgag actggggttg aggtttcagg gctaaactat 840
gagtctgatg agctaaatga gagtggtaaa gcatctgaga gtgttcagaa tggaggaggt 900
aaagggaaga agaaagggat gcctgctaag aatctaatgg ctgagaggag gaggaggaag 960
aagctaaatg atagactcta tatgctaaga tctgttgtac ctaagataag taaaatggat 1020
agagcatcta tactaggaga tgctattgat tacctaaagg agctattaca aagaataaat 1080
gacctacata atgagctaga gtctactcct aatggatctc tacctctagc atcatctagt 1140
ttccatccat taacacctac accacagact ctatcatgtc gtgtaaagga agagttgtgt 1200
ccatcatcat tgcctagtcc taaaggccag caagctagag ttgaggttag attaagggaa 1260
ggaagagcag tgaacattca catgttttgt ggtggtagac ctggtctatt actagctacc 1320
atgaaagcat tggataatct aggattggat gttcagcaag cagtcataag ttgttttaat 1380
gggtttgcat tggatgtttt tcgagctgag caatgccaag aaggacaaga gatattgcct 1440
gatcaaataa aagcagtgct atttgataca gcaggatatg ctggtatgat atga 1494
<210> 67
<211> 1416
<212> DNA
<213> Chrysanthemum dichrum
<220>
<400> 67
atgctaccag aaaatgacac tgcatggatg gataaccacc aagatgaaac atcatcatgg 60
actcctacta ccacacctaa ccaccaccac caaccaccat gcacaaataa ccaaaacacc 120
tacacatcat taactacatt aaaaaacatg ctagaaacag aatggttcca aaataataat 180
aataataacc taaacctcac ctcaacagac actaatccta tgtttttacc actagattca 240
tcatcctcat gttcaccatc acaatcacac tcacactcac atttcactca gtttccattc 300
atacctacta aatataataa taatactaat ctaaatttaa ctgcatttga tttgccattt 360
gaatttgggt gtgatcctag tttcatttct aataatcctc ctactaattt tttgaatttt 420
cctactcaac aaatggctac tagctcagag tatactgaga ctagttacca tgatgattta 480
actggaactg gtacagggtt cactggattt gagccagtga atcctgtttt tccagttagg 540
tctaaagttt tgaggccatt ggaagtttct ccacctgttg gagcacagcc tacattgttt 600
cagaagcgag ctgcactaag acagagtgga gagaagttgg atgggttgga aattgtgaaa 660
aaaaggaaaa ggggtagtgg gagtgggagt ggggcacatg aggatgagat tgatgaaatg 720
ggtaatgggg atggttcagg gtttaattat gattcagatg aagttgagct aactagtgag 780
gttaatggga ataattcagg ttcagttatg gttggggata gtaaagggaa gaggaagggt 840
ttgcctgcta agaatttaat ggcagaaagg cgaaggagga agaagttgaa tgataggttg 900
tatatgctaa gatctgttgt acctaaaatt agtaagatgg atagggcatc tatactaggt 960
gatgctattg attatttgaa ggagttgttg caaaggatta atgatttaca taatgaacta 1020
gaggctacac cacaaggttc attgatgcaa gcatcatcta gtatccatcc attgacacct 1080
acaccaccta cactaccaca acatgttaag gaagagttgt gtcctagtac tctacctagt 1140
cctaaaaacc atccatctaa ggtggaggtg catgctagag aagggagggg tgtgaatatc 1200
cacatggtgt gtgggcgtag accaggtcta ttgctatcta cattgagggc actagagaac 1260
ctaggcctag acattcagca agctgtcata agttgtttta atgggtttgc attggatgta 1320
tttcgagcac agcaatgtag agaagggcag gagatgttac cagagcagat aaaagcagtg 1380
ctactagaga ctgcaggtta tcatggtgcc atttag
1416
<210> 68

<211> 1089
<212> DNA
<213> Thellungiella halophila
<220>
<400> 68
atggttctag atggaaatgg tggtgcagtt tggttaggag gaggagaacg gattcaggag      60
gaggagaatg aggaagcatc atggggtagg aatcaagaag aaggtggagc atcactatct     120
cattttaagc ctatgctaga aggtgattgg tttagtaatc caccacatcc acaagatctc     180
caaatgttac agaatcagca agattttaga ttcctaggtg gttttccttt taatcctagt     240
gataatctac tactacacca gtctattgat tcatcttcat catgctctcc atctcaagca     300
tttagcctag atccatctca gccttcattt ctagcagcta ataataaatc atgtctcctc     360
aatgttccat cctccactaa tccttttgat aatgcatttg agtttggctc tgattctggt     420
tttctaggac aaatccaagc acctatttct atggggtttg gttcattgac acaactagga     480
aatcgggatt tgagctcagt acctgatttt ttatctgctc ggtctctact acctcctgaa     540
aataataact ccacaccatt atgtggtgga ggaggtggtg gtggtggtgg attcactcct     600
ctagagctag aagggtttgg gagtcctgct aatggtggtt ttgtagggaa tagagctaaa     660
gttctaaaac ctttagaggt attagcatca tctggtgcac agcctactct attccagaaa     720
cgtgcagcta tgcgtcagag ctctggaagt aagattggga actcagaagg ctcagggatg     780
aggaagctaa gtgatgatgg agagatggat gagactggga ttgaggtttc tgggttgaat     840
tatgagtctg atgagctaaa tgagagtggt aaagctgctg agagtgttca gaatggagga     900
ggaggatgta aaggtaagaa gaaaggaatg cctgctaaga atctaatggc tgagaggagg     960
aggaggaaga agctaaatga taggctatat atgctaagat cagttgtgcc taagatcagc    1020
aaagtaaata attatttttc atttatctca ttaagggcat tgtcatcatc atttacatat    1080
cgacgttag
1089
<210> 69
<211> 1458
<212> DNA
<213> Daucus carota
<220>
<400> 69
atgctaccta catctaatgg tgtagtttgg atgccacctc aagataccac taacattgaa      60
caacagcaat tgttattaga tacatgtgta gatactaaaa ttagtccatt tgcatcatca     120
tcatcactca ctagttttga atcactacta gaaaatgatt ggttctctaa taattatcta     180
gggaatggta atttagattt ttcagcatct ggttctcagt cacagccatt cactcatgat     240
ccatcacagt cacattctca gttttttcag cctaaatcat gtttctctcc tatgctaaat     300
gggttgtatg ataatgggtt tcagttgggt tctgatccta atttcactaa tgggtcagat     360
tctcatgggt tcatgtcata taatgggttg aattctgtga ctaatttgga tatgggtttg     420
gtttcagatt ttggagggac acgatttgag agtgatccag ttggtgtagg gtttaatcca     480
gttgggtttg gtgggtcatg tttagttgat gataatgttc atgataatga tgataatgca     540
ttgttttgta ataggagtaa tggtaatcag attttaaggc cactagaggt ttcacctagt     600
gtgggtgcag agcctactct atttcagaag agggctgcat tacgacaagg ctcaggtgag     660
ggagaaatag ggaattcagg aaagagtgag ggtttgggga ggaagaggat gagaaatgct     720
agtgaagatg gtgatgaatt tgatgatgga agtatgggtg tttcaggttc atttaattat     780
gattcagatg attttgatac taaaaatgag aatgggggct ctggggatgt ggatggttct     840
aattctgtga aggttggaga tcgaaaggga aagaagaagg gatttcctgc taagaatttg     900
atggctgaga gacgacgaag gaagaagcta aatgataggc tatacatgct aaggtctgtt     960
gttcctaaga taagtaagat ggatcgagca tccatactag gagatgctat tgattattta    1020
aaggatctac tacagaggat aaatgatcta cataatgaac tagaggctat tccaccaggc    1080
tcctcagtaa cagctacatc tagccataat ccattgactc acacatctac cacactacca    1140
catctagtga aggaagaaat atactctagt tctatgccta gtccacaaag cacagctgct    1200
agggtagagg taagggctag agaagggagg gctgtaaaca tccacatgtt ttgtgcacga    1260
agacctggtc tattactatc caccatgagg gcactagata acctagggtt agacattcag    1320
caagctgtca taagttgttt taatgggttt gctatggatg tatttcgagc agagcaatgt    1380
ggtgaaacag aggacattct acctgatcag ataaaagcag ttctactaga ttctgctggt    1440
tttcatggag ctatttag
1458
<210> 70
<211> 1050
<212> DNA
<213> Glycine max
<220>
<400> 70

atgatgtcta gaataaataa tagcctagta tggatggatg aaccagaagc agaaaacact 60
gcctcagcat catgggctag aaactctaag cctaataata ataacatcat cattaccaca 120
ggcactacca ctaccacagg ttttgtagaa acctctaatg atttaggttc tctatgtgca 180
tttaagccta tgctagatgt tgatgatgaa tggtataata acatcactaa ccaccaccac 240
caccaccaag atatggcatt tgatccagat aacctattac tccatccatc agcagtggac 300
tcctcctcct catgctctcc atctcacctc cagtttttcc tacctcctaa tcctaaacta 360
aatccatttg atatgggttt tctagatcct caagcatcat catcattacc actactagca 420
tcagaaacta cacatttcac accacctcag actagcacca ccacagcatt tgcaggtttc 480
cagagcctcc aagagggttc ctcaaatcct ctatttctaa atcgttctaa cattctaaga 540
cctctagagt cactaccacc atctggggca caacctacac tattccagaa gagagcagca 600
ctcaggaaga acatgaacat gagtgataat aagaagagga aggagaagga tgaggtggtg 660
gttgaggatg tgagttttga tgggtctgga ttgaactatg attctgatga tttaactgag 720
agtaattata atgatgctaa ggagaagaat ggtggtgggg gggttagttc taatgctaat 780
agcactgtca caggtttgga tcagaaaggg aagaagaagg gaatgcctgc taagaatttg 840
atggcagaac gacgacgacg aaagaagcta aatgatagac tctacatgct aaggtctgtt 900
gttcctaaga ttagtaaagt aagatttcta tttttttcat ttctattcct ctcaccatct 960
tgccaaattt tgaatctaca gttactattt aagtttttgg agttaaattc atttgaaagg 1020
tctatactat gtggtgtgtg tttggattag
1050
<210> 71
<211> 1266
<212> DNA
<213> Raphanus sativus
<220>
<400> 71
atggttctag atgggaatgg aggagctgtt tggttaggag gtggaggagc aggagaaagg 60
tttcatgagg atgaggagga ggagaatggt gcagctcatt ttaaagctat gctagaaggt 120
ggtgattggt tttttaacca accacaagat ctccacatgt tacagagcca tcatcatcaa 180
gattttagat tcctaggtgg tggttgtggt ggttttgcat ttaatcctaa tgataatcta 240
ctactagatc catttttggc agctgctaat aatagtaagt catgtctcct aaataatgtt 300
gctaatccat ttgataataa taatgcattt gattttgggt catctgattc atgttttcta 360
ggagggtatg gttcattgag ctcctcctca gtacctgagt ttttatctgc tcgggagaat 420
aataataaca cctctacacc attggagtca gaaggttttg ggagtagagc taaggttcta 480
aagccattag aggtgttggc atcttcaggt gcacagccta ctctatttca gaaacgtgca 540
gccatgagga agctaagtga ggatggagag atggatgaga ctggggttga tgtttcaggg 600
ttgaactatg agtctgatga gctaaatgag aattgtaaag ttgttcagaa tggtggagga 660
ggaggaggta ataaaggtaa gaagaaaggg atgcctgcta agaatctaat ggctgagagg 720
agaaggagga agaagctaaa tgataggcta tatatgctaa gatcagttgt acctaagatc 780
agtaaaatgg atagagcatc tatactagga gatgctattg attacctaaa ggaactatta 840
caaaggataa atgatctaca taatgagcta gagtctactc catctggttc attgcctcca 900
acatcatcta gttttcatcc attaactcct acaccacaga ctctatcatg tcgtgtaaag 960
gaagagttgt gtccatcatc attgcctagt cctaaagatc agcaagctag agttgaggta 1020
agattaaggg aaggaagagc agtgaacatt cacatgtttt gtggtcgtag acctggtcta 1080
ttgctagcta ccatgaaagc attggatagt ctaggattgg atattcagca agctgtgatt 1140
agttgtttta atggttttgc attggatgtt tttcgtgctg agcaatgcca agaaggacag 1200
gagataatgc ctgatcaaat aaaagctgtg ctatttgata cagcaggcta tgctggtatg 1260
atatga
1266
<210> 72
<211> 1146
<212> DNA
<213> Triticum aestivum cultivar
<220>
<400> 72
   atggagaact cagctgctgc agtgggggtg gagaaggagg atgagctagt gggggaggga 60
   gggggagatt gggggtacct cacatctgag gctatggcta cagcagggtt tccagcattt 120
   gggtttccat gtggagctag gggtggtgtc acaccagcac ctacctctgc atctctactc 180
   atgtccatgg agcatgcagc tctatttgac tataatgcag catttccatc atcatcatcc 240
   tctgcagcag cagctcctcc agcctaccat gattttgggt ctggaggaaa tccatttaat 300
   gtagatgcac ctccatttct actagaggca ccacctcctc taacagtggc accaggaggg 360
   cagaaagggg ggtttttggc accaccacta tctgcatttg gtgatggcat ggggtgggat 420
   gatgaggatg agctagatca gcagagtgtg gatgcctcct cattgggggt ctcagcctct 480
   ctagagaatg cagtggtagg agctccaggg ggaggtggag gtggaggtgg aggtaatgga 540
   aagggtaaga agaaggggat gccagctaag aacctaatgg cagagcgacg tcgtaggaag 600
   aagctaaatg atcgcctcta catgctacgc tcagtggtgc ctaagataag taagatggat 660
   agggcatcta tcctaggtga tgctatagac tacctaaagg agctcctaca gaggattagt 720
   gatctccact cagagctaga gtctgctcct agctctgcag cactaggtgg accatctaca 780
   gctaatagtt tcctaccatc tacacctact ctacagccat ttccaggacg cataaaggag 840
   gagcggtgtc caccagctcc atttcctagt cctagtggcc agcaggctac agtggaggtg 900
   aggatgaggg aggggcaggc agtgaacatc cacatgtttt gtgcacgtag gccaggcatc 960
   ctactgtcca ccatgagggc actagatagc ctaggcctag acattgagca ggcagtcata 1020
   agttgttttg atggttttgc catggatgta tttcgagcag agcaatgtag ggagggacct 1080
   gggctcctac ctgaggaaat taaggcagtg ctcctacatt gtgcaggtct ccagaatgct 1140
   atgtag
   1146
<210> 73
<211> 315
<212> DNA
<213> Hordeum vulgare
<220>
<400> 73
atggggtggg atgatgagga tgagctagat cagcagagca tggatgcctc ctcattgggg 60
gtctcagcct cactagagaa tgcagcagta ggagcaccag ggggaggtgg aggaggaggt 120
aatgggaagg gtaagaagaa ggggatgcca gctaagaacc taatggcaga gcgacgacgt 180
aggaagaagc taaatgatcg cctctacatg ctacgctcag tggtgcctaa gataagtaag 240
gtgaggaatg ctccaccacg tcatggaaat cgatcaccta catgttcaca tctctactat 300
acatattatg gttga
315
<210> 74
<211> 1410
<212> DNA
<213> Hevea brasiliensis
<220>
<400> 74
atgctagata cagattggta tgatataaat ccatctcctc accatgacct ccatgctctc 60
caaaatcacc atgaaataag agacctaggt ttttgtgcta atcctacacc tgaaaatcta 120
ctattacagc ctatggactc atcctcatca tgctctccat cacaggcatt tactctagat 180
ccagcacagg cacaccagcc attttttcca cctaaatcat gtttctcatc tctactaaat 240
gtcactaatg catttgataa tggttttgat ttgggttgtg aaaatgcatt tctaggccaa 300
taccagccta atcaggcatc taatttgatg ggttttagtg ggccagagtt ccaagcatca 360
cagttgctac cagtgtcaga taatgctgca gcaattggag gtgctgggtt tagtaatgta 420
ggactagagg gttttgatgg ttctactgtg aatgctctat ttttaagtag ggctaaggtt 480
ttgaggccac tagaggtttt gcctccagtg ggttcaccac ctactctatt tcagaagcga 540
gccatgttaa ggcaaagtgg ggataagttg gggaatttgg agatatcagg gctaagatat 600
ggaggagaaa ttggggggaa ttggggagaa atggaaagga aaagaaagaa gagtgaagaa 660
ggtgaaattg aggaagctag ttttgatgtt tcaggtttga attatgattc tgatgaacat 720
aatggagatg gtaagattga agagagtgtg aagaataatg gtggaagtaa ttctaatgct 780
aatagcacag tcactggtgg aggtggtggt gatcaaaagg ggaagaagaa ggggatgcca 840
gctaagaatt tgatggctga gagaaggaga aggaagaggc taaatgatag actctacatg 900
ctaagatctg ttgtacctaa gattagtaag atggatagag catctatact aggggatgcc 960
attgattatt taaaggagct actacaaagg ataaatgatc tacataataa attggagtct 1020
actccacctg gtccattgtt gcctcaatct actagtattc atcctgtgac tcctacacct 1080
ccagccatac catcacgagt gaaggaagaa ctatgtccta gctcattgtc tagtcctaaa 1140
aaccaagcta ctagggtgga agttagggta agggaaggaa gggcagtaaa catccacatg 1200
ttttgtgctc gaagaccagg tttattgctc tccactatga gggctctaga taacctaggg 1260
ttagatattc agcaggctgt cataagttgt tttaatgggt ttgcactaga tgtgtttcga 1320
gctgagcaat gtagggaagg tcaggatgta ttaccagagc aaataaaagc agtactacta 1380
gattcagctg gatttcatgg aataatgtga
1410
<210> 75
<211> 1158
<212> DNA
<213> Juglans regia
<220>
<400> 75
atgctatcaa gagtaaatgg agtatggatg gatgatagag aggaccatga tcctgcatca 60

tgggctagaa ccactaatga tgctaacact agtaatagtt gtggtgtggc agagaagaat 120
gagcatgatt tgggttcact ctccacattt aagtccatgc tagaaataga agatgattgg 180
tataatatgg ctaataatac tctccaaagc cacactgata taagagattt aacattctct 240
cctaacctag ctgaccatcc agataatcta ttgctaaatc cagtggactc ctcctcatca 300
tgctctccat cttcctctgt tttcaccaat ctagatccat ctcaggtgaa ctattttttg 360
cctcctaagc ctacattgtc ctcactaatt aatggcctct ctagtaatcc attagagaat 420
ggatttgatt tgggatgtga ggttgggttt ctagaagcac aaacatctaa tgcatccact 480
ctactaaata ggggaggtgg ggttttgagt aatttcacag atttgagcac taattctcag 540
attaatgctc ctagtatatg ctcagactca caactacgtc tacctaattt tccagaaaca 600
ggtgctgggt ttgtggggtt taaggcattt gatgggggtt ctgggaatgc tctatttatg 660
aataggtcta agattctaag accactagag tcatttccat ctatgggagc acagcctact 720
ctattccaaa agagagcagc tctaaggaaa aacatagcag atagtggagg aaatttgtct 780
atttctacat ctgttgaggg agataagggg aagacagaaa cacatgaacc aggagaggga 840
aagaggaaac taagtaatgg ggatgatgtg gaagatgcta gttttgatgg ctctggtcta 900
aattatgatt cagatgagtt catggagaat gataaggcag agggaaatgc taagaacaca 960
ggagggaata gctctaatgc cactagtaca gttacaggtg gaggagagca aaaggggaag 1020
aagaaaggcc tacctgctaa gaatttgatg gcagagaggc gacgacgaaa gaaactaaat 1080
gatagattat atttgctaag gtcagtggtt cctaagatta gtaaagtaag tgtttttttt 1140
gctgtgtttt tctcatga
1158
<210> 76
<211> 1497
<212> DNA
<213> Lactuca sativa
<220>
<400> 76
atgatgccag atggtggtgc tgtttggatg gaaggaggag gaaaccagga tgaaattgga 60
acatcatcat ggacaccagc caccaccacc accacaacag ctaatcacca tcatgttcta 120
ggaattgaag ctgctgctac atcatgtaat gaagaaacca cagcagctaa cacatatgca 180
tctctcacca cattgaaatc tatgctagaa tcagagtggt atcaacataa taatctaaat 240
ctctctacta atccatctga taataattct atatttttac ctatggattc atcatcatca 300
tgttcaccat cacattcaca ccatccattc tctctaccag atccatcacc atcacactca 360
tatccatttt ttccacctaa atttaataat ccatatgaca ttgggtttga tttcggatgt 420
gatcctggat ttttacctaa tcacacatca aatttgatga atttttctgg gttatgttca 480
caaacacaca tcgccatacc agagctatca tctagttcag attttccagc cactaataat 540
agtaatgaca ctagtgacat tggaggacct gggtttaatc cacagttaac agcatttgat 600
ggttttcatg gagctgcacc tatgaatcct ctatttgcag ctaggtctaa ggttctacga 660
ccactagaca tttcaccacc agtcggagct cagcctacac tatttcaaaa acgagctgca 720
ttacgacaaa gttcactaaa attaggggaa ttgggaggta ctaattgggg gaagagagca 780
gagagtaata gtggtatggg atctaaatta acagtgggaa tagctctaat gcaaacagta 840
ctatcactag cagctatagg gagtaatcaa aaggggaaaa agaaagggtt gccagctaag 900
aatttaatgg cagaaagacg tcgaagaaag aagctaaatg atcgactata catgctaaga 960
tctgtagtac ctaaaataag taagatggat agagcttcta ttctaggaga tgctatagag 1020
tacctaaaag agctactaca gaagataaat gacctaaact atgaactaga atctacacca 1080
tctacctcat cattgacacc taccaccacc ataaccacac caggaagtgg cacacctaca 1140
ggtttctatc ctctaacacc tactcctact agtctaccat ctagaataaa ggaagaacta 1200
tgtccaacag ctatacctag tcctactggc cagcctgcta gagttgaagt aagacagaga 1260
gaaggaagag cagtgaatat ccatatgttt tgtagtagga ggcctggttt gctactatct 1320
accatgagag ctctagataa tctaggttta gatattcaac aagctgtcat aagttgtttt 1380
aatggatttg ctctagatgt ttttcgagct gagcaatgta aagaaggtca agatgttcat 1440
cctgatcaag tgaaagcagt gctactagag tcagctggtt accatggtgt agcatga 1497
<210> 77
<211> 1575
<212> DNA
<213> Oryza sativa
<220>
<400> 77
atgctaccac ggtttcatgg tgccatgtgg atgcaggatg atggaggagg agaccaagaa 60
catgggcagg cagcaccacc tgggcaggag cagcaccacc atgaccagca tctcatggca 120
ttggcagcag cagcagcagg tggtgcaggg tttggtgcag cacaggcacc tgcaccacta 180
ctagatgagg attggtattt tgatgcagca ggtggtggtg gtggtggagc acatgggtcc 240
atgatgctag gtttgtcatc agtccatgga gggattgggg cagggacatc tggtggtggg 300
catgggcagc agttctcact actaaacatg ggagcagcag cagctccatt tgatgtctca 360

gggtttgacc tagggatagc atgtggagga gttggaggtg gtggtgatgt ggtgtcattt 420
ctaggtggtg ggaatgcatc aaacactgca ctactaccag tagggaatgc agggttccta 480
ggcacatttg gagggtttgg cactgcagca tcacaaatgc cagagtttgg agggctagca 540
gggtttgaca tgtttgatgc aggagcagtg aacactgggg gaagctcctc ctcctcatca 600
gcagcagcag cagcagcatc agcctcagca catgtgagta acactgcacc attctcaggg 660
cgaggtaagg ctgctgtgct acggccgcta gatatagtac caccagtggg tgctcagcca 720
actctattcc agaagcgagc gctacgacga aatgctggtg aggatgatga tgataagaag 780
cgtaaggcag cagctggtgc aggtgcagga gcactatcag cagatggtgc agacatggtg 840
ctagatgatg gagatgatga tggcctaagc atagatgcat caggtggtct aaactatgac 900
tcagaggatg ctaggggtgg tgaggatagt ggtgctaaga aggagtcaaa tgctaatagc 960
acagtcactg gagatgggaa ggggaagaag aaggggatgc cagctaagaa cctcatggca 1020
gagcgtcgac gacggaagaa gctaaatgat cgcctctaca tgctacgctc agtagtgcct 1080
aagataagta agatggatag ggcttccatt ctaggtgatg ctattgagta cctaaaggag 1140
ctactacaga agataaatga tctacagaat gagctagagt catcacctgc tacatcatca 1200
ttgcctccta cacctactag tttccatcca ctaacaccta ctctacctac attgccatca 1260
cgcataaagg aagagatatg tcctagtgca ttgcctagtc ctactggaca acagcctagg 1320
gttgaggtta ggctaaggga aggtcgggct gtaaatatcc acatgttttg tgctcggagg 1380
ccaggtctac tactctctgc catgagggca gtagaaggcc taggtctaga tgtccagcaa 1440
gctgtaataa gttgttttaa tggatttaca ttggatattt ttaaggctga gcaatgtaag 1500
gatggacctg ggctattgcc tgaagaaata aaggcagttc taatgcaatc agcagggttc 1560
cataccatga tctag
1575
<210> 78
<211> 1152
<212> DNA
<213> Phalaenopsis aphrodite cultivar
<220>
<400> 78
atgctctcta ggctaagtgg agcagtttgg agggatggag aagcagctga ggaggaagat 60
gacactgcat catggataag atccacctca tgcactgcag ccatgggaga agtgagggat 120
gacatagcac taccatgtct aaaatctatt ctagatgatg attggtatgt agcagccacc 180
tcttctaatg caggtaacct agcagggagc tcattaaacc aggggtttgc aggagactcc 240
tcagcactcc tatcaccacc agtggattcc tcatgctcac catcctcagt attcacacta 300
gatccatctg cacaccagtc attttttcca cataaaccag cactatcatc attcctctct 360
gctgtatgct ctaatccatt tgattcagga tttgattttg tctcagagaa tcctacattc 420
ctaccatctg ctaactcagc agcctccacc tcagtttttg gtggtggaat attgggtttt 480
cctaatgtgc agattaatag tcaagatttt ctacctatga ctggaagctc atcattgttg 540
aatcctatgg catttgattc actagaaaat tcaccatttt tgagtagagg gaaggttttg 600
aggcctctag agatatttcc accagtaggt gctcagccta atctatttca gaaacgtgca 660
gcagcagcac tacgccagaa ttctagttct attggtggtg agaaaggtat gcgtttgggg 720
gttttgggta tggaggatgc agggcagatg aggagctctt gggatggtga gtttgagaag 780
cgtaggagaa tatatgatga ggatgagcta gatgatgcta gcattgatgg ctctggttta 840
aattatgatt ctgaggatat atgtgaagtt gcagagactg taaagttgga tgaagagaag 900
aatgatggtg gaggagcacc tgcaggagga ggaggaggag gtaatgtttc cattactaat 960
agtagtctaa ctggaggggg tggagagctg aagggtagga gaaaggggct acctgctaag 1020
aatctaatgg ctgagaggcg taggaggaag aagctaaatg ataggctata tatgctaaga 1080
tctgttgttc caaagattag taaggtgaga actctcatct cattcatttc attttcacta 1140
gcatcatatt ga
1152
<210> 79
<211> 537
<212> DNA
<213> Prunus mume
<220>
<400> 79
atgtctagta gtgctgtttg gatgtttcca tctaaatcat gtttctcatc tctcctaaat 60
gtagtctcca ctaatccatt tgataatggt tttgatttgg gttgtgatag tggttttcta 120
agctcattcc agggaaacca accatctaat tcatttgcat tgatgggttt cacagccata 180
aattctcaat ctcagatggg tactccagaa ttgagttcta gtgtagagtt tcctactact 240
cggttgctat ctacacctaa taatgacact gagctagatg gtaattttag cctaactggt 300
ttccaaggtt ttgatggctc tgggggtgct cagctactaa atagggctaa gccactaaga 360
ccactacatg tatttcctcc agtgggtgct catcctactc tatttcagaa gggagcagaa 420
ctacgtcaaa attttgctgg tgatgggact gataaattgg ggaatttgga gatttcaggg 480

actgctacca ccactgctgt tggctctgct gctgctgaga gaagaagaca gtgttga 537
<210> 80
<211> 1557
<212> DNA
<213> Camellia sinensis
<220>
<400> 80
atggaagata gaggagatga agaaacacca tcatgggctg ctcaaaataa taataatgga 60
tgtattgtag ataagaaaga tgaactaggt tcactctcta catttaaatc tatgctagga 120
gttgaagatg aagattggta tgtaaacact aataataata acactatcct cacatttcca 180
tctaatttca cagaatctga taataatttg ctattacaat cagtaggttc atcatcatca 240
tgttcaccat catcagcatc agttttcact aatctagatc catctcagga acaatatttt 300
ctaccatcta aaccatccat ttcatcagtc ctaaatccta tctctaataa tccattagac 360
cacagttttg atctaggttg tgaaactggg tttctccaga cacattcatt gacatctcta 420
aataggggag gtgggatttt gactggattc actgatttaa cctcacaatc actaatgggt 480
actcataatt tggcattaga tcctcaattt tctaatactc attcactaca attgactggt 540
tctacatcat ttcgaggttt tcaggaggag ggttttggga atccattgtt tctaaatagg 600
tctaaaatgt tgaagccact acaaaatttt acatctattg gtgcacagcc tactctattc 660
cagaaaagag ctgctctacg taagaatttg gctaataatg ggactaattt gggggttttg 720
ggtactgaaa gaggtgggat atctagtaat gtacaagggt atagtgggaa gatggaagtg 780
agtgaagtga atgagaagaa gaagaggaag tatagcattg gggaagatgt tgaagatgtg 840
agcctagatg ggtctggatt gaattatgac tctgatgagt ttatggagaa taataaggtg 900
gaagagagtg gaaaaaatgg tgggaatagc tctaatgcta atagtactgt cactggagga 960
gatcagaaag ggaagaagag agggctacct gcaaaaaatt tgatggcaga gcgacgacga 1020
cgaaagaagc taaatgatag gctatacatg ctaaggtctg ttgttcctaa aattagtaag 1080
atggataggg catctattct aggggatgct attgaatatt taaaggaact actacaaaag 1140
attaaagacc tacatagtga actagagtct aatcctcctg gctcatcatt gacacctacc 1200
tccactagtt tctatccatt gacacctact ccacattctc taccatgtcg cataaaggaa 1260
gagctatgtc ctagttcatt gcctagtcct aatggactac ctgctagggt agaagtaagg 1320
ttaagtgaac gtagagctgt taatatccac atgttttgta gtcgtagacc aggtctattg 1380
ctctctacta tgagggcatt ggagaaccta gggctagaca ttcaacaagc tgttataagt 1440
tgttttaatg ggtttgctat ggatatattt cgagcagagc aatgtagaga aggccaagat 1500
gtccatcctg accaaataaa agcagtacta ttggattcag ctggattcat gatgtag 1557
<210> 81
<211> 1485
<212> DNA
<213> Arabidopsis thaliana
<220>
<400> 81
atgggtctag atggaaataa tggtggaggg gtttggttaa atggtggtgg tggagaaagg 60
gaagagaatg aggaaggttc atggggaagg aatcatgaag atggttcatc tcagtttaag 120
cctatgctag aaggtgattg gtttagtagt aaccaaccac atccacaaga tctacagatg 180
ttacagaatc agccagattt cagatatttt ggtggttttc catttaatcc taatgataat 240
ctactactac aacactctat tgattcatca tcatcatgtt ctccatctca agcttttagt 300
ctagatccat ctcagcaaaa tcagtttttg tctactaata ataataaggg ttgtctacta 360
aatgttccat catctgctaa tccatttgat aatgcatttg agtttggctc tgaatctggt 420
tttctaaacc aaatccatgc tcctatttct atggggtttg gttcattgac acaattgggg 480
aatagggatt tgagttctgt tcctgatttt ttgtctgctc ggtcactact agcacctgaa 540
agtaataata ataacactat gttgtgtggt ggtttcacag ctccattgga gttggaaggt 600
tttggtagtc ctgctaatgg tggttttgtt gggaatagag ctaaagttct aaagccatta 660
gaggtgttag catcatctgg tgcacagcct actctgttcc agaaacgtgc agctatgcgt 720
cagagctctg gaagtaaaat gggaaattca gagagttcag gaatgaggag gtttagtgat 780
gatggagata tggatgagac tgggattgag gtttctgggt tgaactatga gtctgatgag 840
ataaatgaga gtggtaaagc tgctgagagt gttcagattg gaggaggagg aaagggtaag 900
aagaaaggta tgcctgctaa gaatctaatg gctgagagga gaaggaggaa gaagctaaat 960
gataggctat atatgctaag atcagttgta cctaagataa gtaaaatgga tagagcatct 1020
atactaggag atgctattga ttatctaaag gaactactac aaaggataaa tgatctacat 1080
aatgaactag agtctactcc tcctggatca ttgcctccta catcatcaag tttccatcca 1140
ttgacaccta caccacaaac tctatcatgt cgtgtcaagg aagagttgtg tccatcatca 1200
ttacctagtc ctaaaggaca gcaagctaga gttgaggtta gattaaggga aggaagagca 1260
gtgaacattc atatgttttg tggtcgtaga ccaggtctat tgctagctac catgaaagca 1320
ttggataatc taggattgga tgttcagcaa gctgtgataa gttgttttaa tgggtttgca 1380
ttggatgttt ttcgagctga gcaatgccaa gaaggacaag agatactacc agatcaaata 1440

aaagcagtgc tatttgatac agcagggtat gctggtatga tatga 1485
<210> 82
<211> 1494
<212> DNA
<213> Brassica rapa
<220>
<400> 82
atggttctag atggaaatgg aggtggtgta tggttaggag gaggaggtgg tggtggagga 60
ggagaacgag tgcaggagga ggagaatgag gaagcttcat gggggaggaa tcaagaagat 120
ggtggtcagt ttaagcctat gctagaaggt ggtggtgatt ggtttactag taaccaacca 180
catccacaag atctccagat gctacagagc cagcaagatt ttagattcct aggtgggttt 240
ggttttaatc ctaatgataa tctactacta ctacaacact ccatggactc atcatcatca 300
tgctctccat ctcaggcatt tagcctagat ccatctcaag catcattttt ggctgctgct 360
aataataagt catgtctcct aaatgttgtt ccatcctcag ctaatccatt tgataatgca 420
tttgagtttg ggtcagattc tggtttccta aaccaaatcc aagctccagt ttctatgggg 480
tttggttcat tgacacagct agggagctct gtacctgatt tcttatctgc tcgatcacta 540
ctaccaccag aaaataataa tgcaacacca ttatgtggag gtggaggagg tgggtttaca 600
ccattggagc tagaagggtt tgggagtcca gctagttttg tagggagtag acctaaagtt 660
ctaaagccat tagaggtgtt ggcatcatct ggtgcacagc ctacattgtt ccagaaacgt 720
gcagccatgc gtcagagctc tggaagtaag atggggaact cagagagctc agggatgagg 780
aggctaagtg atgatggaga tatggatgag actggggttg aggtttcagg gctaaactat 840
gagtctgatg agctaaatga gagtggtaaa gcatctgaga gtgttcagaa tggaggaggt 900
aaagggaaga agaaagggat gccagctaag aatctaatgg ctgagaggag gaggaggaag 960
aagctaaatg atagactcta tatgctaaga tctgttgtac ctaagatcag taaaatggat 1020
agagcatcaa tactaggaga tgcaattgat tacctaaagg agctattaca aagaataaat 1080
gacctacata atgagctaga gtctactcct aatggatctc tacctctagc atcatctagt 1140
ttccatccat taacacctac accacagact ctatcatgtc gtgtaaagga agagttgtgt 1200
ccatcatcat tgcctagtcc taaaggccag caagcaagag ttgaggttag attaagggaa 1260
ggaagagcag tgaacattca catgttttgt ggtggtagac ctggtctatt actagctacc 1320
atgaaagcat tggataatct aggattggat gttcagcaag cagtcataag ttgttttaat 1380
gggtttgcat tggatgtttt tcgagctgag caatgccaag aaggacaaga gatattgcct 1440
gatcaaatta aagcagtgct atttgataca gcaggatatg ctggtatgat atga 1494
<210> 83
<211> 1500
<212> DNA
<213> Isatis tinctoria cultivar
<220>
<400> 83
atgggtctag atggaaatgg aggggcagta tggttaggag gaggtggtgg aggaggagga 60
ggaggagaac ggattcagga ggaggagaat ggggaagcat catggggtag gaatcaagat 120
gatggtggag gttctctatc tcattttaag cctatgctag aaggtgattg gtttagtaac 180
acaccacatc cacaagatct ccagatgtta cagagccagc aagattttag atttctaggt 240
ggttttccat ttaatcctaa tgatagccta ctactacatc agtctattga ctcatcatcc 300
tcatgctctc catctcaagc atttagccta gatccatctc agccatcatt tttagcatcc 360
actaataaat catgtctcct aaatgttcca tcctcagcta atccatttga taatgcattt 420
gagtttgggt ctgattcagg ttttctagca caaatccaag ctcctatctc catggggttt 480
ggctcattga cacagttggg gaatcgagaa ctaagctcag tacctgattt tttatctgct 540
cgatcactac caccaccaga aaataataac actactccat tatatggagg aggtgggttc 600
acaccattgg agttggaagg ttttgggagt cctgcaagtg gaggttttgc tgggaataga 660
gctaaagttc taaagccatt agatgtattg gcatcatctg gtgcacagcc tactctattt 720
cagaaacgtg cagctatgcg tcagagctct ggaagtaaga ttgggaactc agagagctca 780
gggatgagga agctaagtga tgatggagag atggatgaga ctgggattga ggtttcaggg 840
ttgaattatg agtctgatga gctaaatgag ggaggtaaag cagcagagag tgttcagaat 900
ggaggaggag gtaaaggtaa gaagaaagga atgcctgcta agaatctaat ggctgagagg 960
aggaggagga agaagctaaa tgataggcta tatatgctaa gatcagttgt acctaagata 1020
agtaaaatgg atagagcatc tatactagga gatgctattg attatctaaa ggaactacta 1080
caaaggatta atgatctaca taatgaacta gagtctactc ctcctggatc attgcctcct 1140
acatcatcta gtttccatcc attaacacct acacctcaga ctctatcatg tcgtgtaaag 1200
gaagagttat gtccatcatc attgcctagt cctaaaggcc agcaagctag agttgaggtt 1260
agattaaggg aaggaagagc agtgaacata cacatgtttt gtggtcgtag accaggtcta 1320
ttgctagcta ccatgaaagc attggataat ctaggattgg atgttcagca agcagtgata 1380
agttgtttta atgggtttgc attggatgtt tttcgagctg agcaatgcca agagggacag 1440
gagatattgc ctgatcaaat aaaagcagtg ctatttgata cagcaggtac actagtatga 1500

<210> 84
<211> 1572
<212> DNA
<213> Eucalyptus camaldulensis
<220>
<400> 84
atggttctag gtggagagga agatgaggtc tcttggacac gagaccacca ccaccaccat 60
gatggaggag gaggtggtaa tgatgatgaa gcagcacgac ataaggaagg agagctagcc 120
atgggagggg cctctacatt taagtccatg ctagatgcag attggtccta ttttgccact 180
aatcctcctc accattcaca gcagcagcag cagctacagc cagctccaga aatggcagac 240
ctatcattct cttctgctgc tccaattcca gataatgcac tcctcctcca ccattctcta 300
gactcatcct cctcatgctc accatcacag gcatttgtcc tagatccatc acagactcac 360
cagccattcc tacagcctaa gcattgtttc tcctctctcc taaatgtagc atggaataat 420
tcatttgata atggatttga tttgggttgt gatgcagcag ggctatttgg ctcagtgcag 480
ggaaatcaga cctctagtgc accactactc atgggaggtt cagagctagg ctcaggttca 540
gagctactac cagtgccaag tcgtctagta ccactaggtg aaaactcaac acaattgcca 600
ggaggagggt ttgggctaac tgggtttgaa ggttttgaga gtcctggaaa tccactattc 660
acaaataggt ctactaaggt cctaaggcct ctagaggttt ttccacagac aggagcacag 720
cctactctat ttcagaagag agctgctcta agaaatgggt ctggaagtaa tggtggtggg 780
gctgagaaat tggggaattt ggagatagat aagaagagaa agaagattga tgatggtatt 840
aatgatgaat tgagttttga tgggtcaggt ttgaattatg atacagatga gggtaatgag 900
agtgggaagg ctatggagga ctctaagcat gaagggtgta attctaatgc taatagcaca 960
gtcacagttg gagatcagaa ggggaagaag aaggggctac cagctaagaa tctaatggct 1020
gagcgacgac gaaggaagaa gctaaatgat aggctataca tgctaaggtc tgtagtgcct 1080
agatcagcta gaatggatag agcatctatt tttggggagg ctattgatta tttgaaggag 1140
gtttgtaaaa ggataaataa cctccataat gagctagatt ctactccacc tggaaccatg 1200
ttgcctccat ctactaattt tcatccattg acacctactc caccaacact accatgtcgt 1260
gtgaaggaag aactatgtcc tagctcattg cctagtccta aaggccagcc tgctagagtg 1320
gaggttagag tgagggaagg gagagcagtg aacatccaca tgttttgtgc acgcagacca 1380
gggctattgc tctccaccat gagggctcta gataacctag ggttggacat tcaacaggct 1440
gtcataagtt gttttaatgc atttgctatg gacatttttc gagctgagca atgtagggaa 1500
ggtcaagatg tcctaccaga acagataaag gcactactat tagagtcagc tggtttccat 1560
gggatggtgt ga
1572
<210> 85
<211> 1629
<212> DNA
<213> Jatropha curcas
<220>
<400> 85
atgctatctg gactaaatgg agctgtatgg atagatgata aagaaccaca ggactctact 60
actaacacca ttaacattaa tgatagtagt tgtagtatta tacagagtaa acaagagatg 120
ggttcactat ctacatttaa atccatgcta gatgtagaag atgattggta tgttgctaac 180
actagtatcc aaaatcatca agatggtatt agagatatca cattttcacc taatctaggt 240
gatccagata atctattgct acattcagtg gattcatcct catcatgctc acctacctcc 300
tctgttttta ataatctaga tccatctcaa gttcactatt ttatgcatcc taaacctaca 360
ttatcatcac tactaaatgt tgtttctaat aatccattag agcatgggtt tgatttgggt 420
gacattgggt ttctagataa ccaggctact aatactacta ctactacagc taatgcctca 480
tcattactaa ataggggagg tggagtatta ggaaatttta ctgatatgag ctctaataat 540
cagattagta gttctaattt atgcactgat ccatcatttt ctactactcg gatgctccaa 600
ttgcctgaaa catgtcctgg gtttgcagga tttaggggtt ttgatgagag ttcaggaaat 660
gcactatttt tgaataggtc taagttgtta aggcctctag aaacatttcc atctatgggt 720
gcacagccta cattgtttca aaagagagca gcattgagga aaaatttggc tgataatggg 780
tctaatttgg gggcattaag tgggattgag ataaataaag ggaagagaga aatgagtcag 840
atgggtgaag agaatgagag aaagagaaag ttgggtaatg tagatgatac atttgaagat 900
gtaagcattg atgggtcagg tttgaattat gactctgatg agttcacaga gaacactaaa 960
atggaggaga ttggaaaaaa tggagggaat agctctaatg caaatagtag tgtcactgga 1020
ggaggtggag atcagaaggg gaagaagaag gggctacctg ctaaaaattt gatggcagaa 1080
aggaggcgac gaaagaagct aaatgatagg ttatatatgc taaggtctgt agttcctaag 1140
attagtaaga tggatagggc atctatacta ggggatgcta ttgagtattt gaaggaacta 1200
ctacaaagga taaatgacct acataatgaa ttggagtcca ctcctcctgg ctcatcattg 1260
acacctacca caagttttca tcctttgaca cctactccat cagcactacc tagtcgtata 1320
aaggataagc tatgtcctac ctcattgcct agtcctaatg gtcaaccagc tagggttgaa 1380

gtgagagtga gagaaggaag agctgtaaac atacacatgt tttgtagtcg aagagcaggt 1440
ctcctactct ccactatgag ggctttggat aacctaggcc tagacatcca gcaagctgtc 1500
ataagttgtt ttaatggttt tgccatggat atatttcgag ctgagcaatg taaagaaggg 1560
caagatgttc atcctgagca aataaaagca gtgctactag attcagctgg tttccatgga 1620
gtgatgtag
1629
<210> 86
<211> 874
<212> DNA
<213> Picea abies
<220>
<400> 86
atgttgtcta gaatgaatct aaatggagga gtgtggggtc tagaagatgc cacagacctc 60
cattgtaata atgatgagaa tttgaatttg cctacattta aggctatgct agatgcagaa 120
gaatggttta catctcatag taatactagt aataatgata ttactactgc tagtattaat 180
aatgctaata gccatcctga tggaccatgt tttaatatag atatgaagga ttttggatgc 240
tattcatctc tagtgaatcc aaatccagag cctcctaata tgctattgca gcatgccatg 300
aattcatgtt cctcatcacc agcatcaatt ttcagcctag atccatcaca ggtccagtca 360
tttctagcag gggggagtaa ccatcatcac caccaccatg tatctccatt tccagatgtt 420
gcaggttcaa gtccattcca ggagttggca tgtgaggggt tggtgagcag taataatgca 480
agtagtaata gtagtagtaa tagctattta tatggggggc taccaggagg aatttttagg 540
cagctaagtc ctagttttgc aggttctagt ccacgcacta atactgttac tcagacacct 600
aatctaagta gctccacata gccagatggg tggtagtagt actcctaatc taagtcctag 660
aatgatgcct actagtaatc taaatctctc agacacagga ttgtttgcta gtagtaatca 720
caccaccact aataataata ataatctaag taatctacta ggtttgagta gcctaatgag 780
tcctcgaata ccagcaccat taaattttag tagtcctaag atgctaagac cactagagat 840
ttgtgcacca gttggtgctc agcctacatt gtttc
875
<210> 87
<211> 1632
<212> DNA
<213> Populus suaveolens
<220>
<400> 87
atgctatgta gactaaatag ccataatatt tgggttgatt atcatgcaga agagcaggat 60
tccacatctg atcgtccaca ccataataac attactaata ctgctgctgg ttgtggaggg 120
gttatgttgg ggggaaaaca agaaatgggt tctctatcta catttaaatc tatgtttgaa 180
gtggaccatg agtggtatgt gactaatgcc tatagtgcta ttcctcgagg tgatcaagat 240
agtattaaag acctcacatt ttcaccacgt ctagtgcacc catataatct attgctacta 300
ccagggcatt catcctcatc atgttcacca tcatcatcag ttgttaataa tttggatgca 360
tcactactac attcatttat gcctcctaca cctacattgt cctctctcct acatgtggtt 420
tcttctaatc cttctaagga tgggtttgat ttgagtgaaa ttggtttcct agagaatcaa 480
ggaactaatt ctactactac agctaatgtt tcatctccat taaataggcc acgtggagtg 540
ttggggaatt ttgggaattt cactgatttg agttctacag tacgattctc attcctattt 600
gggttctcat caccagacta taagtatcca catgcatcac ctgcagagct acctcctgca 660
ctctcatgtt ttacacgact agatgagata tcagtgaatc cattgttctc atcaaggact 720
aagttgctat cacctctaga aacatatcct attcatgggc gctcagctac tctatttcaa 780
agagagcagc tagaggaaag aacatgggga agtgagagag ataaagggaa gagagatatg 840
acacaaatta gagaagagaa tgataagcag agaaactcta gtagtggaga tgattttcta 900
gaggaattag ctcagatggg tcgagtgaga attatgcatc taggaattaa agagtataca 960
ttggtggaag agactggtaa gaatgttgga aatagttcta aggtgaatag tggtgttact 1020
ggtcgtggtg aggatcaaaa ggggaagaaa aggggcctac cagctaagaa tttgatggca 1080
cagtggaggc gacgtatgca gctaaatgat aggctataca caatgaggtc tgttgttcca 1140
cagattagta aaatggatag gccatctatc ctaggtgatg ctattgaata tttgaaagaa 1200
cttctacaaa ggattaatga cctccataat gaattggagt ctactcctcc aagctcatca 1260
ttgacaccta ccactagttt tcatccattg acacctactc catcagcaga gcctagtcgt 1320
atcatggacc aactatgtcc tagctcattg cctagtccta atggccaacc tgctagggta 1380
gaggttagag tgagagaagc acgagcagta aacatccaca tgttttgtgg acgtaaaaca 1440
ggtctattgc tattcactat gagagcattg gataacctag ggctagacat ccagcaagct 1500
gtcattagtt gttttaatgg ttttcctatg gatatattgc gaaatgagca acgtaaagaa 1560
gggcaggaca tgcatccaga tcaaattaaa gcagtcctac tagattcagc tggattccat 1620
ggcactatgt ag
1632

<210> 88
<211> 1194
<212> DNA
<213> Populus trichocarpa
<220>
<400> 88
atgctatata gactaaatag taataatatt tggctagaag atcataaaga agagcaagat 60
tctactacta accatcatca ccataataat attactaata ctgcagctgg ttgtggtggg 120
gttatgttgg agggaaaaga agaaatgggc tctctatcta catttaaatc tatgtttgaa 180
gtggaagatg agtggtatgt gactaataat aatagtacta ttcatcaaaa ccatcaagat 240
agtattaaag acctcacatt ttcacctagt ctagtagatc cagataatct attgctacac 300
caagtggatt catcctcatc atgttcacca tcatcttcag tttttaataa tttggatcca 360
tctcaggtac attattttat gcatcctaag cctacattgt catcactcct aaatgtggtt 420
tctaataatc cattagagca tgggtttgat ttgagtgaaa ttggtttcct agagaatcaa 480
ggaactaatt ctactactac agctaatgtt tcatctctat taaatagggg aagtggtgtt 540
ttggggaatt tggggaattt cactgatttg agctctaata gtcagattag tattcctaat 600
ttatgttctg atccacaatt ttctagttca cgcatgcttc agttgcctga aaatggtcct 660
gggtttaatg gttttagagg gctagatgag atttcaggga atcagttgtt ttttaatagg 720
tctaaattgt taaggcctct agaaacatat ccatctatgg gagcacagcc tacattgttt 780
caaaagagag cagcattgag gaagaatttg ggtgaagttg agagagataa agggaagaga 840
gaaatgactc aaattagtga agagaaagat aagaagagaa aatttagtag tggagatgat 900
tttctagagg atgttagttt tgatgggtca ggtttgaatt atgattctga tgaatttact 960
gagaatacta atttggagga gactggtaag aatggtggaa atagttctaa ggctaatagt 1020
ggtgttactg gtggtggtgt ggatcaaaag ggaagaaaag gggggctacc agctaagaat 1080
ttgatggcag agaggaggcg acgtaagaaa ctaaatgata ggctatacat gctaaggtct 1140
gttgttccta agataagtaa agtaagaatt tttttcctca tcattttttt gtga 1194
<210> 89
<211> 519
<212> DNA
<213> Potamogeton perfoliatus
<220>
<400> 89
atgctaagat ctgttgttcc taaaattagt aagatggata gagcatctat cctaggagat 60
gctatagaat acctaaagga gctactacaa aggattaatg atctccacaa tgaactagaa 120
tcatcaccat caagttctgc tctacctaca cctacaggtt ctagtctacg accattgaca 180
ccaacaccac taacattgcc atgtcgagtt aaggaagagc tatgtcctag ttcattacct 240
agtccragtg gtcaaccagc cacagttgaa gttaaggtga gagaaggagg agcagttaat 300
atccacatgt tttgtgttcg caggcctggc ctattgctct ctactatgcg rgctctagat 360
agcatgggtc tagatatcca gcaggcagtc ataagttgtt ttaaygggtt tgcattagat 420
gtttttcgag ctgagcaatg taaggatgrc cctggagttc ctcctgatga tattaaaact 480
gtactactac attctgctgg tctacatggt tctatgtaa
519
<210> 90
<211> 519
<212> DNA
<213> Potamogeton wrightii
<220>
<400> 90
atgctaagat ctgttgttcc taaaataagt aagatggata gagcatctat cctaggtgat 60
gctatagaat acctaaagga gctactacaa aggattaatg atctccataa tgaactagaa 120
tcatcaccat ctagttctgc tctacctaca cctactggtt ctagtctacg accattgaca 180
cctacaccac tarccttgcc atgtcgggta aaggaagagc tatgtcctag ttcattacct 240
agtcctagta gtcaaccagc cacagttgaa gtaaaggtga gagaaggagg agcagtaaat 300
atccacatgt tttgtgttcg aaggcctggc ctattgctct ctactatgcg ggctctagat 360
agcatgggtc tagatatcca gcaggcagtc ataagttgtt ttaatgggtt tgcattagat 420
gtttttcgag ctgagcaatg taaggatgga cctggtgttc ctcctgatga tattaaaact 480
gtactactac attctgctgg tctacatggt tctatgtaa
519
<210> 91
<211> 1147
<212> DNA
<213> Manihot esculenta
<220>

<400> 91
atgttgtctg gattaaatgg cactgtctgg atggaagata aagaagcgca acactccacc      60
acagacaaca acattcacaa tagctgtgat gtgatagaga gcaaggaaga tatgggttcc     120
ctttctacat tcaaatccat gctcgaagta gaagaagaag actggtatat tgctaatagt     180
agtatccatc atcatcaaga taccataaga gatattacat tttcacctaa tctagcagaa     240
acagataatc tattgctaca tcctgcggat tcctcctcat catgctcacc agcatcctct     300
gtttttaata atatagatcc atctcaggtg ccatatttta tgcactctaa acctacatta     360
tcatcactac taaatgctgt ctctaataat ccattagagc atgacattga gttgggagaa     420
attgggttcc tagataacca gtctactact actactacta ctaatgcctc accattgcta     480
aatagaggag gtggagtttt aagtaactac acagatttga gctctaataa tctatttagc     540
tcatctaatt tatcacctga tccaccattt tctactactc gtgcactaca gttaccagaa     600
aatggtccag ggtttacagg gtttaatggt tttgttgaga gctcagggaa taggctattt     660
tgtaataggt ctaagttgtt gaggcctctt gaaacatttc cttcgatggg tgcacaacca     720
actttgtttc aaaagagagc agctttgaag aagaatttga ctgataatgg aagcaatttg     780
ggggttctga gtggtattga gattgataaa ggaaagagag aaatcactca gttgatcgaa     840
gagaatgaga agaagagcaa attgggtaag ttagatgaca ctgtagagga tgtaagcatt     900
gacgggtcag gtttgaatta tgattctgat gaggtaactg aaaatattaa aatggaggag     960
attggtaaaa atggagggaa tagctcaaat gcaaatagca gcgtgactgg cggtggtgtt    1020
ggggatcaga aggggaagaa gaaggggctc cctgctaaga atttgatggc agaaaggaga    1080
cgccggaaga agctcaatga taggctgtac atgctaaggt ctgtagtccc aaaaataagc    1140
aaggtga
1147
<210> 92
<211> 1416
<212> DNA
<213> Actinidia chinensis
<220>
<400> 92
atgtatgaag agagtgaatg ttttgatcca aattctatgc aaggactgtc taaagatggg      60
ttttcacaaa atccaagttt ctccatggaa gagccctcct accaccaaaa cccctcaccg     120
gaatcagccg cgggtggtgg tgccgctgcc accgtggaag tagaactcca ccagcaaatg     180
ggtttggaca tggcaaatat ggaaccttgt tacaatatca acaacagcaa cagtctcatt     240
gatagtacct atttgatgca agaagttgtt cttgactcta accatgtttt gccctatgac     300
cactctaatt gggatcctaa tctaaaccat aaccaccatc aagatttgaa ccatcatgtt     360
gaaattgcta atgggcatta tcctaatttg attagtaata attcctctat accagaccta     420
ctaaatctac tccatttgcc tacatgttca ccatcctctg tcctacctaa ttcatcacta     480
tcatttttga gtcaagcaca gaaatctact aattttcctc catcatttct aggggatttt     540
gcatctaatg agtcaggaat ggtttctaat ctagtgcatg actctctatt tcatctaaat     600
ctaccatcac ggccaccatt gctaagggag atgtttcagt ctctaccaaa ttgttcattg     660
tttggtggtg gaggtagtgg tggggtggtg gatgagaaag atgggagtga aggagggagg     720
cagtttgata atggggtctt gcagttttgt agagagtggg acatgaattg tatggccaaa     780
gaaggtaatg gtaagggcat tactaccaaa cactttgcca ctgagaggca aaggagagag     840
cacttgaatg gtaagttcac tgctttgagg agcttggtcc ccaaccccac caagactgat     900
agagcatcaa taattgggga tgcaattgag tacatcaaag agctactaag aacagtcaat     960
gagctgaaaa tcctagtcga gaagaagaga tgtggtaaag acagaagtaa gaggcataag    1020
acagaagaag atgggacgac tggagattta gaaaatgtca acaagaaacc cgaccctgac    1080
caatcctaca acgggccatc gcttagaagc tcgtggctgc agagaaaatc caaggatacg    1140
gaggtcgatg ttcggattat tgatgatgag gtgaccataa aactcgcaca gcgcaagaaa    1200
atcaattgcc ttctgattgt gtcgaaggtg atcgatgagc ttcagcttga tatccaccat    1260
gttaccggtg ggcttgttgg ggacaactac agttttctgt tcaacagcaa gatatgtgaa    1320
gggtcttctg tgtatgcaag tgcaatagcc aacaaactca ttgaggttgt gaacagacag    1380
tatgcagcaa tcccaccatc aaccagtagt tattag
1416
<210> 93
<211> 1145
<212> DNA
<213> Musa acuminata
<220>
<400> 93
atgttttcga gaattgatgg cggcttctgg atggatgacg gtggagacga cgacgaccct      60
gtcgcttgga caagaggaaa cgcctccagc tctagcggag gggaatgggg ggagaccaag     120
gaggatgagc taggcctctc aggatttaag tccatgctag tggatgattg gtatgtagga     180
gctgctgctc atcatccatt tgaggcattc cagactcagc aggacctaaa agatgtagca     240

tttctaccta atccatcacc acacgaggca ctattgctac ctcctgtaga tagcctagac 300
caaagccaac catttttttc atctagacct gcagtttcat ctgtatttaa tgcctctaac 360
cattttggag ttggggttga tctaaggagt gatgcaccag ggtttctacc agcagcacca 420
gttttaaatc cccctgactt gatgaacggg ggaggtggcg ccatcgatgg agtgtttggc 480
ttcgtcggaa ttggaaccag cggacaactt ggttgtccgg agctgaactc cgcaagccaa 540
ttctccggcg cccttctgct acctcctgcc gagaactgct ccgtttccag ctctggcacc 600
gccttctgcc ctgttggctt cgacagcttg gagaattcac cgtttctcaa ccggtccaag 660
gtgttgaggc ctttggagat gttccctaca gcgggctcgc agcccaccct gttccagaag 720
cgggcggccg ccgctctact gcagaactcg gcggctgctg cagaaaaagt cggcatcttg 780
ggtccttggg cgtcgaaagg cagctgccaa gggagcagca gtgtggctgc attggaggaa 840
gagaaagaga ggaagaggaa agggaatgag gtcgacgagt tggacgaggg tagcattgac 900
aggtccggct tacattatga ttctgatgac tttgccggag agtgtgccaa agttgaggag 960
aatgcaggga atgtagcagg tggaggaggg aacagttcga gtgctgatgt catggtggcc 1020
gacaggggga agaagaaggg tctcccggcg aagaatctga tggcggagag gaggaggaga 1080
aagaaactca acgacaggct ttacatgctt agatctgttg tccccaagat aagcaagatg 1140
gatag
1145
<210> 94
<211> 1644
<212> DNA
<213> Musa AB Group
<220>
<400> 94
atgttttcga gaattgatgg cggcttctgg atggatgatg gtggagatga tgatgatcct 60
gtagcatgga ctagaggaaa tgcctccagc tctagtggag gggaatgggg ggagactaag 120
gaggatgagc taggcctctc aggatttaag tccatgctag tggatgattg gtatgtagga 180
gcagcagcac atcatccatt tgaggcattc cagactcagc aggacctaaa agatgtagca 240
tttctaccta atccatcacc acatgaggca ctattgctac caccagtaga tagcctagac 300
caaagccaac catttttctc atctagacct gcagtttcat ctgtatttaa tgcctctaac 360
cattttggag ttggggttga tctaaggagt gatgcaccag ggtttctacc agcagcacca 420
gttttaaatc cccctgactt gatgaacggg ggaggtggcg ccatcgatgg agtgtttggc 480
ttcgtcggaa ttggaaccag cggacaactt ggttgtccgg agctgaactc cgcaagccaa 540
ttctccggcg cccttctgct acctcctgcc gagaactgct ccgtttccag ctctggcacc 600
gccttctgcc ctgttggctt cgacagcttg gagaattcac cgtttctcaa ccggtccaag 660
gtgttgaggc ctttggagat gttccctaca gcgggctcgc agcccaccct gttccagaag 720
cgggcggccg ccgctctact gcagaactcg gcggctgctg cagaaaaagt cggcatcttg 780
ggtccttggg cgtcgaaagg cagctgccaa gggagcagca gtgtggctgc attggaggaa 840
gagaaagaga ggaagaggaa agggaatgag gtcgacgagt tggacgaggg tagcattgac 900
aggtccggct tacattatga ttctgatgac tttgccggag agtgtgccaa agttgaggag 960
aatgcaggga atgtagcagg tggaggaggg aacagttcga gtgctgatgt catggtggcc 1020
gacaggggga agaagaaggg tctcccggcg aagaatctga tggcggagag gaggaggaga 1080
aagaaactca acgacaggct ttacatgctt agatctgttg tccccaagat aagcaagatg 1140
gatagagctt ccattcttgg cgatgcgatt gagtacttga aagaacttct ccaaaggatc 1200
aacgaccttc aaaatgagct tgaatcaaca ccttcaagtt cttcactgcc tacaacaaat 1260
gcaacgagct tgcacccctt aactcccaca ctaccaactt tgccatgccg tttgaaggat 1320
gaactcaagc actgctcgag ttctttacca agtccaaata gtcagccagc aagggtcgaa 1380
gttaaggcga gggaaggccg agccgtcgac atacacatgt tctgtgcccg taggcctggt 1440
ctgctgcttt cagctttgcg atcacttgac agccttgggc ttgacattca gcaggcagtc 1500
atcagttgtt tcaatggatt cgcgctagac attttccaag ctgagcaatg caaagacccg 1560
ggtgttctcc tgaagaaatc aaggctgtgc tcttgcactc tgcaggtttg ctcaacacaa 1620
tgtagtagtc agcaatcaag gtag
1644
<210> 95
<211> 1344
<212> DNA
<213> Theobroma cacao
<220>
<400> 95
atgctttcga gggtgaacgg cggtgtcgtt tggatggaag ataaagaaga tgaagaatct 60
gcatcatgga ataggactaa taatagtaat aataataata ataatagtgt tataatggag 120
aataaagaag agatgggttc attgtctaca tttaagtcca tgctagaaga agaatggtat 180
gtagctaata atagtatttc tagccatcaa gatattagag acctatcatt ttcacctaat 240
ctagctgacc atcaagataa tattttgcta catcatcatc atcatcagca gcagcatcat 300

ccagtggatt catcctcatc atgttcacca tcatcctctg ttttcaataa tctagatcca 360
tctcaggtac actatttttt gcaacctaaa cctactttat catcactcct aaatgttgtt 420
tctaataatc ctttagagca tgggtttgat ttgagtgaaa ttgggttcct agataaccaa 480
gctactaatg ctactactct attgaatagg ggaaatgctg gggttttggg tagttttgct 540
gatttaagcc atggtaatca gatagacact gctaatttgt gtcctgagac tcagttttcg 600
agttctagga tggttcagct accggaaaat ggtgcggggt tggcgggttt tcaaggattt 660
gatgagaatc ctggaaatgc tctgttttta aataggtcga aggtgttaag gccactggaa 720
agttttcctt cagtcggagc acaaccaact cttttccaaa aaagagcagc tttgaggaag 780
aatttagctg atagtggagg gaatttcggg ttgttcggtg gaggaaaggt taatgctttg 840
aatggaattg agggagataa agggaaaaaa gaaatggatg aagagaatga gaagaggaaa 900
attatctata gagatgatct agaagatgtg agtattgatg ggtctgcttt aaattatgat 960
tcagatgagt ttactgagaa taacaaggtg gaggaaactc ttaaaaatgg tgggaatact 1020
tcgaatgcaa acagtactgt cactggagga gatcaaaagg gtaaaaagaa ggggcttcct 1080
gctaagaatt tgatggctga gaggcgccgt cgaaagaaac tcaatgatag gctttacatg 1140
ttgcggtctg ttgttccgaa gattagcaaa gtaagaattt ccttcacgtt tctctttttc 1200
tttttttttc cccttctctt ttcgttttta tctcgtgaat tcaagtatga attattgagt 1260
tattttagat cctcagaaat gtcaaatatt ctgacttttt atctttgttt tcatggttgg 1320
tgtttttgta ttatgtgtga atga
1344
<210> 96
<211> 1682
<212> DNA
<213> Citrus trifoliata
<220>
<400> 96
atgctttcta gactaaacgg tgtggtctgg atggacggga aagaagagga aggctcagct 60
gcatgggcta gaaataacat aaataataat agtagtaata gcataaataa taattgtaac 120
actaataaca tgagttgtag taataataat gataataata atggtgtcat agagaatgaa 180
gaagaaatgg gttcactacc tggatttaag tccatgctag aagtggaaga tgattggtat 240
gttactggta atactagttt aaataaccat caagatatta catttccacc aaacctaggt 300
gatcctacta ctgataatct attgctaaat gctgtggatt catcatcatc atgttcacca 360
tcatcctctg tttttaataa ttttgatgca tctcaagttc actatttttt gcctcaaaaa 420
aactcatttt catcattcat gaatgttgtg tctaataata actcattaga gcatggattt 480
gatttgggtg aaatggggtt tctagacaca caagctactc atgcattgaa tagggggaat 540
ggtgggattt tgaatggttt caatgatttg agtgccaata atcagatgaa tgctactaat 600
ttatgttctg atccgcaatt tggaactaac cgtacgcttc aattcccgga aaatggtagc 660
agtagtttcg cgggttttcg gggttttgat gagaataatg ggaattctct gtttttgaat 720
aggtctaagt tgttgaggcc acttgaaact ttcccttcga cgggagcaca acccactctt 780
ttccaaaaaa gagctgcttt gaggaaaaat ttaggtggta atgaggccag tttaggggtt 840
ttaggtacac aaaatagcca acttttgagt gggattgaga gtgataaagg taagaaagaa 900
ctgactgaag ataatgagaa aaagaggaaa ttgagtatta gtgatgattt ggaggatgtg 960
agtgttgatg ggtcgggtct caactatgat tctgatgatt ttttagaaaa taataaggtg 1020
gaggaaatgg gtaaaaatgg tggtagtagc tccaatgcaa ttagcactat cactggtgga 1080
gatcagaagg gcaagaagaa gggtttgcca gcaaagaatc tgatggcaga gaggagacgc 1140
aggaagaagc tcaatgatag gctctacatg ttgcggtcag ttgttccaaa gattagcaaa 1200
atggataggg cttcaatttt gggggatgca attgagtatt tgaaggaact tctccaaagg 1260
atcaatgacc tccacaatga attggagtca acccctcctg gttcagcatt gactccttcc 1320
acaagcttct atcctttgac accgactcca cctgccttgc acagccgtat caaggatgaa 1380
ctttgcccca gctcactgcc aagcccaaat ggccaacctg caaggttgga ggtcagggtg 1440
agggaaggaa gagccgtgaa tatccacatg ttttgcagcc gtagaccagg tcttttgctc 1500
tccacgatga gggcattgga taatcttgga ctagatatcc agcaagctgt catcagttgt 1560
ttcaatggct ttgccatgga tgttttccga gctgagcaat gcaaggaagg ccaggacgtt 1620
catccggagc aaatcaaggc ggtactcttg gattcagccg gcttccatgg catgatgtag 1680
tt
1682
<210> 97
<211> 1092
<212> DNA
<213> Vitis vinifera
<220>
<400> 97
atgttaccca ggtctaatga tgtagtttgg acacgaagtg atgctgtttc atggtctaag 60
aatagtaatg ataataggtt tggtgatgga ggaggtgatg gtgagaggac tagttctgtg 120

tctgcattga aggccattgt tgaggaggag tggttggtga gtggtgtaga aatgaaagga 180
aatttggtgt ctcagagtca gagtggagca gtgaattcac tattgcatcc tatggattgc 240
tcatcatcat gttcaccagc atctgcattt ggtctagatc cagcatttca gggaaaatct 300
tgtttgtcct ctatggtttg tgggtatcca tttgatggct catttgattt ggtatgtgat 360
ccagggtttc tagagcctat acctgaaatt caggctccta gttctagttc attgatgggt 420
tttaatgatt tggcatcaca gcctctaatg ggtgcttcta atctaggctc taattctcag 480
tttcctgcta cacatttggc aggaaatgga gggagtgctg ccactggtgg gtttaatcca 540
tcgggttttg agggttttgt ggggagttct ctgtttgtgg acaggtgtaa ggtgttgaaa 600
ccgctcgaga acttcccttc agttgggtca cagcccacgc tgttccagaa gagagcgatt 660
ctccggcgaa attcgacaga aagagcgggg aattatggag tttcgggcca agaaggcagt 720
gcaattccgg ttagagttgc cggagaagat aagggtaaga ggccggtggt ggaagaagaa 780
atggacaaga tgagaaagga caagagcaat gatgaggatg atatggacga agccagcatt 840
gctcggtccg gtttgatata cgattctgat gatgcaattg agaaccacaa ggtggaggag 900
actgcaaatg atggtggaga caactcaaat ttgaatggat cttccattgg aggagataga 960
aaagggaaga aaaagggcct gccggcgaag aatttgatgg ctgagaggcg ccggagaaag 1020
aagctgaacg ataggctcta catgctgagg tctgttgttc caaagatcag caaggtgaga 1080
attttctggt ga
1092
<210> 98
<211> 1596
<212> DNA
<213> Malus x domestica
<220>
<400> 98
atggacgaca gggaggatag agactctgtt tcctggacta gaacctccgc cactgccact 60
gcaggaaaca cagagaataa ggatgagatg ggttcatctc tatctacatt taagtctatg 120
ctagaggttg aagatgattg gtacatggca gctaataata gcatccaggg ccattcagat 180
gtaggagaca taagtttctc accaagtttt gcagacccag aaagtttgct actaaatcca 240
gtggactcat cctcctcatg ctcaccatca tcatcagttt ttaataatct agatcctaat 300
caggttcatt actacatgcc tcaaaatccc aacttgtctt cgctgcttaa tgttgttcct 360
aacaaccctt tggatcacgg cttcgatttg ggctgtgaca ttgggtttct tgacactcaa 420
gcttcgggtg gcggttcgag tatgatgaac aggggaggtg ggctttatct tggattcaat 480
gatttaagct cgaatagcca ggtgaatgct cagaatttgg gttctaattt acagttttcg 540
actacccgca tgcctcaagc tcttgaaaac agttcaaatt tttcgggttt tcgaggtgtg 600
gatgatggtt cggcaaatgc tttgtttccc aataggccta agttactgag accacttgaa 660
tccttccctt ctgtgggagt ccagccaact cttttccaga aaagagcagc gcttcggaaa 720
aacttatgcg atggtggagg gaatttgggt gttttggtct cacaaggggg cctggttttg 780
aatgaagggg atgagaggaa gagggagatg ggtgtgcaga atgagaagaa gaggaaaatg 840
agcggcggag atgatgtcga tgatctgagt ttcgatggtt cgggtttgaa ctatgattcg 900
gatgagttta ctgagaacac taaggttgat gacggtgcca agaatggtgg gaacagctcg 960
aatgcaaata gcacggttac cggtggagga gggggtcata aggggaagaa gaaagggttg 1020
cctgccaaga atctgatggc tgagaggcgc cgccggaaga agctcaatga taggctgtac 1080
atgctgagat ccgttgttcc gaagattagc aaaatggaca gggcctcaat ccttggggat 1140
gcaattgagt acttgaagga acttctgcag aggatcaaca acctccacaa tgaactggag 1200
tcaatccctc ctggatctgc attgacacct accggaaata ctttccaccc tttgacaccc 1260
actccggcta ctctgccaaa tcgtatcaag gaagaacttt gcctcagctc attacctagc 1320
ccaaatggcc aagctgcaag ggttgaagtt aggctgcgag aaggacgagc agttaacatc 1380
cacatgttct gcggccgaag gccaggtctc ttgctctcaa ccatgagaac tttggataac 1440
cttgggttag acatccagca agccgtcatc agctgtttca atggttttgc tatggatgtc 1500
ttccgtgccg agcaatgcaa ggaaggccaa gacgtccacc cggatcagat aaaggcggta 1560
cttctggatt cgatcgggtt ccatggcatg atgtag
1596
<210> 99
<211> 1479
<212> DNA
<213> Capsella bursa-pastoris
<220>
<400> 99
atggttcttg acggaaacaa cggtggtgcg gtttggttag gcggtggtgg agaacggaa 60
gagaacgagg aaggttcgtg gggaaggaat caagaagatg ggtctcagtt taaaccaatg 120
cttgaaggtg attggtttag caacccacca catccacaag atcttcagat gttacagaat 180
catcaagatt gcagatttct tggtggtttt ccatttaatc ctaatgataa tctactacta 240
cagcactcta ttgattcatc atcatcatgt tctccatctc aagcatttag cctagatcca 300

tctcagcaaa accagttttt gtccactaat aataagtcat gtctcataaa tgttccatca 360
tctgctaatc catttgataa tgcatttgaa tttggctctg aatctgggtt cctaagccaa 420
atacatgctc ctatgtctat gggttttggt tcattgacac agttggggaa tcgagatttg 480
agttctgttc ctgatttttt gtctgctagg tcactactag cacaggatca taatagtagt 540
aattcagtgt tatgtggtgg aggaggtggg ttcacagctc cactggaatt agaaggtttt 600
gggagtcctg ccaatggtgg ttttgttggg aacagagcca aggttctgaa gcctttagag 660
gtgttagctt cgtctggtgc gcagcctact ctgttccaga aacgtgctgc tatgcgtcag 720
agctctagaa gcaaaatggg aaactctgag agctcgggaa tgaggaggtt gagtgatgat 780
ggggatatgg atgagactgg gattggggtt tcagggttga attatgagtc tgatgagcca 840
aacgagagtg ggaaagcagc tgagagtgtt cagattggag gagggaaggg taagaagaaa 900
ggaatgcctg ctaagaatct gatggctgag aggagaagga ggaagaagct taatgatagg 960
ctttatatgc ttagatcagt tgtccccaag atcagcaaaa tggatagagc atcaatactt 1020
ggagatgcaa ttgattatct gaaggaactt ttacaaagga tcaatgatct tcacaacgaa 1080
cttgagtcaa ctcctcctgg atctttgcct ccaacttcat caagcttcca tccattgacg 1140
cctacaccgc agactctttc ttgtcgtgtc aaggaagagt tatgtccctc ttctttgcca 1200
agtcctaaag gccagcaagc aagagttgag gttagattaa gggaaggaag agcagtgaac 1260
attcatatgt tctgtggtcg tagaccgggt cttttacttg ctaccatgaa agctttggat 1320
aatcttggac tggatgttca gcaagccgtg attagctgtt tcaacgggtt tgccttggat 1380
gttttccgcg ctgagcaatg tcaagaagga caggagatac tacctgatca aatcaaagca 1440
gtgctttttg atacagcagg ctatgctggt atgatctga
1479
<210> 100
<211> 981
<212> DNA
<213> Solanum chilense
<220>
<400> 100
atggttgatg atggaggtgt ggattggttc atgggtggtg gtgatagtaa taatcatcac 60
ataaataata ataataataa tggtggtggt ggtggtggtg gtggtagtaa catgcagagc 120
cacatttcat actctactag tttcactgaa gctgaaaata gtttgctact acaacctgtt 180
gattcatcag cttcatgctc acctgtttct ggtaatgttt ttaataacat tgatccatct 240
caggtgaatt ttttcatgcc acaaaaatct actattccat catcactcac tgggttatct 300
aataatccta tggataatag ttttaatttg ggtatgttga atcaagctgg gaatgggatg 360
atgaatactg ggtatcatca tctaggttca cctaaccaaa tggggactaa taatctaagt 420
tcatacactc agttttcatc tcctaatcta ctccagctac ctcaggtagc tggtgggtat 480
agttctatgg gatttggggc taataattct gctaatggaa acacattgtt tctaaatcga 540
tctagaactc ataaaccatt agataatttt gcttccattg gagcacagcc aactcttttc 600
cagaaaagga ttgcaaagaa cttggttagt aatggtgaaa atttaggtac agaaatcggt 660
caatcttcca gtaatctgac tgatagaaaa aggaagtcca gcatcaatga tgagtttgaa 720
gatgttagca tggatggaac attgaattac gactcggatg agttcatgga tatctctaac 780
aaaatggaag atgggatcaa gattggggac agctctaatg cagccagcac tgtcagtggt 840
gctgatcaaa agggaaaaaa gaagggacct ccagctaaga acctgatggc ggagcgacgc 900
cgcagaaaga agctgaatga caggctttac atgctgcgat cggttgtccc aaagataacc 960
aaggtaatga aattctttta a
981
<210> 101
<211> 1596
<212> DNA
<213> Solanum lycopersicum
<220>
<400> 101
   atgctctcaa gagttaacag tatgcaggtt tgggatatgg aaggcaaaca agaagaagaa 60
   aaagaagaaa attttagcaa caaggagaat accactaatg ttgagcttga aaacaaacaa 120
   gatatggaat tgggtgcact ttctacattc aaatctatgt tagatggtac agatgtagat 180
   tggtatcaca acaacatgca aaaccatact gaaaatatct gtttcactca gaatttcact 240
   gaattagctg aaaacagcat gtttctacag ccagtagtac cagtggattc atcatcatca 300
   tgttcaccat catcagtttc tgtttttaat aatctagatc catctcaggt acattatttg 360
   ttagcacaaa aggctattaa taataatcca ttggattata gttttaattt gggttgtgaa 420
   aatggttttc tagaagctca aggtatgggt ggtttgaata aaggggattt tctactagct 480
   ggtggtgggt ttcatgattt gagctcacag aaccaaatgg gtaatcctaa tttgaattca 540
   tttactcaat atccatcatc acatttgcca caaaatacta ctactactgg gtttagtcca 600
   ctagggtttg ttgatggttc tgctaatgag aactctctat ttctaaatag gtctaagttg 660
   ttaaagccac tagataattt tgcatctaat ggagcacaac ctactctatt tcagaaaagg 720
   gctgctctaa ggaagaattt agctaatact actggtggaa gtttggggga ttttggaggt 780
   gaaattggcc agaattccat gaatggagag aatgaaagga agagaaagtg gggtagtggg 840
   gaagaacttg atgatgtgag ctttgatggg tgcacattga gttatgattc agatgacctt 900
   actgaaaatg ttactaataa ggtggatgat actgttaaga atggtggaaa tagctcaaat 960
   gcaactagca ctgtcacctg tgggaatcag aaaggaaaga aaaagggact tccggctaag 1020
   aatttgatgg ctgaacgacg acgtaggaag aagctaaacg ataggcttta catgttgagg 1080
   tctgttgtac caaggattag caagatggac agggcttcaa tcttagggga tgcaattgag 1140
   tacttaaagg agcttttaca gaaaatcaat gacctgcata atgaactgga gtccacgcca 1200
   cctagctctt cactgaccca aacaacaagc ttctatcctc taacaccaac tggaccagct 1260
   ctgccaggcc gcatcaagga agaactctat ccgagttcat ttgcaagccc actgtctagc 1320
   ccgactggac aacctgcaag ggttgaagta aaggcaagag aaggaagagc tgtgaatatc 1380
   catatgtttt gcagccgcag accaggcctc ttactctcaa caatgagggc actcgacaac 1440
   ctcggactgg acatccaaca agctgttatc agctgcttca atggatttgc cttagatatc 1500
   tttagagctg agcaatgcaa agaaggtcaa gactttcatc ctgatcaaat caaagcagta 1560
   ctgctggatt ccgctggttg ccatgggatg atatga
   1596
<210> 102
<211> 1443
<212> DNA
<213> Prunus persica cultivar
<220>
<400> 102
atgcttgatg ttgatgatga ttggtatctg cccaataata gcattcagag ccattcagat 60
gtacgagaca tagcattctc acctagtttt gctgatccag aaagtttgct actaaatcca 120
gtggactcat cctcatcatg ctcaccatca tcctcagttt ttaataatct agatcctaat 180
caggttcatt actatttgcc tcaaaattcc acattgtcat cactcctaaa tgttgttcct 240
aataatccat tggagcatgg ttttgatttg ggatgtgata ttgggtttct agacactcaa 300
gcatctagtg gttctagttt aatgaatagg ggaggtgggg ttttaactgg gtttagtggt 360
ttgagctcta ataatcctat gaatgctcag gatttgtgtt ctaatttgca attttcaacc 420
acacgtctac ctcaattgct agaaaatagt tctaattttt caggatttcg aggttttgag 480
gatacatcag ctaatgcatt gtttcctaat aggcctaaat tattgagacc actagactca 540
gtacctgatg tgggagcacc acctactctt tttcagaaaa gagttcagaa gaacttagct 600
gataatggag gaaatttggg tgctttaggc tcagaagggg gcctggcttt gaatcatatg 660
ggtgaggaga atgagaggaa gaggaaattg agcgggggag atgattttga agatgtgagt 720
atcgatggtt cgggtttgaa ctatgattca gatgacttta ctgaaaacac taagatggat 780
gacggtgcta agaatggtgg taatagctcg aatgcaaata gtactgttac tggcggaggg 840
gatcataagg ggaagaagaa agggttgcct gctaagaatt tgatggctga gaggcgccgc 900
cggaagaagc tcaatgatag gctgtatatg cttagatccg ttgttccaaa gatcagcaaa 960
atggacaggg catcaatcct tggggatgca attgagtact tgaaggaact tctgcaaaag 1020
atcaacaacc tccataatga attggagtca atccctccgg ggtcttcatt gacgcctacc 1080
ggaagtacat tccacccttt gacacccact ccggctaccc tgccaagccg tatcaaggaa 1140
gaactttgcc ccagctcatt acctagccca aacggccaac ccgcaagggt tgaagttagg 1200
ctacgagaag gaagagcagt gaatatccac atgttttgtg ggcgcagacc tggtctcttg 1260
ctctcgacca tgagaacctt agatagcctt gggctagaca tccagcaagc tgtcattagc 1320
tgtttcaatg gttttgctat ggatgtcttc cgtgctgggc aatgcaagga aggtcaagac 1380
gtccacccgg atcagataaa agcagtactt ttggagtcag tcgggttcca tggcttgata 1440
tag
1443
<210> 103
<211> 1131
<212> DNA
<213> Zea mays
<220>
<400> 103
atggacgact cggcggaggt gaagctggtg gacgagatca cgggagaggg aggggcggcg 60
ggggactggg gctacctggg ctcagatggg atgggctcag gctcctatcc agcatttcca 120
ttctctagag atgtcctatc cacacctacc tcagcatcat tgctcctctc catggatcca 180
gcagcactat ttgattttaa tggcacattt cctccatcct cagcagcagc agccactgca 240
ggctcctcac tatcagcatt ccatgatttt agttgcataa atccatttga tgatgcaggc 300
catttcctag gagctccacc tcctgtgcca gcagcagctg ctcctcagca gcaggggcag 360
aaaggtgggt tttttgcacc actaccagcc tcagatttta atgatgcagg gatgagttgg 420
gatgatgagg atgagataga ccaaagtgta gatgcctcct caatggccat ctcagcctcc 480
atggagaatg ctgcaggagc agtggctggg gctagtggtg ctggaggtgg aagtgggaga 540

ggaaagaaga aagggatgcc agctaagaac ctcatggcag agcgacgacg tcgcaagaag 600
ctcaatgacc gcctctacat gctgcgctcg gtggtgccga agatcagcaa gatggatagg 660
gcttctatcc ttggcgacgc aattgagtat ctgaaggagc tcctgcagag gatcagcgat 720
cttcacaatg aacttgagtc agctccgagc tcttcgcttg ttggaccaac atcagctagc 780
ttcaacccat caacaccgac attgcagaca tttcctggcc aggtcaagga agaactttgc 840
ccgggctcat tcccaagccc tactggacaa caagccacag tcgaagtcag gatgagggaa 900
gggcatgcag tcaatatcca catgttctgt gcgcgtaggc caggcatcct gctgtcgaca 960
atgacggccc tcgacagcct aggcctcgac atcgagcagg cggtcatcag ctgcttcaat 1020
ggctttgcga tggacgtgtt ccgagctgag tgcgcggatg gtcctggaat ggtgcctgaa 1080
gaaattaagg ccgtgctgat gcacaccgcc ggcctccaca acgcaatgta g 1131
<210> 104
<211> 1539
<212> DNA
<213> Gossypium hirsutum Ls
<220>
<400> 104
atggagaata aagaagatat gggtacattt tctacattta aatccatgtt agatgatgaa 60
tggtatgtag gaaataatag catttctagc catcaagata ttagagacct atcatttaca 120
tctaatctag gtgaccatca tcaagataat ctattgctcc atcatcattc tctaccatca 180
gtggattcat catcatcatg ttcaccatca tcctcagttt ttaaccatct agatccatct 240
caggtacagt atttttgcca acctaaacct aatttatcat ctatcctaaa ttttgtttct 300
aatagtccat tagaccatgg ttttgatttg agtgaaattg ggttcctaga taaccaagct 360
actaatggta ctacattgtt gaatagggga aatgctgggg ttttgggtag tttaactgat 420
ctaggtcatg ataatcattt agaccctgct aatttgtgtc ctgaggctca gttttcgagt 480
tctcggatag ttcagctacc agaaaatggt accgggtttg ctggttttga agggtttgat 540
gagaatcctg gaaatgctct gtttttaaag agatctaagt tgttaaggcc actagaaagt 600
tgtccttccg ttggtgcaca acctacttta ttccaaaaaa gagcagctat gaggaagaat 660
tcagctgata gtggaccaaa tttcggggtg ttagatggag gaaaggttag tgttcaaagt 720
ggtactgagg gagataaagg gaagaaagaa atgggtaaag aagagactga gaagaggaaa 780
attaacaaca gagatgatgt agaagatgtg agcattgatg ggtcagctct gaactatgat 840
tcagatgagt ttactgagaa taccaaggtg gaggaaactc ttaaaaatgg gggaaatacc 900
ataaatgcaa acactagtgc tactggagga gaccaaaacc aaaagggtaa aaggaaaggg 960
cttcctgcta agaatttgat ggctgagagg cgccgtcgaa agaaactcaa tgataggctt 1020
tacatgttgc gctctgttgt tccaaagatt agcaaaatgg atagagcttc aattctcggg 1080
gatgctatcg agtacctgaa ggaacttcta cagagaatca atgacctcca caatgaattg 1140
gagtcaaccc ttccaagctc ctcactgaca cctacaacca gcagtttcca tccattgaca 1200
cccactccag ctacctttcc tagccgtatc aaggatgaac ttggccccag ttcatcacca 1260
ggtctgaatg gacagccagc aagagttgaa gtaaggctga gagaaggaaa agctgtaaac 1320
atccacatgt tctgtgggcg gagaccaggt cttttgctct ccataatcag ggccttggac 1380
aaccttggac ttgacgccca gcaagcagtc attagctgtt tcaacggttt ttccatggat 1440
atctttcgag ctgagcaatg caaggaaggg caggacatca atcctgagaa tatcaaagct 1500
gtacttttgg attcaactgg cttccccaat atgatatag
1539
<210> 105
<211> 306
<212> DNA
<213> Arachis hypogaea
<220>
<400> 105
atggaggcag ttggtggttc tggaaatgct ctattttcag gtaagggaaa tggattggaa 60
gcattggtag tgagtaattt gaagagggtg caggaagttg aagaagctag taatgaaggt 120
tctatgtgga attctgaaga ggatgatgag agaactgatg aggtggtggt tgaggagagt 180
gggaagaaga agaagaagaa ggggatgccg tccaaaaatc ttatggcgga gaggcggcga 240
aggaagaagc tcaatgatag gttgtacatg cttaggtctg ttgtgccaaa tattagcaag 300
gtataa
306
<210> 106
<211> 1029
<212> DNA
<213> Brassica juncea
<220>
<400> 106
atggttctcg acggaaacgg cggtggtgta tggttaggcg gcggcggcgg cgggggagga 60

gaacgggttc aggaggagga gaacgaggaa gcttcgtggg ggaggaatca agaagacggt 120
ggtcagttca agcctatgct tgaaggtggt ggtgattggt ttactagtag ccaacctcat 180
ccacaagatc tccagatgct gcagagccag caagatttta gatttctagg tgggtttggt 240
tttaatccta atgataatct actactacta caacactcca tggactcatc atcatcatgc 300
tctccatctc aggcatttag cctagatcca tctcaagcat catttttggc tgctgctaac 360
aacaagtcat gtctcctaaa tgttgttcca tcctcagcta atccatttga taatgcattt 420
gagtttggtt cagattctgg tttcctaaac caaatccaag ctccagtttc tatggggttt 480
ggttcattga cacagctagg aagctctgta cctgattttt tatctgctcg atcactacta 540
cctcctgaaa ataataacac agcaccatta tgtggaggag gaggaggtgg aggtgggttc 600
acagcgttgg agctggaagg gtttgggagt ccagcgagtt ttgttgggag cagaccgaaa 660
gttctgaagc ctttagaggt gttggcgtcg tctggcgcac agcctactct gttccagaag 720
cgtgcagcga tgcgtcagag ctctggaagc aagatgggga actcggagag ctcggggatg 780
aggaggctga gcgatgatgg agatatggat gagactgggg ttgaggtttc agggctgaac 840
tatgngtctg atgagctgaa cgagagcggt aaagcttctg agagtgttca gaacggagga 900
ggtaaaggga agaagaaagg gatgcctgct aagaatctga tggctgagag gaggaggagg 960
aagaagctta acgataggct ttacatgctt agatcagttg tccccaagat cagcaaagta 1020
aactattaa
1029
<210> 107
<211> 1494
<212> DNA
<213> Brassica rapa subsp.
<220>
<400> 107
atggttctcg acggaaacgg cggtggtgta tggttaggcg gcggcggcgg cggcggagga 60
ggagaacggg tgcaggagga ggagaacgag gaagcttcgt gggggaggaa tcaagaagac 120
ggtggtcagt tcaagcctat gcttgaaggt ggtggtgatt ggtttactag taaccaacca 180
catccacaag atctccagat gctacagagc cagcaagact tcagattcct cggtgggttt 240
ggtttcaacc ccaacgacaa tcttcttctt cttcaacact ccatggactc gtcttcttcc 300
tgctctccct ctcaggcttt tagcctagat ccatctcaag catcattttt ggctgctgct 360
aataataagt catgtctcct aaatgttgtt ccatcctcag ctaatccatt tgataatgca 420
tttgagtttg ggtcagattc tggtttccta aaccaaatcc aagctccagt ttctatgggg 480
tttggttcat tgacacagct agggagctct gtacctgatt ttttatctgc acggtcacta 540
ctaccaccag aaaataataa tgcaacacca ttatgtggag gtggaggagg tgggttcaca 600
ccattggagc tagaagggtt tgggagtcca gctagttttg tagggagtag acctaaagtt 660
ctaaagccat tagaggtgtt ggcatcatct ggtgcacagc ctacattgtt ccagaaacgt 720
gcagccatgc gtcagagctc tggaagtaag atggggaact cagagagctc agggatgagg 780
aggctaagtg atgatggaga tatggatgag actggggttg aggtttcagg gctaaactat 840
gagtctgatg agctaaatga gagtggtaaa gcatctgaga gtgttcagaa tggaggaggt 900
aaagggaaga agaaagggat gcctgctaag aatctgatgg ctgagaggag gaggaggaag 960
aagcttaatg atagactcta tatgcttaga tctgttgtcc ccaagatcag caaaatggat 1020
agagcatcaa tacttggaga tgcaattgat tacctcaagg agcttttaca aagaatcaat 1080
gaccttcaca acgagcttga gtcaactcca aatggatctc tccctctggc ttcatcaagc 1140
ttccatccat taacgcctac gccgcagact ctttcttgcc gtgtcaagga agagttgtgt 1200
ccctcttctt tgccaagccc taaaggccag caagcgagag ttgaggttag attaagggaa 1260
ggaagagcag tgaacattca catgttctgt ggtggtagac ctggtctttt actagctacc 1320
atgaaagctt tggataatct tggattggat gttcagcaag ccgtcatcag ctgtttcaac 1380
gggtttgcct tggatgtttt ccgcgctgag caatgccaag aaggacaaga gatattgcct 1440
gatcaaatca aagcagtgct tttcgataca gcgggatacg ctggtatgat ctga 1494
<210> 108
<211> 1416
<212> DNA
<213> Chrysanthemum dichrum
<220>
<400> 108
atgctaccag aaaatgacac tgcatggatg gataaccacc aagatgaaac atcatcatgg 60
actcctacta ccacacctaa ccaccaccac caaccaccat gcacaaataa ccaaaacacc 120
tacacatcat taactacatt aaaaaacatg ctagaaacag aatggttcca aaataataat 180
aataataacc taaacctcac ctcaacagac actaatccta tgtttttacc actagattca 240
tcatcctcat gttcaccatc acaatcacac tcacactcac atttcactca gtttccattc 300
atacctacta aatataataa taatactaat ctaaatttaa ctgcatttga tttgccattt 360
gaatttgggt gtgatcctag tttcatttct aataatcctc ctactaattt tttgaatttt 420
cctactcaac aaatggctac tagctcagag tatactgaga ctagttacca tgatgattta 480

actggaactg gtacagggtt cactggcttt gagccagtga atcctgtttt tccggttagg 540
tccaaagttt tgaggccttt ggaagtttct ccacctgttg gagcgcagcc cactttgttt 600
cagaagcggg ctgcgctgag acagagtgga gagaagttgg atgggttgga aattgtgaaa 660
aaaaggaaaa ggggtagtgg gagtgggagt ggggcccatg aggatgagat tgatgaaatg 720
ggtaatgggg atggttcggg gtttaattat gattcggatg aagttgagct tactagtgag 780
gttaatggga ataattcggg ttcggttatg gttggggata gtaaagggaa gaggaagggt 840
ttgcctgcta agaatttaat ggcggaaagg cgaaggagga agaagttgaa tgataggttg 900
tatatgctta gatctgttgt ccctaaaatt agcaagatgg acagggcttc aatacttggt 960
gatgctattg attacttgaa ggagttgttg caaaggatta atgatttaca taatgaactt 1020
gaggcaacac cacaaggttc tttgatgcaa gcttcgtcga gtatccatcc attgacgcct 1080
acaccaccaa cgcttccgca acatgttaag gaagagttgt gtccaagtac tcttcctagc 1140
ccaaaaaacc atccttcaaa ggtggaggtg catgcaagag aagggagggg tgtgaatatc 1200
cacatggtgt gtgggcgcag accaggtctg ttgctttcaa cgttgagggc cctagagaac 1260
cttggcctgg acattcagca agctgtcata agttgtttca atgggtttgc tttggatgta 1320
tttcgagccc agcaatgtag agaagggcag gagatgttac cggagcagat aaaagcagtg 1380
cttctagaga ctgcaggtta tcatggtgcc atttag
1416
<210> 109
<211> 1089
<212> DNA
<213> Thellungiella halophila
<220>
<400> 109
atggttctcg acggaaacgg cggtgcggtt tggttaggcg gcggagaacg gattcaggag 60
gaggagaacg aggaagcttc gtggggtagg aatcaagaag aaggtggagc atcactatct 120
cattttaagc ctatgctaga aggtgattgg tttagtaatc caccacatcc acaagatctc 180
caaatgttac agaatcagca agattttaga ttcctaggtg gttttccttt taatcctagt 240
gataatctac tactacacca gtctattgat tcatcttcat catgctctcc atctcaagca 300
tttagcctag atccatctca gccttcattt ctagcagcta ataataaatc atgtctcctc 360
aatgttccat cctccactaa tccttttgat aatgcatttg agtttggctc tgattctggt 420
tttctaggac aaatccaagc acctatttct atggggtttg gttcattgac acaactagga 480
aatcgggatt tgagctcagt acctgatttt ttatctgctc ggtctctact acctcctgaa 540
aataataact ccacaccatt atgtggtgga ggaggtggtg gtggtggtgg attcactcct 600
ctggagctgg aagggtttgg gagtcctgca aatggcggtt ttgtagggaa cagagcgaaa 660
gttctgaaac ctttagaggt cttagcatcg tctggtgcac agcctactct gttccagaaa 720
cgtgcagcta tgcgtcagag ctctggaagc aagattggga actcggaagg ctcggggatg 780
aggaagctga gcgatgatgg agagatggat gagactggga ttgaggtttc tgggttgaat 840
tatgagtctg atgagctgaa tgagagtggt aaagcggcgg agagtgttca gaatggagga 900
ggaggatgta aaggtaagaa gaaaggaatg cctgctaaga atctgatggc tgagaggagg 960
aggaggaaga agcttaatga taggctttat atgctaagat cagttgtgcc caagatcagc 1020
aaagtaaaca attacttctc ttttatctct ttaagggctt tgtcttcatc gtttacttat 1080
cgccgttag
1089
<210> 110
<211> 1458
<212> DNA
<213> Daucus carota
<220>
<400> 110
atgctaccta catctaatgg tgtagtttgg atgccacctc aagataccac taacattgaa 60
caacagcaat tgttattaga tacatgtgta gatactaaaa ttagtccatt tgcatcatca 120
tcatcactca ctagttttga atcactacta gaaaatgatt ggttctctaa taattatcta 180
gggaatggta atttagattt ttcagcatct ggttctcagt cacagccatt cactcatgat 240
ccatcacagt cacattctca gttttttcag cctaaatcat gtttctctcc tatgctaaat 300
gggttgtatg ataatgggtt tcagttgggt tctgatccta atttcactaa tgggtcagat 360
tctcatgggt tcatgtcata taatgggttg aattctgtga ctaatttgga tatgggtttg 420
gtttcagatt ttggagggac acgatttgag agtgatccag ttggtgtagg gtttaatcca 480
gttgggtttg gtgggtcatg tttagttgat gataatgttc atgataatga tgataatgct 540
ttgttttgta ataggagtaa tggtaatcag attttaaggc cacttgaggt ttcaccaagt 600
gtgggtgcag agccaactct ttttcagaag agggctgctt tacgacaagg ctccggtgag 660
ggcgaaatag ggaattcagg aaagagtgag ggtttgggga ggaagaggat gagaaatgcg 720
agtgaagatg gtgatgaatt tgatgatggc agtatgggtg tttcgggttc ttttaattat 780
gattcggatg actttgatac taaaaacgag aatgggggct ctggggatgt ggatggttcg 840

aattctgtga aggttggaga tcggaaggga aagaagaagg gatttcctgc taagaatttg 900
atggctgaga gacggcggag gaagaagctc aatgataggc tttacatgct gaggtctgtt 960
gttcccaaga tcagcaagat ggatcgagct tccatacttg gcgatgctat tgattattta 1020
aaggatcttc tgcagaggat aaatgatctt cataatgaac tcgaggcaat tccaccaggc 1080
tcctcagtaa cggcaacgtc aagccacaat ccattgactc acacttctac caccctccca 1140
catctcgtga aggaagaaat atactcaagt tcaatgccta gtccacaaag cacagctgca 1200
agggtcgagg taagggcaag agaagggagg gctgtcaaca tccacatgtt ttgtgcacgc 1260
agacctggtc tcttactttc caccatgagg gcccttgaca acctagggtt agacattcag 1320
caagctgtca tcagttgttt caatgggttt gctatggatg tatttcgagc cgagcaatgt 1380
ggtgaaaccg aggacattct gcctgatcag atcaaagcag ttcttctaga ttcggctggt 1440
tttcatggag caatttag
1458
<210> 111
<211> 1050
<212> DNA
<213> Glycine max
<220>
<400> 111
atgatgtcta gaataaataa tagcctagta tggatggatg aaccagaagc agaaaacact 60
gcctcagcat catgggctag aaactctaag cctaataata ataacatcat cattaccaca 120
ggcactacca ctaccacagg ttttgtagaa acctctaatg atttaggttc tctatgtgca 180
tttaagccta tgctagatgt tgatgatgaa tggtataata acatcactaa ccaccaccac 240
caccaccaag atatggcatt tgatccagat aacctattac tccatccatc agcagtggac 300
tcctcctcct catgctctcc atctcacctc cagtttttcc tacctcctaa tcctaaacta 360
aatccatttg atatgggttt tctagatcct caagcatcat catcattacc actactagca 420
tcagaaacta cacatttcac accacctcag actagcacca ccacagcatt tgcaggtttc 480
cagagcctcc aagagggttc ctcaaatcct ctatttctaa atcgttctaa cattctaaga 540
cctctagagt cactaccacc atctggggca caacctaccc ttttccagaa gagagccgcg 600
ctcaggaaga acatgaacat gagtgacaat aagaagagga aggagaagga tgaggtggtg 660
gttgaggatg tgagtttcga cgggtctggc ttgaactacg attctgacga cttaactgag 720
agtaattata atgacgctaa ggagaagaat ggtggtgggg gggttagttc caatgccaac 780
agcaccgtca ccggtttgga tcagaaaggg aagaagaagg gaatgcctgc taagaatttg 840
atggccgaac gacgccgccg gaagaagctc aatgacagac tctacatgct gaggtccgtt 900
gttcccaaga ttagcaaagt aagatttctt ttcttttctt ttcttttcct ctcaccctct 960
tgccaaattt tgaatctgca gttacttttc aagttcttgg agttaaattc atttgaaagg 1020
tcaatacttt gtggtgtgtg tttggattag
1050
<210> 112
<211> 1266
<212> DNA
<213> Raphanus sativus
<220>
<400> 112
atggttctcg acgggaatgg cggcgctgtt tggttaggcg gcggcggagc aggagaaagg 60
tttcatgagg acgaggagga ggagaacggt gcagctcact tcaaagctat gcttgaaggt 120
ggtgactggt tctttaacca accacaagat ctccacatgt tacagagcca tcatcatcaa 180
gatttcagat tcctaggtgg tggttgtggt ggttttgcat ttaatcctaa tgataatcta 240
ctactagatc catttttggc agctgctaat aatagtaagt catgtctcct aaataatgtt 300
gctaatccat ttgataataa taatgcattt gattttgggt catctgattc atgttttcta 360
ggagggtatg gttcattgag ctcctcctca gtacctgagt ttttatctgc tcgggagaat 420
aataataaca cctctacacc attggagtca gaaggttttg ggagtagagc taaggttcta 480
aagccattag aggtgttggc atcttcaggt gcacagccta ctctatttca gaaacgtgca 540
gccatgagga agctaagtga ggatggagag atggatgaga ctggggttga tgtttcaggg 600
ttgaactatg agtctgatga gctaaatgag aattgtaaag ttgttcagaa tggtggagga 660
ggaggaggta ataaaggtaa gaagaaaggg atgcctgcta agaatctgat ggctgagagg 720
agaaggagga agaagcttaa tgataggctt tatatgctta gatcagttgt ccccaagatc 780
agcaaaatgg atagagcatc aatacttgga gatgcaattg attacctcaa ggaactatta 840
caaaggatca atgatcttca caacgagctt gagtcaactc catctggttc tttgcctcca 900
acttcatcaa gcttccatcc gttaacgcct acaccgcaga ctctttcttg ccgtgtcaag 960
gaagagttgt gcccctcttc tttgccaagc cctaaagacc agcaagcaag agttgaggta 1020
agattaaggg aaggaagagc agtgaacatt cacatgtttt gtggtcgtag acctggtctt 1080
ttgctggcta ccatgaaagc tttggatagt cttggattgg atattcagca agctgtgatt 1140
agttgtttca acggttttgc cttggatgtt ttccgcgctg agcaatgcca agaaggacag 1200

gagataatgc ctgatcaaat caaagctgtg cttttcgata cagcaggcta tgctggtatg 1260
atctga
1266
<210> 113
<211> 1146
<212> DNA
<213> Triticum aestivum cultivar
<220>
<400> 113
atggagaact cggcggcggc ggtgggggtg gagaaggagg acgagctggt gggggcggc 60
gggggcgact gggggtacct cacatctgag gctatggcta cagcagggtt tccagcattt 120
gggtttccat gtggagctag gggtggtgtc acaccagcac ctacctctgc atctctactc 180
atgtccatgg agcatgcagc tctatttgac tataatgcag catttccatc atcatcatcc 240
tctgcagcag cagctcctcc agcctaccat gattttgggt ctggaggaaa tccatttaat 300
gtagatgcac ctccatttct actagaggca ccacctcctc taacagtggc accaggaggg 360
cagaaagggg ggtttttggc accaccacta tctgcatttg gtgatggcat ggggtgggat 420
gatgaggatg agctagatca gcagagtgtg gatgcctcct cattgggggt ctcagcctct 480
ctagagaatg cagtggtagg agctccaggg ggaggtggag gtggaggtgg aggtaatggc 540
aagggcaaga agaaggggat gccggccaag aacctgatgg cggagcggcg gcgcaggaag 600
aagctcaacg accgcctcta catgctgcgc tccgtggtgc ccaagatcag caagatggat 660
agggcttcaa tccttggtga tgcaatcgac tacctgaagg agctcctgca gaggattagc 720
gatctccact ccgagctcga gtctgctccc agctctgccg cactcggtgg accatcgaca 780
gctaatagct tcctcccgtc gacgcccacc ctgcagccgt tccccggccg catcaaggag 840
gagcggtgcc cgccggcccc tttccctagc cccagcggcc agcaggcgac ggtggaggtg 900
aggatgaggg aggggcaggc ggtgaacatc cacatgttct gcgcgcgcag gccgggcatc 960
ctgctgtcca ccatgagggc gctggacagc ctcggcctcg acatcgagca ggccgtcatc 1020
agctgcttcg atggcttcgc catggacgtc ttccgcgccg agcaatgcag ggagggccct 1080
gggctcctgc cggaggaaat taaggccgtg ctcctgcact gcgccggtct ccagaacgcg 1140
atgtag
1146
<210> 114
<211> 315
<212> DNA
<213> Hordeum vulgare
<220>
<400> 114
atggggtggg atgatgagga tgagctagat cagcagagca tggatgcctc ctcattgggg 60
gtctcagcct cactagagaa tgcagcagta ggagcaccag ggggaggtgg aggaggaggt 120
aatgggaagg gtaagaagaa ggggatgcca gctaagaacc taatggcaga gcgacgacgt 180
aggaagaagc taaacgaccg cctctacatg ctgcgctccg tggtgcccaa gatcagcaag 240
gtgaggaacg ctcccccccg tcatggaaat cgatcaccta cttgttccca tctctactat 300
acatattatg gttga
315
<210> 115
<211> 1410
<212> DNA
<213> Hevea brasiliensis
<220>
<400> 115
atgctagata cagattggta tgatataaat ccatctcctc accatgacct ccatgctctc 60
caaaatcacc atgaaataag agacctaggt ttttgtgcta atcctacacc tgaaaatcta 120
ctattacagc ctatggactc atcctcatca tgctctccat cacaggcatt tactctagat 180
ccagcacagg cacaccagcc attttttcca cctaaatcat gtttctcatc tctactaaat 240
gtcactaatg catttgataa tggttttgat ttgggttgtg aaaatgcatt tctaggccaa 300
taccagccta atcaggcatc taatttgatg ggttttagtg ggccagagtt ccaagcatca 360
cagttgctac cagtgtcaga taatgctgca gcaattggag gtgctgggtt tagtaatgta 420
ggacttgagg gttttgatgg ttctactgtg aatgctctgt ttttaagcag ggctaaggtt 480
ttgaggccac ttgaggtttt gcctccagtg ggttcaccgc caactctatt tcagaagcga 540
gccatgttaa ggcaaagtgg ggataagttg gggaatttgg agatatcagg gctgagatat 600
ggaggagaaa ttggggggaa ttggggagaa atggaaagga aaagaaagaa gagtgaagaa 660
ggtgaaattg aggaagcgag cttcgatgtt tcgggtttga attatgattc tgatgaacac 720
aatggagatg gtaagattga agagagtgtg aagaataacg gtggaagcaa ttcgaatgcc 780
aatagcaccg tcactggtgg aggtggtggt gatcaaaagg ggaagaagaa ggggatgccg 840

gcaaagaatt tgatggctga gagaaggaga aggaagaggc ttaatgatag actctacatg 900
cttagatctg ttgtccccaa gattagcaag atggatagag cttcaatact tggggatgcc 960
attgattatt taaaggagct tctgcaaagg atcaatgatc ttcataataa attggagtca 1020
accccacctg gtcctttgtt gcctcaatct acaagtattc atccggtgac tccaacacct 1080
cccgccatac cttcccgcgt gaaggaagaa ctatgcccta gctcgttgtc aagccctaaa 1140
aaccaagcta caagggtgga agttagggta agggaaggaa gggcagtcaa catccacatg 1200
ttttgtgctc gcagaccagg tttattgctc tccactatga gggctctgga caaccttggg 1260
ttagatattc agcaggctgt catcagttgt ttcaatgggt ttgcactcga tgtgtttcga 1320
gctgagcaat gcagggaagg tcaggatgtc ttaccagagc aaatcaaagc agtacttctt 1380
gattcagctg gctttcatgg aataatgtga
1410
<210> 116
<211> 1158
<212> DNA
<213> Juglans regia
<220>
<400> 116
atgctatcaa gagtaaatgg agtatggatg gatgatagag aggaccatga tcctgcatca 60
tgggctagaa ccactaatga tgctaacact agtaatagtt gtggtgtggc agagaagaat 120
gagcatgatt tgggttcact ctccacattt aagtccatgc tagaaataga agatgattgg 180
tataatatgg ctaataatac tctccaaagc cacactgata taagagattt aacattctct 240
cctaacctag ctgaccatcc agataatcta ttgctaaatc cagtggactc ctcctcatca 300
tgctctccat cttcctctgt tttcaccaat ctagatccat ctcaggtgaa ctattttttg 360
cctcctaagc ctacattgtc ctcactaatt aatggcctct ctagtaatcc attagagaat 420
ggatttgatt tgggatgtga ggttgggttt ctagaagcac aaacatctaa tgcatccact 480
ctactaaata ggggaggtgg ggttttgagc aacttcaccg atttgagcac caattctcag 540
attaatgctc ccagtatatg ctcggactcc caacttcgtc tgccaaattt tcccgaaaca 600
ggtgctgggt tcgtggggtt taaggctttc gatgggggtt ctgggaatgc cctgtttatg 660
aacaggtcta agattctgag acccctcgag tctttccctt caatgggcgc gcagcccact 720
cttttccaaa agagagcggc tctaaggaaa aacatagcgg atagtggcgg caatttgtcg 780
atttcgactt cggttgaggg agataagggg aagacagaaa cccatgaacc gggcgaggga 840
aagaggaaac tgagcaatgg ggatgatgtg gaagatgcta gttttgatgg ctctggtctg 900
aattacgatt cggatgagtt catggagaac gataaggcgg agggcaatgc caagaacacc 960
ggcgggaata gctccaacgc cactagtacg gttaccggtg gaggagagca aaaggggaag 1020
aagaaaggcc tccctgctaa gaatttgatg gccgagaggc gccgccggaa gaaacttaat 1080
gatagattat acttgctgag gtccgtggtt cccaagatta gcaaagtaag cgtttttttc 1140
gctgtgtttt tctcgtga
1158
<210> 117
<211> 1497
<212> DNA
<213> Lactuca sativa
<220>
<400> 117
atgatgcccg acggtggtgc tgtttggatg gaaggaggag gaaaccagga tgaaattgga 60
acatcttcat ggacgccggc caccaccacc accacaaccg ccaatcacca tcatgttctt 120
ggaattgaag ctgctgcaac atcatgcaac gaagaaacca cagccgccaa cacttacgct 180
tctctcacca ccttgaaatc aatgcttgaa tccgagtggt atcaacacaa caatctcaat 240
ctctctacga atccttctga taacaattca atcttcttac ctatggattc ttcttcttcg 300
tgttcgccgt cgcattccca ccacccgttc tctctccccg acccttcgcc gtcacactca 360
tatcccttct ttccccccaa attcaacaac ccttatgaca ttgggtttga tttcggatgc 420
gaccctggct tcttacctaa tcacacttcc aatttgatga atttttctgg gttatgttca 480
caaacacaca tcgccatacc agagctatca tctagttcag attttccagc cactaataat 540
agtaatgaca ctagtgacat tggaggacct gggtttaatc cacagttaac agcatttgat 600
ggttttcatg gagctgcacc tatgaatcct ctatttgcag ctaggtctaa ggttctacga 660
ccactagaca tttcaccacc agtcggagct cagcctacac tatttcaaaa acgagctgca 720
ttacgacaaa gttcactcaa attaggggaa ttgggaggta caaattgggg gaagagagca 780
gagagtaata gcggtatggg atcaaaatta acggtgggaa tagctctaat gcaaacagta 840
ctgtcactgg cggctatcgg gagcaatcaa aaggggaaaa agaaagggtt gccggcgaag 900
aatttaatgg cggaaagacg tcggagaaag aagctaaacg accggcttta catgctcaga 960
tctgtagtcc ccaaaatcag caagatggac agagcttcaa ttcttggcga tgcgatagag 1020
tacctgaaag agcttcttca gaagatcaac gaccttaact atgaactcga atcaacccca 1080
tcaacctcat cattgacacc caccaccacc ataaccaccc ccggcagcgg cacccccacc 1140

ggtttctacc ctctaacccc aactccgacc agtctccctt caagaatcaa ggaagaactt 1200
tgccccaccg cgatccctag cccaactggc cagcctgcaa gagttgaagt aagacagaga 1260
gaaggaagag cagtgaatat ccatatgttt tgcagtagga ggcctggttt gcttctatca 1320
accatgagag ctcttgataa tcttggttta gatattcaac aagctgtcat aagttgcttc 1380
aatggcttcg ctcttgatgt ttttcgagct gagcaatgca aagaaggtca agatgttcat 1440
cctgatcaag tgaaagcagt gctgctggag tccgctggtt accatggtgt cgcttga 1497
<210> 118
<211> 1575
<212> DNA
<213> Oryza sativa
<220>
<400> 118
atgctgccgc ggtttcacgg cgccatgtgg atgcaggatg atggaggagg agaccaagaa 60
catgggcagg cagcaccacc tgggcaggag cagcaccacc atgaccagca tctcatggca 120
ttggcagcag cagcagcagg tggtgcaggg tttggtgcag cacaggcacc tgcaccacta 180
ctagatgagg attggtattt tgatgcagca ggtggtggtg gtggtggagc acatgggtcc 240
atgatgctag gtttgtcatc agtccatgga gggattgggg cagggacatc tggtggtggg 300
catgggcagc agttctcact actaaacatg ggagcagcag cagctccatt tgatgtctca 360
gggtttgacc tagggatagc atgtggagga gttggaggtg gtggtgatgt ggtgtcattt 420
ctaggtggtg ggaatgcatc aaacactgca ctactaccag tagggaatgc agggttccta 480
ggcacatttg gagggtttgg cactgcagca tcacaaatgc cagagtttgg agggctagca 540
gggttcgaca tgttcgacgc gggcgccgtg aacaccgggg gcagctcctc ctcctcgtcg 600
gcggcggcgg cggcggcgtc cgcctcggcg cacgtgagca acaccgcgcc gttctccggg 660
cgcggcaagg cggcggtgct gcggccgctg gatatcgtcc cgcccgtggg cgcgcagccg 720
acgctgttcc agaagcgcgc gctccgccgc aacgccggcg aggacgacga cgacaagaag 780
cgcaaggccg ccgcgggcgc gggcgcgggc gcgctgtccg ccgacggcgc cgacatggtg 840
ctcgacgacg gcgacgacga cggcctcagc atcgacgcgt cgggcggcct caactacgac 900
tccgaggacg ccaggggcgg cgaggacagc ggcgccaaga aggagtcgaa cgccaacagc 960
acggtcaccg gcgacgggaa ggggaagaag aaggggatgc cggccaagaa cctcatggcg 1020
gagcgccgcc gccggaagaa gctcaacgac cgcctctaca tgctccgctc cgtcgtgccc 1080
aagatcagca agatggacag ggcttccatt ctcggcgacg cgattgagta cctgaaggag 1140
ctgctgcaga agatcaatga tcttcagaat gagctcgagt cgtcccccgc gacgtcgtca 1200
ttgcctccaa cacccacaag cttccatccc ctgacaccga cgctgcccac attgccgtcc 1260
cgcatcaagg aagagatctg cccaagtgca ttgccaagcc ccactggaca acagccaagg 1320
gttgaggtta ggctgaggga aggccgggct gtcaatatcc acatgttctg tgctcggagg 1380
cccggtctac tgctctctgc catgagggcc gtcgaaggcc ttggtctcga tgtccagcaa 1440
gctgtaatca gttgcttcaa tggctttacg ttggatattt ttaaggctga gcaatgcaag 1500
gacggccctg ggctgttgcc tgaagaaatc aaggccgttc tgatgcaatc cgccgggttc 1560
cataccatga tctag
1575
<210> 119
<211> 1152
<212> DNA
<213> Phalaenopsis aphrodite cultivar
<220>
<400> 119
atgctctcga ggctaagtgg agcagtttgg agggatggag aagcagctga ggaggaagat 60
gacactgcat catggataag atccacctca tgcactgcag ccatgggaga agtgagggat 120
gacatagcac taccatgtct aaaatctatt ctagatgatg attggtatgt agcagccacc 180
tcttctaatg caggtaacct agcagggagc tcattaaacc aggggtttgc aggagactcc 240
tcagcactcc tatcaccacc agtggattcc tcatgctcac catcctcagt attcacacta 300
gatccatctg cacaccagtc attttttcca cataaaccag cactatcatc attcctctct 360
gctgtatgct ctaatccatt tgattcagga tttgattttg tctcagagaa tcctacattc 420
ctaccatctg ctaactcagc agcctccacc tcagtttttg gtggtggaat attgggtttt 480
cccaacgtgc agattaatag tcaagatttt ctgcctatga ccggaagctc ttctttgttg 540
aaccccatgg ccttcgattc gcttgaaaat tcgccatttt tgagcagagg gaaggttttg 600
aggcctctgg agatctttcc accagtaggt gctcagccta atctttttca gaaacgtgcg 660
gcggccgcac tgcgccagaa ttcaagttct attggtggtg agaaaggtat gcgtttgggg 720
gttttgggta tggaggatgc agggcagatg aggagctctt gggatggtga gtttgagaag 780
cggaggagaa tatatgatga ggatgagctt gatgatgcca gcattgatgg ctctggttta 840
aattatgatt ctgaggatat ctgtgaagtt gcagagactg taaagttgga tgaagagaag 900
aatgatggtg gaggagcacc tgccggagga ggaggaggag gtaatgtttc cattacgaat 960
agtagtctta ccggaggggg tggagagctg aagggtagga gaaaggggct tcctgctaag 1020

aatctgatgg ctgagaggcg taggaggaag aagctcaatg acaggcttta tatgctaaga 1080
tctgttgttc caaagatcag taaggtgaga accctcatct ccttcatttc attttcgctg 1140
gcttcttact ga
1152
<210> 120
<211> 537
<212> DNA
<213> Prunus mume
<220>
<400> 120
atgtccagca gcgctgtttg gatgtttcca tctaaatcat gtttctcatc tctcctaaat 60
gtagtctcca ctaatccatt tgataatggt tttgatttgg gttgtgatag tggttttcta 120
agctcattcc agggaaacca accatctaat tcatttgcat tgatgggttt cacagccata 180
aattctcaat ctcagatggg tactccagaa ttgagttcta gtgtagagtt tcctactact 240
cggttgctat ctacacctaa taatgacact gagctagatg gtaattttag cctaactggt 300
ttccaaggtt ttgatggctc tgggggtgct cagctactaa atagggctaa gccactaaga 360
ccactacatg tatttcctcc agtgggtgct catcctactc tatttcagaa gggagcagaa 420
ctacgtcaaa attttgctgg tgatgggact gataaattgg ggaatttgga gatttcaggg 480
actgctacca ccactgctgt tggctctgct gctgctgaga gaagaagaca gtgctga 537
<210> 121
<211> 1557
<212> DNA
<213> Camellia sinensis
<220>
<400> 121
atggaagata gaggagatga agaaacacct tcatgggctg ctcaaaataa taataatgga 60
tgtattgtag ataagaaaga tgaactaggt tcactctcta catttaaatc tatgctagga 120
gttgaagatg aagattggta tgtaaacact aataataata acactatcct cacatttcca 180
tctaatttca cagaatctga taataatttg ctattacaat cagtaggttc atcatcatca 240
tgttcaccat catcagcatc agttttcact aatctagatc catctcagga acaatatttt 300
ctaccatcta aaccatccat ttcatcagtc ctaaatccta tctctaataa tccattagac 360
cacagttttg atctaggttg tgaaactggg tttctccaga cacattcatt gacatctcta 420
aataggggag gtgggatttt gactggattc actgatttaa cctcacaatc actaatgggt 480
actcataatt tggcattaga tcctcaattt tctaatactc attcactaca attgactggt 540
tctacatcat ttcgggtttt tcaggaggag ggttttggga atcctttgtt tctaaatagg 600
tccaaaatgt tgaagccact tcaaaatttt acttcaattg gtgcacagcc aactctgttc 660
cagaaaagag ctgctctgcg caagaatttg gccaacaatg ggaccaattt gggggttttg 720
ggtactgaaa gaggtgggat atcaagcaat gtacaagggt atagtgggaa gatggaagtg 780
agtgaagtga atgagaagaa gaagaggaag tacagcattg gggaagatgt tgaagatgtg 840
agccttgatg ggtctggatt gaattatgac tctgatgagt ttatggagaa caataaggtg 900
gaagagagtg gcaaaaatgg tgggaatagc tcgaatgcga atagtactgt cacgggcgga 960
gatcagaaag ggaagaagag agggcttcca gccaaaaatt tgatggcaga gcgacggcgg 1020
cggaagaagc tcaatgatag gctttacatg ctgaggtctg ttgttccaaa aattagcaag 1080
atggataggg cttcaattct tggggatgca attgaatact taaaggaact tctgcaaaag 1140
atcaaagacc ttcacagtga actggagtca aaccctcctg gctcttcatt gacacctacc 1200
tccacaagct tctacccttt gacacccacc ccacattccc tgccatgccg catcaaggaa 1260
gagctttgcc caagttcatt gccaagccca aatggactac ctgcaagggt agaagtaagg 1320
ttaagtgaac gtagagctgt taatatccac atgttttgta gccgcagacc aggtctcttg 1380
ctctctacta tgagggcttt ggagaacctt gggctagaca ttcaacaagc tgttatcagc 1440
tgcttcaatg ggtttgcgat ggatatcttc cgagcagagc aatgcagaga aggccaagat 1500
gtccatcctg accaaatcaa agcggtactc ttggattcag ctggcttcat gatgtag 1557
<210> 122
<211> 1485
<212> DNA
<213> Arabidopsis thaliana
<220>
<400> 122
atgggtcttg acggaaacaa tggtggaggg gtttggttaa acggtggtgg tggagaaagg 60
gaagagaacg aggaaggttc atggggaagg aatcaagaag atggttcttc tcagtttaag 120
cctatgcttg aaggtgattg gtttagtagt aaccaaccac atccacaaga tcttcagatg 180
ttacagaatc agccagattt cagatatttt ggtggttttc catttaatcc taatgataat 240
ctactactac aacactctat tgattcatca tcatcatgtt ctccatctca agcttttagt 300
ctagatccat ctcagcaaaa tcagtttttg tctactaata ataataaggg ttgtctacta 360

aatgttccat catctgctaa tccatttgat aatgcatttg agtttggctc tgaatctggt 420
tttctaaacc aaatccatgc tcctatttct atggggtttg gttcattgac acaattgggg 480
aatagggatt tgagttctgt tcctgatttt ttgtctgctc ggtcactact agcacctgaa 540
agtaataata ataacactat gttgtgtggt ggtttcacag ctccattgga gttggaaggt 600
tttggtagtc ctgctaatgg tggttttgtt gggaatagag ctaaagttct aaagccatta 660
gaggtgttag catcatctgg tgcacagcct actctgttcc agaaacgtgc agctatgcgt 720
cagagctctg gaagcaaaat gggaaattcg gagagttcgg gaatgaggag gtttagtgat 780
gatggagata tggatgagac tgggattgag gtttctgggt tgaactatga gtctgatgag 840
ataaatgaga gcggtaaagc ggctgagagt gttcagattg gaggaggagg aaagggtaag 900
aagaaaggta tgcctgctaa gaatctgatg gctgagagga gaaggaggaa gaagcttaat 960
gataggcttt atatgcttag atcagttgtc cccaagatca gcaaaatgga tagagcatca 1020
atacttggag atgcaattga ttatctgaag gaacttctac aaaggatcaa tgatcttcac 1080
aatgaacttg agtcaactcc tcctggatct ttgcctccaa cttcatcaag cttccatccg 1140
ttgacaccta caccgcaaac tctttcttgt cgtgtcaagg aagagttgtg tccctcttct 1200
ttaccaagtc ctaaaggcca gcaagctaga gttgaggtta gattaaggga aggaagagca 1260
gtgaacattc atatgttctg tggtcgtaga ccgggtctgt tgctcgctac catgaaagct 1320
ttggataatc ttggattgga tgttcagcaa gctgtgatca gctgttttaa tgggtttgcc 1380
ttggatgttt tccgcgctga gcaatgccaa gaaggacaag agatactgcc tgatcaaatc 1440
aaagcagtgc ttttcgatac agcagggtat gctggtatga tctga 1485
<210> 123
<211> 1494
<212> DNA
<213> Brassica rapa
<220>
<400> 123
atggttctcg acggaaacgg cggtggtgta tggttaggcg gcggcggcgg cggcggagga 60
ggagaacggg tgcaggagga ggagaacgag gaagcttcgt gggggaggaa tcaagaagac 120
ggtggtcagt tcaagcctat gcttgaaggt ggtggtgatt ggtttactag taaccaacca 180
catccacaag atctccagat gctacagagc cagcaagatt ttagattcct aggtgggttt 240
ggttttaatc ctaatgataa tctactacta ctacaacact ccatggactc atcatcatca 300
tgctctccat ctcaggcatt tagcctagat ccatctcaag catcattttt ggctgctgct 360
aataataagt catgtctcct aaatgttgtt ccatcctcag ctaatccatt tgataatgca 420
tttgagtttg ggtcagattc tggtttccta aaccaaatcc aagctccagt ttctatgggg 480
tttggttcat tgacacagct agggagctct gtacctgatt tcttatctgc tcgatcacta 540
ctaccaccag aaaataataa tgcaacacca ttatgtggag gtggaggagg tgggtttaca 600
ccattggagc tagaagggtt tgggagtcca gctagttttg tagggagtag acctaaagtt 660
ctaaagccat tagaggtgtt ggcatcatct ggtgcacagc ctactttgtt ccagaaacgt 720
gcagccatgc gtcagagctc tggaagcaag atggggaact cggagagctc ggggatgagg 780
aggctgagcg atgatggaga tatggatgag actggggttg aggtttcagg gctcaactat 840
gagtctgatg agctgaacga gagcggtaaa gcttctgaga gtgttcagaa cggaggaggt 900
aaagggaaga agaaagggat gcctgctaag aatctgatgg ctgagaggag gaggaggaag 960
aagcttaatg atagactcta tatgcttaga tctgttgtcc ccaagatcag caaaatggat 1020
agagcatcaa tacttggaga tgcaattgat tacctcaagg agcttttaca aagaatcaat 1080
gaccttcaca acgagcttga gtcaactcca aatggatctc tccctctggc ttcatcaagc 1140
ttccatccat taacgcctac gccgcagact ctttcttgcc gtgtcaagga agagttgtgt 1200
ccctcttctt tgccaagccc taaaggccag caagcgagag ttgaggttag attaagggaa 1260
ggaagagcag tgaacattca catgttctgt ggtggtagac ctggtctttt actagctacc 1320
atgaaagctt tggataatct tggattggat gttcagcaag ccgtcatcag ctgtttcaac 1380
gggtttgcct tggatgtttt ccgcgctgag caatgccaag aaggacaaga gatattgcct 1440
gatcaaatca aagcagtgct tttcgataca gcgggatacg ctggtatgat ctga 1494
<210> 124
<211> 1500
<212> DNA
<213> Isatis tinctoria cultivar
<220>
<400> 124
atgggtcttg acggaaacgg cggggcggta tggttaggcg gcggcggcgg cggaggagga 60
ggaggagaac ggattcagga ggaggagaac ggggaagctt cgtggggtag gaatcaagat 120
gacggtggag gttctctttc tcacttcaag cctatgcttg aaggtgactg gttcagtaac 180
acaccacacc cacaagatct ccagatgtta cagagccagc aagatttcag atttctcggt 240
ggtttccctt tcaaccccaa cgacagcctt cttcttcatc agtccattga ctcgtcttcc 300
tcttgctctc cctctcaagc ttttagcctc gacccatctc agccttcctt cttagcttcc 360
accaacaaat cttgtctcct caatgttcca tcctcagcta atccatttga taatgcattt 420

gagtttgggt ctgattcagg ttttctagca caaatccaag ctcctatctc catggggttt 480
ggctcattga cacagttggg gaatcgagaa ctaagctcag tacctgattt tttatctgct 540
cgatcactac caccaccaga aaataataac actactccat tatatggagg aggtgggttc 600
acaccattgg agttggaagg ttttgggagt cctgcaagtg gaggttttgc tgggaataga 660
gctaaagttc tgaagccttt agatgtcttg gcatcgtctg gtgcgcagcc tactctgttc 720
cagaaacgtg cagctatgcg tcagagctct ggaagcaaga ttgggaactc ggagagctca 780
gggatgagga agctgagcga tgatggagag atggacgaga cggggattga ggtttcaggg 840
ttgaattatg agtctgatga gctgaacgag ggaggtaaag cggcggagag tgttcagaat 900
ggaggaggag gtaaaggtaa gaagaaagga atgcctgcta agaatctgat ggctgagagg 960
aggaggagga agaagcttaa cgataggctt tatatgctta gatcagttgt ccccaagatc 1020
agcaaaatgg acagagcatc aatacttgga gatgcaattg attatctcaa ggaacttcta 1080
caaaggatca atgatcttca caacgaactt gagtcaactc ctcctggatc tttgcctcca 1140
acttcatcaa gcttccatcc attaacgcct acacctcaga ctctttcgtg ccgtgtcaag 1200
gaagagttat gtccctcttc tttgccaagc cctaaaggcc agcaagcgag agttgaggtt 1260
agattaaggg aaggaagagc agtgaacata cacatgtttt gtggtcgtag accgggtctt 1320
ttgctggcta ccatgaaagc tttggataat cttggattgg atgttcagca agccgtgatc 1380
agctgtttca acgggtttgc cttggatgtt ttccgagctg agcaatgcca agagggacag 1440
gagatattgc ctgatcaaat caaagcagtg ctttttgata cagcgggtac gctggtatga 1500
<210> 125
<211> 1572
<212> DNA
<213> Eucalyptus camaldulensis
<220>
<400> 125
atggttctgg gtggcgagga agacgaggtc tcctggaccc gggaccacca ccaccaccat 60
gatggaggag gaggtggtaa tgatgatgaa gcagcacgac ataaggaagg agagctagcc 120
atgggagggg cctctacatt taagtccatg ctagatgcag attggtccta ttttgccact 180
aatcctcctc accattcaca gcagcagcag cagctacagc cagctccaga aatggcagac 240
ctatcattct cttctgctgc tccaattcca gataatgcac tcctcctcca ccattctcta 300
gactcatcct cctcatgctc accatcacag gcatttgtcc tagatccatc acagactcac 360
cagccattcc tacagcctaa gcattgtttc tcctctctcc taaatgtagc atggaataat 420
tcatttgata atggatttga tttgggttgt gatgcagcag ggctatttgg ctcagtgcag 480
ggaaatcaga cctctagtgc accactactc atgggaggtt cagagctagg ctcaggttca 540
gagctactac cagtgccaag tcgtctagta ccactaggtg aaaactcaac acaattgcca 600
ggaggagggt ttgggctaac tgggtttgaa ggttttgaga gtcctggaaa tccactattc 660
acgaacaggt ctaccaaggt cctaaggcct cttgaggttt tcccccagac gggcgcccag 720
ccgactctgt ttcagaagag agctgccctg agaaatgggt ctggcagtaa tggcggcggg 780
gctgagaaat tggggaattt ggagatcgac aagaagagaa agaagattga tgatggtatt 840
aatgacgaat tgagctttga tgggtccggt ttgaattatg ataccgatga gggtaatgag 900
agcgggaagg ctatggagga ctcgaagcac gaagggtgca attcgaatgc aaacagcacg 960
gtcacagtcg gcgatcagaa ggggaagaag aaggggctgc cggcgaagaa tctgatggct 1020
gagcggcgcc ggaggaagaa gctgaatgat aggctgtaca tgcttaggtc ggtagtgccc 1080
agatcagcaa gaatggatag agcttcaatt ttcggggagg ctattgatta tttgaaggag 1140
gtttgcaaaa ggatcaataa cctccataac gagctagatt cgaccccgcc tggaaccatg 1200
ttgccccctt cgactaattt tcatccattg acaccaactc cgcccaccct tccttgccgt 1260
gtgaaggaag aactgtgccc gagctccttg ccaagtccca aaggccagcc tgcaagagtg 1320
gaggttagag tgagggaagg gagagcagtg aacatccaca tgttttgtgc acgcagaccg 1380
gggctcttgc tctccaccat gagggctcta gacaaccttg ggttggacat tcaacaggct 1440
gtcatcagct gtttcaacgc attcgctatg gacattttcc gagctgagca atgtagggaa 1500
ggtcaagatg tcctcccaga acagatcaag gcacttctct tagagtcagc tggcttccat 1560
gggatggtgt ga
1572
<210> 126
<211> 1629
<212> DNA
<213> Jatropha curcas
<220>
<400> 126
atgctatctg gactaaatgg agctgtatgg atagatgata aagaaccaca ggactctact 60
actaacacca ttaacattaa tgatagtagt tgtagtatta tacagagtaa acaagagatg 120
ggttcactat ctacatttaa atccatgcta gatgtagaag atgattggta tgttgctaac 180
actagtatcc aaaatcatca agatggtatt agagatatca cattttcacc taatctaggt 240
gatccagata atctattgct acattcagtg gattcatcct catcatgctc acctacctcc 300

tctgttttta ataatctaga tccatctcaa gttcactatt ttatgcatcc taaacctaca 360
ttatcatcac tactaaatgt tgtttctaat aatccattag agcatgggtt tgatttgggt 420
gacattgggt ttctagataa ccaggctact aatactacta ctactacagc taatgcctca 480
tcattactaa ataggggagg tggagtatta ggcaacttta ctgatatgag ctcaaacaat 540
cagattagca gttctaattt atgcactgac ccatcctttt ctactacccg gatgctccaa 600
ttgcctgaaa cgtgtcctgg gtttgcagga tttaggggtt ttgatgagag ttcaggaaat 660
gcactgtttt tgaataggtc taagttgtta aggcctcttg aaacattccc ttcaatgggt 720
gcacagccta ccttgtttca aaagagagca gcgttgagga aaaatttggc tgacaatggg 780
tctaatttgg gggctttaag tgggattgag ataaataaag ggaagagaga aatgagtcag 840
atgggtgaag agaatgagag aaagagaaag ttgggtaatg tagatgatac ttttgaagat 900
gtaagcattg atgggtcggg tttgaattat gactctgatg agttcacgga gaacactaaa 960
atggaggaga ttggcaaaaa tggagggaat agctctaatg caaatagcag tgtcactggc 1020
ggcggtggag atcagaaggg gaagaagaag gggcttcctg ctaaaaattt gatggcagaa 1080
aggaggcgcc ggaagaagct caatgatagg ttatatatgc ttaggtctgt agttccaaag 1140
attagcaaga tggacagggc atcaatactt ggggatgcaa ttgagtactt gaaggaactt 1200
ctgcaaagga tcaatgacct acataatgaa ttggagtcca ctcctcctgg ctcttctttg 1260
acacccacca caagttttca ccctttgacg cccactccat cagcccttcc cagccgtatc 1320
aaggataagc tttgcccaac ctcattgcca agtccaaatg gtcaaccggc aagggttgaa 1380
gtgagagtga gagaaggaag agctgtaaac atacacatgt tttgcagtcg aagagcaggt 1440
ctcctgctct ccactatgag ggctttggat aaccttggcc tagacatcca gcaagctgtc 1500
atcagttgtt tcaatggttt tgccatggat atctttcgag ctgagcaatg taaagaaggg 1560
caagatgttc atcctgagca aataaaagca gtgcttctgg attcagctgg cttccatgga 1620
gtgatgtag
1629
<210> 127
<211> 874
<212> DNA
<213> Picea abies
<220>
<400> 127
atgttgtcga gaatgaatct gaatggagga gtgtggggtc tagaagatgc cacagacctc 60
cattgtaata atgatgagaa tttgaatttg cctacattta aggctatgct agatgcagaa 120
gaatggttta catctcatag taatactagt aataatgata ttactactgc tagtattaat 180
aatgctaata gccatcctga tggaccatgt tttaatatag atatgaagga ttttggatgc 240
tattcatctc tagtgaatcc aaatccagag cctcctaata tgctattgca gcatgccatg 300
aattcatgtt cctcatcacc agcatcaatt ttcagcctag atccatcaca ggtccagtca 360
tttctagcag gggggagtaa ccatcatcac caccaccatg tatctccatt tccagatgtt 420
gcaggttcaa gtccattcca ggagttggca tgtgaggggt tggtgagcag taataatgca 480
agtagtaata gtagtagtaa tagctattta tatggggggc taccaggagg aatttttagg 540
cagctaagtc ctagcttcgc aggttcgagt ccccgcacga atactgttac ccagacgcct 600
aatctcagca gcccacacag ccagatgggt ggtagtagta cccccaatct tagccccaga 660
atgatgccca cgagcaatct caatctctcg gacaccggat tgttcgctag cagtaatcac 720
accaccacca acaacaacaa taatctgagc aatcttctgg gtttgagtag ccttatgagt 780
cctcgaatcc cagcgccatt aaattttagc agcccgaaga tgcttagacc ccttgagatt 840
tgcgccccag ttggtgctca gcctactttg tttc
874
<210> 128
<211> 1632
<212> DNA
<213> Populus suaveolens
<220>
<400> 128
atgctttgta gactcaatag ccacaatatt tgggttgatt atcatgccga agagcaggat 60
tccacatcgg accgccccca ccacaacaac attaccaata ctgcggctgg ttgtggcggg 120
gttatgttgg ggggaaaaca agaaatgggt tctctttcta catttaaatc tatgtttgaa 180
gtggaccatg agtggtatgt gactaatgcc tatagtgcta ttcctcgagg tgatcaagat 240
agtattaaag acctcacatt ttcaccacgt ctagtgcacc catataatct attgctacta 300
ccagggcatt catcctcatc atgttcacca tcatcatcag ttgttaataa tttggatgca 360
tcactactac attcatttat gcctcctaca cctacattgt cctctctcct acatgtggtt 420
tcttctaatc cttctaagga tgggtttgat ttgagtgaaa ttggtttcct agagaatcaa 480
ggaactaatt ctactactac agctaatgtt tcatctccat taaataggcc acgtggagtg 540
ttggggaatt ttgggaattt cactgatttg agttctacag tacgattctc attcctattt 600
gggttctcat caccagacta taagtatcca catgcatcac ctgcagagct acctcctgca 660

ctctcatgtt ttacacgact agatgagata tcagtgaatc cattgttctc atcaaggact 720
aagttgctat cacctctaga aacataccct attcatgggc gctcagctac cctgtttcaa 780
agagagcagc tagaggaaag aacttgggga agtgagagag ataaagggaa gagagatatg 840
acacaaatta gagaagagaa tgacaagcag agaaactcta gcagtggcga tgatttcttt 900
gaggaattag ctcagatggg tcgggtgaga attatgcatc tgggaattaa agagtatact 960
ttggtggaag agactggtaa gaatgttgga aacagttcaa aggtgaatag tggtgttact 1020
ggtcgtggtg aggatcaaaa ggggaagaaa aggggccttc cagctaagaa tttgatggca 1080
cagtggaggc gccggatgca gctcaatgat aggctgtaca cgatgaggtc tgttgttcca 1140
cagatcagca aaatggatag gccatcaatc ctcggtgacg caattgaata cttgaaagaa 1200
cttctgcaaa ggattaatga cctccataat gaattggagt caactcctcc cagctcttca 1260
ttgacgccaa ccactagttt tcatcctttg acaccgactc catcagcgga gcccagccgt 1320
atcatggacc aactttgccc cagctcattg ccaagcccaa atggccaacc tgcaagggtc 1380
gaggttagag tgagagaagc acgagccgta aacatccaca tgttttgtgg ccgcaaaaca 1440
ggtcttttgc tcttcactat gagagctttg gataaccttg ggctagacat ccagcaagct 1500
gtcattagct gtttcaatgg ttttcccatg gatatcttgc gaaatgagca acgcaaagaa 1560
gggcaggaca tgcatccaga tcaaattaaa gcagtcctac tggattcagc tggcttccat 1620
ggcacgatgt ag
1632
<210> 129
<211> 1194
<212> DNA
<213> Populus trichocarpa
<220>
<400> 129
atgctttata gactcaatag caacaatatt tggcttgaag atcataaaga agagcaagat 60
tctactacta accatcatca ccataataat attactaata ctgcagctgg ttgtggtggg 120
gttatgttgg agggaaaaga agaaatgggc tctctatcta catttaaatc tatgtttgaa 180
gtggaagatg agtggtatgt gactaataat aatagtacta ttcatcaaaa ccatcaagat 240
agtattaaag acctcacatt ttcacctagt ctagtagatc cagataatct attgctacac 300
caagtggatt catcctcatc atgttcacca tcatcttcag tttttaataa tttggatcca 360
tctcaggtac attattttat gcatcctaag cctacattgt catcactcct aaatgtggtt 420
tctaataatc cattagagca tgggtttgat ttgagtgaaa ttggtttcct agagaatcaa 480
ggaactaatt ctactactac agctaatgtt tcatctctgt taaacagggg aagtggtgtt 540
ttggggaatt tggggaattt cactgatttg agctcaaaca gtcagattag tattcctaat 600
ttatgttctg atccacaatt ttcaagttcc cgcatgcttc agttgcctga aaatggtcct 660
gggtttaatg gttttagagg gcttgatgag atttcgggga atcagttgtt ttttaacagg 720
tctaaattgt taaggcctct tgaaacttac ccttcaatgg gcgcacagcc taccttgttt 780
caaaagagag cagctttgag gaagaacttg ggtgaagttg agagagataa agggaagaga 840
gaaatgactc aaattagtga agagaaagac aagaagagaa aatttagtag tggagatgat 900
tttcttgagg atgttagctt tgatgggtcg ggtttgaatt atgattctga tgaatttact 960
gagaatacta atttggagga gactggcaag aatggtggaa acagttcaaa ggcgaatagt 1020
ggtgttactg gtggtggtgt ggatcaaaag gggaagaaaa gggggcttcc agctaagaat 1080
ttgatggcag agaggaggcg ccggaagaaa ctcaatgata ggctgtacat gctgaggtct 1140
gttgttccaa agatcagcaa agtaagaatt ttcttcctca tcattttctt gtga 1194
<210> 130
<211> 519
<212> DNA
<213> Potamogeton perfoliatus
<220>
<400> 130
atgctcagat ctgttgttcc caaaattagt aagatggata gagcatctat cctaggagat 60
gctatagaat acctaaagga gctactacaa aggattaatg atctccacaa tgaactagaa 120
tcatcaccat caagttctgc tctacctaca cctacaggtt ctagtctacg accattgaca 180
ccaacaccac taacattgcc atgtcgagtt aaggaagagc tatgtcctag ttcattacct 240
agtccragtg gtcaaccagc cacagttgaa gttaaggtga gagaaggagg agcagttaat 300
atccacatgt tttgtgttcg caggcctggc ctattgctct ctactatgcg rgctctagat 360
agcatgggtc tagatatcca gcaggcagtc ataagttgtt ttaayggttt tgcattagat 420
gtttttcgag ctgagcaatg taaggatgrc cctggagttc ctcctgatga tattaaaact 480
gtactgctgc attctgctgg tctacatggt tcgatgtaa
519
<210> 131
<211> 519
<212> DNA

<213> Potamogeton wrightii
<220>
<400> 131
atgctcagat ctgttgttcc caaaataagt aagatggata gagcatctat cctaggtgat 60
gctatagaat acctaaagga gctactacaa aggattaatg atctccataa tgaactagaa 120
tcatcaccat ctagttctgc tctacctaca cctactggtt ctagtctacg accattgaca 180
cctacaccac tarccttgcc atgtcgggta aaggaagagc tatgtcctag ttcattacct 240
agtcctagta gtcaaccagc cacagttgaa gtaaaggtga gagaaggagg agcagtaaat 300
atccacatgt tttgtgttcg aaggcctggc ctattgctct ctactatgcg ggctctagat 360
agcatgggtc tagatatcca gcaggcagtc ataagttgtt ttaatgggtt tgcattagat 420
gtttttcgag ctgagcaatg taaggatgga cctggtgttc ctcctgatga tattaaaact 480
gtactgctgc attctgctgg tctacatggt tcgatgtaa
519